(19) Europäisches Patentamt
European Patent Office
Office européen des brevets

(11) EP 1 666 599 A2

(12) # EUROPEAN PATENT APPLICATION

(43) Date of publication:
**07.06.2006 Bulletin 2006/23**

(21) Application number: **05025393.9**

(22) Date of filing: **22.11.2005**

(51) Int Cl.:
*C12N 15/82* (2006.01) *C12Q 1/68* (2006.01)
*A01H 5/00* (2006.01) *C12N 15/29* (2006.01)
*C12N 5/10* (2006.01)

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(30) Priority: **04.12.2004 EP 04028793**
**03.02.2005 EP 05002267**
**11.02.2005 EP 05002853**

(71) Applicant: **SunGene GmbH**
**06466 Gatersleben (DE)**

(72) Inventors:
- **Keetman, Ulrich, Dr.**
**06484 Quedlinburg (DE)**
- **Herbers, Karin, Dr.**
**67434 Neustadt (DE)**
- **Hillebrand, Helke, Dr.**
**68159 Mannheim (DE)**

(74) Representative: **Bieberbach, Andreas et al**
**BASF Aktiengesellschaft**
**67056 Ludwigshafen (DE)**

(54) **Expression cassettes for mesophyll- and/or epidermis-preferential expression in plants**

(57) The present invention relates to expression cassettes comprising transcription regulating sequences with mesophyll- and/or epidermis-preferential or mesophyll- and/or epidermis-specific expression profiles in plants obtainable from *Arabidopsis thaliana* genes At5g13220, At1g68850, At4g36670, At3g10920, At1g33240, orAt1g28440.

EP 1 666 599 A2

## Description

### FIELD OF THE INVENTION

The present invention relates to expression cassettes comprising transcription regulating nucleotide sequences with mesophyll- and/or epidermis-preferential or mesophyll- and/or epidermis-specific expression profiles in plants obtainable from *Arabidopsis thaliana* genes At5g13220, At1g68850, At4g36670, At3g10920, At1g33240, or At1 g28440.

### BACKGROUND OF THE INVENTION

Manipulation of plants to alter and/or improve phenotypic characteristics (such as productivity or quality) requires the expression of heterologous genes in plant tissues. Such genetic manipulation relies on the availability of a means to drive and to control gene expression as required. For example, genetic manipulation relies on the availability and use of suitable promoters which are effective in plants and which regulate gene expression so as to give the desired effect (s) in the transgenic plant.

Only a very limited number of epidermis-specific promoters is described in the art (US Patent Application No.: US 2002056153 A1). The mesophyll- and/or epidermis-preferential or mesophyll- and/or epidermis-specific promoters are useful for such a stress- or pathogen tolerance.

There is, therefore, a great need in the art for the identification of novel sequences that can be used for expression of selected transgenes in economically important plants. It is thus an objective of the present invention to provide new and alternative expression cassettes for mesophyll- and/or epidermis-preferential or mesophyll- and/or epidermis-specific expression of transgenes in plants. The objective is solved by the present invention.

### SUMMARY OF THE INVENTION

Accordingly, a first embodiment of the invention relates to an expression cassette for mesophyll- and/or epidermis-specific or mesophyll- and/or epidermis-preferential transcription of an operatively linked nucleic acid sequence in plants comprising

i) at least one transcription regulating nucleotide sequence of a plant gene, said plant gene selected from the group of genes described by the GenBank *Arabidopsis thaliana* genome loci At5g13220, At1g68850, At4g36670, At3g10920, At1g33240, or At1g28440, or a functional equivalent thereof, and functionally linked thereto

ii) at least one nucleic acid sequence which is heterologous in relation to said transcription regulating nucleotide sequence.

Preferably, the transcription regulating nucleotide sequence (or the functional equivalent thereof) is selected from the group of sequences consisting of

i) the sequences described by SEQ ID NOs: 1, 2, 3, 4, 5, 6, 9, 10, 11, 12, 13, 14, 15, 16, 19, 20, 21, 22, 23, 24, 25, 26, 27, 28, 29, 30, 33, 34, 35, 36, 37, 38, 39, 40, 43, 44, 45, 46, 47, 48, 49, 50, 53, 54, 55, 56, 57, and 58,

ii) a fragment of at least 50 consecutive bases of a sequence under i) which has substantially the same promoter activity as the corresponding transcription regulating nucleotide sequence described by SEQ ID NO: 1, 2, 3, 4, 5, 6, 9, 10, 11, 12, 13, 14, 15, 16, 19, 20, 21, 22, 23, 24, 25, 26, 27, 28, 29, 30, 33, 34, 35, 36, 37, 38, 39, 40, 43, 44, 45, 46, 47, 48, 49, 50, 53, 54, 55, 56, 57, or 58 ;

iii) a nucleotide sequence having substantial similarity (e.g., with a sequence identity of at least 40, 50, 60, to 70%, e.g., preferably 71%, 72%, 73%, 74%, 75%, 76%, 77%, 78%, to 79%, generally at least 80%, e.g., 81% to 84%, at least 85%, e.g., 86%, 87%, 88%, 89%, 90%, 91%, 92%, 93%, 94%, 95%, 96%, 97%, to 98% and 99%) to a transcription regulating nucleotide sequence described by SEQ ID NO: 1, 2, 3, 4, 5, 6, 9, 10, 11, 12, 13, 14, 15, 16, 19, 20, 21, 22, 23, 24, 25, 26, 27, 28, 29, 30, 33, 34, 35, 36, 37, 38, 39, 40, 43, 44, 45, 46, 47, 48, 49, 50, 53, 54, 55, 56, 57, or 58 ;

iv) a nucleotide sequence capable of hybridizing (preferably under conditions equivalent to hybridization in 7% sodium dodecyl sulfate (SDS), 0.5 M $NaPO_4$, 1 mM EDTA at 50°C with washing in 2 X SSC, 0. 1% SDS at 50°C (more desirably in 7% sodium dodecyl sulfate (SDS), 0.5 M $NaPO_4$, 1 mM EDTA at 50°C with washing in 1 X SSC, 0.1% SDS at 50°C, more desirably still in 7% sodium dodecyl sulfate (SDS), 0.5 M $NaPO_4$, 1 mM EDTA at 50°C with washing in 0.5 X SSC, 0. 1% SDS at 50°C, preferably in 7% sodium dodecyl sulfate (SDS), 0.5 M $NaPO_4$, 1 mM EDTA at 50°C with washing in 0.1 X SSC, 0.1 % SDS at 50°C, more preferably in 7% sodium dodecyl sulfate (SDS), 0.5 M $NaPO_4$, 1 mM EDTA at 50°C with washing in 0.1 X SSC, 0.1% SDS at 65°C) to a transcription regulating nucleotide sequence described by SEQ ID NO: 1, 2, 3, 4, 5, 6, 9, 10, 11, 12, 13, 14, 15, 16, 19, 20, 21, 22, 23, 24, 25, 26, 27, 28, 29, 30, 33, 34, 35, 36, 37, 38, 39, 40, 43, 44, 45, 46, 47, 48, 49, 50, 53, 54, 55, 56, 57, or 58, or the complement thereof;

v) a nucleotide sequence capable of hybridizing (preferably under conditions equivalent to hybridization in 7% sodium dodecyl sulfate (SDS), 0.5 M $NaPO_4$, 1 mM EDTA at 50°C with washing in 2 X SSC, 0. 1% SDS at 50°C (more desirably in 7% sodium dodecyl sulfate (SDS), 0.5 M $NaPO_4$, 1 mM EDTA at 50°C with washing in 1 X SSC, 0.1% SDS at 50°C, more desirably still in 7% sodium dodecyl sulfate (SDS), 0.5 M $NaPO_4$, 1 mM EDTA at 50°C with washing in 0.5 X SSC, 0. 1% SDS at 50°C, preferably in 7% sodium dodecyl sulfate (SDS), 0.5 M $NaPO_4$, 1 mM EDTA at 50°C with washing in 0.1 X SSC, 0.1 % SDS at 50°C, more preferably in 7% sodium dodecyl sulfate (SDS), 0.5 M $NaPO_4$, 1 mM EDTA at 50°C with washing in 0.1 X SSC, 0.1% SDS at 65°C) to a nucleic acid comprising 50 to 200 or more consecutive nucleotides of a transcription regulating nucleotide sequence described by SEQ ID NO: 1, 2, 3, 4, 5, 6, 9, 10, 11, 12, 13, 14, 15, 16, 19, 20, 21, 22, 23, 24, 25, 26, 27, 28, 29, 30, 33, 34, 35, 36, 37, 38, 39, 40, 43, 44, 45, 46, 47, 48, 49, 50, 53, 54, 55, 56, 57, and 58, or the complement thereof;

vi) a nucleotide sequence which is the complement or reverse complement of any of the previously mentioned nucleotide sequences under i) to v).

The functional equivalent of the transcription regulating nucleotide sequence is obtained or obtainable from plant genomic DNA from a gene encoding a polypeptide which has at least 70% amino acid sequence identity to a polypeptide selected from the group described by SEQ ID NO: 8, 18, 32, 42, 52, and 60, respectively.

The expression cassette may be employed for numerous expression purposes such as for example expression of a protein, or expression of a antisense RNA, sense or double-stranded RNA. Preferably, expression of the nucleic acid sequence confers to the plant an agronomically valuable trait.

Other embodiments of the invention relate to vectors comprising an expression cassette of the invention, and transgenic host cell or non-human organism comprising an expression cassette or a vector of the invention. Preferably the organism is a plant.

Another embodiment of the invention relates to a method for identifying and/or isolating a sequence with mesophyll- and/or epidermis-specific or mesophyll- and/or epidermis-preferential transcription regulating activity characterized that said identification and/or isolation utilizes a nucleic acid sequence encoding a amino acid sequence as described by SEQ ID NO: 8, 18, 32, 42, 52, or 60 or a part of at least 15 bases thereof. Preferably the nucleic acid sequences is described by SEQ ID NO: 7, 17, 31, 41, 51, or 59 or a part of at least 15 bases thereof. More preferably, identification and/or isolation is realized by a method selected from polymerase chain reaction, hybridization, and database screening.

Another embodiment of the invention relates to a method for providing a transgenic expression cassette for mesophyll- and/or epidermis-specific or mesophyll- and/or epidermis-preferential expression comprising the steps of:

I. isolating of a mesophyll- and/or epidermis-preferential or mesophyll- and/or epidermis-specific transcription regulating nucleotide sequence utilizing at least one nucleic acid sequence or a part thereof, wherein said sequence is encoding a polypeptide described by SEQ ID NO: 8, 18, 32, 42, 52, or 60, or a part of at least 15 bases thereof, and

II. functionally linking said mesophyll- and/or epidermis-preferential or mesophyll- and/or epidermis-specific transcription regulating nucleotide sequence to another nucleotide sequence of interest, which is heterologous in relation to said mesophyll- and/or epidermis-preferential or mesophyll- and/or epidermis-specific transcription regulating nucleotide sequence.

## DEFINITIONS

It is to be understood that this invention is not limited to the particular methodology, protocols, cell lines, plant species or genera, constructs, and reagents described as such. It is also to be understood that the terminology used herein is for the purpose of describing particular embodiments only, and is not intended to limit the scope of the present invention which will be limited only by the appended claims. It must be noted that as used herein and in the appended claims, the singular forms "a," "and," and "the" include plural reference unless the context clearly dictates otherwise. Thus, for example, reference to "a vector" is a reference to one or more vectors and includes equivalents thereof known to those skilled in the art, and so forth.

The term "about" is used herein to mean approximately, roughly, around, or in the region of. When the term "about" is used in conjunction with a numerical range, it modifies that range by extending the boundaries above and below the numerical values set forth. In general, the term "about" is used herein to modify a numerical value above and below the stated value by a variance of 20 per-cent up or down (higher or lower).

As used herein, the word "or" means any one member of a particular list and also includes any combination of members of that list.

The term "gene" is used broadly to refer to any segment of nucleic acid associated with a biological function. Thus, genes include coding sequences and/or the regulatory sequences required for their expression. For example, gene refers to a nucleic acid fragment that expresses mRNA or functional RNA, or encodes a specific protein, and which includes regulatory sequences. Genes also include non-expressed DNA segments that, for example, form recognition sequences for other proteins. Genes can be obtained from a variety of sources, including cloning from a source of interest or synthesizing from known or predicted sequence information, and may include sequences designed to have desired parameters.

The term "native" or "wild type" gene refers to a gene that is present in the genome of an untransformed cell, i.e., a cell not having a known mutation.

A "marker gene" encodes a selectable or screenable trait.

The term "chimeric gene" refers to any gene that contains

1) DNA sequences, including regulatory and coding sequences, that are not found together in nature, or

2) sequences encoding parts of proteins not naturally adjoined, or

3) parts of promoters that are not naturally adjoined.

Accordingly, a chimeric gene may comprise regulatory sequences and coding sequences that are derived from different sources, or comprise regulatory sequences. and coding sequences derived from the same source, but arranged in a manner different from that found in nature.

A "transgene" refers to a gene that has been introduced into the genome by transformation and is stably maintained. Transgenes may include, for example, genes that are either heterologous or homologous to the genes of a particular plant to be transformed. Additionally, transgenes may comprise native genes inserted into a non-native organism, or chimeric genes. The term "endogenous gene" refers to a native gene in its natural location in the genome of an organism. A "foreign" gene refers to a gene not normally found in the host organism but that is introduced by gene transfer.

An "oligonucleotide" corresponding to a nucleotide sequence of the invention, e.g., for use in probing or amplification reactions, may be about 30 or fewer nucleotides in length (e.g., 9, 12, 15, 18, 20, 21 or 24, or any number between 9 and 30). Generally specific primers are upwards of 14 nucleotides in length. For optimum specificity and cost effectiveness, primers of 16 to 24 nucleotides in length may be preferred. Those skilled in the art are well versed in the design of primers for use processes such as PCR. If required, probing can be done with entire restriction fragments of the gene disclosed herein which may be 100's or even 1000's of nucleotides in length.

The terms "protein," "peptide" and "polypeptide" are used interchangeably herein.

The nucleotide sequences of the invention can be introduced into any plant. The genes to be introduced can be conveniently used in expression cassettes for introduction and expression in any plant of interest. Such expression cassettes will comprise the transcriptional initiation region of the invention linked to a nucleotide sequence of interest. Preferred promoters include constitutive, tissue-specific, developmental-specific, inducible and/or viral promoters, most preferred are the mesophyll- and/or epidermis-preferential or mesophyll- and/or epidermis-specific promoters of the invention. Such an expression cassette is provided with a plurality of restriction sites for insertion of the gene of interest to be under the transcriptional regulation of the regulatory regions. The expression cassette may additionally contain selectable marker genes. The cassette will include in the 5'-3' direction of transcription, a transcriptional and translational initiation region, a DNA sequence of interest, and a transcriptional and translational termination region functional in plants. The termination region may be native with the transcriptional initiation region, may be native with the DNA sequence of interest, or may be derived from another source. Convenient termination regions are available from the Ti-plasmid of A. *tumefaciens,* such, as the octopine synthase and nopaline synthase termination regions (see also, Guerineau 1991; Proudfoot 1991; Sanfacon 1991; Mogen 1990; Munroe 1990; Ballas 1989; Joshi 1987).

"Coding sequence" refers to a DNA or RNA sequence that codes for a specific amino acid sequence and excludes the non-coding sequences. It may constitute an "uninterrupted coding sequence", i.e., lacking an intron, such as in a cDNA or it may include one or more introns bounded by appropriate splice junctions. An "intron" is a sequence of RNA which is contained in the primary transcript but which is removed through cleavage and re-ligation of the RNA within the cell to create the mature mRNA that can be translated into a protein.

The terms "open reading frame" and "ORF" refer to the amino acid sequence encoded between translation initiation and termination codons of a coding sequence. The terms "initiation codon" and "termination codon" refer to a unit of three adjacent nucleotides ('codon') in a coding sequence that specifies initiation and chain termination, respectively, of protein synthesis (mRNA translation).

A "functional RNA" refers to an antisense RNA, ribozyme, or other RNA that is not translated.

The term "RNA transcript" refers to the product resulting from RNA polymerase catalyzed transcription of a DNA sequence. When the RNA transcript is a perfect complementary copy of the DNA sequence, it is referred to as the primary transcript or it may be a RNA sequence derived from posttranscriptional processing of the primary transcript and is referred to as the mature RNA. "Messenger RNA" (mRNA) refers to the RNA that is without introns and that can be translated into protein by the cell. "cDNA" refers to a single- or a double-stranded DNA that is complementary to and derived from mRNA.

"Transcription regulating nucleotide sequence", "regulatory sequences", and "suitable regulatory sequences", each refer to nucleotide sequences influencing the transcription, RNA processing or stability, or translation of the associated (or functionally linked) nucleotide sequence to be transcribed. The transcription regulating nucleotide sequence may have various localizations with the respect to the nucleotide sequences to be transcribed. The transcription regulating nucleotide sequence may be located upstream (5' non-coding sequences), within, or downstream (3' non-coding sequences) of the sequence to be transcribed (e.g., a coding sequence). The transcription regulating nucleotide sequences may be selected from the group comprising enhancers, promoters, translation leader sequences, introns, 5'-untranslated sequences, 3'-untranslated sequences, and polyadenylation signal sequences. They include natural and synthetic sequences as well as sequences, which may be a combination of synthetic and natural sequences. As is noted above, the term "transcription regulating nucleotide sequence" is not limited to promoters. However, preferably a transcription regulating nucleotide sequence of the invention comprises at least one promoter sequence (e.g., a sequence localized upstream of the transcription start of a gene capable to induce transcription of the downstream sequences). In one preferred embodiment the transcription regulating nucleotide sequence of the invention comprises the promoter sequence of the corresponding gene and - optionally and preferably - the native 5'-untranslated region of said gene. Furthermore, the 3'-untranslated region and/or the polyadenylation region of said gene may also be employed.

"5' non-coding sequence" refers to a nucleotide sequence located 5' (upstream) to the coding sequence. It is present in the fully processed mRNA upstream of the initiation codon and may affect processing of the primary transcript to mRNA, mRNA stability or translation efficiency (Turner 1995).

"3' non-coding sequence" refers to nucleotide sequences located 3' (downstream) to a coding sequence and include polyadenylation signal sequences and other sequences encoding regulatory signals capable of affecting mRNA processing or gene expression. The polyadenylation signal is usually characterized by affecting the addition of polyadenylic acid tracts to the 3' end of the mRNA precursor. The use of different 3' non-coding sequences is exemplified by Ingelbrecht et al., 1989.

The term "translation leader sequence" refers to that DNA sequence portion of a gene between the promoter and coding sequence that is transcribed into RNA and is present in the fully processed mRNA upstream (5') of the translation start codon. The translation leader sequence may affect processing of the primary transcript to mRNA, mRNA stability or translation efficiency.

"Signal peptide" refers to the amino terminal extension of a polypeptide, which is translated in conjunction with the polypeptide forming a precursor peptide and which is required for its entrance into the secretory pathway. The term "signal sequence" refers to a nucleotide sequence that encodes the signal peptide. The term "transit peptide" as used herein refers part of a expressed polypeptide (preferably to the amino terminal extension of a polypeptide), which is translated in conjunction with the polypeptide forming a precursor peptide and which is required for its entrance into a cell organelle (such as the plastids (e.g., chloroplasts) or mitochondria). The term "transit sequence" refers to a nucleotide sequence that encodes the transit peptide.

"Promoter" refers to a nucleotide sequence, usually upstream (5') to its coding sequence, which controls the expression of the coding sequence by providing the recognition for RNA polymerase and other factors required for proper transcription. "Promoter" includes a minimal promoter that is a short DNA sequence comprised of a TATA box and other sequences that serve to specify the site of transcription initiation, to which regulatory elements are added for control of expression. "Promoter" also refers to a nucleotide sequence that includes a minimal promoter plus regulatory elements that is capable of controlling the expression of a coding sequence or functional RNA. This type of promoter sequence consists of proximal and more distal upstream elements, the latter elements often referred to as enhancers. Accordingly, an "enhancer" is a DNA sequence which can stimulate promoter activity and may be an innate element of the promoter or a heterologous element inserted to enhance the level or tissue specificity of a promoter. It is capable of operating in both orientations (normal or flipped), and is capable of functioning even when moved either upstream or down-stream from the promoter. Both enhancers and other upstream promoter elements bind sequence-specific DNA-binding proteins that mediate their effects. Promoters may be derived in their entirety from a native gene, or be composed of different elements, derived from different promoters found in nature, or even be comprised of synthetic DNA segments. A promoter may also contain DNA sequences that are involved in the binding of protein factors which control the effectiveness of transcription initiation in response to physiological or developmental conditions.

The "initiation site" is the position surrounding the first nucleotide that is part of the transcribed sequence, which is also defined as position +1. With respect to this site all other sequences of the gene and its controlling regions are numbered. Downstream sequences (i.e., further protein encoding sequences in the 3' direction) are denominated positive, while upstream sequences (mostly of the controlling regions in the 5' direction) are denominated negative.

Promoter elements, particularly a TATA element, that are inactive or that have greatly reduced promoter activity in the absence of upstream activation are referred to as "minimal or core promoters." In the presence of a suitable transcription factor, the minimal promoter functions to permit transcription. A "minimal or core promoter" thus consists only of all basal elements needed for transcription initiation, e.g., a TATA box and/or an initiator.

"Constitutive expression" refers to expression using a constitutive or regulated promoter. "Conditional" and "regulated expression" refer to expression controlled by a regulated promoter.

"Constitutive promoter" refers to a promoter that is able to express the open reading frame (ORF) that it controls in all or nearly all of the plant tissues during all or nearly all developmental stages of the plant. Each of the transcription-activating elements do not exhibit an absolute tissue-specificity, but mediate transcriptional activation in most plant parts at a level of at least 1 % of the level reached in the part of the plant in which transcription is most active.

"Regulated promoter" refers to promoters that direct gene expression not constitutively, but in a temporally- and/or spatially-regulated manner, and includes both tissue-specific and inducible promoters. It includes natural and synthetic sequences as well as sequences which may be a combination of synthetic and natural sequences. Different promoters may direct the expression of a gene in different tissues or cell types, or at different stages of development, or in response to different environmental conditions. New promoters of various types useful in plant cells are constantly being discovered, numerous examples may be found in the compilation by Okamuro et al. (1989). Typical regulated promoters useful in plants include but are not limited to safener-inducible promoters, promoters derived from the tetracycline-inducible system, promoters derived from salicylate-inducible systems, promoters derived from alcohol-inducible systems, promoters derived from glucocorticoid-inducible system, promoters derived from pathogen-inducible systems, and promoters derived from ecdysone-inducible systems.

"Tissue-specific promoter" refers to regulated promoters that are not expressed in all plant cells but only in one or more cell types in specific organs (such as leaves or seeds), specific tissues (such as embryo or cotyledon), or specific cell types (such as leaf parenchyma or seed storage cells). These also include promoters that are temporally regulated, such as in early or late embryogenesis, during fruit ripening in developing seeds or fruit, in fully differentiated leaf, or at the onset of senescence.

"Inducible promoter" refers to those regulated promoters that can be turned on in one or more cell types by an external stimulus, such as a chemical, light, hormone, stress, or a pathogen.

"Operably-linked" or "functionally linked" refers preferably to the association of nucleic acid sequences on single nucleic acid fragment so that the function of one is affected by the other. For example, a regulatory DNA sequence is said to be "operably linked to" or "associated with" a DNA sequence that codes for an RNA or a polypeptide if the two sequences are situated such that the regulatory DNA sequence affects expression of the coding DNA sequence (i.e., that the coding sequence or functional RNA is under the transcriptional control of the promoter). Coding sequences can be operably-linked to regulatory sequences in sense or antisense orientation.

"Expression" refers to the transcription and/or translation of an endogenous gene, ORF or portion thereof, or a transgene in plants. For example, in the case of antisense constructs, expression may refer to the transcription of the antisense DNA only. In addition, expression refers to the transcription and stable accumulation of sense (mRNA) or functional RNA. Expression may also refer to the production of protein.

"Specific expression" is the expression of gene products which is limited to one or a few plant tissues (spatial limitation) and/or to one or a few plant developmental stages (temporal limitation). It is acknowledged that hardly a true specificity exists: promoters seem to be preferably switch on in some tissues, while in other tissues there can be no or only little activity. This phenomenon is known as leaky expression. However, with specific expression in this invention is meant preferable expression in one or a few plant tissues.

The "expression pattern" of a promoter (with or without enhancer) is the pattern of expression levels which shows where in the plant and in what developmental stage transcription is initiated by said promoter. Expression patterns of a set of promoters are said to be complementary when the expression pattern of one promoter shows little overlap with the expression pattern of the other promoter. The level of expression of a promoter can be determined by measuring the 'steady state' concentration of a standard transcribed reporter mRNA. This measurement is indirect since the concentration of the reporter mRNA is dependent not only on its synthesis rate, but also on the rate with which the mRNA is degraded. Therefore, the steady state level is the product of synthesis rates and degradation rates.

The rate of degradation can however be considered to proceed at a fixed rate when the transcribed sequences are identical, and thus this value can serve as a measure of synthesis rates. When promoters are compared in this way techniques available to those skilled in the art are hybridization S1-RNAse analysis, northern blots and competitive RT-PCR. This list of techniques in no way represents all available techniques, but rather describes commonly used procedures used to analyze transcription activity and expression levels of mRNA.

The analysis of transcription start points in practically all promoters has revealed that there is usually no single base at which transcription starts, but rather a more or less clustered set of initiation sites, each of which accounts for some start points of the mRNA. Since this distribution varies from promoter to promoter the sequences of the reporter mRNA in each of the populations would differ from each other. Since each mRNA species is more or less prone to degradation, no single degradation rate can be expected for different reporter mRNAs. It has been shown for various eukaryotic promoter sequences that the sequence surrounding the initiation site ('initiator') plays an important role in determining the level of RNA expression directed by that specific promoter. This includes also part of the transcribed sequences. The direct fusion of promoter to reporter sequences would therefore lead to suboptimal levels of transcription.

A commonly used procedure to analyze expression patterns and levels is through determination of the 'steady state' level of protein accumulation in a cell. Commonly used candidates for the reporter gene, known to those skilled in the art are beta-glucuronidase (GUS), chloramphenicol acetyl transferase (CAT) and proteins with fluorescent properties, such as green fluorescent protein (GFP) from Aequora victoria. In principle, however, many more proteins are suitable for this purpose, provided the protein does not interfere with essential plant functions. For quantification and determination of localization a number of tools are suited. Detection systems can readily be created or are available which are based on, e.g., immunochemical, enzymatic, fluorescent detection and quantification. Protein levels can be determined in plant tissue extracts or in intact tissue using in situ analysis of protein expression.

Generally, individual transformed lines with one chimeric promoter reporter construct will vary in their levels of expression of the reporter gene. Also frequently observed is the phenomenon that such transformants do not express any detectable product (RNA or protein). The variability in expression is commonly ascribed to 'position effects', although the molecular mechanisms underlying this inactivity are usually not clear.

"Overexpression" refers to the level of expression in transgenic cells or organisms that exceeds levels of expression in normal or untransformed (non-transgenic) cells or organisms.

"Antisense inhibition" refers to the production of antisense RNA transcripts capable of suppressing the expression of protein from an endogenous gene or a transgene.

"Gene silencing" refers to homology-dependent suppression of viral genes, transgenes, or endogenous nuclear genes. Gene silencing may be transcriptional, when the suppression is due to decreased transcription of the affected genes, or post-transcriptional, when the suppression is due to increased turnover (degradation) of RNA species homologous to the affected genes (English 1996). Gene silencing includes virus-induced gene silencing (Ruiz et al. 1998).

The terms "heterologous DNA sequence," "exogenous DNA segment" or "heterologous nucleic acid," as used herein, each refer to a sequence that originates from a source foreign to the particular host cell or, if from the same source, is modified from its original form. Thus, a heterologous gene in a host cell includes a gene that is endogenous to the particular host cell but has been modified through, for example, the use of DNA shuffling. The terms also include non-naturally occurring multiple copies of a naturally occurring DNA sequence. Thus, the terms refer to a DNA segment that is foreign or heterologous to the cell, or homologous to the cell but in a position within the host cell nucleic acid in which the element is not ordinarily found. Exogenous DNA segments are expressed to yield exogenous polypeptides. A "homologous" DNA sequence is a DNA sequence that is naturally associated with a host cell into which it is introduced.

"Homologous to" in the context of nucleotide sequence identity refers to the similarity between the nucleotide sequence of two nucleic acid molecules or between the amino acid sequences of two protein molecules. Estimates of such homology are provided by either DNA-DNA or DNA-RNA hybridization under conditions of stringency as is well understood by those skilled in the art (as described in Haines and Higgins (eds.), Nucleic Acid Hybridization, IRL Press, Oxford, U.K.), or by the comparison of sequence similarity between two nucleic acids or proteins.

The term "substantially similar" refers to nucleotide and amino acid sequences that represent functional and/or structural equivalents of *Arabidopsis* sequences disclosed herein.

In its broadest sense, the term "substantially similar" when used herein with respect to a nucleotide sequence means that the nucleotide sequence is part of a gene which encodes a polypeptide having substantially the same structure and function as a polypeptide encoded by a gene for the reference nucleotide sequence, e.g., the nucleotide sequence comprises a promoter from a gene that is the ortholog of the gene corresponding to the reference nucleotide sequence, as well as promoter sequences that are structurally related the promoter sequences particularly exemplified herein, i.e., the substantially similar promoter sequences hybridize to the complement of the promoter sequences exemplified herein under high or very high stringency conditions. For example, altered nucleotide sequences which simply reflect the degeneracy of the genetic code but nonetheless encode amino acid sequences that are identical to a particular amino acid sequence are substantially similar to the particular sequences. The term "substantially similar" also includes nucleotide sequences wherein the sequence has been modified, for example, to optimize expression in particular cells, as well as nucleotide sequences encoding a variant polypeptide having one or more amino acid substitutions relative to the (unmodified) polypeptide encoded by the reference sequence, which substitution(s) does not alter the activity of the variant polypeptide relative to the unmodified polypeptide.

In its broadest sense, the term "substantially similar" when used herein with respect to polypeptide means that the polypeptide has substantially the same structure and function as the reference polypeptide. In addition, amino acid sequences that are substantially similar to a particular sequence are those wherein overall amino acid identity is at least 65% or greater to the instant sequences. Modifications that result in equivalent nucleotide or amino acid sequences are well within the routine skill in the art. The percentage of amino acid sequence identity between the substantially similar and the reference polypeptide is at least 65%, 66%, 67%, 68%, 69%, 70%, e.g., 71%, 72%, 73%, 74%, 75%, 76%, 77%, 78%, 79%, 80%, 81%, 82%, 83%, 84%, 85%, 86%, 87%, 88%, 89%, and even 90% or more, e.g., 91%, 92%, 93%, 94%, 95%, 96%, 97%, 98%, up to at least 99%, wherein the reference polypeptide is an *Arabidopsis* polypeptide encoded by a gene with a promoter having any one of SEQ ID NOs: 1, 2, 3, 4, 5, 6, 9, 10, 11, 12, 13, 14, 15, 16, 19, 20, 21, 22, 23, 24, 25, 26, 27, 28, 29, 30, 33, 34, 35, 36, 37, 38, 39, 40, 43, 44, 45, 46, 47, 48, 49, 50, 53, 54, 55, 56, 57, or 58, a nucleotide sequence comprising an open reading frame having any one of SEQ ID NOs: 7, 17, 31, 41, 51, or 59, which encodes one of SEQ ID NOs: 8, 18, 32, 42, 52, or 60. One indication that two polypeptides are substantially similar to each other, besides having substantially the same function, is that an agent, e.g., an antibody, which specifically binds to one of the polypeptides, also specifically binds to the other.

Sequence comparisons maybe carried out using a Smith-Waterman sequence alignment algorithm (see e.g., Waterman (1995)). The localS program, version 1.16, is preferably used with following parameters: match: 1, mismatch penalty: 0.33, open-gap penalty: 2, extended-gap penalty: 2.

Moreover, a nucleotide sequence that is "substantially similar" to a reference nucleotide sequence is said to be "equivalent" to the reference nucleotide sequence. The skilled artisan recognizes that equivalent nucleotide sequences encompassed by this invention can also be defined by their ability to hybridize, under low, moderate and/or stringent conditions (e.g., 0.1 X SSC, 0.1 % SDS, 65°C), with the nucleotide sequences that are within the literal scope of the instant claims.

What is meant by "substantially the same activity" when used in reference to a polynucleotide or polypeptide fragment is that the fragment has at least 65%, 66%, 67%, 68%, 69%, 70%, e.g., 71%, 72%, 73%, 74%, 75%, 76%, 77%, 78%, 79%, 80%, 81%, 82%, 83%, 84%, 85%, 86%, 87%, 88%, 89%, and even 90% or more, e.g., 91%, 92%, 93%, 94%, 95%, 96%, 97%, 98%, up to at least 99% of the activity of the full length polynucleotide or full length polypeptide.

"Target gene" refers to a gene on the replicon that expresses the desired target coding sequence, functional RNA, or protein. The target gene is not essential for replicon replication. Additionally, target genes may comprise native non-viral genes inserted into a non-native organism, or chimeric genes, and will be under the control of suitable regulatory sequences. Thus, the regulatory sequences in the target gene may come from any source, including the virus. Target genes may include coding sequences that are either heterologous or homologous to the genes of a particular plant to be transformed. However, target genes do not include native viral genes. Typical target genes include, but are not limited to genes encoding a structural protein, a seed storage protein, a protein that conveys herbicide resistance, and a protein that conveys insect resistance. Proteins encoded by target genes are known as "foreign proteins". The expression of a target gene in a plant will typically produce an altered plant trait.

The term "altered plant trait" means any phenotypic or genotypic change in a transgenic plant relative to the wild-type or non-transgenic plant host.

"Replication gene" refers to a gene encoding a viral replication protein. In addition to the ORF of the replication protein, the replication gene may also contain other overlapping or non-overlapping ORF(s), as are found in viral sequences in nature. While not essential for replication, these additional ORFs may enhance replication and/or viral DNA accumulation. Examples of such additional ORFs are AC3 and AL3 in ACMV and TGMV geminiviruses, respectively.

"Chimeric trans-acting replication gene" refers either to a replication gene in which the coding sequence of a replication protein is under the control of a regulated plant promoter other than that in the native viral replication gene, or a modified native viral replication gene, for example, in which a site specific sequence(s) is inserted in the 5' transcribed but untranslated region. Such chimeric genes also include insertion of the known sites of replication protein binding between the promoter and the transcription start site that attenuate transcription of viral replication protein gene.

"Chromosomally-integrated" refers to the integration of a foreign gene or DNA construct into the host DNA by covalent bonds. Where genes are not "chromosomally integrated" they may be "transiently expressed." Transient expression of a gene refers to the expression of a gene that is not integrated into the host chromosome but functions independently, either as part of an autonomously replicating plasmid or expression cassette, for example, or as part of another biological system such as a virus.

The term "transformation" refers to the transfer of a nucleic acid fragment into the genome of a host cell, resulting in genetically stable inheritance. Host cells containing the transformed nucleic acid fragments are referred to as "transgenic" cells, and organisms comprising transgenic cells are referred to as "transgenic organisms". Examples of methods of transformation of plants and plant cells include Agrobacterium-mediated transformation (De Blaere 1987) and particle bombardment technology (US 4,945,050). Whole plants may be regenerated from transgenic cells by methods well known to the skilled artisan (see, for example, Fromm 1990).

"Transformed," "transgenic," and "recombinant" refer to a host organism such as a bacterium or a plant into which a heterologous nucleic acid molecule has been introduced. The nucleic acid molecule can be stably integrated into the genome generally known in the art and are disclosed (Sambrook 1989; Innis 1995; Gelfand 1995; Innis & Gelfand 1999. Known methods of PCR include, but are not limited to, methods using paired primers, nested primers, single specific primers, degenerate primers, gene-specific primers, vector-specific primers, partially mismatched primers, and the like. For example, "transformed," "transformant," and "transgenic" plants or calli have been through the transformation process and contain a foreign gene integrated into their chromosome. The term "untransformed" refers to normal plants that have not been through the transformation process.

"Transiently transformed" refers to cells in which transgenes and foreign DNA have been introduced (for example, by such methods as Agrobacterium-mediated transformation or biolistic bombardment), but not selected for stable maintenance.

"Stably transformed" refers to cells that have been selected and regenerated on a selection media following transformation.

"Transient expression" refers to expression in cells in which a virus or a transgene is introduced by viral infection or by such methods as Agrobacterium-mediated transformation, electroporation, or biolistic bombardment, but not selected for its stable maintenance.

"Genetically stable" and "heritable" refer to chromosomally-integrated genetic elements that are stably maintained in the plant and stably inherited by progeny through successive generations.

"Primary transformant" and "T0 generation" refer to transgenic plants that are of the same genetic generation as the tissue which was initially transformed (i.e., not having gone through meiosis and fertilization since transformation).

"Secondary transformants" and the "T1, T2, T3, etc. generations" refer to transgenic plants derived from primary transformants through one or more meiotic and fertilization cycles. They may be derived by self-fertilization of primary or secondary transformants or crosses of primary or secondary transformants with other transformed or untransformed plants.

"Wild-type" refers to a virus or organism found in nature without any known mutation.

"Genome" refers to the complete genetic material of an organism.

The term "nucleic acid" refers to deoxyribonucleotides or ribonucleotides and polymers thereof in either single- or double-stranded form, composed of monomers (nucleotides) containing a sugar, phosphate and a base which is either a purine or pyrimidine. Unless specifically limited, the term encompasses nucleic acids containing known analogs of natural nucleotides which have similar binding properties as the reference nucleic acid and are metabolized in a manner similar to naturally occurring nucleotides. Unless otherwise indicated, a particular nucleic acid sequence also implicitly encompasses conservatively modified variants thereof (e.g., degenerate codon substitutions) and complementary sequences as well as the sequence explicitly indicated. Specifically, degenerate codon substitutions may be achieved by generating sequences in which the third position of one or more selected (or all) codons is substituted with mixed-base and/or deoxyinosine residues (Batzer 1991; Ohtsuka 1985; Rossolini 1994). A "nucleic acid fragment" is a fraction of a given nucleic acid molecule. In higher plants, deoxyribonucleic acid (DNA) is the genetic material while ribonucleic acid (RNA) is involved in the transfer of information contained within DNA into proteins. The term "nucleotide sequence" refers to a polymer of DNA or RNA which can be single- or double-stranded, optionally containing synthetic, non-natural or altered nucleotide bases capable of incorporation into DNA or RNA polymers. The terms "nucleic acid" or "nucleic acid sequence" may also be used interchangeably with gene, cDNA, DNA and RNA encoded by a gene.

The invention encompasses isolated or substantially purified nucleic acid or protein compositions. In the context of the present invention, an "isolated" or "purified" DNA molecule or an "isolated" or "purified" polypeptide is a DNA molecule or polypeptide that, by the hand of man, exists apart from its native environment and is therefore not a product of nature. An isolated DNA molecule or polypeptide may exist in a purified form or may exist in a non-native environment such as, for example, a transgenic host cell. For example, an "isolated" or "purified" nucleic acid molecule or protein, or biologically active portion thereof, is substantially free of other cellular material, or culture medium when produced by recombinant techniques, or substantially free of chemical precursors or other chemicals when chemically synthesized. Preferably, an "isolated" nucleic acid is free of sequences (preferably protein encoding sequences) that naturally flank the nucleic acid (i.e., sequences located at the 5' and 3' ends of the nucleic acid) in the genomic DNA of the organism from which the nucleic acid is derived. For example, in various embodiments, the isolated nucleic acid molecule can contain less than about 5 kb, 4 kb, 3 kb, 2 kb, 1 kb, 0.5 kb, or 0.1 kb of nucleotide sequences that naturally flank the nucleic acid molecule in genomic DNA of the cell from which the nucleic acid is derived. A protein that is substantially free of cellular material includes preparations of protein or polypeptide having less than about 30%, 20%, 10%, 5%, (by dry weight) of contaminating protein. When the protein of the invention, or biologically active portion thereof, is recombinantly produced, preferably culture medium represents less than about 30%, 20%, 10%, or 5% (by dry weight) of chemical precursors or non-protein of interest chemicals.

The nucleotide sequences of the invention include both the naturally occurring sequences as well as mutant (variant) forms. Such variants will continue to possess the desired activity, i.e., either promoter activity or the activity of the product encoded by the open reading frame of the non-variant nucleotide sequence.

The term "variant" with respect to a sequence (e.g., a polypeptide or nucleic acid sequence such as - for example - a transcription regulating nucleotide sequence of the invention) is intended to mean substantially similar sequences. For nucleotide sequences comprising an open reading frame, variants include those sequences that, because of the degeneracy of the genetic code, encode the identical amino acid sequence of the native protein. Naturally occurring allelic variants such as these can be identified with the use of well-known molecular biology techniques, as, for example, with polymerase chain reaction (PCR) and hybridization techniques. Variant nucleotide sequences also include synthetically derived nucleotide sequences, such as those generated, for example, by using site-directed mutagenesis and for open reading frames, encode the native protein, as well as those that encode a polypeptide having amino acid substitutions relative to the native protein. Generally, nucleotide sequence variants of the invention will have at least 40, 50, 60, to 70%, e.g., preferably 71%, 72%, 73%, 74%, 75%, 76%, 77%, 78%, to 79%, generally at least 80%, e.g., 81%-84%, at least 85%, e.g., 86%, 87%, 88%, 89%, 90%, 91%, 92%, 93%, 94%, 95%, 96%, 97%, to 98% and 99% nucleotide sequence identity to the native (wild type or endogenous) nucleotide sequence.

"Conservatively modified variations" of a particular nucleic acid sequence refers to those nucleic acid sequences that encode identical or essentially identical amino acid sequences, or where the nucleic acid sequence does not encode an amino acid sequence, to essentially identical sequences. Because of the degeneracy of the genetic code, a large number of functionally identical nucleic acids encode any given polypeptide. For instance the codons CGT, CGC, CGA, CGG, AGA, and AGG all encode the amino acid arginine. Thus, at every position where an arginine is specified by a codon, the codon can be altered to any of the corresponding codons described without altering the encoded protein. Such nucleic acid variations are "silent variations" which are one species of "conservatively modified variations." Every nucleic acid sequence described herein which encodes a polypeptide also describes every possible silent variation, except where otherwise noted. One of skill will recognize that each codon in a nucleic acid (except ATG, which is ordinarily the only codon for methionine) can be modified to yield a functionally identical molecule by standard techniques. Accordingly, each "silent variation" of a nucleic acid which encodes a polypeptide is implicit in each described sequence.

The nucleic acid molecules of the invention can be "optimized" for enhanced expression in plants of interest (see, for example, WO 91/16432; Perlak 1991; Murray 1989). In this manner, the open reading frames in genes or gene fragments can be synthesized utilizing plant-preferred codons (see, for example, Campbell & Gowri, 1990 for a discussion of host-preferred codon usage). Thus, the nucleotide sequences can be optimized for expression in any plant. It is recognized that all or any part of the gene sequence may be optimized or synthetic. That is, synthetic or partially optimized sequences may also be used. Variant nucleotide sequences and proteins also encompass, sequences and protein derived from a mutagenic and recombinogenic procedure such as DNA shuffling. With such a procedure, one or more different coding sequences can be manipulated to create a new polypeptide possessing the desired properties. In this manner, libraries of recombinant polynucleotides are generated from a population of related sequence polynucleotides comprising sequence regions that have substantial sequence identity and can be homologously recombined in vitro or in vivo. Strategies for such DNA shuffling are known in the art (see, for example, Stemmer.1994; Stemmer 1994; Crameri 1997; Moore 1997; Zhang 1997; Crameri 1998; and US 5,605,793 and 5,837,458).

By "variant" polypeptide is intended a polypeptide derived from the native protein by deletion (so-called truncation) or addition of one or more amino acids to the N-terminal and/or C-terminal end of the native protein; deletion or addition of one or more amino acids at one or more sites in the native protein; or substitution of one or more amino acids at one or more sites in the native protein. Such variants may result from, for example, genetic polymorphism or from human manipulation. Methods for such manipulations are generally known in the art.

Thus, the polypeptides may be altered in various ways including amino acid substitutions, deletions, truncations, and insertions. Methods for such manipulations are generally known in the art. For example, amino acid sequence variants of the polypeptides can be prepared by mutations in the DNA. Methods for mutagenesis and nucleotide sequence alterations are well known in the art (see, for example, Kunkel 1985; Kunkel 1987; US 4,873,192; Walker & Gaastra, 1983 and the references cited therein). Guidance as to appropriate amino acid substitutions that do not affect biological activity of the protein of interest may be found in the model of Dayhoff et al. (1978). Conservative substitutions, such as exchanging one amino acid with another having similar properties, are preferred.

Individual substitutions deletions or additions that alter, add or delete a single amino acid or a small percentage of amino acids (typically less than 5%, more typically less than 1%) in an encoded sequence are "conservatively modified variations," where the alterations result in the substitution of an amino acid with a chemically similar amino acid. Conservative substitution tables providing functionally similar amino acids are well known in the art. The following five groups each contain amino acids that are conservative substitutions for one another: Aliphatic: Glycine (G), Alanine (A), Valine (V), Leucine (L), Isoleucine (I); Aromatic: Phenylalanine (F), Tyrosine (Y), Tryptophan (W); Sulfur-containing: Methionine (M), Cysteine (C); Basic: Arginine (R), Lysine (K), Histidine (H); Acidic: Aspartic acid (D), Glutamic acid (E), Asparagine (N), Glutamine (Q). See also, Creighton, 1984. In addition, individual substitutions, deletions or additions which alter, add or delete a single amino acid or a small percentage of amino acids in an encoded sequence are also "conservatively modified variations."

"Expression cassette" as used herein means a DNA sequence capable of directing expression of a particular nucleotide sequence in an appropriate host cell, comprising a promoter operably linked to a nucleotide sequence of interest, which is - optionally - operably linked to termination signals and/or other regulatory elements. An expression cassette may also comprise sequences required for proper translation of the nucleotide sequence. The coding region usually codes for a protein of interest but may also code for a functional RNA of interest, for example antisense RNA or a nontranslated RNA, in the sense or antisense direction. The expression cassette comprising the nucleotide sequence of interest may be chimeric, meaning that at least one of its components is heterologous with respect to at least one of its other components. The expression cassette may also be one, which is naturally occurring but has been obtained in a recombinant form useful for heterologous expression. An expression cassette may be assembled entirely extracellularly (e.g., by recombinant cloning techniques). However, an expression cassette may also be assembled using in part endogenous components. For example, an expression cassette may be obtained by placing (or inserting) a promoter sequence upstream of an endogenous sequence, which thereby becomes functionally linked and controlled by said promoter sequences. Likewise, a nucleic acid sequence to be expressed may be placed (or inserted) downstream of an endogenous promoter sequence thereby forming an expression cassette. The expression of the nucleotide sequence in the expression cassette may be under the control of a constitutive promoter or of an inducible promoter which initiates transcription only when the host cell is exposed to some particular external stimulus. In the case of a multicellular organism, the promoter can also be specific to a particular tissue or organ or stage of development (e.g., the mesophyll- and/or epidermis-specific or mesophyll- and/or epidermis-preferential promoters of the invention).

"Vector" is defined to include, inter alia, any plasmid, cosmid, phage or Agrobacterium binary vector in double or single stranded linear or circular form which may or may not be self transmissible or mobilizable, and which can transform prokaryotic or eukaryotic host either by integration into the cellular genome or exist extrachromosomally (e.g. autonomous replicating plasmid with an origin of replication).

Specifically included are shuttle vectors by which is meant a DNA vehicle capable, naturally or by design, of replication in two different host organisms, which may be selected from actinomycetes and related species, bacteria and eukaryotic (e.g. higher plant, mammalian, yeast or fungal cells).

Preferably the nucleic acid in the vector is under the control of, and operably linked to, an appropriate promoter or other regulatory elements for transcription in a host cell such as a microbial, e.g. bacterial, or plant cell. The vector may be a bi-functional expression vector which functions in multiple hosts. In the case of genomic DNA, this may contain its own promoter or other regulatory elements and in the case of cDNA this may be under the control of an appropriate promoter or other regulatory elements for expression in the host cell.

"Cloning vectors" typically contain one or a small number of restriction endonuclease recognition sites at which foreign DNA sequences can be inserted in a determinable fashion without loss of essential biological function of the vector, as well as a marker gene that is suitable for use in the identification and selection of cells transformed with the cloning vector. Marker genes typically include genes that provide tetracycline resistance, hygromycin resistance or ampicillin resistance.

A "transgenic plant" is a plant having one or more plant cells that contain an expression vector.

"Plant tissue" includes differentiated and undifferentiated tissues or plants, including but not limited to roots, stems, shoots, leaves, pollen, seeds, tumor tissue and various forms of cells and culture such as single cells, protoplast, embryos, and callus tissue. The plant tissue may be in plants or in organ, tissue or cell culture.

The following terms are used to describe the sequence relationships between two or more nucleic acids or polynucleotides: (a) "reference sequence", (b) "comparison window", (c) "sequence identity", (d) "percentage of sequence identity", and (e) "substantial identity".

(a) As used herein, "reference sequence" is a defined sequence used as a basis for sequence comparison. A reference sequence may be a subset or the entirety of a specified sequence; for example, as a segment of a full length cDNA or gene sequence, or the complete cDNA or gene sequence.

(b) As used herein, "comparison window" makes reference to a contiguous and specified segment of a polynucleotide sequence, wherein the polynucleotide sequence in the comparison window may comprise additions or deletions (i.e., gaps) compared to the reference sequence (which does not comprise additions or deletions) for optimal alignment of the two sequences. Generally, the comparison window is at least 20 contiguous nucleotides in length, and optionally can be 30, 40, 50, 100, or longer. Those of skill in the art understand that to avoid a high similarity to a reference sequence due to inclusion of gaps in the polynucleotide sequence a gap penalty is typically introduced and is subtracted from the number of matches.

Methods of alignment of sequences for comparison are well known in the art. Thus, the determination of percent identity between any two sequences can be accomplished using a mathematical algorithm. Preferred, non-limiting examples of such mathematical algorithms are the algorithm of Myers and Miller, 1988; the local homology algorithm of Smith et al. 1981; the homology alignment algorithm of Needleman and Wunsch 1970; the search-for-similarity-method of Pearson and Lipman 1988; the algorithm of Karlin and Altschul, 1990, modified as in Karlin and Altschul, 1993.

Computer implementations of these mathematical algorithms can be utilized for comparison of sequences to determine sequence identity. Such implementations include, but are not limited to: CLUSTAL in the PC/Gene program (available from Intelligenetics, Mountain View, Calif.); the ALIGN program (Version 2.0) and GAP, BESTFIT, BLAST, FASTA, and TFASTA in the Wisconsin Genetics Software Package, Version 8 (available from Genetics Computer Group (GCG), 575 Science Drive, Madison, Wis., USA). Alignments using these programs can be performed using the default parameters. The CLUSTAL program is well described (Higgins 1988, 1989; Corpet 1988; Huang 1992; Pearson 1994). The ALIGN program is based on the algorithm of Myers and Miller, supra. The BLAST programs of Altschul et al., 1990, are based on the algorithm of Karlin and Altschul, supra.

Software for performing BLAST analyses is publicly available through the National Center for Biotechnology Information (http://www.ncbi.n)m.nih.gov/). This algorithm involves first identifying high scoring sequence pairs (HSPs) by identifying short words of length W in the query sequence, which either match or satisfy some positive-valued threshold score T when aligned with a word of the same length in a database sequence. T is referred to as the neighborhood word score threshold (Altschul 1990). These initial neighborhood word hits act as seeds for initiating searches to find longer HSPs containing them. The word hits are then extended in both directions along each sequence for as far as the cumulative alignment score can be increased. Cumulative scores are calculated using, for nucleotide sequences, the parameters M (reward score for a pair of matching residues; always >0) and N (penalty score for mismatching residues; always <0). For amino acid sequences, a scoring matrix is used to calculate the cumulative score. Extension of the word hits in each direction are halted when the cumulative alignment score falls off by the quantity X from its maximum achieved value, the cumulative score goes to zero or below due to the accumulation of one or more negative-scoring residue alignments, or the end of either sequence is reached.

In addition to calculating percent sequence identity, the BLAST algorithm also performs a statistical analysis of the similarity between two sequences (see, e.g., Karlin & Altschul (1993). One measure of similarity provided by the BLAST algorithm is the smallest sum probability (P(N)), which provides an indication of the probability by which a match between two nucleotide or amino acid sequences would occur by chance. For example, a test nucleic acid sequence is considered similar to a reference sequence if the smallest sum probability in a comparison of the test nucleic acid sequence to the reference nucleic acid sequence is less than about 0.1, more preferably less than about 0.01, and most preferably less than about 0.001.

To obtain gapped alignments for comparison purposes, Gapped BLAST (in BLAST 2.0) can be utilized as described in Altschul et al. 1997. Alternatively, PSI-BLAST (in BLAST 2.0) can be used to perform an iterated search that detects distant relationships between molecules. See Altschul et al., supra. When utilizing BLAST, Gapped BLAST, PSI-BLAST, the default parameters of the respective programs (e.g. BLASTN for nucleotide sequences, BLASTX for proteins) can be used. The BLASTN program (for nucleotide sequences) uses as defaults a wordlength (W) of 11, an expectation (E) of 10, a cutoff of 100, M=5, N=-4, and a comparison of both strands. For amino acid sequences, the BLASTP program uses as defaults a wordlength (W) of 3, an expectation (E) of 10, and the BLOSUM62 scoring matrix (see Henikoff & Henikoff, 1989). See http://www.ncbi.nlm.nih.gov. Alignment may also be performed manually by inspection.

For purposes of the present invention, comparison of nucleotide sequences for determination of percent sequence identity to the promoter sequences disclosed herein is preferably made using the BlastN program (version 1.4.7 or later) with its default parameters or any equivalent program. By "equivalent program" is intended any sequence comparison program that, for any two sequences in question, generates an alignment having identical nucleotide or amino acid residue matches and an identical percent sequence identity when compared to the corresponding alignment generated by the preferred program.

(c) As used herein, "sequence identity" or "identity" in the context of two nucleic acid or polypeptide sequences makes reference to the residues in the two sequences that are the same when aligned for maximum correspondence over a specified comparison window. When percentage of sequence identity is used in reference to proteins it is recognized that residue positions which are not identical often differ by conservative amino acid substitutions, where amino acid residues are substituted for other amino acid residues with similar chemical properties (e.g., charge or hydrophobicity) and therefore do not change the functional properties of the molecule. When sequences differ in conservative substitutions, the percent sequence identity may be adjusted upwards to correct for the conservative nature of the substitution. Sequences that differ by such conservative substitutions are said to have "sequence similarity" or "similarity." Means for making this adjustment are well known to those of skill in the art. Typically this involves scoring a conservative substitution as a partial rather than a full mismatch, thereby increasing the percentage sequence identity. Thus, for example, where an identical amino acid is given a score of 1 and a non-conservative substitution is given a score of zero, a conservative substitution is given a score between zero and 1. The scoring of conservative substitutions is calculated, e.g., as implemented in the program PC/GENE (Intelligenetics, Mountain View, Calif.).

(d) As used herein, "percentage of sequence identity" means the value determined by comparing two optimally aligned sequences over a comparison window, wherein the portion of the polynucleotide sequence in the comparison window may comprise additions or deletions (i.e., gaps) as compared to the reference sequence (which does not comprise additions or deletions) for optimal alignment of the two sequences. The percentage is calculated by determining the number of positions at which the identical nucleic acid base or amino acid residue occurs in both sequences to yield the number of matched positions, dividing the number of matched positions by the total number of positions in the window of comparison, and multiplying the result by 100 to yield the percentage of sequence identity.

(e) (i) The term "substantial identity" or "substantial similarity" of polynucleotide sequences (preferably for a protein encoding sequence) means that a polynucleotide comprises a sequence that has at least 70%, 71%, 72%, 73%, 74%, 75%, 76%, 77%, 78%, or 79%, preferably at least 80%, 81%, 82%, 83%, 84%, 85%, 86%, 87%, 88%, or 89%, more preferably at least 90%, 91%, 92%, 93%, or 94%, and most preferably at least 95%, 96%, 97%, 98%, or 99% sequence identity, compared to a reference sequence using one of the alignment programs described using standard parameters. The term "substantial identity" or "substantial similarity" of polynucleotide sequences (preferably for promoter sequence) means (as described above for variants) that a polynucleotide comprises a sequence that has at least 40, 50, 60, to 70%, e.g., preferably 71%, 72%, 73%, 74%, 75%, 76%, 77%, 78%, to 79%, generally at least 80%, e.g., 81%-84%, at least 85%, e.g., 86%, 87%, 88%, 89%, 90%, 91%, 92%, 93%, 94%, 95%, 96%, 97%, to 98% and 99% nucleotide sequence identity compared to a reference sequence using one of the alignment programs described using standard parameters. One of skill in the art will recognize that these values can be appropriately adjusted to determine corresponding identity of proteins encoded by two nucleotide sequences by taking into account codon degeneracy, amino acid similarity, reading frame positioning, and the like. Substantial identity of amino acid sequences for these purposes normally means sequence identity of at least 70%, more preferably at least 80%, 90%, and most preferably at least 95%.

Another indication that nucleotide sequences are substantially identical is if two molecules hybridize to each other under stringent conditions (see below). Generally, stringent conditions are selected to be about 5°C lower than the thermal melting point ($T_m$) for the specific sequence at a defined ionic strength and pH. However, stringent conditions encompass temperatures in the range of about 1 °C to about 20°C, depending upon the desired degree of stringency as otherwise qualified herein. Nucleic acids that do not hybridize to each other under stringent conditions are still substantially identical if the polypeptides they encode are substantially identical. This may occur, e.g., when a copy of a nucleic acid is created using the maximum codon degeneracy permitted by the genetic code. One indication that two nucleic acid sequences are substantially identical is when the polypeptide encoded by the first nucleic acid is immunologically cross reactive with the polypeptide encoded by the second nucleic acid.

(ii) The term "substantial identity" in the context of a peptide indicates that a peptide comprises a sequence with at least 70%, 71%, 72%, 73%, 74%, 75%, 76%, 77%, 78%, or 79%, preferably 80%, 81%, 82%, 83%, 84%, 85%, 86%, 87%, 88%, or 89%, more preferably at least 90%, 91%, 92%, 93%, or 94%, or even more preferably, 95%, 96%, 97%, 98% or 99%, sequence identity to the reference sequence over a specified comparison window. Preferably, optimal alignment is conducted using the homology alignment algorithm of Needleman and Wunsch (1970). An indication that two peptide sequences are substantially identical is that one peptide is immunologically reactive with antibodies raised against the second peptide. Thus, a peptide is substantially identical to a second peptide, for example, where the two peptides differ only by a conservative substitution.

For sequence comparison, typically one sequence acts as a reference sequence to which test sequences are compared. When using a sequence comparison algorithm, test and reference sequences are input into a computer, subsequence coordinates are designated if necessary, and sequence algorithm program parameters are designated. The sequence comparison algorithm then calculates the percent sequence identity for the test sequence(s) relative to the reference sequence, based on the designated program parameters.

As noted above, another indication that two nucleic acid sequences are substantially identical is that the two molecules hybridize to each other under stringent conditions. The phrase "hybridizing specifically to" refers to the binding, duplexing, or hybridizing of a molecule only to a particular nucleotide sequence under stringent conditions when that sequence is present in a complex mixture (e.g., total cellular) DNA or RNA. "Bind(s) substantially" refers to complementary hybridization between a probe nucleic acid and a target nucleic acid and embraces minor mismatches that can be accommodated by reducing the stringency of the hybridization media to achieve the desired detection of the target nucleic acid sequence.

"Stringent hybridization conditions" and "stringent hybridization wash conditions" in the context of nucleic acid hybridization experiments such as Southern and Northern hybridization are sequence dependent, and are different under different environmental parameters. The $T_m$ is the temperature (under defined ionic strength and pH) at which 50% of the target sequence hybridizes to a perfectly matched probe. Specificity is typically the function of post-hybridization washes, the critical factors being the ionic strength and temperature of the final wash solution. For DNA-DNA hybrids, the $T_m$ can be approximated from the equation of Meinkoth and Wahl, 1984:

$$T_m = 81.5°C + 16.6 (\log_{10} M)+0.41 (\%GC) - 0.61 (\% form) - 500 / L$$

where M is the molarity of monovalent cations, %GC is the percentage of guanosine and cytosine nucleotides in the DNA, % form is the percentage of formamide in the hybridization solution, and L is the length of the hybrid in base pairs. $T_m$ is reduced by about 1 °C for each 1% of mismatching; thus, $T_m$, hybridization, and/or wash conditions can be adjusted to hybridize to sequences of the desired identity. For example, if sequences with >90% identity are sought, the $T_m$ can be decreased 10°C. Generally, stringent conditions are selected to be about 5°C lower than the thermal melting point I for the specific sequence and its complement at a defined ionic strength and pH. However, severely stringent conditions can utilize a hybridization and/or wash at 1, 2, 3, or 4°C lower than the thermal melting point I; moderately stringent conditions can utilize a hybridization and/or wash at 6, 7, 8, 9, or 10°C lower than the thermal melting point I; low stringency conditions can utilize a hybridization and/or wash at 11, 12, 13, 14, 15, or 20°C lower than the thermal melting point I. Using the equation, hybridization and wash compositions, and desired T, those of ordinary skill will understand that variations in the stringency of hybridization and/or wash solutions are inherently described. If the desired degree of mismatching results in a T of less than 45°C (aqueous solution) or 32°C (formamide solution), it is preferred to increase the SSC concentration so that a higher temperature can be used. An extensive guide to the hybridization of nucleic acids is found in Tijssen, 1993. Generally, highly stringent hybridization and wash conditions are selected to be about 5°C lower than the thermal melting point $T_m$ for the specific sequence at a defined ionic strength and pH.

An example of highly stringent wash conditions is 0.15 M NaCl at 72°C for about 15 minutes. An example of stringent wash conditions is a 0.2 X SSC wash at 65°C for 15 minutes (see, Sambrook, infra, for a description of SSC buffer). Often, a high stringency wash is preceded by a low stringency wash to remove background probe signal. An example medium stringency wash for a duplex of, e.g., more than 100 nucleotides, is 1 X SSC at 45°C for 15 minutes. An example low stringency wash for a duplex of, e.g., more than 100 nucleotides, is 4 to 6 X SSC at 40°C for 15 minutes. For short probes (e.g., about 10 to 50 nucleotides), stringent conditions typically involve salt concentrations of less than about 1.5 M, more preferably about 0.01 to 1.0 M, Na ion concentration (or other salts) at pH 7.0 to 8.3, and the temperature is typically at least about 30°C and at least about 60°C for long robes (e.g., >50 nucleotides). Stringent conditions may also be achieved with the addition of destabilizing agents such as formamide. In general, a signal to noise ratio of 2 X (or higher) than that observed for an unrelated probe in the particular hybridization assay indicates detection of a specific hybridization. Nucleic acids that do not hybridize to each other under stringent conditions are still substantially identical if the proteins that they encode are substantially identical. This occurs, e.g., when a copy of a nucleic acid is created using the maximum codon degeneracy permitted by the genetic code.

Very stringent conditions are selected to be equal to the $T_m$ for a particular probe. An example of stringent conditions for hybridization of complementary nucleic acids which have more than 100 complementary residues on a filter in a Southern or Northern blot is 50% formamide, e.g., hybridization in 50% formamide, 1 M NaCl, 1% SDS at 37°C, and a wash in 0.1 x SSC at 60 to 65°C. Exemplary low stringency conditions include hybridization with a buffer solution of 30 to 35% formamide, 1 M NaCl, 1% SDS (sodium dodecyl sulphate) at 37°C, and a wash in 1 X to 2 X SSC (20 X SSC=3.0 M NaCl/0.3 M trisodium citrate) at 50 to 55°C. Exemplary moderate stringency conditions include hybridization in 40 to 45% formamide, 1.0 M NaCl, 1% SDS at 37°C, and a wash in 0.5 X to 1 X SSC at 55 to 60°C.

The following are examples of sets of hybridization/wash conditions that may be used to clone orthologous nucleotide sequences that are substantially identical to reference nucleotide sequences of the present invention: a reference nucleotide sequence preferably hybridizes to the reference nucleotide sequence in 7% sodium dodecyl sulfate (SDS), 0.5 M $NaPO_4$, 1 mM EDTA at 50°C with washing in 2 X SSC, 0. 1% SDS at 50°C, more desirably in 7% sodium dodecyl sulfate (SDS), 0.5 M $NaPO_4$, 1 mM EDTA at 50°C with washing in 1 X SSC, 0.1 % SDS at 50°C, more desirably still in 7% sodium dodecyl sulfate (SDS), 0.5 M $NaPO_4$, 1 mM EDTA at 50°C with washing in 0.5 X SSC, 0. 1% SDS at 50°C, preferably in 7% sodium dodecyl sulfate (SDS), 0.5 M $NaPO_4$, 1 mM EDTA at 50°C with washing in 0.1 X SSC, 0.1 % SDS at 50°C, more preferably in 7% sodium dodecyl sulfate (SDS), 0.5 M $NaPO_4$, 1 mM EDTA at 50°C with washing in 0.1 X SSC, 0.1% SDS at 65°C.

"DNA shuffling" is a method to introduce mutations or rearrangements, preferably randomly, in a DNA molecule or to generate exchanges of DNA sequences between two or more DNA molecules, preferably randomly. The DNA molecule resulting from DNA shuffling is a shuffled DNA molecule that is a non-naturally occurring DNA molecule derived from at least one template DNA molecule. The shuffled DNA preferably encodes a variant polypeptide modified with respect to the polypeptide encoded by the template DNA, and may have an altered biological activity with respect to the polypeptide encoded by the template DNA.

"Recombinant DNA molecule' is a combination of DNA sequences that are joined together using recombinant DNA technology and procedures used to join together DNA sequences as described, for example, in Sambrook et al., 1989.

The word "plant" refers to any plant, particularly to agronomically useful plants (e.g., seed plants), and "plant cell" is a structural and physiological unit of the plant, which comprises a cell wall but may also refer to a protoplast. The plant cell may be in form of an isolated single cell or a cultured cell, or as a part of higher organized unit such as, for example, a plant tissue, or a plant organ differentiated into a structure that is present at any stage of a plant's development. Such structures include one or more plant organs including, but are not limited to, fruit, shoot, stem, leaf, flower petal, etc. Preferably, the term "plant" includes whole plants, shoot vegetative organs/structures (e.g. leaves, stems and tubers), roots, flowers and floral organs/structures (e.g. bracts, sepals, petals, stamens, carpels, anthers and ovules), seeds (including embryo, endosperm, and seed coat) and fruits (the mature ovary), plant tissues (e.g. vascular tissue, ground tissue, and the like) and cells (e.g. guard cells, egg cells, trichomes and the like), and progeny of same.

The class of plants that can be used in the method of the invention is generally as broad as the class of higher and lower plants amenable to transformation techniques, including angiosperms (monocotyledonous and dicotyledonous plants), gymnosperms, ferns, and multicellular algae. It includes plants of a variety of ploidy levels, including aneuploid, polyploid, diploid, haploid and hemizygous. Included within the scope of the invention are all genera and species of higher and lower plants of the plant kingdom. Included are furthermore the mature plants, seed, shoots and seedlings, and parts, propagation material (for example seeds and fruit) and cultures, for example cell cultures, derived therefrom. Preferred are plants and plant materials of the following plant families: *Amaranthaceae, Brassicaceae, Carophyllaceae, Chenopodiaceae, Compositae, Cucurbitaceae, Labiatae, Leguminosae, Papilionoideae, Liliaceae, Linaceae, Malvaceae, Rosaceae, Saxifragaceae, Scrophulariaceae, Solanaceae, Tetragoniaceae.*

Annual, perennial, monocotyledonous and dicotyledonous plants are preferred host organisms for the generation of transgenic plants. The use of the recombination system, or method according to the invention is furthermore advantageous in all omamental plants, forestry, fruit, or omamental trees, flowers, cut flowers, shrubs or turf. Said plant may include - but shall not be limited to - bryophytes such as, for example, Hepaticae (hepaticas) and Musci (mosses); pteridophytes such as ferns, horsetail and clubmosses; gymnosperms such as conifers, cycads, ginkgo and *Gnetaeae;* algae such as *Chlorophyceae, Phaeophpyceae, Rhodophyceae, Myxophyceae, Xanthophy*ceae, *Bacillariophyceae* (diatoms) and *Euglenophyceae.*

Plants for the purposes of the invention may comprise the families of the Rosaceae such as rose, Ericaceae such as rhododendrons and azaleas, *Euphorbiaceae* such as poinsettias and croton, *Caryophyllaceae* such as pinks, Solanaceae such as petunias, *Gesneriaceae* such as African violet, *Balsaminaceae* such as touch-me-not, Orchidaceae such as orchids, *Iridaceae* such as gladioli, iris, freesia and crocus, *Compositae* such as marigold, *Geraniaceae* such as geraniums, *Liliaceae* such as *Drachaena,* Moraceae such as ficus, Araceae such as philodendron and many others. The transgenic plants according to the invention are furthermore selected in particular from among dicotyledonous crop plants such as, for example, from the families of the Leguminosae such as pea, alfalfa and soybean; the family of the *Umbelliferae*, particularly the genus *Daucus* (very particularly the species carota (carrot)) and *Apium* (very particularly the species *graveoiens* var. dulce (celery)) and many others; the family of the Solanaceae, particularly the genus *Lycopersicon,* very particularly the species *esculentum* (tomato) and the genus *Solanum,* very particularly the species *tuberosum* (potato) and *melongena* (aubergine), tobacco and many others; and the genus *Capsicum,* very particularly the species *annum* (pepper) and many others; the family of the *Leguminosae,* particularly the genus *Glycine,* very particularly the species *max* (soybean) and many others; and the family of the *Cruciferae,* particularly the genus *Brassica,* very particularly the species *napus* (oilseed rape), *campestris* (beet), *oleracea* cv Tastie (cabbage), *oleracea* cv Snowball Y (cauliflower) and *oleracea* cv Emperor (broccoli); and the genus *Arabidopsis,* very particularly the species *thaliana* and many others; the family of the Compositae, particularly the genus *Lactuca*, very particularly the species *sativa* (lettuce) and many others.

The transgenic plants according to the invention may be selected among monocotyledonous crop plants, such as, for example, cereals such as wheat, barley, sorghum and millet, rye, triticale, maize, rice or oats, and sugarcane. Further preferred are trees such as apple, pear, quince, plum, cherry, peach, nectarine, apricot, papaya, mango, and other woody species including coniferous and deciduous trees such as poplar, pine, sequoia, cedar, oak, etc. Especially preferred are *Arabidopsis thaliana, Nicotiana tabacum,* oilseed rape, soybean, corn (maize), wheat, linseed, potato and tagetes.

"Significant increase" is an increase that is larger than the margin of error inherent in the measurement technique, preferably an increase by about 2-fold or greater.

"Significantly less" means that the decrease is larger than the margin of error inherent in the measurement technique, preferably a decrease by about 2-fold or greater.

## DETAILED DESCRIPTION OF THE INVENTION

The present invention thus provides for isolated nucleic acid molecules comprising a plant nucleotide sequence that directs mesophyll- and/or epidermis-preferential or mesophyll- and/or epidermis-specific transcription of an operably linked nucleic acid fragment in a plant cell.

Specifically, the present invention provides transgenic expression cassettes for regulating mesophyll- and/or epidermis-preferential or mesophyll- and/or epidermis-specific expression in plants comprising

i) at least one transcription regulating nucleotide sequence of a plant gene, said plant gene selected from the group of genes described by the GenBank *Arabidopsis thaliana* genome locii At5g13220, At1g68850, At4g36670, At3g10920, At1g33240, or At1g28440, or a functional equivalent thereof, and functionally linked thereto

ii) at least one nucleic acid sequence which is heterologous in relation to said transcription regulating nucleotide sequence.

The term "epidermis" as used herein refers to the outermost layer of cells; the "skin" of a plant, covering the leaves, stem, and roots. This tissue may contain specialized cells for defense, gas exchange, or secretion. The epidermis is usually consisting of a single layer but sometimes several layers thick. The cells of the epidermis are characterized by several properties: They generally do not photosynthesize, i.e. they are not green, they are of flat, often isodiametric and particularly in leaves of jigsaw puzzle piece-like shape. They are usually covered by a wax or cutin layer, which confers water repellence and provides protection against adverse environmental conditions. Embedded into the epidermis cell layer are pairs of bean-like shaped cells forming stomata through which the bulk of gas exchange is mediated. Trichomes, i.e. hair-like structures that can be branched or unbranched often protrude from the epidermal cell layer into the environment. Because of their morphological and biochemical features, epidermis cells prevent uncontrolled water loss and are the first line of defense against invading pathogens.

Since the epidermis as the outer cell layer is the contact interphase of the plant with the environment, the epidermis-preferential or epidermis-specific promoters may be especially useful for transgenic approaches in which e.g. invasion by fungal or other pathogens should be minimized. This could be facilitated by epidermis-specific expression of effect genes, which alter e.g. cell wall characteristics or lead to the synthesis and/or secretion of toxic or pathogen-repelling substances. It might also be possible to enhance detection mechanisms towards pathogen attack, e.g. by installing or improving signal transduction chains involved in sensing those attacks. Furthermore, resistance against other biotic or abiotic stress factors (such as draught or freezing) may be enhanced. Another field of application of those promoters could be to enhance the barrier function against water loss in order to engineer plants to better withstand periodical or permanent drought conditions. These latter approaches might be combined with strategies in which promoters specific for other key cells, tissues or organs involved in water use efficiency are exploited (e.g. guard cell-specific, vasculature-specific, root-specific). Additional useful applications for epidermis-preferential or -specific promoters are disclosed in US Patent Application No.: US 2002056153 A1, herein incorporated by reference.

The term "mesophyll" as used here in refers to the internal non-vascular tissue (i.e. ground tissue) of a plant leaf, which is sandwiched between the upper and lower epidermis and specialized for photosynthesis. The mesophyll is covered by epidermal cells on the adaxial as well as on the abaxial side of the leave blade. Mesophyll tissue also contains numerous intercellular spaces, which communicate with the atmosphere outside the leaf via stomata. The mesophyll constitutes the bulk of photosynthetically active tissue of plants. The mesophyll is made up of parenchyma cells and often comprises two layers, palisade mesophyll and spongy mesophyll. While mesophyll cells in the palisade parenchyma are tightly packed in a way which is sufficiently described by the term "palisade", spongy parenchyma cells form a loosely connected three dimensional cellular network with large gas-filled spaces in between the cells. All mesophyll cells contain numerous chloroplasts, for photosynthesis, which lie close to the edge of the cell to gain maximum light and gas supply. Mesophyll cells of source organs (e.g. fully grown leaves) provide photosynthates to all of the sink organs of the plant (e.g. growing leaves, roots, flowers). In order to fulfill their function, mesophyll cells need to be connected to each other by cell-cell junctions, and to the remainder of the plant via long distance transport by the vascular system.

Mesophyll-preferential or mesophyll-specific transcription regulating nucleotide sequence of the invention may be especially useful to drive the expression of effect genes which are intended to alter photosynthetic performance of plants. This could also include approaches in which transgenes are expressed in plants which confer tolerance or resistance to chemicals applied to control weeds by inhibiting photosynthetic processes. It might also be advantageous to use mesophyll-specific promoters to control the expression of transgenes encoding enzymes or regulators from the C4 type of photosynthesis in C3 plants. C4 photosynthesis is distinguished from the C3 type by mechanisms of carbon dioxide enrichment associated with spatial, i.e. morphologically distinct separation of primary and secondary carbon dioxide fixation. Preferred genes in for such applications are described (see e.g., Matsuoka 2001) Mesophyll is also the target of numerous pathogens striving for tapping into the supply of photosynthates for their own benefit. Mesophyll-specific expression of transgenes deterring or entirely preventing pathogens from invading this tissue might therefore be another application of mesophyll-specific promoters.

"Mesophyll-specific transcription" in the context of this invention means the transcription of a nucleic acid sequence by a transcription regulating element in a way that transcription of said nucleic acid sequence in the mesophyll contribute to more than 90%, preferably more than 95%, more preferably more than 99% of the entire quantity of the RNA transcribed from said nucleic acid sequence in the entire plant during any of its developmental stage. The transcription regulating nucleotide sequences designated pSUK460L, pSUK460LGB, pSUK460S, pSUK460SGB, pSUK462L, pSUK462LGB, pSUK462S, and pSUK462SGB and their respective shorter and longer variants are considered to be mesophyll-specific transcription regulating nucleotide sequences.

"Epidermis-specific transcription" in the context of this invention means the transcription of a nucleic acid sequence by a transcription regulating element in a way that transcription of said nucleic acid sequence in the epidermis contribute to more than 90%, preferably more than 95%, more preferably more than 99% of the entire quantity of the RNA transcribed from said nucleic acid sequence in the entire plant during any of its developmental stage.

"Mesophyll- and epidermis specific transcription" in the context of this invention means the transcription of a nucleic acid sequence by a transcription regulating element in a way that transcription of said nucleic acid sequence in the mesophyll and epidermis together contribute to more than 90%, preferably more than 95%, more preferably more than 99% of the entire quantity of the RNA transcribed from said nucleic acid sequence in the entire plant during any of its developmental stage.

"Mesophyll-preferential transcription" in the context of this invention means the transcription of a nucleic acid sequence by a transcription regulating element in a way that transcription of said nucleic acid sequence in the mesophyll contribute to more than 50%, preferably more than 70%, more preferably more than 80% of the entire quantity of the RNA transcribed from said nucleic acid sequence in the entire plant during any of its developmental stage. The transcription regulating nucleotide sequences designated pSUK468L, pSUK468LGB, pSUK468S, pSUK468SGB, pSUK470LGB, pSUK470SGB, pSUK464L, pSUK464LGB, pSUK464S, pSUK464SGB, pSUK466L, pSUK466LGB, pSUK466S, pSUK466SGB, pSUK398L, pSUK398LGB, pSUK398S, pSUK398SGB, pSUK399L, pSUK399LGB, pSUK399S, pSUK399SGB, pSUK400L, pSUK400LGB, pSUK400S and pSUK400SGB and their respective shorter and longer variants are considered to be mesophyll-preferential transcription regulating nucleotide sequences.

"Epidermis-preferential transcription" in the context of this invention means the transcription of a nucleic acid sequence by a transcription regulating element in a way that transcription of said nucleic acid sequence in the epidermis contribute to more than 50%, preferably more than 70%, more preferably more than 80% of the entire quantity of the RNA transcribed from said nucleic acid sequence in the entire plant during any of its developmental stage.

"Mesophyll- and epidermis preferential transcription" in the context of this invention means the transcription of a nucleic acid sequence by a transcription regulating element in a way that transcription of said nucleic acid sequence in the mesophyll and epidermis together contribute to more than 50%, preferably more than 70%, more preferably more than 80% of the entire quantity of the RNA transcribed from said nucleic acid sequence in the entire plant during any of its developmental stage. The transcription regulating nucleotide sequences designated pSUK440L, pSUK440LGB, pSUK440S, pSUK440SGB, pSUK442L, pSUK442LGB, pSUK442S, pSUK442SGB, pSUK402L, pSUK402LGB, pSUK402S, pSUK402SGB, pSUK404LGB, and pSUK404SGB and their respective shorter and longer variants are considered to be mesophyll- and epidermis preferential transcription regulating nucleotide sequences.

Preferably a transcription regulating nucleotide sequence of the invention comprises at least one promoter sequence of the respective gene (e.g., a sequence localized upstream of the transcription start of the respective gene capable to induce transcription of the downstream sequences). The transcription regulating nucleotide sequence may comprise the promoter sequence of said genes but may further comprise other elements such as the 5'-untranslated sequence, enhancer, introns etc. Preferably, said promoter sequence directs mesophyll- and/or epidermis-preferential or mesophyll- and/or epidermis-specific transcription of an operably linked nucleic acid segment in a plant or plant cell e.g., a linked plant DNA comprising an open reading frame for a structural or regulatory gene.

The following Table 1 illustrates the genes from which the promoters of the invention are preferably isolated, the function of said genes, the cDNA encoded by said genes, and the protein (ORF) encoded by said genes.

Table 1: Genes from which the promoters of the invention are preferably isolated, putative function of said genes, cDNA and the protein encoded bv said genes.

| **Gene Locus** | **Putative function** | **Promoter SEQ ID** | **mRNA locus ID cDNA SEQ ID** | **Proteine ID Protein SEQ ID** |
|---|---|---|---|---|
| At5g13220 | encoding expressed protein | SEQ ID NO: 1,2,3,4,5,6 | NM_203046 SEQ ID NO: 7 | NP_974775 SEQ ID NO: 8 |
| At1g68850 | encoding putative peroxidase | SEQ ID NO: 9,10,11,12,13,14, 15,16 | NM_105559 SEQ ID NO: 17 | NP_564948 SEQ ID NO: 18 |

Table continued

| Gene Locus | Putative function | Promoter SEQ ID | mRNA locus ID cDNA SEQ ID | Proteine ID Protein SEQ ID |
|---|---|---|---|---|
| At4g36670 | encoding putative Arabidopsis thaliana mannitol transporter | SEQ ID NO: 19,20,21,22,23, 24,25,26,27,28 29,30 | NM_119831 SEQ ID NO: 31 | NP_195385 SEQ ID NO: 32 |
| At3g10920 | encoding superoxide dismutase [Mn], mitochondrial (SODA) / manganese superoxide dismutase (MSD1) | SEQ ID NO: 33,34,35,36,37, 38, 39,40 | NM_111929 SEQ ID NO: 41 | NP_187703 SEQ ID NO: 42 |
| At1g33240 | encoding putative trihelixbinding protein (GTL1) | SEQ ID NO: 43,44,45,46,47 48,49,50 | NM_103052 SEQ ID NO: 51 | NP_174594.1 SEQ ID NO: 52 |
| At1g28440 | encoding putative leucinerich repeat transmembrane protein kinase | SEQ ID NO: 53,54,55,56,57, 58 | NM_102612 SEQ ID NO: 59 | NP_174166 SEQ ID NO: 60 |

Preferably the transcription regulating nucleotide sequence (or the functional equivalent thereof) is selected from the group of sequences consisting of

i) the sequences described by SEQ ID NOs: 1, 2, 3, 4, 5, 6, 9, 10, 11, 12, 13, 14, 15, 16, 19, 20, 21, 22, 23, 24, 25, 26, 27, 28, 29, 30, 33, 34, 35, 36, 37, 38, 39, 40, 43, 44, 45, 46, 47, 48, 49, 50, 53, 54, 55, 56, 57, and 58,

ii) a fragment of at least 50 consecutive bases of a sequence under i) which has substantially the same promoter activity as the corresponding transcription regulating nucleotide sequence described by 1, 2, 3, 4, 5, 6, 9, 10, 11, 12, 13, 14, 15, 16, 19, 20, 21, 22, 23, 24, 25, 26, 27, 28, 29, 30, 33, 34, 35, 36, 37, 38, 39, 40, 43, 44, 45, 46, 47, 48, 49, 50, 53, 54, 55, 56, 57, or 58;

iii) a nucleotide sequence having substantial similarity (e.g., with a sequence identity of at least 40, 50, 60, to 70%, e.g., preferably 71%, 72%, 73%, 74%, 75%, 76%, 77%, 78%, to 79%, generally at least 80%, e.g., 81% to 84%, at least 85%, e.g., 86%, 87%, 88%, 89%, 90%, 91%, 92%, 93%, 94%, 95%, 96%, 97%, to 98% and 99%) to a transcription regulating nucleotide sequence described by SEQ ID NO: 1, 2, 3, 4, 5, 6, 9, 10, 11, 12, 13, 14, 15, 16, 19, 20, 21, 22, 23, 24, 25, 26, 27, 28, 29, 30, 33, 34, 35, 36, 37, 38, 39, 40, 43, 44, 45, 46, 47, 48, 49, 50, 53, 54, 55, 56, 57, or 58;

iv) a nucleotide sequence capable of hybridizing (preferably under conditions equivalent to hybridization in 7% sodium dodecyl sulfate (SDS), 0.5 M $NaPO_4$, 1 mM EDTA at 50°C with washing in 2 X SSC, 0. 1% SDS at 50°C (more desirably in 7% sodium dodecyl sulfate (SDS), 0.5 M $NaPO_4$, 1 mM EDTA at 50°C with washing in 1 X SSC, 0.1% SDS at 50°C, more desirably still in 7% sodium dodecyl sulfate (SDS), 0.5 M $NaPO_4$, 1 mM EDTA at 50°C with washing in 0.5 X SSC, 0. 1 % SDS at 50°C, preferably in 7% sodium dodecyl sulfate (SDS), 0.5 M $NaPO_4$, 1 mM EDTA at 50°C with washing in 0.1 X SSC, 0.1% SDS at 50°C, more preferably in 7% sodium dodecyl sulfate (SDS), 0.5 M $NaPO_4$, 1 mM EDTA at 50°C with washing in 0.1 X SSC, 0.1% SDS at 65°C) to a transcription regulating nucleotide sequence described by SEQ ID NO: 1, 2, 3, 4, 5, 6, 9, 10, 11, 12, 13, 14, 15, 16, 19, 20, 21, 22, 23, 24, 25, 26, 27, 28, 29, 30, 33, 34, 35, 36, 37, 38, 39, 40, 43, 44, 45, 46, 47, 48, 49, 50, 53, 54, 55, 56, 57, or 58, or the complement thereof;

v) a nucleotide sequence capable of hybridizing (preferably under conditions equivalent to hybridization in 7% sodium dodecyl sulfate (SDS), 0.5 M $NaPO_4$, 1 mM EDTA at 50°C with washing in 2 X SSC, 0. 1% SDS at 50°C (more desirably in 7% sodium dodecyl sulfate (SDS), 0.5 M $NaPO_4$, 1 mM EDTA at 50°C with washing in 1 X SSC, 0.1%

SDS at 50°C, more desirably still in 7% sodium dodecyl sulfate (SDS), 0.5 M $NaPO_4$, 1 mM EDTA at 50°C with washing in 0.5 X SSC, 0. 1 % SDS at 50°C, preferably in 7% sodium dodecyl sulfate (SDS), 0.5 M $NaPO_4$, 1 mM EDTA at 50°C with washing in 0.1 X SSC, 0.1% SDS at 50°C, more preferably in 7% sodium dodecyl sulfate (SDS), 0.5 M $NaPO_4$, 1 mM EDTA at 50°C with washing in 0.1 X SSC, 0.1 % SDS at 65°C) to a nucleic acid comprising 50 to 200 or more consecutive nucleotides of a transcription regulating nucleotide sequence described by SEQ ID NO: 1, 2, 3, 4, 5, 6, 9, 10, 11, 12, 13, 14, 15, 16, 19, 20, 21, 22, 23, 24, 25, 26, 27, 28, 29, 30, 33, 34, 35, 36, 37, 38, 39, 40, 43, 44, 45, 46, 47, 48, 49, 50, 53, 54, 55, 56, 57, or 58, or the complement thereof;

vi) a nucleotide sequence which is the complement or reverse complement of any of the previously mentioned nucleotide sequences under i) to v).

A functional equivalent of the transcription regulating nucleotide sequence can also be obtained or is obtainable from plant genomic DNA from a gene encoding a polypeptide which is substantially similar and preferably has at least 70%, preferably 80%, more preferably 90%, most preferably 95% amino acid sequence identity to a polypeptide encoded by an *Arabidopsis thaliana* gene comprising any one of SEQ ID NOs: 8, 18, 32, 42, 52, or 60, respectively, or a fragment of said transcription regulating nucleotide sequence which exhibits promoter activity in a mesophyll- and/or epidermis-preferential or mesophyll- and/or epidermis-specific fashion.

The activity of a transcription regulating nucleotide sequence is considered equivalent if transcription is initiated in a mesophyll- and/or epidermis-preferential or mesophyll- and/or epidermis-specific fashion (as defined above). Such expression profile is preferably demonstrated using reporter genes operably linked to said transcription regulating nucleotide sequence. Preferred reporter genes (Schenborn 1999) in this context are green fluorescence protein (GFP) (Chui 1996; Leffel 1997), chloramphenicol transferase, luciferase (Millar 1992), β-glucuronidase or β-galactosidase. Especially preferred is β-glucuronidase (Jefferson 1987).

Beside this the transcription regulating activity of a function equivalent may vary from the activity of its parent sequence, especially with respect to expression level. The expression level may be higher or lower than the expression level of the parent sequence. Both derivations may be advantageous depending on the nucleic acid sequence of interest to be expressed. Preferred are such functional equivalent sequences which - in comparison with its parent sequence - does not derivate from the expression level of said parent sequence by more than 50%, preferably 25%, more preferably 10% (as to be preferably judged by either mRNA expression or protein (e.g., reporter gene) expression). Furthermore preferred are equivalent sequences which demonstrate an increased expression in comparison to its parent sequence, preferably an increase my at least 50%, more preferably by at least 100%, most preferably by at least 500%.

Preferably functional equivalent of the transcription regulating nucleotide sequence can be obtained or is obtainable from plant genomic DNA from a gene expressing a mRNA described by a cDNA which is substantially similar and preferably has at least 70%, preferably 80%, more preferably 90%, most preferably 95% sequence identity to a sequence described by any one of SEQ ID NOs: 7, 17, 31, 41, 51, or 59, respectively, or a fragment of said transcription regulating nucleotide sequence which exhibits promoter activity in a mesophyll- and/or epidermis-preferential or mesophyll- and/or epidermis-specific fashion.

Such functional equivalent of the transcription regulating nucleotide sequence may be obtained from other plant species by using the mesophyll- and/or epidermis-preferential or mesophyll- and/or epidermis-specific *Arabidopsis* promoter sequences described herein as probes to screen for homologous structural genes in other plants by hybridization under low, moderate or stringent hybridization conditions. Regions of the mesophyll- and/or epidermis-preferential or mesophyll- and/or epidermis-specific promoter sequences of the present invention which are conserved among species could also be used as PCR primers to amplify a segment from a species other than *Arabidopsis,* and that segment used as a hybridization probe (the latter approach permitting higher stringency screening) or in a transcription assay to determine promoter activity. Moreover, the mesophyll- and/or epidermis-preferential or mesophyll- and/or epidermis-specific promoter sequences could be employed to identify structurally related sequences in a database using computer algorithms.

More specifically, based on the *Arabidopsis* nucleic acid sequences of the present invention, orthologs may be identified or isolated from the genome of any desired organism, preferably from another plant, according to well known techniques based on their sequence similarity to the *Arabidopsis* nucleic acid sequences, e.g., hybridization, PCR or computer generated sequence comparisons. For example, all or a portion of a particular *Arabidopsis* nucleic acid sequence is used as a probe that selectively hybridizes to other gene sequences present in a population of cloned genomic DNA fragments or cDNA fragments (i.e., genomic or cDNA libraries) from a chosen source organism. Further, suitable genomic and cDNA libraries may be prepared from any cell or tissue of an organism. Such techniques include hybridization screening of plated DNA libraries (either plaques or colonies; see, e.g., Sambrook 1989) and amplification by PCR using oligonucleotide primers preferably corresponding to sequence domains conserved among related polypeptide or subsequences of the nucleotide sequences provided herein (see, e.g., Innis 1990). These methods are particularly well suited to the isolation of gene sequences from organisms closely related to the organism from which the probe sequence is derived. The application of these methods using the *Arabidopsis* sequences as probes is well suited for the isolation of gene sequences from any source organism, preferably other plant species. In a PCR approach, oligonucleotide primers can be designed for use in PCR reactions to amplify corresponding DNA sequences from cDNA or genomic DNA extracted from any plant of interest. Methods for designing PCR primers and PCR cloning are generally known in the art.

In hybridization techniques, all or part of a known nucleotide sequence is used as a probe that selectively hybridizes to other corresponding nucleotide sequences present in a population of cloned genomic DNA fragments or cDNA fragments (i.e., genomic or cDNA libraries) from a chosen organism. The hybridization probes may be genomic DNA fragments, cDNA fragments, RNA fragments, or other oligonucleotides, and may be labeled with a detectable group such as $^{32}P$, or any other detectable marker. Thus, for example, probes for hybridization can be made by labeling synthetic oligonucleotides based on the sequence of the invention. Methods for preparation of probes for hybridization and for construction of cDNA and genomic libraries are generally known in the art and are disclosed in Sambrook et al. (1989). In general, sequences that hybridize to the sequences disclosed herein will have at least 40% to 50%, about 60% to 70% and even about 80% 85%, 90%, 95% to 98% or more identity with the disclosed sequences. That is, the sequence similarity of sequences may range, sharing at least about 40% to 50%, about 60% to 70%, and even about 80%, 85%, 90%, 95% to 98% sequence similarity.

The nucleic acid molecules of the invention can also be identified by, for example, a search of known databases for genes encoding polypeptides having a specified amino acid sequence identity or DNA having a specified nucleotide sequence identity. Methods of alignment of sequences for comparison are well known in the art and are described hereinabove.

Hence, the isolated nucleic acid molecules of the invention include the orthologs of the *Arabidopsis* sequences disclosed herein, i.e., the corresponding nucleotide sequences in organisms other than *Arabidopsis,* including, but not limited to, plants other than *Arabidopsis,* preferably dicotyledonous plants, e.g., Brassica napus, alfalfa, sunflower, soybean, cotton, peanut, tobacco or sugar beet, but also cereal plants such as corn, wheat, rye, turfgrass, sorghum, millet, sugarcane, barley and banana. An orthologous gene is a gene from a different species that encodes a product having the same or similar function, e.g., catalyzing the same reaction as a product encoded by a gene from a reference organism. Thus, an ortholog includes polypeptides having less than, e.g., 65% amino acid sequence identity, but which ortholog encodes a polypeptide having the same or similar function. Databases such GenBank may be employed to identify sequences related to the *Arabidopsis* sequences, e.g., orthologs in other dicotyledonous plants such as Brassica napus and others. Alternatively, recombinant DNA techniques such as hybridization or PCR may be employed to identify sequences related to the *Arabidopsis* sequences or to clone the equivalent sequences from different *Arabidopsis* DNAs.

The transcription regulating nucleotide sequences of the invention or their functional equivalents can be obtained or isolated from any plant or non-plant source, or produced synthetically by purely chemical means. Preferred sources include, but are not limited to the plants defined in the DEFINITION section above.

Thus, another embodiment of the invention relates to a method for identifying and/or isolating a sequence with mesophyll- and/or epidermis-preferential or mesophyll- and/or epidermis-specific transcription regulating activity utilizing a nucleic acid sequence encoding a amino acid sequence as described by SEQ ID NO: 8, 18, 32, 42, 52, or 60 or a part thereof. Preferred are nucleic acid sequences described by SEQ ID NO: 7, 17, 31, 41, 51, or 59 or parts thereof. "Part" in this context means a nucleic acid sequence of at least 15 bases preferably at least 25 bases, more preferably at least 50 bases. The method can be based on (but is not limited to) the methods described above such as polymerase chain reaction, hybridization or database screening. Preferably, this method of the invention is based on a polymerase chain reaction, wherein said nucleic acid sequence or its part is utilized as oligonucleotide primer. The person skilled in the art is aware of several methods to amplify and isolate the promoter of a gene starting from part of its coding sequence (such as, for example, part of a cDNA). Such methods may include but are not limited to method such as inverse PCR ("iPCR") or "thermal asymmetric interlaced PCR" ("TAIL PCR").

Another embodiment of the invention is related to a method for providing a transgenic expression cassette for mesophyll- and/or epidermis-preferential or mesophyll- and/or epidermis-specific expression comprising the steps of:

I. isolating of a mesophyll- and/or epidermis-preferential or mesophyll- and/or epidermis-specific transcription regulating nucleotide sequence utilizing at least one nucleic acid sequence or a part thereof, wherein said sequence is encoding a polypeptide described by SEQ ID NO: 8, 18, 32, 42, 52, or 60, or a part of at least 15 bases thereof, and

II. functionally linking said mesophyll- and/or epidermis-preferential or mesophyll- and/or epidermis-specific transcription regulating nucleotide sequence to another nucleotide sequence of interest, which is heterologous in relation to said mesophyll- and/or epidermis-preferential or mesophyll- and/or epidermis-specific transcription regulating nucleotide sequence.

Preferably, the nucleic acid sequence employed for the isolation comprises at least 15 base, preferably at least 25 bases, more preferably at least 50 bases of a sequence described by SEQ ID NO: 7, 17, 31, 41, 51, or 59. Preferably, the isolation of the mesophyll- and/or epidermis-preferential or mesophyll- and/or epidermis-specific transcription regulating nucleotide sequence is realized by a polymerase chain reaction utilizing said nucleic acid sequence as a primer. The operable linkage can be realized by standard cloning method known in the art such as ligation-mediated cloning or recombination-mediated cloning.

Preferably, the transcription regulating nucleotide sequences and promoters of the invention include a consecutive stretch of about 25 to 2000, including 50 to 500 or 100 to 250, and up to 1000 or 1500, contiguous nucleotides, e.g., 40 to about 743, 60 to about 743, 125 to about 743, 250 to about 743, 400 to about 743, 600 to about 743, of any one of SEQ ID NOs: 1, 2, 3, 4, 5, 6, 9, 10, 11, 12, 13, 14, 15, 16, 19, 20, 21, 22, 23, 24, 25, 26, 27, 28, 29, 30, 33, 34, 35, 36, 37, 38, 39, 40, 43, 44, 45, 46, 47, 48, 49, 50, 53, 54, 55, 56, 57, and 58, or the promoter orthologs thereof, which include the minimal promoter region.

In a particular embodiment of the invention said consecutive stretch of about 25 to 2000, including 50 to 500 or 100 to 250, and up to 1000 or 1500, contiguous nucleotides, e.g., 40 to about 743, 60 to about 743, 125 to about 743, 250 to about 743, 400 to about 743, 600 to about 743, has at least 75%, preferably 80%, more preferably 90% and most preferably 95%, nucleic acid sequence identity with a corresponding consecutive stretch of about 25 to 2000, including 50 to 500 or 100 to 250, and up to 1000 or 1500, contiguous nucleotides, e.g., 40 to about 743, 60 to about 743, 125 to about 743, 250 to about 743, 400 to about 743, 600 to about 743, of any one of SEQ ID NOs: 1, 2, 3, 4, 5, 6, 9, 10, 11, 12, 13, 14, 15, 16, 19, 20, 21, 22, 23, 24, 25, 26, 27, 28, 29, 30, 33, 34, 35, 36, 37, 38, 39, 40, 43, 44, 45, 46, 47, 48, 49, 50, 53, 54, 55, 56, 57, and 58, or the promoter orthologs thereof, which include the minimal promoter region. The above defined stretch of contiguous nucleotides preferably comprises one or more promoter motifs selected from the group consisting of TATA box, GC-box, CAAT-box and a transcription start site.

The transcription regulating nucleotide sequences of the invention or their functional equivalents are capable of driving mesophyll- and/or epidermis-preferential or mesophyll- and/or epidermis-specific expression of a coding sequence in a target cell, particularly in a plant cell. The promoter sequences and methods disclosed herein are useful in regulating mesophyll- and/or epidermis-preferential or mesophyll- and/or epidermis-specific expression, respectively, of any heterologous nucleotide sequence in a host plant in order to vary the phenotype of that plant. These promoters can be used with combinations of enhancer, upstream elements, and/or activating sequences from the 5' flanking regions of plant expressible structural genes. Similarly the upstream element can be used in combination with various plant promoter sequences.

The transcription regulating nucleotide sequences and promoters of the invention are useful to modify the phenotype of a plant. Various changes in the phenotype of a transgenic plant are desirable, i.e., modifying the fatty acid composition in a plant, altering the amino acid content of a plant, altering a plant's pathogen defense mechanism, and the like. These results can be achieved by providing expression of heterologous products or increased expression of endogenous products in plants. Alternatively, the results can be achieved by providing for a reduction of expression of one or more endogenous products, particularly enzymes or cofactors in the plant. These changes result in an alteration in the phenotype of the transformed plant.

Generally, the transcription regulating nucleotide sequences and promoters of the invention may be employed to express a nucleic acid segment that is operably linked to said promoter such as, for example, an open reading frame, or a portion thereof, an anti-sense sequence, a sequence encoding for a double-stranded RNA sequence, or a transgene in plants.

An operable linkage may - for example - comprise an sequential arrangement of the transcription regulating nucleotide sequence of the invention (for example a sequence as described by SEQ ID NO: 1, 2, 3, 4, 5, 6, 9, 10, 11, 12, 13, 14, 15, 16, 19, 20, 21, 22, 23, 24, 25, 26, 27, 28, 29, 30, 33, 34, 35, 36, 37, 38, 39, 40, 43, 44, 45, 46, 47, 48, 49, 50, 53, 54, 55, 56, 57, or 58) with a nucleic acid sequence to be expressed, and - optionally - additional regulatory elements such as for example polyadenylation or transcription termination elements, enhancers, introns etc, in a way that the transcription regulating nucleotide sequence can fulfill its function in the process of expression the nucleic acid sequence of interest under the appropriate conditions. the term "appropriate conditions" mean preferably the presence of the expression cassette in a plant cell. Preferred are arrangements, in which the nucleic acid sequence of interest to be expressed is placed down-stream (i.e., in 3'-direction) of the transcription regulating nucleotide sequence of the invention in a way, that both sequences are covalently linked. Optionally additional sequences may be inserted in-between the two sequences. Such sequences may be for example linker or multiple cloning sites. Furthermore, sequences can be inserted coding for parts of fusion proteins (in case a fusion protein of the protein encoded by the nucleic acid of interest is intended to be expressed). Preferably, the distance between the nucleic acid sequence of interest to be expressed and the transcription regulating nucleotide sequence of the invention is not more than 200 base pairs, preferably not more than 100 base pairs, more preferably no more than 50 base pairs.

An operable linkage in relation to any expression cassette or of the invention may be realized by various methods known in the art, comprising both *in vitro* and *in vivo* procedure. Thus, an expression cassette of the invention or an vector comprising such expression cassette may by realized using standard recombination and cloning techniques well known in the art (see e.g., Maniatis 1989; Silhavy 1984; Ausubel 1987).

An expression cassette may also be assembled by inserting a transcription regulating nucleotide sequence of the invention (for example a sequence as described by SEQ ID NO: 1, 2, 3, 4, 5, 6, 9, 10, 11, 12, 13, 14, 15, 16, 19, 20, 21, 22, 23, 24, 25, 26, 27, 28, 29, 30, 33, 34, 35, 36, 37, 38, 39, 40, 43, 44, 45, 46, 47, 48, 49, 50, 53, 54, 55, 56, 57, or 58) into the plant genome. Such insertion will result in an operable linkage to a nucleic acid sequence of interest which as such already existed in the genome. By the insertion the nucleic acid of interest is expressed in a mesophyll- and/or epidermis-preferential or mesophyll- and/or epidermis-specific way due to the transcription regulating properties of the transcription regulating nucleotide sequence. The insertion may be directed or by chance. Preferably the insertion is directed and realized by for example homologous recombination. By this procedure a natural promoter may be exchanged against the transcription regulating nucleotide sequence of the invention, thereby modifying the expression profile of an endogenous gene. The transcription regulating nucleotide sequence may also be inserted in a way, that antisense mRNA of an endogenous gene is expressed, thereby inducing gene silencing.

Similar, a nucleic acid sequence of interest to be expressed may by inserted into a plant genome comprising the transcription regulating nucleotide sequence in its natural genomic environment (i.e. linked to its natural gene) in a way that the inserted sequence becomes operably linked to the transcription regulating nucleotide sequence, thereby forming an expression cassette of the invention.

The open reading frame to be linked to the transcription regulating nucleotide sequence of the invention may be obtained from an insect resistance gene, a disease resistance gene such as, for example, a bacterial disease resistance gene, a fungal disease resistance gene, a viral disease resistance gene, a nematode disease resistance gene, a herbicide resistance gene, a gene affecting grain composition or quality, a nutrient utilization gene, a mycotoxin reduction gene, a male sterility gene, a selectable marker gene, a screenable marker gene, a negative selectable marker, a positive selectable marker, a gene affecting plant agronomic characteristics, i.e., yield, standability, and the like, or an environment or stress resistance gene, i.e., one or more genes that confer herbicide resistance or tolerance, insect resistance or tolerance, disease resistance or tolerance (viral, bacterial, fungal, oomycete, or nematode), stress tolerance or resistance (as exemplified by resistance or tolerance to drought, heat, chilling, freezing, excessive moisture, salt stress, or oxidative stress), increased yields, food content and makeup, physical appearance, male sterility, drydown, standability, prolificacy, starch properties or quantity, oil quantity and quality, amino acid or protein composition, and the like. By "resistant" is meant a plant which exhibits substantially no phenotypic changes as a consequence of agent administration, infection with a pathogen, or exposure to stress. By "tolerant" is meant a plant which, although it may exhibit some phenotypic changes as a consequence of infection, does not have a substantially decreased reproductive capacity or substantially altered metabolism.

Mesophyll- and/or epidermis-preferential or mesophyll- and/or epidermis-specific transcription regulating nucleotide sequences (e.g., promoters) are useful for expressing a wide variety of genes including those which alter metabolic pathways, confer disease resistance, for protein production, e.g., antibody production, or to improve nutrient uptake and the like. Mesophyll- and/or epidermis-preferential or mesophyll- and/or epidermis-specific transcription regulating nucleotide sequences (e.g., promoters) may be modified so as to be regulatable, e.g., inducible. The genes and transcription regulating nucleotide sequences (e.g., promoters) described hereinabove can be used to identify orthologous genes and their promoters which are also likely expressed in a particular tissue and/or development manner. Moreover, the orthologous transcription regulating nucleotide sequences (e.g., promoters) are useful to express linked open reading frames. In addition, by aligning the transcription regulating nucleotide sequences (e.g., promoters) of these orthologs, novel cis elements can be identified that are useful to generate synthetic transcription regulating nucleotide sequences (e.g., promoters).

The expression regulating nucleotide sequences specified above may be optionally operably linked to other suitable regulatory sequences, e.g., a transcription terminator sequence, operator, repressor binding site, transcription factor binding site and/or an enhancer.

The present invention further provides a recombinant vector containing the expression cassette of the invention, and host cells comprising the expression cassette or vector, e.g., comprising a plasmid. The expression cassette or vector may augment the genome of a transformed plant or may be maintained extra chromosomally. The expression cassette or vector of the invention may be present in the nucleus, chloroplast, mitochondria and/or plastid of the cells of the plant. Preferably, the expression cassette or vector of the invention is comprised in the chromosomal DNA of the plant nucleus. The present invention also provides a transgenic plant prepared by this method, a seed from such a plant and progeny plants from such a plant including hybrids and inbreds. The expression cassette may be operatively linked to a structural gene, the open reading frame thereof, or a portion thereof. The expression cassette may further comprise a Ti plasmid and be contained in an Agrobacterium tumefaciens cell; it may be carried on a microparticle, wherein the microparticle is suitable for ballistic transformation of a plant cell; or it may be contained in a plant cell or protoplast. Further, the expression cassette or vector can be contained in a transformed plant or cells thereof, and the plant may be a dicot or a monocot. In particular, the plant may be a dicotyledonous plant. Preferred transgenic plants are transgenic maize, soybean, barley, alfalfa, sunflower, canola, soybean, cotton, peanut, sorghum, tobacco, sugarbeet, rice, wheat, rye, turfgrass, millet, sugarcane, tomato, or potato.

The invention also provides a method of plant breeding, e.g., to prepare a crossed fertile transgenic plant. The method comprises crossing a fertile transgenic plant comprising a particular expression cassette of the invention with itself or with a second plant, e.g., one lacking the particular expression cassette, to prepare the seed of a crossed fertile transgenic plant comprising the particular expression cassette. The seed is then planted to obtain a crossed fertile transgenic plant. The plant may be a monocot or a dicot. In a particular embodiment, the plant is a dicotyledonous plant. The crossed fertile transgenic plant may have the particular expression cassette inherited through a female parent or through a male parent. The second plant may be an inbred plant. The crossed fertile transgenic may be a hybrid. Also included within the present invention are seeds of any of these crossed fertile transgenic plants.

The transcription regulating nucleotide sequences of the invention further comprise sequences which are complementary to one (hereinafter "test" sequence) which hybridizes under stringent conditions with a nucleic acid molecule as described by SEQ ID NO: 1, 2, 3, 4, 5, 6, 9, 10, 11, 12, 13, 14, 15, 16, 19, 20, 21, 22, 23, 24, 25, 26, 27, 28, 29, 30, 33, 34, 35, 36, 37, 38, 39, 40, 43, 44, 45, 46, 47, 48, 49, 50, 53, 54, 55, 56, 57, or 58 as well as RNA which is transcribed from the nucleic acid molecule. When the hybridization is performed under stringent conditions, either the test or nucleic acid molecule of invention is preferably supported, e.g., on a membrane or DNA chip. Thus, either a denatured test or nucleic acid molecule of the invention is preferably first bound to a support and hybridization is effected for a specified period of time at a temperature of, e.g., between 55 and 70°C, in double strength citrate buffered saline (SC) containing 0.1 % SDS followed by rinsing of the support at the same temperature but with a buffer having a reduced SC concentration. Depending upon the degree of stringency required such reduced concentration buffers are typically single strength SC containing 0.1% SDS, half strength SC containing 0.1% SDS and one-tenth strength SC containing 0.1 % SDS. More preferably hybridization is carried out under high stringency conditions (as defined above).

Virtually any DNA composition may be used for delivery to recipient plant cells, e.g., dicotyledonous cells, to ultimately produce fertile transgenic plants in accordance with the present invention. For example, DNA segments or fragments in the form of vectors and plasmids, or linear DNA segments or fragments, in some instances containing only the DNA element to be expressed in the plant, and the like, may be employed. The construction of vectors which may be employed in conjunction with the present invention will be known to those of skill of the art in light of the present disclosure (see, e.g., Sambrook 1989; Gelvin 1990).

Vectors, plasmids, cosmids, YACs (yeast artificial chromosomes), BACs (bacterial artificial chromosomes) and DNA segments for use in transforming such cells will, of course, generally comprise the cDNA, gene or genes which one desires to introduce into the cells. These DNA constructs can further include structures such as promoters, enhancers, polylinkers, or even regulatory genes as desired. The DNA segment, fragment or gene chosen for cellular introduction will often encode a protein which will be expressed in the resultant recombinant cells, such as will result in a screenable or selectable trait and/or which will impart an improved phenotype to the regenerated plant. However, this may not always be the case, and the present invention also encompasses transgenic plants incorporating non-expressed transgenes.

In certain embodiments, it is contemplated that one may wish to employ replication-competent viral vectors in monocot transformation. Such vectors include, for example, wheat dwarf virus (WDV) "shuttle" vectors, such as pW1-11 and PW1-GUS (Ugaki 1991). These vectors are capable of autonomous replication in maize cells as well as E. coli, and as such may provide increased sensitivity for detecting DNA delivered to transgenic cells. A replicating vector may also be useful for delivery of genes flanked by DNA sequences from transposable elements such as Ac, Ds, or Mu. It has been proposed (Laufs 1990) that transposition of these elements within the maize genome requires DNA replication. It is also contemplated that transposable elements would be useful for introducing DNA segments or fragments lacking elements necessary for selection and maintenance of the plasmid vector in bacteria, e.g., antibiotic resistance genes and origins of DNA replication. It is also proposed that use of a transposable element such as Ac, Ds, or Mu would actively promote integration of the desired DNA and hence increase the frequency of stably transformed cells. The use of a transposable element such as Ac, Ds, or Mu may actively promote integration of the DNA of interest and hence increase the frequency of stably transformed cells. Transposable elements may be useful to allow separation of genes of interest from elements necessary for selection and maintenance of a plasmid vector in bacteria or selection of a transformant. By use of a transposable element, desirable and undesirable DNA sequences may be transposed apart from each other in the genome, such that through genetic segregation in progeny, one may identify plants with either the desirable undesirable DNA sequences.

The nucleotide sequence of interest linked to one or more of the transcription regulating nucleotide sequences of the invention can, for example, code for a ribosomal RNA, an antisense RNA or any other type of RNA that is not translated into protein. In another preferred embodiment of the invention, said nucleotide sequence of interest is translated into a protein product. The transcription regulating nucleotide sequence and/or nucleotide sequence of interest linked thereto may be of homologous or heterologous origin with respect to the plant to be transformed. A recombinant DNA molecule useful for introduction into plant cells includes that which has been derived or isolated from any source, that may be subsequently characterized as to structure, size and/or function, chemically altered, and later introduced into plants. An example of a nucleotide sequence or segment of interest "derived" from a source, would be a nucleotide sequence or segment that is identified as a useful fragment within a given organism, and which is then chemically synthesized in essentially pure form. An example of such a nucleotide sequence or segment of interest "isolated" from a source, would be nucleotide sequence or segment that is excised or removed from said source by chemical means, e.g., by the use of restriction endonucleases, so that it can be further manipulated, e.g., amplified, for use in the invention, by the methodology of genetic engineering. Such a nucleotide sequence or segment is commonly referred to as "recombinant."

Therefore a useful nucleotide sequence, segment or fragment of interest includes completely synthetic DNA, semi-synthetic DNA, DNA isolated from biological sources, and DNA derived from introduced RNA. Generally, the introduced DNA is not originally resident in the plant genotype which is the recipient of the DNA, but it is within the scope of the invention to isolate a gene from a given plant genotype, and to subsequently introduce multiple copies of the gene into the same genotype, e.g., to enhance production of a given gene product such as a storage protein or a protein that confers tolerance or resistance to water deficit.

The introduced recombinant DNA molecule includes but is not limited to, DNA from plant genes, and non-plant genes such as those from bacteria, yeasts, animals or viruses. The introduced DNA can include modified genes, portions of genes, or chimeric genes, including genes from the same or different genotype. The term "chimeric gene" or "chimeric DNA" is defined as a gene or DNA sequence or segment comprising at least two DNA sequences or segments from species which do not combine DNA under natural conditions, or which DNA sequences or segments are positioned or linked in a manner which does not normally occur in the native genome of untransformed plant.

The introduced recombinant DNA molecule used for transformation herein may be circular or linear, double-stranded or single-stranded. Generally, the DNA is in the form of chimeric DNA, such as plasmid DNA, that can also contain coding regions flanked by regulatory sequences which promote the expression of the recombinant DNA present in the resultant plant. Generally, the introduced recombinant DNA molecule will be relatively small, i.e., less than about 30 kb to minimize any susceptibility to physical, chemical, or enzymatic degradation which is known to increase as the size of the nucleotide molecule increases. As noted above, the number of proteins, RNA transcripts or mixtures thereof which is introduced into the plant genome is preferably preselected and defined, e.g., from one to about 5-10 such products of the introduced DNA may be formed.

Two principal methods for the control of expression are known, viz.: overexpression and underexpression. Overexpression can be achieved by insertion of one or more than one extra copy of the selected gene. It is, however, not unknown for plants or their progeny, originally transformed with one or more than one extra copy of a nucleotide sequence, to exhibit the effects of underexpression as well as overexpression. For underexpression there are two principle methods which are commonly referred to in the art as "antisense downregulation" and "sense downregulation" (sense downregulation is also referred to as "cosuppression"). Generically these processes are referred to as "gene silencing". Both of these methods lead to an inhibition of expression of the target gene.

Obtaining sufficient levels of transgene expression in the appropriate plant tissues is an important aspect in the production of genetically engineered crops. Expression of heterologous DNA sequences in a plant host is dependent upon the presence of an operably linked promoter that is functional within the plant host. Choice of the promoter sequence will determine when and where within the organism the heterologous DNA sequence is expressed.

It is specifically contemplated by the inventors that one could mutagenize a promoter to potentially improve the utility of the elements for the expression of transgenes in plants. The mutagenesis of these elements can be carried out at random and the mutagenized promoter sequences screened for activity in a trial-by-error procedure. Alternatively, particular sequences which provide the promoter with desirable expression characteristics, or the promoter with expression enhancement activity, could be identified and these or similar sequences introduced into the sequences via mutation. It is further contemplated that one could mutagenize these sequences in order to enhance their expression of transgenes in a particular species.

The means for mutagenizing a DNA segment encoding a promoter sequence of the current invention are well-known to those of skill in the art. As indicated, modifications to promoter or other regulatory element may be made by random, or site-specific mutagenesis procedures. The promoter and other regulatory element may be modified by altering their structure through the addition or deletion of one or more nucleotides from the sequence which encodes the corresponding unmodified sequences.

Mutagenesis may be performed in accordance with any of the techniques known in the art, such as, and not limited to, synthesizing an oligonucleotide having one or more mutations within the sequence of a particular regulatory region. In particular, site-specific mutagenesis is a technique useful in the preparation of promoter mutants, through specific mutagenesis of the underlying DNA. The technique further provides a ready ability to prepare and test sequence variants, for example, incorporating one or more of the foregoing considerations, by introducing one or more nucleotide sequence changes into the DNA. Site-specific mutagenesis allows the production of mutants through the use of specific oligonucleotide sequences which encode the DNA sequence of the desired mutation, as well as a sufficient number of adjacent nucleotides, to provide a primer sequence of sufficient size and sequence complexity to form a stable duplex on both sides of the deletion junction being traversed. Typically, a primer of about 17 to about 75 nucleotides or more in length is preferred, with about 10 to about 25 or more residues on both sides of the junction of the sequence being altered.

In general, the technique of site-specific mutagenesis is well known in the art, as exemplified by various publications. As will be appreciated, the technique typically employs a phage vector which exists in both a single stranded and double stranded form. Typical vectors useful in site-directed mutagenesis include vectors such as the M13 phage. These phage are readily commercially available and their use is generally well known to those skilled in the art. Double stranded plasmids also are routinely employed in site directed mutagenesis which eliminates the step of transferring the gene of interest from a plasmid to a phage.

In general, site-directed mutagenesis in accordance herewith is performed by first obtaining a single-stranded vector or melting apart of two strands of a double stranded vector which includes within its sequence a DNA sequence which encodes the promoter. An oligonucleotide primer bearing the desired mutated sequence is prepared, generally synthetically. This primer is then annealed with the single-stranded vector, and subjected to DNA polymerizing enzymes such as E. coli polymerase I Klenow fragment, in order to complete the synthesis of the mutation-bearing strand. Thus, a heteroduplex is formed wherein one strand encodes the original non-mutated sequence and the second strand bears the desired mutation. This heteroduplex vector is then used to transform or transfect appropriate cells, such as E. coli cells, and cells are selected which include recombinant vectors bearing the mutated sequence arrangement. Vector DNA can then be isolated from these cells and used for plant transformation. A genetic selection scheme was devised by Kunkel et al. (1987) to enrich for clones incorporating mutagenic oligonucleotides. Alternatively, the use of PCR with commercially available thermostable enzymes such as Taq polymerase may be used to incorporate a mutagenic oligonucleotide primer into an amplified DNA fragment that can then be cloned into an appropriate cloning or expression vector. The PCR-mediated mutagenesis procedures of Tomic et al. (1990) and Upender et al. (1995) provide two examples of such protocols. A PCR employing a thermostable ligase in addition to a thermostable polymerase also may be used to incorporate a phosphorylated mutagenic oligonucleotide into an amplified DNA fragment that may then be cloned into an appropriate cloning or expression vector. The mutagenesis procedure described by Michael (1994) provides an example of one such protocol.

The preparation of sequence variants of the selected promoter-encoding DNA segments using site-directed mutagenesis is provided as a means of producing potentially useful species and is not meant to be limiting as there are other ways in which sequence variants of DNA sequences may be obtained. For example, recombinant vectors encoding the desired promoter sequence may be treated with mutagenic agents, such as hydroxylamine, to obtain sequence variants.

As used herein; the term "oligonucleotide directed mutagenesis procedure" refers to template-dependent processes and vector-mediated propagation which result in an increase in the concentration of a specific nucleic acid molecule relative to its initial concentration, or in an increase in the concentration of a detectable signal, such as amplification. As used herein, the term "oligonucleotide directed mutagenesis procedure" also is intended to refer to a process that involves the template-dependent extension of a primer molecule. The term template-dependent process refers to nucleic acid synthesis of an RNA or a DNA molecule wherein the sequence of the newly synthesized strand of nucleic acid is dictated by the well-known rules of complementary base pairing (see, for example, Watson and Rarnstad, 1987). Typically, vector mediated methodologies involve the introduction of the nucleic acid fragment into a DNA or RNA vector, the clonal amplification of the vector, and the recovery of the amplified nucleic acid fragment. Examples of such methodologies are provided by U.S. Pat. No. 4,237,224. A number of template dependent processes are available to amplify the target sequences of interest present in a sample, such methods being well known in the art and specifically disclosed herein below.

Where a clone comprising a promoter has been isolated in accordance with the instant invention, one may wish to delimit the essential promoter regions within the clone. One efficient, targeted means for preparing mutagenizing promoters relies upon the identification of putative regulatory elements within the promoter sequence. This can be initiated by comparison with promoter sequences known to be expressed in similar tissue-specific or developmentally unique manner. Sequences which are shared among promoters with similar expression patterns are likely candidates for the binding of transcription factors and are thus likely elements which confer expression patterns. Confirmation of these putative regulatory elements can be achieved by deletion analysis of each putative regulatory region followed by functional analysis of each deletion construct by assay of a reporter gene which is functionally attached to each construct. As such, once a starting promoter sequence is provided, any of a number of different deletion mutants of the starting promoter could be readily prepared.

Functionally equivalent fragments of a transcription regulating nucleotide sequence of the invention can also be obtained by removing or deleting non-essential sequences without deleting the essential one. Narrowing the transcription regulating nucleotide sequence to its essential, transcription mediating elements can be realized in vitro by trial-and-arrow deletion mutations, or in silico using promoter element search routines. Regions essential for promoter activity often demonstrate clusters of certain, known promoter elements. Such analysis can be performed using available computer algorithms such as PLACE ("Plant Cis-acting Regulatory DNA Elements"; Higo 1999), the BIOBASE database "Transfac" (Biologische Datenbanken GmbH, Braunschweig; Wingender 2001) or the database PlantCARE (Lescot 2002).

Preferably, functional equivalent fragments of one of the transcription regulating nucleotide sequences of the invention comprises at least 100 base pairs, preferably, at least 200 base pairs, more preferably at least 500 base pairs of a transcription regulating nucleotide sequence as described by SEQ ID NO: 1, 2, 3, 4, 5, 6, 9, 10, 11, 12, 13, 14, 15, 16, 19, 20, 21, 22, 23, 24, 25, 26, 27, 28, 29, 30, 33, 34, 35, 36, 37, 38, 39, 40, 43, 44, 45, 46, 47, 48, 49, 50, 53, 54, 55, 56, 57, or 58 . More preferably this fragment is starting from the 3'-end of the indicated sequences.

Especially preferred are equivalent fragments of transcription regulating nucleotide sequences, which are obtained by deleting the region encoding the 5'-untranslated region of the mRNA, thus only providing the (untranscribed) promoter region. The 5'-untranslated region can be easily determined by methods known in the art (such as 5'-RACE analysis). Accordingly, some of the transcription regulating nucleotide sequences of the invention are equivalent fragments of other sequences (see Table 2 below).

Table 2: Relationship of transcription regulating nucleotide sequences of the invention

| Transcription regulating sequence | Equivalent sequence | Equivalent fragment |
|---|---|---|
| SEQ ID NO: 5 (2173 bp) | | SEQ ID NO: 1 (1068 bp)<br>SEQ ID NO: 2 (1076 bp)<br>SEQ ID NO: 3 (986 bp)<br>SEQ ID NO: 4 (992 bp)<br>SEQ ID NO: 6 (2089 bp) |
| SEQ ID NO: 13 (2056 bp) | SEQ ID NO: 14 (2080 bp) | SEQ ID NO: 9 (1289bp)<br>SEQ ID NO: 10 (1297 bp)<br>SEQ ID NO: 11 (1235 bp)<br>SEQ ID NO: 12 (1241 bp)<br>SEQ ID NO: 15 (2002 bp)<br>SEQ ID NO: 16 (2014 bp) |
| SEQ ID NO: 23 (2254 bp) | SEQ ID NO: 24 (2250 bp) | SEQ ID NO: 19 (1023 bp)<br>SEQ ID NO: 20 (1036 bp)<br>SEQ ID NO: 21 (918 bp)<br>SEQ ID NO: 22 (928 bp)<br>SEQ ID NO: 25 (2149 bp)<br>SEQ ID NO: 26 (2143 bp)<br>SEQ ID NO: 27 (1280 bp)<br>SEQ ID NO: 28 (1283 bp)<br>SEQ ID NO: 29 (1175 bp)<br>SEQ ID NO: 30 (1176 bp) |
| SEQ ID NO: 37 (2419 bp) | SEQ ID NO: 38 (2427 bp) | SEQ ID NO: 33 (1179 bp)<br>SEQ ID NO: 34 (1183 bp)<br>SEQ ID NO: 35 (1143 bp)<br>SEQ ID NO: 36 (1149 bp)<br>SEQ ID NO: 39 (2383 bp)<br>SEQ ID NO: 40 (2389 bp) |
| SEQ ID NO: 47 (2819 bp) | SEQ ID NO: 48 (2833 bp) | SEQ ID NO: 43 (1009 bp)<br>SEQ ID NO: 44 (1023 bp)<br>SEQ ID NO: 45 (785 bp)<br>SEQ ID NO: 46 (797 bp)<br>SEQ ID NO: 49 (2595 bp)<br>SEQ ID NO: 50 (2607 bp) |
| SEQ ID NO: 57 (2258 bp) | | SEQ ID NO: 53 (993 bp)<br>SEQ ID NO: 54 (1010 bp)<br>SEQ ID NO: 55 (905 bp)<br>SEQ ID NO: 56 (920 bp) |

Table continued

| Transcription regulating sequence | Equivalent sequence | Equivalent fragment |
|---|---|---|
| | | SEQ ID NO: 58 (2168 bp) |

As indicated above, deletion mutants, deletion mutants of the promoter of the invention also could be randomly prepared and then assayed. With this strategy, a series of constructs are prepared, each containing a different portion of the clone (a subclone), and these constructs are then screened for activity. A suitable means for screening for activity is to attach a deleted promoter or intron construct which contains a deleted segment to a selectable or screenable marker, and to isolate only those cells expressing the marker gene. In this way, a number of different, deleted promoter constructs are identified which still retain the desired, or even enhanced, activity. The smallest segment which is required for activity is thereby identified through comparison of the selected constructs. This segment may then be used for the construction of vectors for the expression of exogenous genes.

An expression cassette of the invention may comprise further regulatory elements. The term in this context is to be understood in the a broad meaning comprising all sequences which may influence construction or function of the expression cassette. Regulatory elements may for example modify transcription and/or translation in prokaryotic or eukaryotic organism. In an preferred embodiment the expression cassette of the invention comprised downstream (in 3'-direction) of the nucleic acid sequence to be expressed a transcription termination sequence and - optionally additional regulatory elements - each operably liked to the nucleic acid sequence to be expressed (or the transcription regulating nucleotide sequence).

Additional regulatory elements may comprise additional promoter, minimal promoters, or promoter elements, which may modify the expression regulating properties. For example the expression may be made depending on certain stress factors such water stress, abscisin (Lam 1991) or heat stress (Schoffl 1989). Furthermore additional promoters or promoter elements may be employed, which may realized expression in other organisms (such as E.coli or Agrobacterium). Such regulatory elements can be find in the promoter sequences or bacteria such as amy and SPO2 or in the promoter sequences of yeast or fungal promoters (such as ADC1, MFa, AC, P-60, CYC1, GAPDH, TEF, rp28, and ADH).

Furthermore, it is contemplated that promoters combining elements from more than one promoter may be useful. For example, US 5,491,288 discloses combining a Cauliflower Mosaic Virus promoter with a histone promoter. Thus, the elements from the promoters disclosed herein may be combined with elements from other promoters. Promoters which are useful for plant transgene expression include those that are inducible, viral, synthetic, constitutive (Odell 1985), temporally regulated, spatially regulated, tissue-specific, and spatial-temporally regulated.

Where expression in specific tissues or organs is desired, tissue-specific promoters may be used. In contrast, where gene expression in response to a stimulus is desired, inducible promoters are the regulatory elements of choice. Where continuous expression is desired throughout the cells of a plant, constitutive promoters are utilized. Additional regulatory sequences upstream and/or downstream from the core promoter sequence may be included in expression constructs of transformation vectors to bring about varying levels of expression of heterologous nucleotide sequences in a transgenic plant.

A variety of 5' and 3' transcriptional regulatory sequences are available for use in the present invention. Transcriptional terminators are responsible for the termination of transcription and correct mRNA polyadenylation. The 3' nontranslated regulatory DNA sequence preferably includes from about 50 to about 1,000, more preferably about 100 to about 1,000, nucleotide base pairs and contains plant transcriptional and translational termination sequences. Appropriate transcriptional terminators and those which are known to function in plants include the CaMV 35S terminator, the tml terminator, the nopaline synthase terminator, the pea rbcS E9 terminator, the terminator for the T7 transcript from the octopine synthase gene of Agrobacterium tumefaciens, and the 3' end of the protease inhibitor I or II genes from potato or tomato, although other 3' elements known to those of skill in the art can also be employed. Alternatively, one also could use a gamma coixin, oleosin 3 or other terminator from the genus Coix.

Preferred 3' elements include those from the nopaline synthase gene of Agrobacterium tumefaciens (Bevan 1983), the terminator for the T7 transcript from the octopine synthase gene of Agrobacterium tumefaciens, and the 3' end of the protease inhibitor I or II genes from potato or tomato.

As the DNA sequence between the transcription initiation site and the start of the coding sequence, i.e., the untranslated leader sequence, can influence gene expression, one may also wish to employ a particular leader sequence. Preferred leader sequences are contemplated to include those which include sequences predicted to direct optimum expression of the attached gene, i.e., to include a preferred consensus leader sequence which may increase or maintain mRNA stability and prevent inappropriate initiation of translation. The choice of such sequences will be known to those of skill in the art in light of the present disclosure. Sequences that are derived from genes that are highly expressed in plants will be most preferred.

Preferred regulatory elements also include the 5'-untranslated region, introns and the 3'-untranslated region of genes. Such sequences that have been found to enhance gene expression in transgenic plants include intron sequences (e.g., from Adh1, bronze1, actin1, actin 2 (WO 00/760067), or the sucrose synthase intron; see: The Maize Handbook, Chapter 116, Freeling and Walbot, Eds., Springer, New York (1994)) and viral leader sequences (e.g., from TMV, MCMV and AMV; Gallie 1987). For example, a number of non-translated leader sequences derived from viruses are known to enhance expression. Specifically, leader sequences from Tobacco Mosaic Virus (TMV), Maize Chlorotic Mottle Virus (MCMV), and Alfalfa Mosaic Virus (AMV) have been shown to be effective in enhancing expression (e.g., Gallie 1987; Skuzeski 1990). Other leaders known in the art include but are not limited to: Picomavirus leaders, for example, EMCV leader (Encephalomyocarditis 5' noncoding region) (Elroy-Stein 1989); Potyvirus leaders, for example, TEV leader (Tobacco Etch Virus); MDMV leader (Maize

Dwarf Mosaic Virus); Human immunoglobulin heavy-chain binding protein (BiP) leader, (Macejak 1991); Untranslated leader from the coat protein mRNA of alfalfa mosaic virus (AMV RNA 4), (Jobling 1987; Tobacco mosaic virus leader (TMV), (Gallie 1989; and Maize Chlorotic Mottle Virus leader (MCMV) (Lommel 1991. See also, Della-Cioppa 1987. Regulatory elements such as Adh intron 1 (Callis 1987), sucrose synthase intron (Vasil 1989) or TMV omega element (Gallie 1989), may further be included where desired. Especially preferred are the 5'-untranslated region, introns and the 3'-untranslated region from the genes described by the GenBank *Arabidopsis thaliana* genome locii At5g13220, At1g68850, At4g36670, At3g10920, At1g33240, or At1g28440, or of functional equivalent thereof.

Additional preferred regulatory elements are enhancer sequences or polyadenylation sequences. Preferred polyadenylation sequences are those from plant genes or Agrobacterium T-DNA genes (such as for example the terminator sequences of the OCS (octopine synthase) or NOS (nopaline synthase) genes).

Examples of enhancers include elements from the CaMV 35S promoter, octopine synthase genes (Ellis el al., 1987), the rice actin I gene, the maize alcohol dehydrogenase gene (Callis 1987), the maize shrunken I gene (Vasil 1989), TMV Omega element (Gallie 1989) and promoters from non-plant eukaryotes (e.g. yeast; Ma 1988). Vectors for use in accordance with the present invention may be constructed to include the ocs enhancer element. This element was first identified as a 16 bp palindromic enhancer from the octopine synthase (ocs) gene of ultilane (Ellis 1987), and is present in at least 10 other promoters (Bouchez 1989). The use of an enhancer element, such as the ocs elements and particularly multiple copies of the element, will act to increase the level of transcription from adjacent promoters when applied in the context of plant transformation.

An expression cassette of the invention (or a vector derived therefrom) may comprise additional functional elements, which are to be understood in the broad sense as all elements which influence construction, propagation, or function of an expression cassette or a vector or a transgenic organism comprising them. Such functional elements may include origin of replications (to allow replication in bacteria; for the ORI of pBR322 or the P15A ori; Sambrook 1989), or elements required for Agrobacterium T-DNA transfer (such as for example the left and/or rights border of the T-DNA).

Ultimately, the most desirable DNA segments for introduction into, for example, a dicot genome, may be homologous genes or gene families which encode a desired trait (e.g., increased yield per acre) and which are introduced under the control of novel promoters or enhancers, etc., or perhaps even homologous or tissue specific (e.g., root-, collar/sheath-, whorl-, stalk-, earshank-, kernel- or leaf-specific) promoters or control elements. Indeed, it is envisioned that a particular use of the present invention will be the expression of a gene in a mesophyll- and/or epidermis-preferential or mesophyll- and/or epidermis-specific manner.

Additionally, vectors may be constructed and employed in the intracellular targeting of a specific gene product within the cells of a transgenic plant or in directing a protein to the extracellular environment. This will generally be achieved by joining a DNA sequence encoding a transit or signal peptide sequence to the coding sequence of a particular gene. The resultant transit or signal peptide will transport the protein to a particular intracellular or extracellular destination, respectively, and will then be post-translationally removed. Transit or signal peptides act by facilitating the transport of proteins through intracellular membranes, e.g., vacuole, vesicle, plastid and mitochondrial membranes, whereas signal peptides direct proteins through the extracellular membrane.

A particular example of such a use concerns the direction of a herbicide resistance gene, such as the EPSPS gene, to a particular organelle such as the chloroplast rather than to the cytoplasm. This is exemplified by the use of the rbcs transit peptide which confers plastid-specific targeting of proteins. In addition, it is proposed that it may be desirable to target certain genes responsible for male sterility to the mitochondria, or to target certain genes for resistance to phytopathogenic organisms to the extracellular spaces, or to target proteins to the vacuole.

By facilitating the transport of the protein into compartments inside and outside the cell, these sequences may increase the accumulation of gene product protecting them from proteolytic degradation. These sequences also allow for additional mRNA sequences from highly expressed genes to be attached to the coding sequence of the genes. Since mRNA being translated by ribosomes is more stable than naked mRNA, the presence of translatable mRNA in front of the gene may increase the overall stability of the mRNA transcript from the gene and thereby increase synthesis of the gene product. Since transit and signal sequences are usually post-translationally removed from the initial translation product, the use of these sequences allows for the addition of extra translated sequences that may not appear on the final polypeptide. Targeting of certain proteins may be desirable in order to enhance the stability of the protein (US 5,545,818).

It may be useful to target DNA itself within a cell. For example, it may be useful to target introduced DNA to the nucleus as this may increase the frequency of transformation. Within the nucleus itself it would be useful to target a gene in order to achieve site specific integration. For example, it would be useful to have an gene introduced through transformation replace an existing gene in the cell. Other elements include those that can be regulated by endogenous or exogenous agents, e.g., by zinc finger proteins, including naturally occurring zinc finger proteins or chimeric zinc finger proteins (see, e.g., US 5,789,538, WO 99/48909; WO 99/45132; WO 98/53060; WO 98/53057; WO 98/53058; WO 00/23464; WO 95/19431; and WO 98/54311) or myb-like transcription factors. For example, a chimeric zinc finger protein may include amino acid sequences which bind to a specific DNA sequence (the zinc finger) and amino acid sequences that activate (e.g., GAL 4 sequences) or repress the transcription of the sequences linked to the specific DNA sequence.

It is one of the objects of the present invention to provide recombinant DNA molecules comprising a nucleotide sequence according to the invention operably linked to a nucleotide segment of interest.

A nucleotide segment of interest is reflective of the commercial markets and interests of those involved in the development of the crop. Crops and markets of interest changes, and as developing nations open up world markets, new crops and technologies will also emerge. In addition, as the understanding of agronomic traits and characteristics such as yield and heterosis increase, the choice of genes for transformation will change accordingly. General categories of nucleotides of interest include, for example, genes involved in information, such as zinc fingers, those involved in communication, such as kinases, and those involved in housekeeping, such as heat shock proteins. More specific categories of transgenes, for example, include genes encoding important traits for agronomics, insect resistance, disease resistance, herbicide resistance, sterility, grain characteristics, and commercial products. Genes of interest include, generally, those involved in starch, oil, carbohydrate, or nutrient metabolism, as well as those affecting kernel size, sucrose loading, zinc finger proteins, see, e.g., US 5,789,538, WO 99/48909; WO 99/45132; WO 98/53060; WO 98/53057; WO 98/53058; WO 00/23464; WO 95/19431; and WO 98/54311, and the like.

One skilled in the art recognizes that the expression level and regulation of a transgene in a plant can vary significantly from line to line. Thus, one has to test several lines to find one with the desired expression level and regulation. Once a line is identified with the desired regulation specificity of a chimeric Cre transgene, it can be crossed with lines carrying different inactive replicons or inactive transgene for activation.

Other sequences which may be linked to the gene of interest which encodes a polypeptide are those which can target to a specific organelle, e.g., to the mitochondria, nucleus, or plastid, within the plant cell. Targeting can be achieved by providing the polypeptide with an appropriate targeting peptide sequence, such as a secretory signal peptide (for secretion or cell wall or membrane targeting, a plastid transit peptide, a chloroplast transit peptide, e.g., the chlorophyll a/b binding protein, a mitochondrial target peptide, a vacuole targeting peptide, or a nuclear targeting peptide, and the like. For example, the small subunit of ribulose bisphosphate carboxylase transit peptide, the EPSPS transit peptide or the dihydrodipicolinic acid synthase transit peptide may be used. For examples of plastid organelle targeting sequences (see WO 00/12732). Plastids are a class of plant organelles derived from proplastids and include chloroplasts, leucoplasts, amyloplasts, and chromoplasts. The plastids are major sites of biosynthesis in plants. In addition to photosynthesis in the chloroplast, plastids are also sites of lipid biosynthesis, nitrate reduction to ammonium, and starch storage. And while plastids contain their own circular, genome, most of the proteins localized to the plastids are encoded by the nuclear genome and are imported into the organelle from the cytoplasm.

Transgenes used with the present invention will often be genes that direct the expression of a particular protein or polypeptide product, but they may also be non-expressible DNA segments, e.g., transposons such as Ds that do no direct their own transposition. As used herein, an "expressible gene" is any gene that is capable of being transcribed into RNA (e.g., mRNA, antisense RNA, etc.) or translated into a protein, expressed as a trait of interest, or the like, etc., and is not limited to selectable, screenable or non-selectable marker genes. The invention also contemplates that, where both an expressible gene that is not necessarily a marker gene is employed in combination with a marker gene, one may employ the separate genes on either the same or different DNA segments for transformation. In the latter case, the different vectors are delivered concurrently to recipient cells to maximize cotransformation.

The choice of the particular DNA segments to be delivered to the recipient cells will often depend on the purpose of the transformation. One of the major purposes of transformation of crop plants is to add some commercially desirable, agronomically important traits to the plant. Such traits include, but are not limited to, herbicide resistance or tolerance; insect resistance or tolerance; disease resistance or tolerance (viral, bacterial, fungal, nematode); stress tolerance and/or resistance, as exemplified by resistance or tolerance to drought, heat, chilling, freezing, excessive moisture, salt stress; oxidative stress; increased yields; food content and makeup; physical appearance; male sterility; drydown; standability; prolificacy; starch properties; oil quantity and quality; and the like. One may desire to incorporate one or more genes conferring any such desirable trait or traits, such as, for example, a gene or genes encoding pathogen resistance.

In certain embodiments, the present invention contemplates the transformation of a recipient cell with more than one advantageous transgene. Two or more transgenes can be supplied in a single transformation event using either distinct transgene-encoding vectors, or using a single vector incorporating two or more gene coding sequences. For example, plasmids bearing the bar and aroA expression units in either convergent, divergent, or colinear orientation, are considered to be particularly useful. Further preferred combinations are those of an insect resistance gene, such as a Bt gene, along with a protease inhibitor gene such as pinII, or the use of bar in combination with either of the above genes. Of course, any two or more transgenes of any description, such as those conferring herbicide, insect, disease (viral, bacterial, fungal, nematode) or drought resistance, male sterility, drydown, standability, prolificacy, starch properties, oil quantity and quality, or those increasing yield or nutritional quality may be employed as desired.

### 1. Exemplary Transgenes

#### 1.1. Herbicide Resistance

The genes encoding phosphinothricin acetyltransferase (bar and pat), glyphosate tolerant EPSP synthase genes, the glyphosate degradative enzyme gene gox encoding glyphosate oxidoreductase, deh (encoding a dehalogenase enzyme that inactivates dalapon), herbicide resistant (e.g., sulfonylurea and imidazolinone) acetolactate synthase, and bxn genes (encoding a nitrilase enzyme that degrades bromoxynil) are good examples of herbicide resistant genes for use in transformation. The bar and pat genes code for an enzyme, phosphinothricin acetyltransferase (PAT), which inactivates the herbicide phosphinothricin and prevents this compound from inhibiting glutamine synthetase enzymes. The enzyme 5-enolpyruvylshikimate 3-phosphate synthase (EPSP Synthase), is normally inhibited by the herbicide N-(phosphonomethyl)glycine (glyphosate). However, genes are known that encode glyphosate-resistant EPSP Synthase enzymes. The deh gene encodes the enzyme dalapon dehalogenase and confers resistance to the herbicide dalapon. The bxn gene codes for a specific nitrilase enzyme that converts bromoxynil to a non-herbicidal degradation product.

#### 1.2 Insect Resistance

An important aspect of the present invention concerns the introduction of insect resistance-conferring genes into plants. Potential insect resistance genes which can be introduced include Bacillus thuringiensis crystal toxin genes or Bt genes (Watrud 1985). Bt genes may provide resistance to lepidopteran or coleopteran pests such as European Corn Borer (ECB) and corn rootworm (CRW). Preferred Bt toxin genes for use in such embodiments include the CrylA (b) and CrylA(c) genes. Endotoxin genes from other species of B. thuringiensis which affect insect growth or development may also be employed in this regard. Protease inhibitors may also provide insect resistance (Johnson 1989), and will thus have utility in plant transformation. The use of a protease inhibitor II gene, pinII, from tomato or potato is envisioned to be particularly useful. Even more advantageous is the use of a pinII gene in combination with a Bt toxin gene, the combined effect of which has been discovered by the present inventors to produce synergistic insecticidal activity. Other genes which encode inhibitors of the insects' digestive system, or those that encode enzymes or co-factors that facilitate the production of inhibitors, may also be useful. This group may be exemplified by cystatin and amylase inhibitors, such as those from wheat and barley.

Also, genes encoding lectins may confer additional or alternative insecticide properties. Lectins (originally termed phytohemagglutinins) are multivalent carbohydrate-binding proteins which have the ability to agglutinate red blood cells from a range of species. Lectins have been identified recently as insecticidal agents with activity against weevils, ECB and rootworm (Murdock 1990; Czapla & Lang, 1990). Lectin genes contemplated to be useful include, for example, barley and wheat germ agglutinin (WGA) and rice lectins (Gatehouse 1984), with WGA being preferred.

Genes controlling the production of large or small polypeptides active against insects when introduced into the insect pests, such as, e.g., lytic peptides, peptide hormones and toxins and venoms, form another aspect of the invention. For example, it is contemplated, that the expression of juvenile hormone esterase, directed towards specific insect pests, may also result in insecticidal activity, or perhaps cause cessation of metamorphosis (Hammock 1990).

Transgenic plants expressing genes which encode enzymes that affect the integrity of the insect cuticle form yet another aspect of the invention. Such genes include those encoding, e.g., chitinase, proteases, lipases and also genes for the production of nik-komycin, a compound that inhibits chitin synthesis, the introduction of any of which is contemplated to produce insect resistant maize plants. Genes that code for activities that affect insect molting, such those affecting the production of ecdysteroid UDP-glucosyl transferase, also fall within the scope of the useful transgenes of the present invention.

Genes that code for enzymes that facilitate the production of compounds that reduce the nutritional quality of the host plant to insect pests are also encompassed by the present invention. It may be possible, for instance, to confer insecticidal activity on a plant by altering its sterol composition. Sterols are obtained by insects from their diet and are used for hormone synthesis and membrane stability. Therefore alterations in plant sterol composition by expression of novel genes, e.g., those that directly promote the production of undesirable sterols or those that convert desirable sterols into undesirable forms, could have a negative effect on insect growth and/or development and hence endow the plant with insecticidal activity. Lipoxygenases are naturally occurring plant enzymes that have been shown to exhibit anti-nutritional effects on insects and to reduce the nutritional quality of their diet. Therefore, further embodiments of the invention concem transgenic plants with enhanced lipoxygenase activity which may be resistant to insect feeding.

The present invention also provides methods and compositions by which to achieve qualitative or quantitative changes in plant secondary metabolites. One example concerns transforming plants to produce DIMBOA which, it is contemplated, will confer resistance to European corn borer, rootworm and several other maize insect pests. Candidate genes that are particularly considered for use in this regard include those genes at the bx locus known to be involved in the synthetic DIMBOA pathway (Dunn 1981). The introduction of genes that can regulate the production of maysin, and genes involved in the production of dhurrin in sorghum, is also contemplated to be of use in facilitating resistance to earworm and rootworm, respectively.

Tripsacum dactyloides is a species of grass that is resistant to certain insects, including corn root worm. It is anticipated that genes encoding proteins that are toxic to insects or are involved in the biosynthesis of compounds toxic to insects will be isolated from Tripsacum and that these novel genes will be useful in conferring resistance to insects. It is known that the basis of insect resistance in Tripsacum is genetic, because said resistance has been transferred to Zea mays via sexual crosses (Branson & Guss, 1972).

Further genes encoding proteins characterized as having potential insecticidal activity may also be used as transgenes in accordance herewith. Such genes include, for example, the cowpea trypsin inhibitor (CpTI; Hilder 1987) which may be used as a rootworm deterrent; genes encoding avermectin (Campbell 1989; Ikeda 1987) which may prove particularly useful as a corn rootworm deterrent; ribosome inactivating protein genes; and even genes that regulate plant structures. Transgenic maize including antiinsect antibody genes and genes that code for enzymes that can covert a non-toxic insecticide (pro-insecticide) applied to the outside of the plant into an insecticide inside the plant are also contemplated.

### 1.3 Environment or Stress Resistance

Improvement of a plant's ability to tolerate various environmental stresses such as, but not limited to, drought, excess moisture, chilling, freezing, high temperature, salt, and oxidative stress, can also be effected through expression of heterologous, or overexpression of homologous genes. Benefits may be realized in terms of increased resistance to freezing temperatures through the introduction of an "antifreeze" protein such as that of the Winter Flounder (Cutler 1989) or synthetic gene derivatives thereof. Improved chilling tolerance may also be conferred through increased expression of glycerol-3-phosphate acetyltransferase in chloroplasts (Murata 1992; Wolter 1992). Resistance to oxidative stress (often exacerbated by conditions such as chilling temperatures in combination with high light intensities) can be conferred by expression of superoxide dismutase (Gupta 1993), and may be improved by glutathione reductase (Bowler 1992). Such strategies may allow for tolerance to freezing in newly emerged fields as well as extending later maturity higher yielding varieties to earlier relative maturity zones.

Expression of novel genes that favorably effect plant water content, total water potential, osmotic potential, and turgor can enhance the ability of the plant to tolerate drought. As used herein, the terms "drought resistance" and "drought tolerance" are used to refer to a plants increased resistance or tolerance to stress induced by a reduction in water availability, as compared to normal circumstances, and the ability of the plant to function and survive in lower-water environments, and perform in a relatively superior manner. In this aspect of the invention it is proposed, for example, that the expression of a gene encoding the biosynthesis of osmotically-active solutes can impart protection against drought. Within this class of genes are DNAs encoding mannitol dehydrogenase (Lee and Saier, 1982) and trehalose-6-phosphate synthase (Kaasen 1992). Through the subsequent action of native phosphatases in the cell or by the introduction and coexpression of a specific phosphatase, these introduced genes will result in the accumulation of either mannitol or trehalose, respectively, both of which have been well documented as protective compounds able to mitigate the effects of stress. Mannitol accumulation in transgenic tobacco has been verified and preliminary results indicate that plants expressing high levels of this metabolite are able to tolerate an applied osmotic stress (Tarczynski 1992).

Similarly, the efficacy of other metabolites in protecting either enzyme function (e.g. alanopine or propionic acid) or membrane integrity (e.g., alanopine) has been documented (Loomis 1989), and therefore expression of gene encoding the biosynthesis of these compounds can confer drought resistance in a manner similar to or complimentary to mannitol. Other examples of naturally occurring metabolites that are osmotically active and/or provide some direct protective effect during drought and/or desiccation include sugars and sugar derivatives such as fructose, erythritol (Coxson 1992), sorbitol, dulcitol (Karsten 1992), glucosylglycerol (Reed 1984; Erdmann 1992), sucrose, stachyose (Koster & Leopold 1988; Blackman 1992), ononitol and pinitol (Vernon & Bohnert 1992), and raffinose (Bernal-Lugo & Leopold 1992). Other osmotically active solutes which are not sugars include, but are not limited to, proline and glycine-betaine (Wyn-Jones and Storey, 1981). Continued canopy growth and increased reproductive fitness during times of stress can be augmented by introduction and expression of genes such as those controlling the osmotically active compounds discussed above and other such compounds, as represented in one exemplary embodiment by the enzyme myoinositol 0-methyltransferase.

It is contemplated that the expression of specific proteins may also increase drought tolerance. Three classes of Late Embryogenic Proteins have been assigned based on structural similarities (see Dure 1989). All three classes of these proteins have been demonstrated in maturing (i.e., desiccating) seeds. Within these 3 types of proteins, the Type-II (dehydrin-type) have generally been implicated in drought and/or desiccation tolerance in vegetative plant parts (e.g.. Mundy and Chua, 1988; Piatkowski 1990; Yamaguchi-Shinozaki 1992). Recently, expression of a Type-III LEA (HVA-1) in tobacco was found to influence plant height, maturity and drought tolerance (Fitzpatrick, 1993). Expression of structural genes from all three groups may therefore confer drought tolerance. Other types of proteins induced during water stress include thiol proteases, aldolases and transmembrane transporters (Guerrero 1990), which may confer various protective and/or repair-type functions during drought stress. The expression of a gene that effects lipid biosynthesis and hence membrane composition can also be useful in conferring drought resistance on the plant.

Many genes that improve drought resistance have complementary modes of action. Thus, combinations of these genes might have additive and/or synergistic effects in improving drought resistance in maize. Many of these genes also improve freezing tolerance (or resistance); the physical stresses incurred during freezing and drought are similar in nature and may be mitigated in similar fashion. Benefit may be conferred via constitutive or tissue-specific expression of these genes, but the preferred means of expressing these novel genes may be through the use of a turgor-induced promoter (such as the promoters for the turgor-induced genes described in Guerrero et al. 1990 and Shagan 1993). Spatial and temporal expression patterns of these genes may enable maize to better withstand stress.

Expression of genes that are involved with specific morphological traits that allow for increased water extractions from drying soil would be of benefit. For example, introduction and expression of genes that alter root characteristics may enhance water uptake. Expression of genes that enhance reproductive fitness during times of stress would be of significant value. For example, expression of DNAs that improve the synchrony of pollen shed and receptiveness of the female flower parts, i.e., silks, would be of benefit. In addition, expression of genes that minimize kernel abortion during times of stress would increase the amount of grain to be harvested and hence be of value. Regulation of cytokinin levels in monocots, such as maize, by introduction and expression of an isopentenyl transferase gene with appropriate regulatory sequences can improve monocot stress resistance and yield (Gan 1995).

Given the overall role of water in determining yield, it is contemplated that enabling plants to utilize water more efficiently, through the introduction and expression of novel genes, will improve overall performance even when soil water availability is not limiting. By introducing genes that improve the ability of plants to maximize water usage across a full range of stresses relating to water availability, yield stability or consistency of yield performance may be realized.

Improved protection of the plant to abiotic stress factors such as drought, heat or chill, can also be achieved - for example - by overexpressing antifreeze polypeptides from Myoxocephalus Scorpius (WO 00/00512), Myoxocephalus octodecemspinosus, the

Arabidopsis thaliana transcription activator CBF1, glutamate dehydrogenases (WO 97/12983, WO 98/11240), calcium-dependent protein kinase genes (WO 98/26045), calcineurins (WO 99/05902), casein kinase from yeast (WO 02/052012), famesyltransferases (WO 99/06580; Pei ZM et al. (1998) Science 282:287-290), ferritin (Deak M et al. (1999) Nature Biotechnology 17:192-196), oxalate oxidase (WO 99/04013; Dunwell JM (1998) Biotechn Genet Eng Rev 15:1-32), DREB1A factor ("dehydration response element B 1A"; Kasuga M et al. (1999) Nature Biotech 17:276-286), genes of mannitol or trehalose synthesis such as trehalose-phosphate synthase or trehalose-phosphate phosphatase (WO 97/42326) or by inhibiting genes such as trehalase (WO 97/50561).

### 1.4 Disease Resistance

It is proposed that increased resistance to diseases may be realized through introduction of genes into plants period. It is possible to produce resistance to diseases caused, by viruses, bacteria, fungi, root pathogens, insects and nematodes. It is also contemplated that control of mycotoxin producing organisms may be realized through expression of introduced genes.

Resistance to viruses may be produced through expression of novel genes. For example, it has been demonstrated that expression of a viral coat protein in a transgenic plant can impart resistance to infection of the plant by that virus and perhaps other closely related viruses (Cuozzo 1988, Hemenway 1988, Abel 1986). It is contemplated that expression of antisense genes targeted at essential viral functions may impart resistance to said virus. For example, an antisense gene targeted at the gene responsible for replication of viral nucleic acid may inhibit said replication and lead to resistance to the virus. It is believed that interference with other viral functions through the use of antisense genes may also increase resistance to viruses. Further it is proposed that it may be possible to achieve resistance to viruses through other approaches, including, but not limited to the use of satellite viruses.

It is proposed that increased resistance to diseases caused by bacteria and fungi may be realized through introduction of novel genes. It is contemplated that genes encoding so-called "peptide antibiotics," pathogenesis related (PR) proteins, toxin resistance, and proteins affecting host-pathogen interactions such as morphological characteristics will be useful. Peptide antibiotics are polypeptide sequences which are inhibitory to growth of bacteria and other microorganisms. For example, the classes of peptides referred to as cecropins and magainins inhibit growth of many species of bacteria and fungi. It is proposed that expression of PR proteins in plants may be useful in conferring resistance to bacterial disease. These genes are induced following pathogen attack on a host plant and have been divided into at least five classes of proteins (Bol 1990). Included amongst the PR proteins are beta-1,3-glucanases, chitinases, and osmotin and other proteins that are believed to function in plant resistance to disease organisms. Other genes have been identified that have antifungal properties, e.g., UDA (stinging nettle lectin) and hevein (Broakgert 1989; Barkai-Golan 1978). It is known that certain plant diseases are caused by the production of phytotoxins. Resistance to these diseases could be achieved through expression of a novel gene that encodes an enzyme capable of degrading or otherwise inactivating the phytotoxin. Expression novel genes that alter the interactions between the host plant and pathogen may be useful in reducing the ability the disease organism to invade the tissues of the host plant, e.g., an increase in the waxiness of the leaf cuticle or other morphological characteristics.

Plant parasitic nematodes are a cause of disease in many plants. It is proposed that it would be possible to make the plant resistant to these organisms through the expression of novel genes. It is anticipated that control of nematode infestations would be accomplished by altering the ability of the nematode to recognize or attach to a host plant and/or enabling the plant to produce nematicidal compounds, including but not limited to proteins.

Furthermore, a resistance to fungi, insects, nematodes and diseases, can be achieved by by targeted accumulation of certain metabolites or proteins. Such proteins include but are not limited to glucosinolates (defense against herbivores), chitinases or glucanases and other enzymes which destroy the cell wall of parasites, ribosome-inactivating proteins (RIPs) and other proteins of the plant resistance and stress reaction as are induced when plants are wounded or attacked by microbes, or chemically, by, for example, salicylic acid, jasmonic acid or ethylene, or lysozymes from nonplant sources such as, for example, T4-lysozyme or lysozyme from a variety of mammals, insecticidal proteins such as *Bacillus thuringiensis* endotoxin, a-amylase inhibitor or protease inhibitors (cowpea trypsin inhibitor), lectins such as wheatgerm agglutinin, RNAses or ribozymes. Further examples are nucleic acids which encode the Trichoderma harzianum chit42 endochitinase (GenBank Acc. No.: S78423) or the N-hydroxylating, multi-functional cytochrome P-450 (CYP79) protein from Sorghum bicolor (GenBank Acc. No.: U32624), or functional equivalents of these. The accumulation of glucosinolates as protection from pests (Rask L et al. (2000) Plant Mol Biol 42:93-113; Menard R et al. (1999) Phytochemistry 52:29-35), the expression of *Bacillus thuringiensis* endotoxins (Vaeck et al. (1987) Nature 328:33-37) or the protection against attack by fungi, by expression of chitinases, for example from beans (Broglie et al. (1991) Science 254:1194-1197), is advantageous. Resistance to pests such as, for example, the rice pest *Nilaparvata lugens* in rice plants can be achieved by expressing the snowdrop *(Galanthus nivalis*) lectin agglutinin (Rao et al. (1998) Plant J 15(4):469-77).The expression of synthetic crylA(b) and crylA(c) genes, which encode lepidoptera-specific *Bacillus thuringiensis* D-endotoxins can bring about a resistance to insect pests in various plants (Goyal RK et al. (2000) Crop Protection 19(5):307-312). Further target genes which are suitable for pathogen defense comprise "polygalacturonase-inhibiting protein" (PGIP), thaumatine, invertase and antimicrobial peptides such as lactoferrin (Lee TJ et al. (2002) J Amer Soc Horticult Sci 127(2):158-164).

### 1.5 Plant Agronomic Characteristics

Two of the factors determining where plants can be grown are the average daily temperature during the growing season and the length of time between frosts. Within the areas where it is possible to grow a particular plant, there are varying limitations on the maximal time it is allowed to grow to maturity and be harvested. The plant to be grown in a particular area is selected for its ability to mature and dry down to harvestable moisture content within the required period of time with maximum possible yield. Therefore, plant of varying maturities are developed for different growing locations. Apart from the need to dry down sufficiently to permit harvest is the desirability of having maximal drying take place in the field to minimize the amount of energy required for additional drying post-harvest. Also the more readily the grain can dry down, the more time there is available for growth and kernel fill. Genes that influence maturity and/or dry down can be identified and introduced into plant lines using transformation techniques to create new varieties adapted to different growing locations or the same growing location but having improved yield to moisture ratio at harvest. Expression of genes that are involved in regulation of plant development may be especially useful, e.g., the liguleless and rough sheath genes that have been identified in plants.

Genes may be introduced into plants that would improve standability and other plant growth characteristics. For example, expression of novel genes which confer stronger stalks, improved root systems, or prevent or reduce ear droppage would be of great value to the corn farmer. Introduction and expression of genes that increase the total amount of photoassimilate available by, for example, increasing light distribution and/or interception would be advantageous. In addition the expression of genes that increase the efficiency of photosynthesis and/or the leaf canopy would further increase gains in productivity. Such approaches would allow for increased plant populations in the field.

Delay of late season vegetative senescence would increase the flow of assimilate into the grain and thus increase yield. Overexpression of genes within plants that are associated with "stay green" or the expression of any gene that delays senescence would be advantageous. For example, a non-yellowing mutant has been identified in Festuca pratensis (Davies 1990). Expression of this gene as well as others may prevent premature breakdown of chlorophyll and thus maintain canopy function.

### 1.6 Nutrient Utilization

The ability to utilize available nutrients and minerals may be a limiting factor in growth of many plants. It is proposed that it would be possible to alter nutrient uptake, tolerate pH extremes, mobilization through the plant, storage pools, and availability for metabolic activities by the introduction of novel genes. These modifications would allow a plant to more efficiently utilize available nutrients. It is contemplated that an increase in the activity of, for example, an enzyme that is normally present in the plant and involved in nutrient utilization would increase the availability of a nutrient. An example of such an enzyme would be phytase. It is also contemplated that expression of a novel gene may make a nutrient source available that was previously not accessible, e.g., an enzyme that releases a component of nutrient value from a more complex molecule, perhaps a macromolecule.

### 1.7. Non-Protein-Expressing Sequences

#### 1.7.1 RNA-Expressing

DNA may be introduced into plants for the purpose of expressing RNA transcripts that function to affect plant phenotype yet are not translated into protein. Two examples are antisense RNA and RNA with ribozyme activity. Both may serve possible functions in reducing or eliminating expression of native or introduced plant genes.

Genes may be constructed or isolated, which when transcribed, produce antisense RNA or double-stranded RNA that is complementary to all or part(s) of a targeted messenger RNA(s). The antisense RNA reduces production of the polypeptide product of the messenger RNA. The polypeptide product may be any protein encoded by the plant genome. The aforementioned genes will be referred to as antisense genes. An antisense gene may thus be introduced into a plant by transformation methods to produce a novel transgenic plant with reduced expression of a selected protein of interest. For example, the protein may be an enzyme that catalyzes a reaction in the plant. Reduction of the enzyme activity may reduce or eliminate products of the reaction which include any enzymatically synthesized compound in the plant such as fatty acids, amino acids, carbohydrates, nucleic acids and the like. Alternatively, the protein may be a storage protein, such as a zein, or a structural protein, the decreased expression of which may lead to changes in seed amino acid composition or plant morphological changes respectively. The possibilities cited above are provided only by way of example and do not represent the full range of applications.

Expression of antisense-RNA or double-stranded RNA by one of the expression cassettes of the invention is especially preferred. Also expression of sense RNA can be employed for gene silencing (co-suppression). This RNA is preferably a non-translatable RNA. Gene regulation by double-stranded RNA ("double-stranded RNA interference"; dsRNAi) is well known in the arte and described for various organism including plants (e.g., Matzke 2000; Fire A et al 1998; WO 99/32619; WO 99/53050; WO 00/68374; WO 00/44914; WO 00/44895; WO 00/49035; WO 00/63364).

Genes may also be constructed or isolated, which when transcribed produce RNA enzymes, or ribozymes, which can act as endoribonucleases and catalyze the cleavage of RNA molecules with selected sequences. The cleavage of selected messenger RNA's can result in the reduced production of their encoded polypeptide products. These genes may be used to prepare novel transgenic plants which possess them. The transgenic plants may possess reduced levels of polypeptides including but not limited to the polypeptides cited above that may be affected by antisense RNA.

It is also possible that genes may be introduced to produce novel transgenic plants which have reduced expression of a native gene product by a mechanism of cosuppression. It has been demonstrated in tobacco, tomato, and petunia (Goring 1991; Smith 1990; Napoli 1990; van der Krol 1990) that expression of the sense transcript of a native gene will reduce or eliminate expression of the native gene in a manner similar to that observed for antisense genes. The introduced gene may encode all or part of the targeted native protein but its translation may not be required for reduction of levels of that native protein.

#### 1.7.2 Non-RNA-Expressing

For example, DNA elements including those of transposable elements such as Ds, Ac, or Mu, may be, inserted into a gene and cause mutations. These DNA elements may be inserted in order to inactivate (or activate) a gene and thereby "tag" a particular trait. In this instance the transposable element does not cause instability of the tagged mutation, because the utility of the element does not depend on its ability to move in the genome. Once a desired trait is tagged, the introduced DNA sequence may be used to clone the corresponding gene, e.g., using the introduced DNA sequence as a PCR primer together with PCR gene cloning techniques (Shapiro, 1983; Dellaporta 1988). Once identified, the entire gene(s) for the particular trait, including control or regulatory regions where desired may be isolated, cloned and manipulated as desired. The utility of DNA elements introduced into an organism for purposed of gene tagging is independent of the DNA sequence and does not depend on any biological activity of the DNA sequence, i.e., transcription into RNA or translation into protein. The sole function of the DNA element is to disrupt the DNA sequence of a gene.

It is contemplated that unexpressed DNA sequences, including novel synthetic sequences could be introduced into cells as proprietary "labels" of those cells and plants and seeds thereof. It would not be necessary for a label DNA element to disrupt the function of a gene endogenous to the host organism, as the sole function of this DNA would be to identify the origin of the organism. For example, one could introduce a unique DNA sequence into a plant and this DNA element would identify all cells, plants, and progeny of these cells as having arisen from that labeled source. It is proposed that inclusion of label DNAs would enable one to distinguish proprietary germplasm or germplasm derived from such, from unlabelled germplasm.

Another possible element which may be introduced is a matrix attachment region element (MAR), such as the chicken lysozyme A element (Stief 1989), which can be positioned around an expressible gene of interest to effect an increase in overall expression of the gene and diminish position dependant effects upon incorporation into the plant genome (Stief 1989; Phi-Van 1990).

Further nucleotide sequences of interest that may be contemplated for use within the scope of the present invention in operable linkage with the promoter sequences according to the invention are isolated nucleic acid molecules, e.g., DNA or RNA, comprising a plant nucleotide sequence according to the invention comprising an open reading frame that is preferentially expressed in a specific tissue, i.e., mesophyll- and/or epidermis-, root, green tissue (leaf and stem), panicle-, or pollen, or is expressed constitutively.

**2. Marker Genes**

In order to improve the ability to identify transformants, one may desire to employ a selectable or screenable marker gene as, or in addition to, the expressible gene of interest. "Marker genes" are genes that impart a distinct phenotype to cells expressing the marker gene and thus allow such transformed cells to be distinguished from cells that do not have the marker. Such genes may encode either a selectable or screenable marker, depending on whether the marker confers a trait which one can 'select' for by chemical means, i.e., through the use of a selective agent (e.g., a herbicide, antibiotic, or the like), or whether it is simply a trait that one can identify through observation or testing, i.e., by 'screening' (e.g., the R-locus trait, the green fluorescent protein (GFP)). Of course, many examples of suitable marker genes are known to the art and can be employed in the practice of the invention.

Included within the terms selectable or screenable marker genes are also genes which encode a "secretable marker" whose secretion can be detected as a means of identifying or selecting for transformed cells. Examples include markers which encode a secretable antigen that can be identified by antibody interaction, or even secretable enzymes which can be detected by their catalytic activity. Secretable proteins fall into a number of classes, including small, diffusible proteins detectable, e.g., by ELISA; small active enzymes detectable in extracellular solution (e.g., alpha-amylase, beta-lactamase, phosphinothricin acetyltransferase); and proteins that are inserted or trapped in the cell wall (e.g., proteins that include a leader sequence such as that found in the expression unit of extensin or tobacco PR-S).

With regard to selectable secretable markers, the use of a gene that encodes a protein that becomes sequestered in the cell wall, and which protein includes a unique epitope is considered to be particularly advantageous. Such a secreted antigen marker would ideally employ an epitope sequence that would provide low background in plant tissue, a promoter-leader sequence that would impart efficient expression and targeting across the plasma membrane, and would produce protein that is bound in the cell wall and yet accessible to antibodies. A normally secreted wall protein modified to include a unique epitope would satisfy all such requirements.

One example of a protein suitable for modification in this manner is extensin, or hydroxyproline rich glycoprotein (HPRG). For example, the maize HPRG (Steifel 1990) molecule is well characterized in terms of molecular biology, expression and protein structure. However, any one of a variety of ultilane and/or glycine-rich wall proteins (Keller 1989) could be modified by the addition of an antigenic site to create a screenable marker.

One exemplary embodiment of a secretable screenable marker concerns the use of a maize sequence encoding the wall protein HPRG, modified to include a 15 residue epitope from the pro-region of murine interleukin, however, virtually any detectable epitope may be employed in such embodiments, as selected from the extremely wide variety of antigen-antibody combinations known to those of skill in the art. The unique extracellular epitope can then be straightforwardly detected using antibody labeling in conjunction with chromogenic or fluorescent adjuncts.

Elements of the present disclosure may be exemplified in detail through the use of the bar and/or GUS genes, and also through the use of various other markers. Of course, in light of this disclosure, numerous other possible selectable and/or screenable marker genes will be apparent to those of skill in the art in addition to the one set forth herein below. Therefore, it will be understood that the following discussion is exemplary rather than exhaustive. In light of the techniques disclosed herein and the general recombinant techniques which are known in the art, the present invention renders possible the introduction of any gene, including marker genes, into a recipient cell to generate a transformed plant.

### 2.1 Selectable Markers

Various selectable markers are known in the art suitable for plant transformation. Such markers may include but are not limited to:

#### 2.1.1 Negative selection markers

Negative selection markers confer a resistance to a biocidal compound such as a metabolic inhibitor (e.g., 2-deoxyglucose-6-phosphate, WO 98/45456), antibiotics (e.g., kanamycin, G 418, bleomycin or hygromycin) or herbicides (e.g., phosphinothricin or glyphosate). Transformed plant material (e.g., cells, tissues or plantlets), which express marker genes, are capable of developing in the presence of concentrations of a corresponding selection compound (e.g., antibiotic or herbicide) which suppresses growth of an untransformed wild type tissue. Especially preferred negative selection markers are those which confer resistance to herbicides. Examples which may be mentioned are:

- Phosphinothricin acetyltransferases (PAT; also named Bialophos ® resistance; bar; de Block 1987; Vasil 1992, 1993; Weeks 1993; Becker 1994; Nehra 1994; Wan & Lemaux 1994; EP 0 333 033; US 4,975,374). Preferred are the bar gene from Streptomyces hygroscopicus or the pat gene from Streptomyces viridochromogenes. PAT inactivates the active ingredient in the herbicide bialaphos, phosphinothricin (PPT). PPT inhibits glutamine synthetase, (Murakami 1986; Twell 1989) causing rapid accumulation of ammonia and cell death..
- altered 5-enolpyruvylshikimate-3-phosphate synthase (EPSPS) conferring resistance to Glyphosate® (N-(phosphonomethyl)glycine) (Hinchee 1988; Shah 1986; Della-Cioppa 1987). Where a mutant EPSP synthase gene is employed, additional benefit may be realized through the incorporation of a suitable chloroplast transit peptide, CTP (EP-A1 0 218 571).
- Glyphosate® degrading enzymes (Glyphosate® oxidoreductase; gox),
- Dalapon® inactivating dehalogenases (deh)
- sulfonylurea- and/or imidazolinone-inactivating acetolactate synthases (*ahas* or ALS; for example mutated ahas/ALS variants with, for example, the S4, XI12, XA17, and/or Hra mutation (EP-A1 154 204)
- Bromoxynil® degrading nitrilases (bxn; Stalker 1988)
- Kanamycin- or. geneticin (G418) resistance genes (NPTII; NPT or neo; Potrykus 1985) coding e.g., for neomycin phosphotransferases (Fraley 1983; Nehra 1994)
- 2-Desoxyglucose-6-phosphate phosphatase ($DOG^R1$-Gene product; WO 98/45456; EP 0 807 836) conferring resistance against 2-desoxyglucose (Randez-Gil 1995).
- hygromycin phosphotransferase (HPT), which mediates resistance to hygromycin (Vanden Elzen 1985).
- altered dihydrofolate reductase (Eichholtz 1987) conferring resistance against methotrexat (Thillet 1988);
- mutated anthranilate synthase genes that confers resistance to 5-methyl tryptophan.

Additional negative selectable marker genes of bacterial origin that confer resistance to antibiotics include the aadA gene, which confers resistance to the antibiotic spectinomycin, gentamycin acetyl transferase, streptomycin phosphotransferase (SPT), aminoglycoside-3-adenyl transferase and the bleomycin resistance determinant (Hayford 1988; Jones 1987; Svab 1990; Hille 1986).

Especially preferred are negative selection markers that confer resistance against the toxic effects imposed by D-amino acids like e.g., D-alanine and D-serine (WO 03/060133; Erikson 2004). Especially preferred as negative selection marker in this contest are the daol gene (EC: 1.4. 3.3 : GenBank Acc.-No.: U60066) from the yeast *Rhodotorula gracilis* (*Rhodosporidium toruloides*) and the *E. coli* gene *dsd*A (D-serine dehydratase (D-serine deaminase) [EC: 4.3. 1.18; GenBank Acc.-No.: J01603).

Transformed plant material (e.g., cells, embryos, tissues or plantlets) which express such marker genes are capable of developing in the presence of concentrations of a corresponding selection compound (e.g., antibiotic or herbicide) which suppresses growth of an untransformed wild type tissue. The resulting plants can be bred and hybridized in the customary fashion. Two or more generations should be grown in order to ensure that the genomic integration is stable and hereditary. Corresponding methods are described (Jenes 1993; Potrykus 1991).

Furthermore, reporter genes can be employed to allow visual screening, which may or may not (depending on the type of reporter gene) require supplementation with a substrate as a selection compound.

Various time schemes can be employed for the various negative selection marker genes. In case of resistance genes (e.g., against herbicides or D-amino acids) selection is preferably applied throughout callus induction phase for about 4 weeks and beyond at least 4 weeks into regeneration. Such a selection scheme can be applied for all selection regimes. It is furthermore possible (although not explicitly preferred) to remain the selection also throughout the entire regeneration scheme including rooting.

For example, with the phosphinotricin resistance gene (bar) as the selective marker, phosphinotricin at a concentration of from about 1 to 50 mg/l may be included in the medium. For example, with the *dao1* gene as the selective marker, D-serine or D-alanine at a concentration of from about 3 to 100 mg/l may be included in the medium. Typical concentrations for selection are 20 to 40 mg/l. For example, with the mutated ahas genes as the selective marker, PURSUIT™ at a concentration of from about 3 to 100 mg/l may be included in the medium. Typical concentrations for selection are 20 to 40 mg/l.

### 2.1.2 Positive selection marker

Furthermore, positive selection marker can be employed. Genes like isopentenyltransferase from *Agrobacterium* tumefaciens (strain:PO22; Genbank Acc.-No.: AB025109) may - as a key enzyme of the cytokinin biosynthesis - facilitate regeneration of transformed plants (e.g., by selection on cytokinin-free medium). Corresponding selection methods are described (Ebinuma 2000a,b). Additional positive selection markers, which confer a growth advantage to a transformed plant in comparison with a non-transformed one, are described e.g., in EP-A 0 601 092. Growth stimulation selection markers may include (but shall not be limited to) β-Glucuronidase (in combination with e.g., a cytokinin glucuronide), mannose-6-phosphate isomerase (in combination with mannose), UDP-galactose-4-epimerase (in combination with e.g., galactose), wherein mannose-6-phosphate isomerase in combination with mannose is especially preferred.

### 2.1.3 Counter-selection marker

Counter-selection markers are especially suitable to select organisms with defined deleted sequences comprising said marker (Koprek 1999). Examples for counter- selection marker comprise thymidin kinases (TK), cytosine deaminases (Gleave 1999; Perera 1993; Stougaard 1993), cytochrom P450 proteins (Koprek 1999), haloalkan dehalogenases (Naested 1999), iaaH gene products (Sundaresan 1995), cytosine deaminase codA (Schlaman & Hooykaas 1997), tms2 gene products (Fedoroff & Smith 1993), or α-naphthalene acetamide (NAM; Depicker 1988). Counter selection markers may be useful in the construction of transposon tagging lines. For example, by marking an autonomous transposable element such as Ac, Master Mu, or En/Spn with a counter selection marker, one could select for transformants in which the autonomous element is not stably integrated into the genome. This would be desirable, for example, when transient expression of the autonomous element is desired to activate in trans the transposition of a defective transposable element, such as Ds, but stable integration of the autonomous element is not desired. The presence of the autonomous element may not be desired in order to stabilize the defective element, i.e., prevent it from further transposing. However, it is proposed that if stable integration of an autonomous transposable element is desired in a plant the presence of a negative selectable marker may make it possible to eliminate the autonomous element during the breeding process.

### 2.2. Screenable Markers

Screenable markers that may be employed include, but are not limited to, a beta-glucuronidase (GUS) or uidA gene which encodes an enzyme for which various chromogenic substrates are known; an R-locus gene, which encodes a product that regulates the production of anthocyanin pigments (red color) in plant tissues (Dellaporta 1988); a beta-lactamase gene (Sutcliffe 1978), which encodes an enzyme for which various chromogenic substrates are known (e.g., PADAC, a chromogenic cephalosporin); a xylE gene (Zukowsky 1983) which encodes a catechol dioxygenase that can convert chromogenic catechols; an α-amylase gene (Ikuta 1990); a tyrosinase gene (Katz 1983) which encodes an enzyme capable of oxidizing tyrosine to DOPA and dopaquinone which in turn condenses to form the easily detectable compound melanin; β-galactosidase gene, which encodes an enzyme for which there are chromogenic substrates; a luciferase (lux) gene (Ow 1986), which allows for bioluminescence detection; or even an aequorin gene (Prasher 1985), which may be employed in calcium-sensitive bioluminescence detection, or a green fluorescent protein gene (Niedz 1995).

Genes from the maize R gene complex are contemplated to be particularly useful as screenable markers. The R gene complex in maize encodes a protein that acts to regulate the production of anthocyanin pigments in most seed and plant tissue. A gene from the R gene complex was applied to maize transformation, because the expression of this gene in transformed cells does not harm the cells. Thus, an R gene introduced into such cells will cause the expression of a red pigment and, if stably incorporated, can be visually scored as a red sector. If a maize line carries dominant genes encoding the enzymatic intermediates in the anthocyanin biosynthetic pathway (C2, A1, A2, Bz1 and Bz2), but carries a recessive allele at the R locus, transformation of any cell from that line with R will result in red pigment formation. Exemplary lines include Wisconsin 22 which contains the rg-Stadler allele and TR112, a K55 derivative which is r-g, b, P1. Alternatively any genotype of maize can be utilized if the C1 and R alleles are introduced together.

It is further proposed that R gene regulatory regions may be employed in chimeric constructs in order to provide mechanisms for controlling the expression of chimeric genes. More diversity of phenotypic expression is known at the R locus than at any other locus (Coe 1988). It is contemplated that regulatory regions obtained from regions 5' to the structural R gene would be valuable in directing the expression of genes, e.g., insect resistance, drought resistance, herbicide tolerance or other protein coding regions. For the purposes of the present invention, it is believed that any of the various R gene family members may be successfully employed (e.g., P, S, Lc, etc.). However, the most preferred will generally be Sn (particularly Sn:bol3). Sn is a dominant member of the R gene complex and is functionally similar to the R and B loci in that Sn controls the tissue specific deposition of anthocyanin pigments in certain seedling and plant cells, therefore, its phenotype is similar to R.

A further screenable marker contemplated for use in the present invention is firefly luciferase, encoded by the lux gene. The presence of the lux gene in transformed cells may be detected using, for example, X-ray film, scintillation counting, fluorescent spectrophotometry, low-light video cameras, photon counting cameras or multiwell luminometry. It is also envisioned that this system may be developed for populational screening for bioluminescence, such as on tissue culture plates, or even for whole plant screening. Where use of a screenable marker gene such as lux or GFP is desired, benefit may be realized by creating a gene fusion between the screenable marker gene and a selectable marker gene, for example, a GFP-NPTII gene fusion. This could allow, for example, selection of transformed cells followed by screening of transgenic plants or seeds.

### 3. Exemplary DNA Molecules

The invention provides an isolated nucleic acid molecule, e.g., DNA or RNA, comprising a plant nucleotide sequence comprising an open reading frame that is preferentially expressed in a specific plant tissue, i.e., in seeds, roots, green tissue (leaf and stem), panicles or pollen, or is expressed constitutively, or a promoter thereof.

These promoters include, but are not limited to, constitutive, inducible, temporally regulated, developmentally regulated, spatially-regulated, chemically regulated, stress-responsive, tissue-specific, viral and synthetic promoters. Promoter sequences are known to be strong or weak. A strong promoter provides for a high level of gene expression, whereas a weak promoter provides for a very low level of gene expression.

An inducible promoter is a promoter that provides for the turning on and off of gene expression in response to an exogenously added agent, or to an environmental or developmental stimulus. A bacterial promoter such as the $P_{tac}$ promoter can be induced to varying levels of gene expression depending on the level of isothiopropylgalactoside added to the transformed bacterial cells. An isolated promoter sequence that is a strong promoter for heterologous nucleic acid is advantageous because it provides for a sufficient level of gene expression to allow for easy detection and selection of transformed cells and provides for a high level of gene expression when desired.

Within a plant promoter region there are several domains that are necessary for full function of the promoter. The first of these domains lies immediately upstream of the structural gene and forms the "core promoter region" containing consensus sequences, normally 70 base pairs immediately upstream of the gene. The core promoter region contains the characteristic CAAT and TATA boxes plus surrounding sequences, and represents a transcription initiation sequence that defines the transcription start point for the structural gene.

The presence of the core promoter region defines a sequence as being a promoter: if the region is absent, the promoter is non-functional. Furthermore, the core promoter region is insufficient to provide full promoter activity. A series of regulatory sequences upstream of the core constitute the remainder of the promoter. The regulatory sequences determine expression level, the spatial and temporal pattern of expression and, for an important subset of promoters, expression under inductive conditions (regulation by external factors such as light, temperature, chemicals, hormones).

Regulated expression of the chimeric transacting viral replication protein can be further regulated by other genetic strategies. For example, Cre-mediated gene activation as described by Odell et al. 1990. Thus, a DNA fragment containing 3' regulatory sequence bound by lox sites between the promoter and the replication protein coding sequence that blocks the expression of a chimeric replication gene from the promoter can be removed by Cre-mediated excision and result in the expression of the transacting replication gene. In this case, the chimeric Cre gene, the chimeric transacting replication gene, or both can be under the control of tissue- and developmental-specific or inducible promoters. An alternate genetic strategy is the use of tRNA suppressor gene. For example, the regulated expression of a tRNA suppressor gene can conditionally control expression of a trans-acting replication protein coding sequence containing an appropriate termination codon as described by Ulmasov et al. 1997. Again, either the chimeric tRNA suppressor gene, the chimeric transacting replication gene, or both can be under the control of tissue- and developmental-specific or inducible promoters.

Frequently it is desirable to have continuous or inducible expression of a DNA sequence throughout the cells of an organism in a tissue-independent manner. For example, increased resistance of a plant t6 infection by soil- and airbome-pathogens might be accomplished by genetic manipulation of the plant's genome to comprise a continuous promoter operably linked to a heterologous pathogen-resistance gene such that pathogen-resistance proteins are continuously expressed throughout the plant's tissues.

Alternatively, it might be desirable to inhibit expression of a native DNA sequence within the seeds of a plant to achieve a desired phenotype. In this case, such inhibition might be accomplished with transformation of the plant to comprise a promoter operably linked to an antisense nucleotide sequence, such that mesophyll- and/or epidermis-preferential or mesophyll- and/or epidermis-specific expression of the antisense sequence produces an RNA transcript that interferes with translation of the mRNA of the native DNA sequence.

To define a minimal promoter region, a DNA segment representing the promoter region is removed from the 5' region of the gene of interest and operably linked to the coding sequence of a marker (reporter) gene by recombinant DNA techniques well known to the art. The reporter gene is operably linked downstream of the promoter, so that transcripts initiating at the promoter proceed through the reporter gene. Reporter genes generally encode proteins which are easily measured, including, but not limited to, chloramphenicol acetyl transferase (CAT), beta-glucuronidase (GUS), green fluorescent protein (GFP), beta-galactosidase (beta-GAL), and luciferase.

The construct containing the reporter gene under the control of the promoter is then introduced into an appropriate cell type by transfection techniques well known to the art. To assay for the reporter protein, cell lysates are prepared and appropriate assays, which are well known in the art, for the reporter protein are performed. For example, if CAT were the reporter gene of choice, the lysates from cells transfected with constructs containing CAT under the control of a promoter under study are mixed with isotopically labeled chloramphenicol and acetyl-coenzyme A (acetyl-CoA). The CAT enzyme transfers the acetyl group from acetyl-CoA to the 2- or 3-position of chloramphenicol. The reaction is monitored by thin-layer chromatography, which separates acetylated chloramphenicol from unreacted material. The reaction products are then visualized by autoradiography.

The level of enzyme activity corresponds to the amount of enzyme that was made, which in turn reveals the level of expression from the promoter of interest. This level of expression can be compared to other promoters to determine the relative strength of the promoter under study. In order to be sure that the level of expression is determined by the promoter, rather than by the stability of the mRNA, the level of the reporter mRNA can be measured directly, such as by Northern blot analysis.

Once activity is detected, mutational and/or deletional analyses may be employed to determine the minimal region and/or sequences required to initiate transcription. Thus, sequences can be deleted at the 5' end of the promoter region and/or at the 3' end of the promoter region, and nucleotide substitutions introduced. These constructs are then introduced to cells and their activity determined.

In one embodiment, the promoter may be a gamma zein promoter, an oleosin ole16 promoter, a globulins promoter, an actin I promoter, an actin cl promoter, a sucrose synthetase promoter, an INOPS promoter, an EXM5 promoter, a globulin2 promoter, a b-32, ADPG-pyrophosphorylase promoter, an LtpI promoter, an Ltp2 promoter, an oleosin ole17 promoter, an oleosin ole18 promoter, an actin 2 promoter, a pollen-specific protein promoter, a pollen-specific pectate lyase promoter, an anther-specific protein promoter, an anther-specific gene RTS2 promoter, a pollen-specific gene promoter, a tapetum-specific gene promoter, tapeturn-specific gene RAB24 promoter, a anthranilate synthase alpha subunit promoter, an alpha zein promoter, an anthranilate synthase beta subunit promoter, a dihydrodipicolinate synthase promoter, a Thil promoter, an alcohol dehydrogenase promoter, a cab binding protein promoter, an H3C4 promoter, a RUBISCO SS starch branching enzyme promoter, an ACCase promoter, an actin3 promoter, an actin7 promoter, a regulatory protein GF14-12 promoter, a ribosomal protein L9 promoter, a cellulose biosynthetic enzyme promoter, an S-adenosyl-L-homocysteine hydrolase promoter, a superoxide dismutase promoter, a C-kinase receptor promoter, a phosphoglycerate mutase promoter, a root-specific RCc3 mRNA promoter, a glucose-6 phosphate isomerase promoter, a pyrophosphate-fructose 6-phosphatelphosphotransferase promoter, an ubiquitin promoter, a beta-ketoacyl-ACP synthase promoter, a 33 kDa photosystem 11 promoter, an oxygen evolving protein promoter, a 69 kDa vacuolar ATPase subunit promoter, a metallothionein-like protein promoter, a glyceraldehyde-3-phosphate dehydrogenase promoter, an ABA- and ripening-inducible-like protein promoter, a phenylalanine ammonia lyase promoter, an adenosine triphosphatase S-adenosyl-L-homocysteine hydrolase promoter, an a-tubulin promoter, a cab promoter, a PEPCase promoter, an R gene promoter, a lectin promoter, a light harvesting complex promoter, a heat shock protein promoter, a chalcone synthase promoter, a zein promoter, a globulin-1 promoter, an ABA promoter, an auxin-binding protein promoter, a UDP glucose flavonoid glycosyl-transferase gene promoter, an NTI promoter, an actin promoter, an opaque 2 promoter, a b70 promoter, an oleosin promoter, a CaMV 35S promoter, a CaMV 34S promoter, a CaMV 19S promoter, a histone promoter, a turgor-inducible promoter, a pea small subunit RuBP carboxylase promoter, a Ti plasmid mannopine synthase promoter, Ti plasmid nopaline synthase promoter, a petunia chalcone isomerase promoter, a bean glycine rich protein I promoter, a CaMV 35S transcript promoter, a potato patatin promoter, or a S-E9 small subunit RuBP carboxylase promoter.

**4. Transformed (Transgenic) Plants of the Invention and Methods of Preparation**

Plant species may be transformed with the DNA construct of the present invention by the DNA-mediated transformation of plant cell protoplasts and subsequent regeneration of the plant from the transformed protoplasts in accordance with procedures well known in the art.

Any plant tissue capable of subsequent clonal propagation, whether by organogenesis or embryogenesis, may be transformed with a vector of the present invention. The term "organogenesis," as used herein, means a process by which shoots and roots are developed sequentially from meristematic centers; the term "embryogenesis," as used herein, means a process by which shoots and roots develop together in a concerted fashion (not sequentially), whether from somatic cells or gametes. The particular tissue chosen will vary depending on the clonal propagation systems available for, and best suited to, the particular species being transformed. Exemplary tissue targets include leaf disks, pollen, embryos, cotyledons, hypocotyls, megagametophytes, callus tissue, existing meristematic tissue (e.g., apical meristems, axillary buds, and root meristems), and induced meristem tissue (e.g., cotyledon meristem and ultilane meristem).

Plants of the present invention may take a variety of forms. The plants may be chimeras of transformed cells and non-transformed cells; the plants may be clonal transformants (e.g., all cells transformed to contain the expression cassette); the plants may comprise grafts of transformed and untransformed tissues (e.g., a transformed root stock grafted to an untransformed scion in citrus species). The transformed plants may be propagated by a variety of means, such as by clonal propagation or classical breeding techniques. For example, first generation (or T1) transformed plants may be selfed to give homozygous second generation (or T2) transformed plants, and the T2 plants further propagated through classical breeding techniques. A dominant selectable marker (such as npt II) can be associated with the expression cassette to assist in breeding.

Thus, the present invention provides a transformed (transgenic) plant cell, *in planta* or *ex planta*, including a transformed plastid or other organelle, e.g., nucleus, mitochondria or chloroplast. The present invention may be used for transformation of any plant species, including, but not limited to, cells from the plant species specified above in the DEFINITION section. Preferably, transgenic plants of the present invention are crop plants and in particular cereals (for example, com, alfalfa, sunflower, rice, Brassica, canola, soybean, barley, soybean, sugarbeet, cotton, safflower, peanut, sorghum, wheat, millet, tobacco, etc.), and even more preferably corn, rice and soybean. Other embodiments of the invention are related to cells, cell cultures, tissues, parts (such as plants organs, leaves, roots, etc.) and propagation material (such as seeds) of such plants.

The transgenic expression cassette of the invention may not only be comprised in plants or plant cells but may advantageously also be containing in other organisms such for example bacteria. Thus, another embodiment of the invention relates to transgenic cells or non-human, transgenic organisms comprising an expression cassette of the invention. Preferred are prokaryotic and eukaryotic organism. Both microorganism and higher organisms are comprised. Preferred microorganism are bacteria, yeast, algae, and fungi. Preferred bacteria are those of the genus *Escherichia, Erwinia, Agrobacterium, Flavobacterium, Alcaligenes, Pseudomonas, Bacillus or Cyanobacterim* such as - for example - *Synechocystis* and other bacteria described in Brock Biology of Microorganisms Eighth Edition (pages A-8, A-9, A10 and A11).

Especially preferred are microorganisms capable to infect plants and to transfer DNA into their genome, especially bacteria of the genus Agrobacterium, preferably Agrobacterium tumefaciens and rhizogenes. Preferred yeasts are Candida, Saccharomyces, Hansenula and Pichia. Preferred Fungi are Aspergillus, Trichoderma, Ashbya, Neurospora, Fusarium, and Beauveria. Most preferred are plant organisms as defined above.

Transformation of plants can be undertaken with a single DNA molecule or multiple DNA molecules (i.e., co-transformation), and both these techniques are suitable for use with the expression cassettes of the present invention. Numerous transformation vectors are available for plant transformation, and the expression cassettes of this invention can be used in conjunction with any such vectors. The selection of vector will depend upon the preferred transformation technique and the target species for transformation.

A variety of techniques are available and known to those skilled in the art for introduction of constructs into a plant cell host. These techniques generally include transformation with DNA employing *A. tumefaciens* or A. rhizogenes as the transforming agent, liposomes, PEG precipitation, electroporation, DNA injection, direct DNA uptake, microprojectile bombardment, particle acceleration, and the like (See, for example, EP 295959 and EP 138341) (see below). However, cells other than plant cells may be transformed with the expression cassettes of the invention. The general descriptions of plant expression vectors and reporter genes, and Agrobacterium and Agrobacterium-mediated gene transfer, can be found in Gruber et al. (1993).

Expression vectors containing genomic or synthetic fragments can be introduced into protoplasts or into intact tissues or isolated cells. Preferably expression vectors are introduced into intact tissue. General methods of culturing plant tissues are provided for example by Maki et al., (1993); and by Phillips et al. (1988). Preferably, expression vectors are introduced into maize or other plant tissues using a direct gene transfer method such as microprojectile-mediated delivery, DNA injection, electroporation and the like. More preferably expression vectors are introduced into plant tissues using the microprojectile media delivery with the biolistic device. See, for example, Tomes et al. (1995). The vectors of the invention can not only be used for expression of structural genes but may also be used in exon-trap cloning, or promoter trap procedures to detect differential gene expression in varieties of tissues (Lindsey 1993; Auch & Reth 1990).

It is particularly preferred to use the binary type vectors of Ti and Ri plasmids of Agrobacterium spp. Ti-derived vectors transform a wide variety of higher plants, including monocotyledonous and dicotyledonous plants, such as soybean, cotton, rape, tobacco, and rice (Pacciotti 1985: Byme 1987; Sukhapinda 1987; Lorz 1985; Potrykus, 1985; Park 1985: Hiei 1994). The use of T-DNA to transform plant cells has received extensive study and is amply described (EP 120516; Hoekema, 1985; Knauf, 1983; and An 1985). For introduction into plants, the chimeric genes of the invention can be inserted into binary vectors as described in the examples.

Other transformation methods are available to those skilled in the art, such as direct uptake of foreign DNA constructs (see EP 295959), techniques of electroporation (Fromm 1986) or high velocity ballistic bombardment with metal particles coated with the nucleic acid constructs (Kline 1987, and US 4,945,050). Once transformed, the cells can be regenerated by those skilled in the art. Of particular relevance are the recently described methods to transform foreign genes into commercially important crops, such as rapeseed (De Block 1989), sunflower (Everett 1987), soybean (McCabe 1988; Hinchee 1988; Chee 1989; Christou 1989; EP 301749), rice (Hiei 1994), and corn (Gordon-Kamm 1990; Fromm 1990).

Those skilled in the art will appreciate that the choice of method might depend on the type of plant, i.e., monocotyledonous or dicotyledonous, targeted for transformation. Suitable methods of transforming plant cells include, but are not limited to, microinjection (Crossway 1986), electroporation (Riggs 1986), Agrobacterium-mediated transformation (Hinchee 1988), direct gene transfer (Paszkowski 1984), and ballistic particle acceleration using devices available from Agracetus, Inc., Madison, Wis. And BioRad, Hercules, Calif. (see, for example, US 4,945,050; and McCabe 1988). Also see, Weissinger 1988; Sanford 1987 (onion); Christou 1988 (soybean); McCabe 1988 (soybean); Datta 1990 (rice); Klein 1988 (maize); Klein 1988 (maize); Klein 1988 (maize); Fromm 1990 (maize); and Gordon-Kamm 1990 (maize); Svab 1990 (tobacco chloroplast); Koziel 1993 (maize); Shimamoto 1989 (rice); Christou 1991 (rice); European Patent Application EP 0 332 581 (orchardgrass and other Pooideae); Vasil 1993 (wheat); Weeks 1993 (wheat).

In another embodiment, a nucleotide sequence of the present invention is directly transformed into the plastid genome. Plastid transformation technology is extensively described in US 5,451,513, 5,545,817, and 5,545,818, in PCT application no. WO 95/16783, and in McBnde et al., 1994. The basic technique for chloroplast transformation involves introducing regions of cloned plastid DNA flanking a selectable marker together with the gene of interest into a suitable target tissue, e.g., using biolistics or protoplast transformation (e.g., calcium chloride or PEG mediated transformation). The 1 to 1.5 kb flanking regions, termed targeting sequences, facilitate orthologous recombination with the plastid genome and thus allow the replacement or modification of specific regions of the plastome. Initially, point mutations in the chloroplast 16S rRNA and rps12 genes conferring resistance to spectinomycin and/or streptomycin are utilized as selectable markers for transformation (Svab 1990; Staub 1992). This resulted in stable homoplasmic transformants at a frequency of approximately one per 100 bombardments of target leaves. The presence of cloning sites between these markers allowed creation of a plastid targeting vector for introduction of foreign genes (Staub 1993). Substantial increases in transformation frequency are obtained by replacement of the recessive rRNA or r-protein antibiotic resistance genes with a dominant selectable marker, the bacterial aadA gene encoding the spectinomycin-detoxifying enzyme aminoglycoside-3N-adenyltransferase (Svab 1993). Other selectable markers useful for plastid transformation are known in the art and encompassed within the scope of the invention. Typically, approximately 15-20 cell division cycles following transformation are required to reach a homoplastidic state. Plastid expression, in which genes are inserted by orthologous recombination into all of the several thousand copies of the circular plastid genome present in each plant cell, takes advantage of the enormous copy number advantage over nuclear-expressed genes to permit expression levels that can readily exceed 10% of the total soluble plant protein. In a preferred embodiment, a nucleotide sequence of the present invention is inserted into a plastid targeting vector and transformed into the plastid genome of a desired plant host. Plants homoplastic for plastid genomes containing a nucleotide sequence of the present invention are obtained, and are preferentially capable of high expression of the nucleotide sequence.

*Agrobacterium tumefaciens* cells containing a vector comprising an expression cassette of the present invention, wherein the vector comprises a Ti plasmid, are useful in methods of making transformed plants. Plant cells are infected with an *Agrobacterium tumefaciens* as described above to produce a transformed plant cell, and then a plant is regenerated from the transformed plant cell. Numerous Agrobacterium vector systems useful in carrying out the present invention are known.

Various *Agrobacterium* strains can be employed, preferably disarmed *Agrobacterium tumefaciens* or *rhizogenes* strains. In a preferred embodiment, *Agrobacterium* strains for use in the practice of the invention include octopine strains, e.g., LBA4404 or agropine strains, e.g., EHA101 or EHA105. Suitable strains of *A. tumefaciens* for DNA transfer are for example EHA101[pEHA101] (Hood 1986), EHA105[pEHA105] (Li 1992), LBA4404[pAL4404] (Hoekema 1983), C58C1[pMP90] (Koncz & Schell 1986), and C58C1[pGV2260] (Deblaere 1985). Other suitable strains are *Agrobacterium* tumefaciens C58, a nopaline strain. Other suitable strains are *A. tumefaciens* C58C1 (Van Larebeke 1974), A136 (Watson 1975) or LBA4011 (Klapwijk 1980). In another preferred embodiment the soil-borne bacterium is a disarmed variant of *Agrobacterium rhizogenes* strain K599 (NCPPB 2659). Preferably, these strains are comprising a disarmed plasmid variant of a Ti- or Ri-plasmid providing the functions required for T-DNA transfer into plant cells (e.g., the vir genes). In a preferred embodiment, the *Agrobacterium* strain used to transform the plant tissue pre-cultured with the plant phenolic compound contains a L,L-succinamopine type Ti-plasmid, preferably disarmed, such as pEHA101. In another preferred embodiment, the *Agrobacterium* strain used to transform the plant tissue pre-cultured with the plant phenolic compound contains an octopine-type Ti-plasmid, preferably disarmed, such as pAL4404. Generally, when using octopine-type Ti-plasmids or helper plasmids, it is preferred that the virF gene be deleted or inactivated (Jarschow 1991).

The method of the invention can also be used in combination with particular *Agrobacterium* strains, to further increase the transformation efficiency, such as *Agrobacterium* strains wherein the vir gene expression and/or induction thereof is altered due to the presence of mutant or chimeric virA or virG genes (e.g. Hansen 1994; Chen and Winans 1991; Scheeren-Groot, 1994). Preferred are further combinations of *Agrobacterium* tumefaciens strain LBA4404 (Hiei 1994) with super-virulent plasmids. These are preferably pTOK246-based vectors (Ishida 1996).

A binary vector or any other vector can be modified by common DNA recombination techniques, multiplied in E. coli, and introduced into *Agrobacterium* by e.g., electroporation or other transformation techniques (Mozo & Hooykaas 1991).

*Agrobacterium* is grown and used in a manner similar to that described in Ishida (1996). The vector comprising *Agrobacterium* strain may, for example, be grown for 3 days on YP medium (5 g/l yeast extract, 10 g/l peptone, 5 g/l NaCl, 15 g/l agar, pH 6.8) supplemented with the appropriate antibiotic (e.g., 50 mg/l spectinomycin). Bacteria are collected with a loop from the solid medium and resuspended. In a preferred embodiment of the invention, *Agrobacterium* cultures are started by use of aliquots frozen at - 80°C.

The transformation of the target tissue (e.g., an immature embryo) by the *Agrobacterium* may be carried out by merely contacting the target tissue with the *Agrobacterium*. The concentration of *Agrobacterium* used for infection and co-cultivation may need to be varied. For example, a cell suspension of the *Agrobacterium* having a population density of approximately from $10^5$ - 10", preferably $10^6$ to $10^{10}$, more preferably about $10^8$ cells or cfu / ml is prepared and the target tissue is immersed in this suspension for about 3 to 10 minutes. The resulting target tissue is then cultured on a solid medium for several days together with the *Agrobacterium*.

Preferably, the bacterium is employed in concentration of $10^6$ to $10^{10}$ cfu/ml. In a preferred embodiment for the co-cultivation step about 1 to 10 μl of a suspension of the soil-bome bacterium (e.g., *Agrobacteria*) in the co-cultivation medium are directly applied to each target tissue explant and air-dried. This is saving labor and time and is reducing unintended Agrobacterium-mediated damage by excess Agrobacterium usage.

For *Agrobacterium* treatment, the bacteria are resuspended in a plant compatible co-cultivation medium. Supplementation of the co-culture medium with antioxidants (e.g., silver nitrate), phenol-absorbing compounds (like polyvinylpyrrolidone, Perl 1996) or thiol compounds (e.g., dithiothreitol, L-cysteine, Olhoft 2001) which can decrease tissue necrosis due to plant defence responses (like phenolic oxidation) may further improve the efficiency of *Agrobacterium*-mediated transformation. In another preferred embodiment, the co-cultivation medium of comprises least one thiol compound, preferably selected from the group consisting of sodium thiolsulfate, dithiotrietol (DTT) and cysteine. Preferably the concentration is between about 1 mM and 10mM of L-Cysteine, 0.1 mM to 5 mM DTT, and/or 0.1 mM to 5 mM sodium thiolsulfate. Preferably, the medium employed during co-cultivation comprises from about 1 μM to about 10 μM of silver nitrate and from about 50 mg/L to about 1,000 mg/L of L-Cystein. This results in a highly reduced vulnerability of the target tissue against Agrobacterium-mediated damage (such as induced necrosis) and highly improves overall transformation efficiency.

Various vector systems can be used in combination with Agrobacteria. Preferred are binary vector systems. Common binary vectors are based on "broad host range"-plasmids like pRK252 (Bevan 1984) or pTJS75 (Watson 1985) derived from the P-type plasmid RK2. Most of these vectors are derivatives of pBIN19 (Bevan 1984). Various binary vectors are known, some of which are commercially available such as, for example, pBI101.2 or pBIN19 (Clontech Laboratories, Inc. USA). Additional vectors were improved with regard to size and handling (e.g. pPZP; Hajdukiewicz 1994). Improved vector systems are described also in WO 02/00900.

Methods using either a form of direct gene transfer or Agrobacterium-mediated transfer usually, but not necessarily, are undertaken with a selectable marker which may provide resistance to an antibiotic (e.g., kanamycin, hygromycin or methotrexate) or a herbicide (e.g., phosphinothricin). The choice of selectable marker for plant transformation is not, however, critical to the invention.

For certain plant species, different antibiotic or herbicide selection markers may be preferred. Selection markers used routinely in transformation include the nptII gene which confers resistance to kanamycin and related antibiotics (Messing & Vierra, 1982; Bevan 1983), the bar gene which confers resistance to the herbicide phosphinothricin (White 1990, Spencer 1990), the hph gene which confers resistance to the antibiotic hygromycin (Blochlinger & Diggelmann), and the dhfr gene, which confers resistance to methotrexate (Bourouis 1983).

### 5. Production and Characterization of Stably Transformed Plants

Transgenic plant cells are then placed in an appropriate selective medium for selection of transgenic cells which are then grown to callus. Shoots are grown from callus and plantlets generated from the shoot by growing in rooting medium. The various constructs normally will be joined to a marker for selection in plant cells. Conveniently, the marker may be resistance to a biocide (particularly an antibiotic, such as kanamycin, G418, bleomycin, hygromycin, chloramphenicol, herbicide, or the like). The particular marker used will allow for selection of transformed cells as compared to cells lacking the DNA which has been introduced. Components of DNA constructs including transcription cassettes of this invention may be prepared from sequences which are native (endogenous) or foreign (exogenous) to the host. By "foreign" it is meant that the sequence is not found in the wild-type host into which the construct is introduced. Heterologous constructs will contain at least one region which is not native to the gene from which the transcription-initiation-region is derived.

To confirm the presence of the transgenes in transgenic cells and plants, a variety of assays may be performed. Such assays include, for example, "molecular biological" assays well known to those of skill in the art, such as Southern and Northern blotting, in situ hybridization and nucleic acid-based amplification methods such as PCR or RT-PCR; "biochemical" assays, such as detecting the presence of a protein product, e.g., by immunological means (ELISAs and Western blots) or by enzymatic function; plant part assays, such as seed assays; and also, by analyzing the phenotype of the whole regenerated plant, e.g., for disease or pest resistance.

DNA may be isolated from cell lines or any plant parts to determine the presence of the preselected nucleic acid segment through the use of techniques well known to those skilled in the art. Note that intact sequences will not always be present, presumably due to rearrangement or deletion of sequences in the cell.

The presence of nucleic acid elements introduced through the methods of this invention may be determined by polymerase chain reaction (PCR). Using this technique discreet fragments of nucleic acid are amplified and detected by gel electrophoresis. This type of analysis permits one to determine whether a preselected nucleic acid segment is present in a stable transformant, but does not prove integration of the introduced preselected nucleic acid segment into the host cell genome. In addition, it is not possible using PCR techniques to determine whether transformants have exogenous genes introduced into different sites in the, genome, i.e., whether transformants are of independent origin. It is contemplated that using PCR techniques it would be possible to clone fragments of the host genomic DNA adjacent to an introduced preselected DNA segment.

Positive proof of DNA integration into the host genome and the independent identities of transformants may be determined using the technique of Southern hybridization. Using this technique specific DNA sequences that were introduced into the host genome and flanking host DNA sequences can be identified. Hence the Southern hybridization pattern of a given transformant serves as an identifying characteristic of that transformant. In addition it is possible through Southern hybridization to demonstrate the presence of introduced preselected DNA segments in high molecular weight DNA, i.e., confirm that the introduced preselected, DNA segment has been integrated into the host cell genome. The technique of Southern hybridization provides information that is obtained using PCR, e.g., the presence of a preselected DNA segment, but also demonstrates integration into the genome and characterizes each individual transformant.

It is contemplated that using the techniques of dot or slot blot hybridization which are modifications of Southern hybridization techniques one could obtain the same information that is derived from PCR, e.g., the presence of a preselected DNA segment.

Both PCR and Southern hybridization techniques can be used to demonstrate transmission of a preselected DNA segment to progeny. In most instances the characteristic Southern hybridization pattern for a given transformant will segregate in progeny as one or more Mendelian genes (Spencer 1992); Laursen 1994) indicating stable inheritance of the gene. The non-chimeric nature of the callus and the parental transformants ($R_0$) was suggested by germline transmission and the identical Southern blot hybridization patterns and intensities of the transforming DNA in callus, $R_0$ plants and $R_1$ progeny that segregated for the transformed gene.

Whereas DNA analysis techniques may be conducted using DNA isolated from any part of a plant, RNA may only be expressed in particular cells or tissue types and hence it will be necessary to prepare RNA for analysis from these tissues. PCR techniques may also be used for detection and quantitation of RNA produced from introduced preselected DNA segments. In this application of PCR it is first necessary to reverse transcribe RNA into DNA, using enzymes such as reverse transcriptase, and then through the use of conventional PCR techniques amplify the DNA. In most instances PCR techniques, while useful, will not demonstrate integrity of the RNA product. Further information about the nature of the RNA product may be obtained by Northern blotting. This technique will demonstrate the presence of an RNA species and give information about the integrity of that RNA. The presence or absence of an RNA species can also be determined using dot or slot blot Northern hybridizations. These techniques are modifications of Northern blotting and will only demonstrate the presence or absence of an RNA species.

While Southern blotting and PCR may be used to detect the preselected DNA segment in question, they do not provide information as to whether the preselected DNA segment is being expressed. Expression may be evaluated by specifically identifying the protein products of the introduced preselected DNA segments or evaluating the phenotypic changes brought about by their expression.

Assays for the production and identification of specific proteins may make use of physical-chemical, structural, functional, or other properties of the proteins. Unique physical-chemical or structural properties allow the proteins to be separated and identified by electrophoretic procedures, such as native or denaturing gel electrophoresis or isoelectric focusing, or by chromatographic techniques such as ion exchange or gel exclusion chromatography. The unique structures of individual proteins offer opportunities for use of specific antibodies to detect their presence in formats such as an ELISA assay. Combinations of approaches may be employed with even greater specificity such as Western blotting in which antibodies are used to locate individual gene products that have been separated by electrophoretic techniques. Additional techniques may be employed to absolutely confirm the identity of the product of interest such as evaluation by amino acid sequencing following purification. Although these are among the most commonly employed, other procedures may be additionally used.

Assay procedures may also be used to identify the expression of proteins by their functionality, especially the ability of enzymes to catalyze specific chemical reactions involving specific substrates and products. These reactions may be followed by providing and quantifying the loss of substrates or the generation of products of the reactions by physical or chemical procedures. Examples are as varied as the enzyme to be analyzed.

Very frequently the expression of a gene product is determined by evaluating the phenotypic results of its expression. These assays also may take many forms including but not limited to analyzing changes in the chemical composition, morphology, or physiological properties of the plant. Morphological changes may include greater stature or thicker stalks. Most often changes in response of plants or plant parts to imposed treatments are evaluated under carefully controlled conditions termed bioassays.

### 6. Uses of Transgenic Plants

Once an expression cassette of the invention has been transformed into a particular plant species, it may be propagated in that species or moved into other varieties of the same species, particularly including commercial varieties, using traditional breeding techniques. Particularly preferred plants of the invention include the agronomically important crops listed above. The genetic properties engineered into the transgenic seeds and plants described above are passed on by sexual reproduction and can thus be maintained and propagated in progeny plants. The present invention also relates to a transgenic plant cell, tissue, organ, seed or plant part obtained from the transgenic plant. Also included within the invention are transgenic descendants of the plant as well as transgenic plant cells, tissues, organs, seeds and plant parts obtained from the descendants.

Preferably, the expression cassette in the transgenic plant is sexually transmitted. In one preferred embodiment, the coding sequence is sexually transmitted through a complete normal sexual cycle of the R0 plant to the R1 generation. Additionally preferred, the expression cassette is expressed in the cells, tissues, seeds or plant of a transgenic plant in an amount that is different than the amount in the cells, tissues, seeds or plant of a plant which only differs in that the expression cassette is absent.

The transgenic plants produced herein are thus expected to be useful for a variety of commercial and research purposes. Transgenic plants can be created for use in traditional agriculture to possess traits beneficial to the grower (e.g., agronomic traits such as resistance to water deficit, pest resistance, herbicide resistance or increased yield), beneficial to the consumer of the grain harvested from the plant (e.g., improved nutritive content in human food or animal feed; increased vitamin, amino acid, and antioxidant content; the production of antibodies (passive immunization) and nutriceuticals), or beneficial to the food processor (e.g., improved processing traits). In such uses, the plants are generally grown for the use of their grain in human or animal foods. Additionally, the use of root-specific promoters in transgenic plants can provide beneficial traits that are localized in the consumable (by animals and humans) roots of plants such as carrots, parsnips, and beets. However, other parts of the plants, including stalks, husks, vegetative parts, and the like, may also have utility, including use as part of animal silage or for omamental purposes. Often, chemical constituents (e.g., oils or starches) of maize and other crops are extracted for foods or industrial use and transgenic plants may be created which have enhanced or modified levels of such components.

Transgenic plants may also find use in the commercial manufacture of proteins or other molecules, where the molecule of interest is extracted or purified from plant parts, seeds, and the like. Cells or tissue from the plants may also be cultured, grown in vitro, or fermented to manufacture such molecules. The transgenic plants may also be used in commercial breeding programs, or may be crossed or bred to plants of related crop species. Improvements encoded by the expression cassette may be transferred, e.g., from maize cells to cells of other species, e.g., by protoplast fusion.

The transgenic plants may have many uses in research or breeding, including creation of new mutant plants through insertional mutagenesis, in order to identify beneficial mutants that might later be created by traditional mutation and selection. An example would be the introduction of a recombinant DNA sequence encoding a transposable element that may be used for generating genetic variation. The methods of the invention may also be used to create plants having unique "signature sequences" or other marker sequences which can be used to identify proprietary lines or varieties.

Thus, the transgenic plants and seeds according to the invention can be used in plant breeding which aims at the development of plants with improved properties conferred by the expression cassette, such as tolerance of drought, disease, or other stresses. The various breeding steps are characterized by well-defined human intervention such as selecting the lines to be crossed, directing pollination of the parental lines, or selecting appropriate descendant plants. Depending on the desired properties different breeding measures are taken. The relevant techniques are well known in the art and include but are not limited to hybridization, inbreeding, backcross breeding, multilane breeding, variety blend, interspecific hybridization, aneuploid techniques, etc. Hybridization techniques also include the sterilization of plants to yield male or female sterile plants by mechanical, chemical or biochemical means. Cross pollination of a male sterile plant with pollen of a different line assures that the genome of the male sterile but female fertile plant will uniformly obtain properties of both parental lines. Thus, the transgenic seeds and plants according to the invention can be used for the breeding of improved plant lines which for example increase the effectiveness of conventional methods such as herbicide or pesticide treatment or allow to dispense with said methods due to their modified genetic properties. Altematively new crops with improved stress tolerance can be obtained which, due to their optimized genetic "equipment", yield harvested product of better quality than products which were not able to tolerate comparable adverse developmental conditions.

## EXAMPLES

### Materials and General Methods

Unless indicated otherwise, chemicals and reagents in the Examples were obtained from Sigma Chemical Company (St. Louis, MO), restriction endonucleases were from New England Biolabs (Beverly, MA) or Roche (Indianapolis, IN), oligonucleotides were synthesized by MWG Biotech Inc. (High Point, NC), and other modifying enzymes or kits regarding biochemicals and molecular biological assays were from Clontech (Palo Alto, CA), Pharmacia Biotech (Piscataway, NJ), Promega Corporation (Madison, WI), or Stratagene (La Jolla, CA). Materials for cell culture media were obtained from Gibco/BRL (Gaithersburg, MD) or DIFCO (Detroit, MI). The cloning steps carried out for the purposes of the present invention, such as, for example, restriction cleavages, agarose gel electrophoresis, purification of DNA fragments, transfer of nucleic acids to nitrocellulose and nylon membranes, linking DNA fragments, transformation of *E. coli* cells, growing bacteria, multiplying phages and sequence analysis of recombinant DNA, are carried out as described by Sambrook (1989). The sequencing of recombinant DNA molecules is carried out using ABI laser fluorescence DNA sequencer following the method of Sanger (Sanger 1977).

For generating transgenic *Arabidopsis* plants *Agrobacterium tumefaciens* (strain C58C1[pMP90]) is transformed with the various promoter::GUS vector constructs (see below). Resulting *Agrobacterium* strains are subsequently employed to obtain transgenic plants. For this purpose a isolated transformed *Agrobacterium* colony is incubated in 4 ml culture (Medium: YEB medium with 50 μg/ml Kanamycin and 25 μg/ml Rifampicin) over night at 28°C. With this culture a 400 ml culture of the same medium is inoculated and incubated over night (28 °C, 220 rpm). The bacteria a precipitated by centrifugation (GSA-Rotor, 8.000 U/min, 20 min) and the pellet is resuspended in infiltration medium (1/2 MS-Medium; 0,5 g/l MES, pH 5,8; 50 g/l sucrose). The suspension is placed in a plant box (Duchefa) and 100 ml SILVET L-77 (Osi Special-ties Inc., Cat. P030196) are added to a final concentration of 0.02%. The plant box with 8 to 12 Plants is placed into an exsiccator for 10 to 15 min. under vacuum with subsequent, spontaneous ventilation (expansion). This process is repeated 2-3 times. Thereafter all plants are transferred into pods with wet-soil and grown under long daytime conditions (16 h light; day temperature 22-24°C, night temperature 19°C; 65% rel. humidity). Seeds are harvested after 6 weeks.

### EXAMPLE 1: Growth conditions for plants for tissue-specific expression analysis

To obtain 4 and 7 days old seedlings, about 400 seeds *(Arabidopsis* thaliana ecotype Columbia) are sterilized with a 80% (v/v) ethanol:water solution for 2 minutes, treated with a sodium hypochlorite solution (0.5% v/v) for 5 minutes, washed three times with distillated water and incubated at 4°C for 4 days to ensure a standardized germination. Subsequently, seeds are incubated on Petri dishes with MS medium (Sigma M5519) supplemented with 1% sucrose, 0.5 g/l MES (Sigma M8652), 0.8% Difco-BactoAgar (Difco 0140-01), adjusted to pH 5.7. The seedlings are grown under 16 h light / 8 h dark cyklus (Philips 58W/33 white light) at 22°C and harvested after 4 or 7 days, respectively. To obtain root tissue, 100 seeds are sterilized as described above, incubated at 4°C for 4 days, and transferred into 250ml flasks with MS medium (Sigma M5519) supplemented with additional 3% sucrose and 0.5 g/l MES (Sigma M8652), adjusted to pH 5.7 for further growing. The seedlings are grown at a 16 h light / 8 h dark cycle (Philips 58W/33 white light) at 22°C and 120 rpm and harvested after 3 weeks. For all other plant organs employed, seeds are sown on standard soil (Type VM, Manna-Italia, Via S. Giacomo 42, 39050 San Giacomo/ Laives, Bolzano, Italien), incubated for 4 days at 4°C to ensure uniform germination, and subsequently grown under a 16 h light / 8 darkness regime (OSRAM Lumi-lux Daylight 36W/

12) at 22°C. Young rosette leaves are harvested at the 8-leaf stage (after about 3 weeks), mature rosette leaves are harvested after 8 weeks briefly before stem formation. Apices of out-shooting stems are harvested briefly after out-shooting. Stem, stem leaves, and flower buds are harvested in development stage 12 (Bowmann J (ed.), *Arabidopsis,* Atlas of Morphology, Springer New York, 1995) prior to stamen development. Open flowers are harvested in development stage 14 immediately after stamen development. Wilting flowers are harvested in stage 15 to 16. Green and yellow shoots used for the analysis have a length of 10 to 13 mm.

**EXAMPLE 2: Demonstration of expression profile**

To demonstrate and analyze the transcription regulating properties of a promoter of the useful to operably link the promoter or its fragments to a reporter gene, which can be employed to monitor its expression both qualitatively and quantitatively. Preferably bacterial β-glucuronidase is used (Jefferson 1987). β-glucuronidase activity can be monitored *in planta* with chromogenic substrates such as 5-bromo-4-Chloro-3-indolyl-β-D-glucuronic acid during corresponding activity assays (Jefferson 1987). For determination of promoter activity and tissue specificity plant tissue is dissected, embedded, stained and analyzed as described (e.g., Bäumlein 1991).

For quantitative β-glucuronidase activity analysis MUG (methylumbelliferyl glucuronide) is used as a substrate, which is converted into MU (methylumbelliferone) and glucuronic acid. Under alkaline conditions this conversion can be quantitatively monitored fluorometrically (excitation at 365 nm, measurement at 455 nm; SpectroFluorimeter Thermo Life Sciences Fluoroscan) as described (Bustos 1989).

**EXAMPLE 3: Cloning of the promoter fragments**

To isolate the promoter fragments described by SEQ ID NO: 1, 2, 3, 4, 5, 6, 9, 10, 11, 12, 13, 14, 15, 16, 19, 20, 21, 22, 23, 24, 25, 26, 27, 28, 29, 30, 33, 34, 35, 36, 37, 38, 39, 40, 43, 44, 45, 46, 47, 48, 49, 50, 53, 54, 55, 56, 57, and 58, genomic DNA is isolated from *Arabidopsis thaliana* (ecotype Columbia) as described (Galbiati 2000). The isolated genomic DNA is employed as matrix DNA for a polymerase chain reaction (PCR) mediated amplification using the oligonucleotide primers and protocols indicated below (Table 3).

Table 3: PCR oligonucleotide primers for amplification of the various transcription regulating nucleotide sequences and restriction enzymes for modifying the resulting PCR products

| SEQ ID | Promoter | Forward Primer | Reverse Primer | Restriction enzymes |
|---|---|---|---|---|
| SEQ ID NO: 1 | pSUK402L | SUK402for SEQ ID NO: 61 | SUK402Lrev SEQ ID NO: 62 | EcoRI/NcoI |
| SEQ ID NO: 2 | pSUK402LGB | SUK402for SEQ ID NO: 61 | SUK402Lrev SEQ ID NO: 62 | EcoRI/NcoI |
| SEQ ID NO: 3 | pSUK402S | SUK402for SEQ ID NO: 61 | SUK402Srev SEQ ID NO: 63 | EcoRI/NcoI |
| SEQ ID NO: 4 | pSUK402SGB | SUK402for SEQ ID NO: 61 | SUK402Srev SEQ ID NO: 63 | EcoRI/NcoI |
| SEQ ID NO: 5 | pSUK404L | SUK404for SEQ ID NO: 64 | SUK404Lrev SEQ ID NO: 65 | EcoRI/NcoI |
| SEQ ID NO: 6 | pSUK404LGB | SUK404for SEQ ID NO: 64 | SUK404Srev SEQ ID NO: 66 | EcoRI/NcoI |
| SEQ ID NO: 9 | pSUK440L | SUK440for SEQ ID NO: 67 | SUK440Lrev SEQ ID NO: 68 | XhoI/BamHI |
| SEQ ID NO: 10 | pSUK440LGB | SUK440for SEQ ID NO: 67 | SUK440Lrev SEQ ID NO: 68 | XhoI/BamHI |
| SEQ ID NO: 11 | pSUK440S | SUK440for SEQ ID NO: 67 | SUK440Srev SEQ ID NO: 69 | XhoI/BamHI |
| SEQ ID NO: 12 | pSUK440SGB | SUK440for SEQ ID NO: 67 | SUK440Srev SEQ ID NO: 69 | XhoI/BamHI |

Table continued

| SEQ ID | Promoter | Forward Primer | Reverse Primer | Restriction enzymes |
|---|---|---|---|---|
| SEQ ID NO: 13 | pSUK442L | SUK442for SEQ ID NO: 70 | SUK442Lrev SEQ ID NO: 71 | Smal/BamHI |
| SEQ ID NO: 14 | pSUK442LGB | SUK442for SEQ ID NO: 70 | SUK442Lrev SEQ ID NO: 71 | Smal/BamHI |
| SEQ ID NO: 15 | pSUK442S | SUK442for SEQ ID NO: 70 | SUK442Srev SEQ ID NO: 72 | Smal/BamHI |
| SEQ ID NO: 16 | pSUK442SGB | SUK442for SEQ ID NO: 70 | SUK442Srev SEQ ID NO: 72 | Smal/BamHI |
| SEQ ID NO: 19 | pSUK398L | SUK398for SEQ ID NO: 73 | SUK398Lrev SEQ ID NO: 74 | BamHI/Ncol |
| SEQ ID NO: 20 | pSUK398LGB | SUK398for SEQ ID NO: 73 | SUK398Lrev SEQ ID NO: 74 | BamHI/Ncol |
| SEQ ID NO: 21 | pSUK398SGB | SUK398for SEQ ID NO: 73 | SUK398Srev SEQ ID NO: 75 | BamHI/Ncol |
| SEQ ID NO: 22 | pSUK398SGB | SUK398for SEQ ID NO: 73 | SUK398Srev SEQ ID NO: 75 | BamHI/Ncol |
| SEQ ID NO: 23 | pSUK399L | SUK399for SEQ ID NO: 76 | SUK399Lrev SEQ ID NO: 77 | EcoRI/Ncol |
| SEQ ID NO: 24 | pSUK399LGB | SUK399for SEQ ID NO: 76 | SUK399Lrev SEQ ID NO: 77 | EcoRI/Ncol |
| SEQ ID NO: 25 | pSUK399S | SUK399for SEQ ID NO: 76 | SUK399Srev SEQ ID NO: 78 | EcoRI/Ncol |
| SEQ ID NO: 26 | pSUK399SGB | SUK399for SEQ ID NO: 76 | SUK399Srev SEQ ID NO: 78 | EcoRI/Ncol |
| SEQ ID NO: 27 | pSUK400L | SUK400for SEQ ID NO: 79 | SUK400Lrev SEQ ID NO: 80 | Spel/Ncol |
| SEQ ID NO: 28 | pSUK400LGB | SUK400for SEQ ID NO: 79 | SUK400Lrev SEQ ID NO: 80 | Spel/Ncol |
| SEQ ID NO: 29 | pSUK400S | SUK400for SEQ ID NO: 79 | SUK400Srev SEQ ID NO: 81 | Spel/Ncol |
| SEQ ID NO: 30 | pSUK400SGB | SUK400for SEQ ID NO: 79 | SUK400Srev SEQ ID NO: 81 | Spel/Ncol |
| SEQ ID NO: 33 | pSUK460L | SUK460for SEQ ID NO: 82 | SUK460Lrev SEQ ID NO: 83 | Spel/Ncol |
| SEQ ID NO: 34 | pSUK460LGB | SUK460for SEQ ID NO: 82 | SUK460Lrev SEQ ID NO: 83 | Spel/Ncol |
| SEQ ID NO: 35 | pSUK460S | SUK460for SEQ ID NO: 82 | SUK460Srev SEQ ID NO: 84 | Spel/Ncol |
| SEQ ID NO: 36 | pSUK460SGB | SUK460for SEQ ID NO: 82 | SUK460Srev SEQ ID NO: 84 | Spel/Ncol |
| SEQ ID NO: 37 | pSUK462L | SUK462for SEQ ID NO: 85 | SUK462Lrev SEQ ID NO: 86 | EcoRI/Ncol |
| SEQ ID NO: 38 | pSUK462LGB | SUK462for SEQ ID NO: 85 | SUK462Lrev SEQ ID NO: 86 | EcoRI/Ncol |

Table continued

| SEQ ID | Promoter | Forward Primer | Reverse Primer | Restriction enzymes |
|---|---|---|---|---|
| SEQ ID NO: 39 | pSUK462S | SUK462for SEQ ID NO: 85 | SUK462Srev SEQ ID NO: 87 | EcoRI/Ncol |
| SEQ ID NO: 40 | pSUK462SGB | SUK462for SEQ ID NO: 85 | SUK462Srev SEQ ID NO: 87 | EcoRI/Ncol |
| SEQ ID NO: 43 | pSUK464L | SUK464for SEQ ID NO: 88 | SUK464Lrev SEQ ID NO: 89 | Spel/Ncol |
| SEQ ID NO: 44 | pSUK464LGB | SUK464for SEQ ID NO: 88 | SUK464Lrev SEQ ID NO: 89 | Spel/Ncol |
| SEQ ID NO: 45 | pSUK464S | SUK464for SEQ ID NO: 88 | SUK464Srev SEQ ID NO: 90 | Spel/Ncol |
| SEQ ID NO: 46 | pSUK464SGB | SUK464for SEQ ID NO: 88 | SUK464Srev SEQ ID NO: 90 | Spel/Ncol |
| SEQ ID NO: 47 | pSUK466L | SUK466for SEQ ID NO: 91 | SUK466Lrev SEQ ID NO: 92 | Spel/Ncol |
| SEQ ID NO: 48 | pSUK466LGB | SUK466for SEQ ID NO: 91 | SUK466Lrev SEQ ID NO: 92 | Spel/Ncol |
| SEQ ID NO: 49 | pSUK466S | SUK466for SEQ ID NO: 91 | SUK466Srev SEQ ID NO: 93 | Spel/Ncol |
| SEQ ID NO: 50 | pSUK466SGB | SUK466for SEQ ID NO: 91 | SUK466Srev SEQ ID NO: 93 | Spel/Ncol |
| SEQ ID NO: 53 | pSUK468L | SUK468for SEQ ID NO: 94 | SUK468Lrev SEQ ID NO: 95 | BamHI/Ncol |
| SEQ ID NO: 54 | pSUK468LGB | SUK468for SEQ ID NO: 94 | SUK468Lrev SEQ ID NO: 95 | BamHI/Ncol |
| SEQ ID NO: 55 | pSUK468S | SUK468for SEQ ID NO: 94 | SUK468Srev SEQ ID NO: 96 | BamHI/Ncol |
| SEQ ID NO: 56 | pSUK468SGB | SUK468for SEQ ID NO: 94 | SUK468rev SEQ ID NO: 96 | BamHI/Ncol |
| SEQ ID NO: 57 | pSUK470LGB | SUK470for SEQ ID NO: 97 | SUK470Lrev SEQ ID NO: 98 | BamHI/Ncol |
| SEQ ID NO: 58 | pSUK470SGB | SUK470for SEQ ID NO: 97 | SUK470Srev SEQ ID NO: 99 | BamHI/Ncol |

Amplification is carried out as follows:

100 ng genomic DNA
1X PCR buffer
2,5 mM MgCl2,
200 μM each of dATP, dCTP, dGTP und dTTP
10 pmol of each oligonucleotide primers
2,5 Units Pfu DNA Polymerase (Stratagene)
in a final volume of 50 μl

The following temperature program is employed for the various amplifications (BIORAD Thermocycler).

1. 95°C for 5 min
2. 54°C for 1 min, followed by 72°C for 5 min and 95°C for 30 sec. Repeated 25 times.
3. 54°C for 1 min, followed by 72°C for 10min.

4. Storage at 4°C

The resulting PCR-products are digested with the restriction endonucleases specified in the Table above (Table 3) and cloned into the vector pSUN0301 (SEQ ID NO: 100) (pre-digested with the same enzymes) upstream and in operable linkage to the glucuronidase (GUS) gene. Following stable transformation of each of these constructs into *Arabidopsis* thaliana tissue specificity and expression profile was analyzed by a histochemical and quantitative GUS-assay, respectively.

## EXAMPLE 4: Expression profile of the various promoter::GUS constructs in stably transformed *A. thaliana* plants

### 4.1 pSUK402L, pSUK402LGB, pSUK402S, pSUK402SGB, pSUK404LGB, pSUK404SGB

This promoter confers expression to genes in epidermis cells but also in mesophyll and phloem tissue. Weak expression was also observed in roots of seedlings but confined to root tips and vascular tissue. The expression in above ground organs is weak to medium in strength and is detectable in all organs analyzed. Reporter gene expression was not observed in guard cells.

### 4.2 pSUK440L, pSUK440LGB, pSUK440S, pSUK440SGB, pSUK442L, pSUK442LGB, pSUK442S, pSUK442SGB

This promoter confers expression to genes in epidermis cells but also in mesophyll and phloem tissue of above ground organs. Expression of the reporter gene was also detected in trichomes of young leaves and carpel walls.

### 4.3 pSUK398L, pSUK398LGB, pSUK398S, pSUK398SGB, pSUK399L, pSUK399LGB, pSUK399S, pSUK399SGB, pSUK400L, pSUK400LGB, pSUK400S, pSUK400SGB

This promoter confers medium-strong mesophyll-preferential expression in leaves accompanied by side activities in trichomes and clusters of guard cells and hydathodes of seedlings and adult plants as well as in roots of seedlings.

### 4.4 pSUK460L, pSUK460LGB, pSUK460S, pSUK460SGB, pSUK462L, pSUK462LGB, pSUK462S, pSUK462SGB

This promoter confers mesophyll-specific expression of weak-medium strength to the controlled gene. Reporter gene activity was confined to mesophyll tissue of leaves, flower sepals and seedling hypocotyl.

### 4.5 pSUK464L, pSUK464LGB, pSUK464S, pSUK464SGB, pSUK466L, pSUK466LGB, pSUK466S, pSUK466SGB

This promoter confers strong mesophyll-preferential expression in seedlings accompanied by weaker side activities in roots of seedlings. Reporter gene expression in leaves of adult plants was markedly weaker and was also detected in sepals, petals and stalks.

### 4.6 pSUK468L, pSUK468LGB, pSUK468S, pSUK468SGB, pSUK470LGB

This promoter confers strong mesophyll-preferential expression in all above ground organs of seedlings and adult plants. Reporter gene expression driven by the promoter was also detected in the entire stalk except its vascular tissue.

## EXAMPLE 5 Vector Construction for Overexpression and Gene "Knockout" Experiments

### 5.1 Overexpression

Vectors used for expression of full-length "candidate genes" of interest in plants (overexpression) are designed to overexpress the protein of interest and are of two general types, biolistic and binary, depending on the plant transformation method to be used.

For biolistic transformation (biolistic vectors), the requirements are as follows:

1. a backbone with a bacterial selectable marker (typically, an antibiotic resistance gene) and origin of replication functional in Escherichia coli (*E. coli ; e.g.,* ColE1), and

2. a plant-specific portion consisting of:

a. a gene expression cassette consisting of a promoter (eg. ZmUBlint MOD), the gene of interest (typically, a full-length cDNA) and a transcriptional terminator (e.g., Agrobacterium tumefaciens nos terminator);

b. a plant selectable marker cassette, consisting of a suitable promoter, selectable marker gene (*e.g.*, D-amino acid oxidase; dao1) and transcriptional terminator (eg. nos terminator).

Vectors designed for transformation by Agrobacterium tumefaciens (*A. tumefaciens*; binary vectors) consist of:

1. a backbone with a bacterial selectable marker functional in both E. coli and A. *tumefaciens (e.g.,* spectinomycin resistance mediated by the aadA gene) and two origins of replication, functional in each of aforementioned bacterial hosts, plus the *A. tumefaciens* virG gene;

2. a plant-specific portion as described for biolistic vectors above, except in this instance this portion is flanked by *A. tumefaciens* right and left border sequences which mediate transfer of the DNA flanked by these two sequences to the plant.

**5.2 Gene Silencing Vectors**

Vectors designed for reducing or abolishing expression of a single gene or of a family or related genes (gene silencing vectors) are also of two general types corresponding to the methodology used to downregulate gene expression: antisense or double-stranded RNA interference (dsRNAi).

(a) Anti-sense

For antisense vectors, a full-length or partial gene fragment (typically, a portion of the cDNA) can be used in the same vectors described for full-length expression, as part of the gene expression cassette. For antisense-mediated down-regulation of gene expression, the coding region of the gene or gene fragment will be in the opposite orientation relative to the promoter; thus, mRNA will be made from the non-coding (antisense) strand in planta.

(b) dsRNAi

For dsRNAi vectors, a partial gene fragment (typically, 300 to 500 basepairs long) is used in the gene expression cassette, and is expressed in both the sense and antisense orientations, separated by a spacer region (typically, a plant intron, eg. the OsSH1 intron 1, or a selectable marker, eg. conferring kanamycin resistance). Vectors of this type are designed to form a double-stranded mRNA stem, resulting from the basepairing of the two complementary gene fragments *in planta.*

Biolistic or binary vectors designed for overexpression or knockout can vary in a number of different ways, including eg. the selectable markers used in plant and bacteria, the transcriptional terminators used in the gene expression and plant selectable marker cassettes, and the methodologies used for cloning in gene or gene fragments of interest (typically, conventional restriction enzyme-mediated or Gateway™ recombinase-based cloning).

**References**

1. Abel et al., Science, 232:738 (1986).
2. Altschul et al., Nucleic Acids Res., 25:3389 (1997).
3. Altschul et al., J. Mol. Biol., 215:403 (1990).
4. An et al., EMBO J., 4:277 (1985).
5. Auch & Reth, Nucleic Acids Research, 18:6743 (1990).
6. Ausubel et al. (1987) Current Protocols in Molecular Biology, Greene Publishing Assoc. and Wiley Interscience
7. Ballas et al., Nucleic Acids Res., 17:7891 (1989).
8. Barkai-Golan et al., Arch. Microbiol., 116:119 (1978).
9. Batzer et al., Nucleic Acid Res., 19:5081 (1991).
10. Bäumlein et al. Mol Gen Genet 225:121-128 (1991)
11. Becker et al. (1994) Plant J., 5:299-307,

12. Bernal-Lugo and Leopold, Plant Physiol., 98:1207 (1992).
13. Bevan et al., Nature, 304:184 (1983).
14. Bevan et al., Nucl. Acids Res., 11:369 (1983).
15. Bevan, Nucl. Acids Res., 12:8711 (1984).
16. Blackman et al., Plant Physiol., 100:225 (1992).
17. Blochlinger & Diggelmann, Mol Cell Biol, 4:2929 (1984).
18. Bol et al., Ann. Rev. Phytopath., 28:113 (1990).
19. Bouchez et al., EMBO J., 8:4197 (1989).
20. Bourouis et al., EMBO J., 2:1099 (1983).
21. Bowler et al., Ann. Rev. Plant Physiol., 43:83 (1992).
22. Branson and Guss, Proc. North Central Branch Entomological Society of America (1972).
23. Broakgert et al., Science, 245:110 (1989).
24. Bustos MM et al. (1989) Plant Gell 1:839-853
25. Byrne et al. Plant Cell Tissue and Organ Culture, 8:3 (1987).
26. Callis et al., Genes and Develop., 1:1183 (1987).
27. Campbell and Gowri, Plant Physiol., 92:1 (1990).
28. Campbell, W. C., ed. Ivermectin and Abamectin, Springer-Verlag, New York, 1989.
29. Chee et al. Plant Physiol., 91:1212 (1989).
30. Chen and Winans (1991) J. Bacteriol. 173: 1139-1144
31. Christou et al. Proc. Natl. Acad. Sci USA, 86:7500 (1989).
32. Christou et al., Biotechnology, 9:957 (1991).
33. Christou et al., Plant Physiol., 87:671 (1988).
34. Chui et al. (1996) Curr Biol 6:325-330
35. Coe et al., In: Corn and Corn Improvement, Sprague et al. (eds.) pp. 81-258 (1988).
36. Corpet et al. Nucleic Acids Res., 16:10881 (1988).
37. Coxson et al., Biotropica, 24:121 (1992).
38. Crameri et al., Nature Biotech., 15:436 (1997).
39. Crameri et al., Nature, 391:288 (1998).
40. Crossway et al., BioTechniques, 4:320 (1986).
41. Cuozzo et al., Bio/Technology, 6:549 (1988).
42. Cutler et al., J. Plant Physiol., 135:351 (1989).
43. Czapla and Lang, J. Econ. Entomol., 83:2480 (1990).
44. Datta et al., Bio/Technology, 8:736 (1990).
45. Davies et al., Plant Physiol., 93:588 (1990).
46. Dayhoff et al., Atlas of Protein Sequence and Structure, Natl. Biomed. Res. Found., Washington, C. D. (1978).
47. De Blaere et al., Meth. Enzymol., 143:277 (1987).
48. De Block et al. Plant Physiol., 91:694 (1989).
49. De Block et al., EMBO Journal, 6:2513 (1987).
50. Deblaere et al. Nucl Acids Res 13:4777-4788 (1985)
51. Della-Cioppa et al. Bio/Technology 5:579-584 (1987)
52. Della-Cioppa et al., Plant Physiology, 84:965-968 (1987).
53. Dellaporta et al., in Chromosome Structure and Function, Plenum Press, 263-282 (1988).
54. Depicker et al., Plant Cell Reports, 7:63 (1988).
55. Dunn et al., Can. J. Plant Sci., 61:583 (1981).
56. Dure et al., Plant Mol. Biol., 12:475 (1989).
57. Ebinuma et al. Proc Natl Acad Sci USA 94:2117-2121 (2000a)
58. Ebinuma et al. Selection of Marker-free transgenic plants using the oncogenes *(ipt, rol* A, B, C) of *Agrobacterium* as selectable markers, In Molecular Biology of Woody Plants. Kluwer Academic Publishers (2000b)
59. Eichholtz et al. Somatic Cell and Molecular Genetics 13, 67-76 (1987)
60. Ellis et al., EMBO Journal, 6:3203 (1987).
61. Elroy-Stein et al., Proc. Natl. Acad. Sci. U.S.A., 86:6126 (1989).
62. English et al., Plant Cell, 8:179 (1996).
63. Erdmann et al., J. Gen. Microbiol., 138:363 (1992).
64. Erikson et al. Nat Biotechnol. 22(4):455-8 (2004)
65. Everett et al., Bio/Technology, 5:1201(1987).
66. Fedoroff NV & Smith DL Plant J 3:273- 289 (1993)
67. Fire A et al Nature 391:806-811 (1998)
68. Fitzpatrick, Gen. Engineering News, 22:7 (1993).

69. Fraley et al. Proc Natl Acad Sci USA 80:4803 (1983)
70. Fromm et al., Bio/Technology, 8:833 (1990).
71. Fromm et al., Nature (London), 319:791 (1986).
72. Galbiati et al. Funct. Integr Genozides 2000, 20 1:25-34
73. Gallie et al. Nucl Acids Res 15:8693-8711 (1987)
74. Gallie et al., Nucleic Acids Res., 15:3257 (1987).
75. Gallie et al., The Plant Cell, 1:301 (1989).
76. Gan et al., Science, 270:1986 (1995).
77. Gatehouse et al., J. Sci. Food Agric., 35:373 (1984).
78. Gelfand, eds., PCR Strategies Academic Press, New York (1995).
79. Gelvin et al., Plant Molecular Biology Manual, (1990).
80. Gleave et al. Plant Mol Biol. 40(2):223-35 (1999)
81. Gordon-Kamm et al., Plant Cell, 2:603 (1990).
82. Goring et al, PNAS, 88:1770 (1991).
83. Gruber, et al., Vectors for Plant Transformation, in: Methods in Plant Molecular Biology & Biotechnology" in Glich et al., (Eds. pp. 89-119, CRC Press, 1993).
84. Guerineau et al., Mol. Gen. Genet., 262:141 (1991).
85. Guerrero et al., Plant Mol. Biol., 15:11 (1990).
86. Gupta et al., PNAS, 90:1629 (1993).
87. Haines and Higgins (eds.), Nucleic Acid Hybridization, IRL Press, Oxford, U.K.
88. Hajdukiewicz et al. Plant Mol Biol 25:989-994 (1994)
89. Hammock et al., Nature, 344:458 (1990).
90. Hansen et al. Proc. Natl. Acad. Sci. USA 91:7603-7607 (1994)
91. Hayford et al. Plant Physiol. 86:1216 (1988)
92. Hemenway et al., EMBO Journal, 7:1273 (1988).
93. Henikoff & Henikoff, Proc. Natl. Acad. Sci. USA, 89:10915 (1989).
94. Hiei et al. Plant J 6: 271-282 (1994)
95. Higgins et al., Gene, 73:237 (1988).
96. Higo et al. (1999) Nucl Acids Res 27(1): 297-300
97. Hilder et al., Nature, 330:160 (1987).
98. Hille et al. Plant Mol. Biol. 7:171 (1986)
99. Hinchee et al. Bio/Technology 6:915 (1988).
100. Hoekema *et al*. (1983) Nature 303:179-181
101. Hoekema, In: The Binary Plant Vector System. Offset-drukkerij Kanters B.V.; Alblasserdam (1985).
102. Hood et al. J Bacteriol 168:1291-1301 (1986)
103. Huang et al., CABIOS, 8:155 (1992).
104. Ikeda et al., J. Bacteriol., 169:5612 (1987).
105. Ikuta et al., Biotech., 8:241 (1990).
106. Ingelbrecht et al., Plant Cell, 1:671 (1989).
107. Innis and Gelfand, eds., PCR Methods Manual (Academic Press, New York) (1999).
108. Innis et al., eds., PCR Protocols: A Guide to Methods and Applications (Academic Press, New York (1995).
109. Innis et al., PCR Protocols: A Guide to Methods and Applications, Academic Press, Inc., San Diego, Calif. (1990).
110. Ishida Y et al. Nature Biotech 745-750 (1996)
111. Jefferson et al. EMBO J 6:3901-3907 (1987)
112. Jefferson et al. Plant Mol Biol Rep 5:387-405 (1987)
113. Jenes B et al. Techniques for Gene Transfer, in: Recombinant Plants, Vol. 1, Engineering and Utilization, edited by SD Kung and R Wu, Academic Press, pp. 128-143 (1993)
114. Jobling et al., Nature, 325:622 (1987).
115. Johnson et al., PNAS USA, 86:9871 (1989)
116. Jones et al. Mol. Gen. Genet., 210:86 (1987)
117. Joshi et al., Nucleic Acid Res., 15:9627 (1987).
118. Kaasen et al., J. Bacteriol., 174:889 (1992).
119. Karlin and Altschul, Proc. Natl. Acad Sci. USA, 87:2264 (1990).
120. Karlin and Altschul, Proc. Natl. Acad. Sci. USA, 90:5873 (1993).
121. Karsten et al., Botanica Marina, 35:11 (1992).
122. Katz et al., J. Gen. Microbiol., 129:2703 (1983).
123. Keller et al., EMBO Journal, 8:1309 (1989).
124. Keller et al., Genes Dev., 3:1639 (1989).

125. Klapwijk et al. J. Bacteriol., 141,128-136 (1980)
126. Klein et al., Bio/Technoloy, 6:559 (1988).
127. Klein et al., Plant Physiol., 91:440 (1988).
128. Klein et al., Proc. Natl. Acad. Sci. USA, 85:4305 (1988).
129. Knauf, et al., Genetic Analysis of Host Range Expression by Agrobacterium In: Molecular Genetics of the Bacteria-Plant Interaction, Puhler, A. ed., Springer-Verlag, New York, 1983.
130. Koncz & Schell Mol Gen Genet 204:383-396 (1986)
131. Koprek T et al. Plant J 19(6): 719-726 (1999)
132. Koster and Leopold, Plant Physiol., 88:829 (1988).
133. Koziel et al., Biotechnology, 11:194 (1993).
134. Kunkel et al., Methods in Enzymol., 154:367 (1987).
135. Kunkel, Proc. Natl. Acad. Sci. USA, 82:488 (1985).
136. Lam E und Chua NH, J Biol Chem; 266(26):17131-17135 (1991)
137. Laufs et al., PNAS, 87:7752 (1990).
138. Lawton et al., Mol. Cell Biol., 7:335 (1987).
139. Lee and Saier, J. Bacteriol., 153 (1982).
140. Leffel et al. Biotechniques 23(5):912-8 (1997)
141. Lescot et al. Nucleic Acids Res 30(1):325-7 (2002)
142. Li et al. Plant Mol Biol 20:1037-1048 (1992)
143. Lindsey et al., Transgenic Research, 2:3347 (1993).
144. Liu et al.,. Plant J. 8, 457-463 (1995)
145. Lommel et al., Virology, 181:382 (1991).
146. Loomis et al., J. Expt. Zool., 252:9 (1989).
147. Lorz et al., Mol. Gen. Genet., 199:178 (1985).
148. Ma et al., Nature, 334 :631 (1988).
149. Macejak et al., Nature, 353:90 (1991).
150. Maki et al., Methods in Plant Mol Biol & Biotechnol, Glich et al., 67-88 CRC Press, (1993).
151. Maniatis T, Fritsch EF, and Sambrook J Molecular Cloning: A Laboratory Manual, Cold Spring Harbor Laboratory, Cold Spring Harbor (NY), (1989)
152. Matzke et al. (2000) Plant Mol Biol 43:401-415;
153. Matsuoka M et al. (2001) High-level expression of C4 photosynthetic genes in transgenic rice. In: Rice genetics IV. Proceedings of the Fourth International Rice Genetics Symposium, Los Banos, Philippines, 22-27.10. 2000. eds. Khush; Brar; Hardy pp. 439-447
154. McBride et al., PNAS USA, 91:7301 (1994).
155. McCabe et al., Bio/Technology, 6:923 (1988).
156. Meinkoth and Wahl, Anal. Biochem., 138:267 (1984).
157. Messing and Vierra, Gene, 19:259 (1982).
158. Michael et al., J. Mol. Biol., 26 :585 (1990). (im Text steht: Michael et al. 1994)
159. Millar et al. Plant Mol Biol Rep 10:324-414 (1992)
160. Mogen et al., Plant Cell, 2:1261 (1990).
161. Moore et al., J. Mol. Biol., 272:336 (1997).
162. Mozo & Hooykaas Plant Mol. Biol. 16:917-918 (1991)
163. Mundy and Chua, EMBO J., 7:2279 (1988).
164. Munroe et al., Gene, 91:151 (1990).
165. Murakami et al., Mol. Gen. Genet., 205:42 (1986).
166. Murata et al., FEBS Lett., 296:187 (1992).
167. Murdock et al., Phytochemistry, 29:85 (1990).
168. Murray et al., Nucleic Acids Res., 17:477 (1989).
169. Myers and Miller, CABIOS, 4:11 (1988).
170. Naested H Plant J 18:571-576 (1999)
171. Napoli et al., Plant Cell, 2:279 (1990).
172. Needleman and Wunsch, J. Mol. Biol., 48:443-453 (1970).
173. Nehra et al. Plant J. 5:285-297 (1994)
174. Niedz et al., Plant Cell Reports, 14:403 (1995).
175. Odell et al., Mol. Gen. Genet., 113:369 (1990).
176. Odell et al., Nature, 313:810 (1985).
177. Ohtsuka et al., J. Biol. Chem., 260:2605 (1985)
178. Olhoft et al. Plant Cell Rep 20: 706-711 (2001)

179. Ow et al., Science, 234:856 (1986).
180. Pacciotti et al., Bio/Technology, 3:241 (1985).
181. Park et al., J. Plant Biol., 38:365 (1985).
182. Paszkowski et al., EMBO J., 3:2717 (1984).
183. Pearson and Lipman, Proc. Natl. Acad. Sci., 85:2444 (1988).
184. Pearson et al., Meth. Mol. Biol., 24:307 (1994).
185. Perera RJ et al. Plant Mol. Biol 23(4): 793-799 (1993)
186. Perlak et al., Proc. Natl. Acad. Sci. USA, 88:3324 (1991).
187. Phillips et al., In Corn & Corn Improvement, 3rd Edition 10, Sprague et al. (Eds.) pp. 345-387 (1988).
188. Phi-Van et al., Mol. Cell. Biol., 10:2302 (1990).
189. Piatkowski et al., Plant Physiol., 94:1682 (1990).
190. Potrykus et al., Mol. Gen. Genet., 199:183 (1985).
191. Potrykus, Trends Biotech., 7:269 (1989).
192. Prasher et al., Biochem. Biophys. Res. Comm., 126:1259 (1985).
193. Proudfoot, Cell, 64:671 (1991).
194. Reed et al., J. Gen. Microbiol., 130:1 (1984).
195. Riggs et al., Proc. Natl. Acad. Sci. USA, 83:5602 (1986).
196. Rossolini et al., Mol. Cell. Probes, 8:91 (1994).
197. Ruiz, Plant Cell, 10:937 (1998).
198. Sambrook et al., Molecular Cloning: A Laboratory Manual (2d ed., Cold Spring Harbor Laboratory Press, Plainview, N.Y.) (1989).
199. Sanfacon et al., Genes Dev., 5:141 (1991).
200. Sanford et al., Particulate Science and Technology, 5:27 (1987).
201. Scheeren-Groot et al. J. Bacteriol 176: 6418-6426 (1994)
202. Schenborn and Groskreutz Mol Biotechnol 13(1): 29-44 (1999)
203. Schlaman and Hooykaas Plant J 11:1377-1385 (1997)
204. Schoffl F et al. (1989) Mol Gen Genetics 217(2-3):246-53
205. Shagan et al., Plant Physiol., 101:1397 (1993).
206. Shah et al. Science 233: 478 (1986)
207. Shapiro, Mobile Genetic Elements, Academic Press, N.Y. (1983).
208. Shimamoto et al., Nature, 338:274 (1989).
209. Silhavy TJ, Berman ML, and Enquist LW Experiments with Gene Fusions, Cold Spring Harbor Laboratory Press, Cold Spring Harbor (NY), (1984)
210. Skuzeski et al., Plant Molec. Biol. 15: 65-79 (1990).
211. Smith et al., Adv. Appl. Math., 2:482.(1981).
212. Smith et al., Mol. Gen. Genet., 224:447 (1990).
213. Spencer et al., Theor. Appl. Genet, 79:625 (1990). Spencer 1992 Referenz fehlt
214. Stalker et al., Science, 242:419 (1988).
215. Staub et al., EMBO J., 12:601 (1993).
216. Staub et al., Plant Cell, 4:39 (1992).
217. Steifel et al., The Plant Cell, 2:785 (1990).
218. Stemmer, Nature, 370:389 (1994).
219. Stemmer, Proc. Natl. Acad. Sci. USA, 91:10747 (1994).
220. Stief et al., Nature, 341:343 (1989).
221. Stougaard Plant J 3:755-761 (1993)
222. Sukhapinda et al., Plant Mol. Biol., 8:209 (1987).
223. Sundaresan et al. Gene Develop 9: 1797-1810 (1995)
224. Sutcliffe, PNAS USA, 75:3737 (1978).
225. Svab et al., Plant Mol. Biol. 14:197 (1990)
226. Svab et al., Proc. Natl. Acad. Sci. USA, 87:8526 (1990).
227. Svab et al., Proc. Natl. Acad. Sci. USA, 90:913 (1993).
228. Tarczynski et al., PNAS USA, 89:2600 (1992).
229. Thillet et al., J. Biol. Chem., 263:12500 (1988).
230. Tijssen, Laboratory Techniques in Biochemistry and Molecular Biology-Hybridization with Nucleic Acid Probes, Elsevier, N.Y. (1993).
231. Tomes et al., Plant Cell, Tissue and Organ Culture: Fundamental Methods, Springer Verlag, Berlin (1995).
232. Tomic et al., NAR, 12:1656 (1990).
233. Turner et al., Molecular Biotechnology, 3:225 (1995).

234. Twell et al., Plant Physiol., 91:1270 (1989).
235. Ugaki et al., Nucl. Acids Res., 19:371 (1991).
236. Ulmasov et al., Plant Mol. Biol., 35:417 (1997).
237. Upender et al., Biotechniques, 18:29 (1995).
238. van der Krol et al., Plant Cell, 2:291 (1990).
239. Vanden Elzen et al. Plant Mol Biol. 5:299 (1985)
240. Vasil et al. Bio/Technology, 10:667-674 (1992)
241. Vasil et al. Bio/Technology, 11:1153-1158 (1993)
242. Vasil et al., Mol. Microbiol., 3:371 (1989).
243. Vasil et al., Plant Physiol., 91:1575 (1989).
244. Vernon and Bohnert, EMBO J., 11:2077 (1992).
245. Walker and Gaastra, eds., Techniques in Molecular Biology, MacMillan Publishing Company, New York (1983).
246. Wan & Lemaux (1994) Plant Physiol., 104:3748
247. Wang et al., Mol. Cell. Biol., 12:3399 (1992).
248. Waterman, M. S. Introduction to Computational Biology: Maps, sequences and genomes. Chapman & Hall. London (1995).
249. Watrud et al., in Engineered Organisms and the Environment (1985).
250. Watson et al. J. Bacteriol 123, 255-264 (1975)
251. Watson et al., Corn: Chemistry and Technology (1987).
252. Weeks et al. Plant Physiol 102:1077-1084 (1993)
253. Weissinger et al., Annual Rev. Genet., 22:421 (1988).
254. White et al, Nucl Acids Res, 18, 1062 (1990).
255. Wingender E et al. Nucleic Acids Res 29(1):281-3 (2001)
256. Wolter et al., EMBO Journal, 11:4685 (1992).
257. Wyn-Jones and Storey, Physiology and Biochemistry of Drought Resistance in Plants, Paleg et al. (eds.), pp. 171-204 (1981).
258. Yamaguchi-Shinozaki et al., Plant Cell Physiol., 33:217 (1992).
259. Zhang et al., Proc. Natl. Acad. Sci. USA, 94:4504 (1997).
260. Zukowsky et al., PNAS USA, 80:1101 (1983).

All publications, patents and patent applications are incorporated herein by reference. While in the foregoing specification this invention has been described in relation to certain preferred embodiments thereof, and many details have been set forth for purposes of illustration, it will be apparent to those skilled in the art that the invention is susceptible to additional embodiments and that certain of the details described herein may be varied considerably without departing from the basic principles of the invention.

## Claims

1. An expression cassette for regulating mesophyll- and/or epidermis-preferential or mesophyll- and/or epidermis-specific expression in plants comprising

    i) at least one transcription regulating nucleotide sequence of a plant gene, said plant gene selected from the group of genes described by the GenBank *Arabidopsis thaliana* genome loci At5g13220, At1g68850, At4g36670, At3g10920, At1g33240, and At1g28440, or a functional equivalent thereof, and functionally linked thereto
    ii) at least one nucleic acid sequence which is heterologous in relation to said transcription regulating nucleotide sequence.

2. The expression cassette of Claim 1, wherein the transcription regulating nucleotide sequence is selected from the group of sequences consisting of

    i) the sequences described by SEQ ID NOs: 1, 2, 3, 4, 5, 6, 9, 10, 11, 12, 13, 14, 15, 16, 19, 20, 21, 22, 23, 24, 25, 26, 27, 28, 29, 30, 33, 34, 35, 36, 37, 38, 39, 40, 43, 44, 45, 46, 47, 48, 49, 50, 53, 54, 55, 56, 57, and 58,
    ii) a fragment of at least 50 consecutive bases of a sequence under i) which has substantially the same promoter activity as the corresponding transcription regulating nucleotide sequence described by SEQ ID NO: 1, 2, 3, 4, 5, 6, 9, 10, 11, 12, 13, 14, 15, 16, 19, 20, 21, 22, 23, 24, 25, 26, 27, 28, 29, 30, 33, 34, 35, 36, 37, 38, 39, 40, 43, 44, 45, 46, 47, 48, 49, 50, 53, 54, 55, 56, 57, or 58;
    iii) a nucleotide sequence having substantial similarity with a sequence identity of at least 40% to a transcription regulating nucleotide sequence described by SEQ ID NO: 1, 2, 3, 4, 5, 6, 9, 10, 11, 12, 13, 14, 15, 16, 19, 20, 21, 22, 23, 24, 25, 26, 27, 28, 29, 30, 33, 34, 35, 36, 37, 38, 39, 40, 43, 44, 45, 46, 47, 48, 49, 50, 53, 54, 55, 56, 57, or 58;
    iv) a nucleotide sequence capable of hybridizing under conditions equivalent to hybridization in 7% sodium dodecyl sulfate (SDS), 0.5 M $NaPO_4$, 1 mM EDTA at 50°C with washing in 2 X SSC, 0. 1% SDS at 50°C to a transcription regulating nucleotide sequence described by SEQ ID NO: 1, 2, 3, 4, 5, 6, 9, 10, 11, 12, 13, 14, 15, 16, 19, 20, 21, 22, 23, 24, 25, 26, 27, 28, 29, 30, 33, 34, 35, 36, 37, 38, 39, 40, 43, 44, 45, 46, 47, 48, 49, 50, 53, 54, 55, 56, 57, or 58, or the complement thereof;
    v) a nucleotide sequence capable of hybridizing under conditions equivalent to hybridization in 7% sodium dodecyl sulfate (SDS), 0.5 M $NaPO_4$, 1 mM EDTA at 50°C with washing in 2 X SSC, 0. 1% SDS at 50°C to a nucleic acid comprising 50 to 200 or more consecutive nucleotides of a transcription regulating nucleotide sequence described by SEQ ID NO: 1, 2, 3, 4, 5, 6, 9, 10, 11, 12, 13, 14, 15, 16, 19, 20, 21, 22, 23, 24, 25, 26, 27, 28, 29, 30, 33, 34, 35, 36, 37, 38, 39, 40, 43, 44, 45, 46, 47, 48, 49, 50, 53, 54, 55, 56, 57, or 58, or the complement thereof;
    vi) a nucleotide sequence which is the complement or reverse complement of any of the previously mentioned nucleotide sequences under i) to v).

3. The expression cassette of Claim 1, wherein the functional equivalent of the transcription regulating nucleotide sequence is obtained or obtainable from plant genomic DNA from a gene encoding a polypeptide which has at least 70% amino acid sequence identity to a polypeptide selected from the group described by SEQ ID NO: 8, 18, 32, 42, 52, and 60, respectively.

4. The expression cassette of any of Claim 1 to 3, wherein expression of the nucleic acid sequence results in expression of a protein, or expression of a antisense RNA, sense or double-stranded RNA.

5. The expression cassette of any of Claim 1 to 4, wherein expression of the nucleic acid sequence confers to the plant an agronomically valuable trait.

6. A vector comprising an expression cassette of any of Claim 1 to 5.

7. A transgenic host cell or non-human organism comprising an expression cassette of any of Claim 1 to 5, or a vector of Claim 6.

8. A transgenic plant comprising the expression cassette of any of Claim 1 to 5, a vector of Claim 6, or a cell of claim 7..

9. A method for identifying and/or isolating a sequence with mesophyll- and/or epidermis-preferential or mesophyll- and/or epidermis-specific transcription regulating activity **characterized** that said identification and/or isolation utilizes a nucleic acid sequence encoding a amino acid sequence as described by SEQ ID NO: 8, 18, 32, 42, 52, or 60 or a part of at least 15 bases thereof.

**10.** The method of Claim 9, wherein the nucleic acid sequences is described by SEQ ID NO: 7, 17, 31, 41, 51, or 59, or a part of at least 15 bases thereof.

**11.** The method of Claim 9 or 10, wherein said identification and/or isolation is realized by a method selected from polymerase chain reaction, hybridization, and database screening.

**12.** A method for providing a transgenic expression cassette for mesophyll- and/or epidermis-preferential or mesophyll- and/or epidermis-specific expression comprising the steps of:

I. isolating of a mesophyll- and/or epidermis-preferential or mesophyll- and/or epidermis-specific transcription regulating nucleotide sequence utilizing at least one nucleic acid sequence or a part thereof, wherein said sequence is encoding a polypeptide described by SEQ ID NO: 8, 18, 32, 42, 52, or 60, or a part of at least 15 bases thereof, and
II. functionally linking said mesophyll- and/or epidermis-preferential or mesophyll- and/or epidermis-specific transcription regulating nucleotide sequence to another nucleotide sequence of interest, which is heterolog in relation to said mesophyll- and/or epidermis-preferential or mesophyll- and/or epidermis-specific transcription regulating nucleotide sequence.

SEQUENCE LISTING

<110> SunGene GmbH

<120> Expression cassettes for mesophyll- and/or epidermis-preferential expression in plants

<130> AE20040915 AE20040916 / PF 56110 (AT)

<160> 100

<170> PatentIn version 3.3

<210> 1
<211> 1068
<212> DNA
<213> Arabidopsis thaliana

<220>
<221> promoter
<222> (1)..(1068)
<223> transcription regulating sequence from Arabidopsis thaliana gene At5g13220

<400> 1
```
gaattcagca gccacatatg ctttaaacaa gagaacaaga aaaagagcgg ccacatgtga      60
aaaaaaaaca ccagccaaaa tgtgaaacta agaaaagaaa agaaaaacca tcgacatcgt     120
ccaaaggatc gacccgtctt ttctggtttg gcagaaaaca aacttccaag ttccgacttg     180
acctagtctt tatgcatctg cagtggtgtt ttctttaaat cttttttga atggatttga      240
aaatcttatt ttttcttctt ctttcaaaac actaactgtg aatcaccacc aacattttat     300
tgttctaaaa tccctatttg tatgttttat tggttacttt cccaaatatg atgaaagttt     360
attggtgtac tttagtttag aaatgtaaga aatttcagtt gtattattag gtcaaccaaa     420
tattattctt gaatagtaat gtcaaatttt gtttatattg aaaaatcaat aagactgata     480
tctactaaat gtgatttgct atactagtag agctagtagt agcaagatta aaattcatat     540
taagaaaaaa tggaaaagaa aaacatgttt cgagaaaaat atacagatca gagactagag     600
tagaaaacaa atataatgtc agaagaaccc aatgtgacga acagtaacag aaacatgtta     660
ccaaattttt gaagatatat ttttataaaa cattacaatg ttcattggac ttccgttatt     720
tccggatata aaatatcttc tttctgcgta gcaagcaatt acattacatt aggtatatta     780
agaatttaaa catgtgtttt gaatcattaa attaattaga taagtaagag aagaggttgg     840
gagcctaata aaaacaaaac aaaaaggaaa aagtggatag gttgccaatt gtgccgaaaa     900
gaaagagaaa gcatgaacct cgtgcgttaa ataagtatca gagacatact acattttaaa     960
aactctcaca tgagaaatca gaatccgtta ttattcctcc atttattcat ctcaaaaccc    1020
atatctctct gtcttgatct ctctctcact ttctaataag atcaaaga                 1068
```

<210> 2
<211> 1076
<212> DNA
<213> Arabidopsis thaliana

<220>
<221> promoter
<222> (1)..(1076)
<223> transcription regulating sequence from Arabidopsis thaliana gene At5g13220

<400> 2
```
tgttaggaaa cgagcagcca catatgcttt aaacaagaga acaagaaaaa gagcggccac     60
atgtgaaaaa aaaacaccag ccaaaatgtg aaactaagaa aagaaaagaa aaaccatcga    120
catcgtccaa aggatcgacc cgtcttttct ggtttggcag aaaacaaact tccaagttcc    180
gacttgacct agtctttatg catctgcagt ggtgttttct ttaaatcttt ttttgaatgg    240
atttgaaaat cttatttttt cttcttcttt caaaacacta actgtgaatc accaccaaca    300
ttttattgtt ctaaaatccc tatttgtatg ttttattggt tactttccca aatatgatga    360
aagtttattg gtgtacttta gtttagaaat gtaagaaatt tcagttgtat tattaggtca    420
accaaatatt attcttgaat agtaatgtca aattttgttt atattgaaaa atcaataaga    480
ctgatatcta ctaaatgtga tttgctatac tagtagagct agtagtagca agattaaaat    540
tcatattaag aaaaaatgga aaagaaaaac atgtttcgag aaaaatatac agatcagaga    600
ctagagtaga aaacaaatat aatgtcagaa gaacccaatg tgacgaacag taacagaaac    660
atgttaccaa atttttgaag atatattttt ataaaacatt acaatgttca ttggacttcc    720
gttatttccg gatataaaat atcttctttc tgcgtagcaa gcaattacat tacattaggt    780
atattaagaa tttaaacatg tgttttgaat cattaaatta attagataag taagagaaga    840
ggttgggagc ctaataaaaa caaaacaaaa aggaaaaagt ggataggttg ccaattgtgc    900
cgaaaagaaa gagaaagcat gaacctcgtg cgttaaataa gtatcagaga catactacat    960
tttaaaaact ctcacatgag aaatcagaat ccgttattat tcctccattt attcatctca   1020
aaacccatat ctctctgtct tgatctctct ctcactttct aataagatca aagaag       1076
```

<210> 3
<211> 986
<212> DNA
<213> Arabidopsis thaliana

<220>
<221> promoter
<222> (1)..(986)
<223> transcription regulating sequence from Arabidopsis thaliana gene At5g13220

<400> 3
```
gaattcagca gccacatatg ctttaaacaa gagaacaaga aaaagagcgg ccacatgtga     60
aaaaaaaaca ccagccaaaa tgtgaaacta agaaaagaaa agaaaaacca tcgacatcgt    120
ccaaaggatc gacccgtctt ttctggtttg gcagaaaaca aacttccaag ttccgacttg    180
acctagtctt tatgcatctg cagtggtgtt ttctttaaat cttttttga atggatttga    240
aaatcttatt ttttcttctt ctttcaaaac actaactgtg aatcaccacc aacattttat    300
tgttctaaaa tccctatttg tatgttttat tggttacttt cccaaatatg atgaaagttt    360
attggtgtac tttagtttag aaatgtaaga aatttcagtt gtattattag gtcaaccaaa    420
```

```
tattattctt gaatagtaat gtcaaatttt gtttatattg aaaaatcaat aagactgata    480
tctactaaat gtgatttgct atactagtag agctagtagt agcaagatta aaattcatat    540
taagaaaaaa tggaaaagaa aaacatgttt cgagaaaaat atacagatca gagactagag    600
tagaaaacaa atataatgtc agaagaaccc aatgtgacga acagtaacag aaacatgtta    660
ccaaattttt gaagatatat ttttataaaa cattacaatg ttcattggac ttccgttatt    720
tccggatata aaatatcttc tttctgcgta gcaagcaatt acattacatt aggtatatta    780
agaatttaaa catgtgtttt gaatcattaa attaattaga taagtaagag aagaggttgg    840
gagcctaata aaaacaaaac aaaaaggaaa aagtggatag gttgccaatt gtgccgaaaa    900
gaaagagaaa gcatgaacct cgtgcgttaa ataagtatca gagacatact acattttaaa    960
aactctcaca tgagaaatca gaatcc                                         986
```

<210> 4
<211> 992
<212> DNA
<213> Arabidopsis thaliana

<220>
<221> promoter
<222> (1)..(992)
<223> transcription regulating sequence from Arabidopsis thaliana gene At5g13220

<400> 4

```
tgttaggaaa cgagcagcca catatgcttt aaacaagaga acaagaaaaa gagcggccac     60
atgtgaaaaa aaaacaccag ccaaaatgtg aaactaagaa aagaaaagaa aaaccatcga    120
catcgtccaa aggatcgacc cgtcttttct ggtttggcag aaaacaaact tccaagttcc    180
gacttgacct agtctttatg catctgcagt ggtgttttct ttaaatcttt ttttgaatgg    240
atttgaaaat cttatttttt cttcttcttt caaaacacta actgtgaatc accaccaaca    300
ttttattgtt ctaaaatccc tatttgtatg ttttattggt tactttccca aatatgatga    360
aagtttattg gtgtacttta gtttagaaat gtaagaaatt tcagttgtat tattaggtca    420
accaaatatt attcttgaat agtaatgtca aattttgttt atattgaaaa atcaataaga    480
ctgatatcta ctaaatgtga tttgctatac tagtagagct agtagtagca agattaaaat    540
tcatattaag aaaaaatgga aaagaaaaac atgtttcgag aaaaatatac agatcagaga    600
ctagagtaga aaacaaatat aatgtcagaa gaacccaatg tgacgaacag taacagaaac    660
atgttaccaa atttttgaag atatattttt ataaaacatt acaatgttca ttggacttcc    720
gttatttccg gatataaaat atcttctttc tgcgtagcaa gcaattacat tacattaggt    780
atattaagaa tttaaacatg tgttttgaat cattaaatta attagataag taagagaaga    840
ggttgggagc ctaataaaaa caaaacaaaa aggaaaaagt ggataggttg ccaattgtgc    900
cgaaaagaaa gagaaagcat gaacctcgtg cgttaaataa gtatcagaga catactacat    960
tttaaaaact ctcacatgag aaatcagaat cc                                 992
```

<210> 5
<211> 2173
<212> DNA
<213> Arabidopsis thaliana

<220>
<221> promoter
<222> (1)..(2173)
<223> transcription regulating sequence from Arabidopsis thaliana gene At5g13220

<400> 5

```
cgtttagaat tcagaatccg acgactttgg cgagcaaacc ttacgcaaac tatggatggt      60
attggaaatg aacgacaaca agaacaaccg tttcatttac agatttactt cttcaaatgg     120
gcttttcttc tctaaatggg tttactacct taaggggctt ttcaactata ggttcaatta     180
tattcttatg taagggtact aactagtgtg ggctgtaaac gacttaaagt ttcggccatt     240
tagttttatg caagaaaaaa aatatagata atacagtaat gatgaatcca ctatacatgt     300
taaatccaat ataataaatc cactatacat tagtgttctc atatttcgtt tcaagattcg     360
attcaaaaaa tttgaaaatg ttaaatatgt gtttctgaga ttaaaggcca aaagaagggt     420
aaggtcgtat atgaaaggtg gacagagggg caaattagtc agcttgaaaa atacatcgac     480
attaaaaatc ggcagcactg acaaaacgtg tgataacaca aataaaaata atcggaagaa     540
aaagaataaa taaggcgtat tgactctgga acgtgagaag atgaaagaga gcgacgaaaa     600
cctctaaaga cttatcgaga agtgcacctt agcctagtga ataatgatat gtattgacat     660
taactactac tactacagct cccaacacgt gaaagacatg catctaactc tttcactttc     720
ggttgccaag aaaaaaaaaa aaaaaaaact ttcactttcg gcatcaaata tatactacta     780
aaaaagtttt ttatacgtaa ttcttttta atatttgatt agttcagaat attaattaaa     840
aaaattactt ttcgtaatca ctacattttg tagttatatg atttttaatt taaaatagta     900
aaaaggagaa atgtaactaa ttaagctaac atgcgcgcga tagaataagg tgaagtttaa     960
atttgagcac aagatagaaa attgaggtca atttaattcg gaagtggttc aaggtatatt    1020
ttcacaagaa agaaaaacgt gagttcattg aatccctcta taactttact tgagaacaat    1080
gtaataatat gtaaatctgt taggaaacga gcagccacat atgctttaaa caagagaaca    1140
agaaaaagag cggccacatg tgaaaaaaaa acaccagcca aaatgtgaaa ctaagaaaag    1200
aaaagaaaaa ccatcgacat cgtccaaagg atcgacccgt cttttctggt ttggcagaaa    1260
acaaacttcc aagttccgac ttgacctagt ctttatgcat ctgcagtggt gttttcttta    1320
aatctttttt tgaatggatt tgaaaatctt attttttctt cttctttcaa aacactaact    1380
gtgaatcacc accaacattt tattgttcta aaatccctat ttgtatgttt tattggttac    1440
tttcccaaat atgatgaaag tttattggtg tactttagtt tagaaatgta agaaatttca    1500
gttgtattat taggtcaacc aaatattatt cttgaatagt aatgtcaaat tttgtttata    1560
ttgaaaaatc aataagactg atatctacta aatgtgattt gctatactag tagagctagt    1620
agtagcaaga ttaaaattca tattaagaaa aaatggaaaa gaaaaacatg tttcgagaaa    1680
aatatacaga tcagagacta gagtagaaaa caaatataat gtcagaagaa cccaatgtga    1740
cgaacagtaa cagaaacatg ttaccaaatt tttgaagata tatttttata aaacattaca    1800
atgttcattg gacttccgtt atttccggat ataaaatatc ttctttctgc gtagcaagca    1860
attacattac attaggtata ttaagaattt aaacatgtgt tttgaatcat taaattaatt    1920
agataagtaa gagaagaggt tgggagccta ataaaaacaa aacaaaaagg aaaaagtgga    1980
taggttgcca attgtgccga aaagaaagag aaagcatgaa cctcgtgcgt taaataagta    2040
tcagagacat actacatttt aaaaactctc acatgagaaa tcagaatccg ttattattcc    2100
tccatttatt catctcaaaa cccatatctc tctgtcttga tctctctctc actttctaat    2160
aagatcaaag aag                                                       2173
```

<210> 6
<211> 2089
<212> DNA
<213> Arabidopsis thaliana

<220>
<221> promoter
<222> (1)..(2089)
<223> transcription regulating sequence from Arabidopsis thaliana gene At5g13220

<400> 6
```
cgtttagaat tcagaatccg acgactttgg cgagcaaacc ttacgcaaac tatggatggt     60
attggaaatg aacgacaaca agaacaaccg tttcatttac agatttactt cttcaaatgg    120
gcttttcttc tctaaatggg tttactacct taaggggctt ttcaactata ggttcaatta    180
tattcttatg taagggtact aactagtgtg ggctgtaaac gacttaaagt ttcggccatt    240
tagttttatg caagaaaaaa aatatagata atacagtaat gatgaatcca ctatacatgt    300
taaatccaat ataataaatc cactatacat tagtgttctc atatttcgtt tcaagattcg    360
attcaaaaaa tttgaaaatg ttaaatatgt gtttctgaga ttaaaggcca aaagaagggt    420
aaggtcgtat atgaaaggtg gacagagggg caaattagtc agcttgaaaa atacatcgac    480
attaaaaatc ggcagcactg acaaaacgtg tgataacaca aataaaaata atcggaagaa    540
aaagaataaa taaggcgtat tgactctgga acgtgagaag atgaaagaga gcgacgaaaa    600
cctctaaaga cttatcgaga agtgcacctt agcctagtga ataatgatat gtattgacat    660
taactactac tactacagct cccaacacgt gaaagacatg catctaactc tttcactttc    720
ggttgccaag aaaaaaaaaa aaaaaaaact ttcactttcg gcatcaaata tatactacta    780
aaaaagtttt ttatacgtaa ttctttttta atatttgatt agttcagaat attaattaaa    840
aaaattactt ttcgtaatca ctacattttg tagttatatg atttttaatt taaaatagta    900
aaaaggagaa atgtaactaa ttaagctaac atgcgcgcga tagaataagg tgaagtttaa    960
atttgagcac aagatagaaa attgaggtca atttaattcg gaagtggttc aaggtatatt   1020
ttcacaagaa agaaaaacgt gagttcattg aatccctcta taactttact tgagaacaat   1080
gtaataatat gtaaatctgt taggaaacga gcagccacat atgctttaaa caagagaaca   1140
agaaaaagag cggccacatg tgaaaaaaaa acaccagcca aaatgtgaaa ctaagaaaag   1200
aaaagaaaaa ccatcgacat cgtccaaagg atcgacccgt cttttctggt ttggcagaaa   1260
acaaacttcc aagttccgac ttgacctagt ctttatgcat ctgcagtggt gttttcttta   1320
aatctttttt tgaatggatt tgaaaatctt atttttttctt cttctttcaa aacactaact   1380
gtgaatcacc accaacattt tattgttcta aaatccctat ttgtatgttt tattggttac   1440
tttcccaaat atgatgaaag tttattggtg tactttagtt tagaaatgta agaaatttca   1500
gttgtattat taggtcaacc aaatattatt cttgaatagt aatgtcaaat tttgtttata   1560
ttgaaaaatc aataagactg atatctacta aatgtgattt gctatactag tagagctagt   1620
agtagcaaga ttaaaattca tattaagaaa aaatggaaaa gaaaaacatg tttcgagaaa   1680
aatatacaga tcagagacta gagtagaaaa caaatataat gtcagaagaa cccaatgtga   1740
cgaacagtaa cagaaacatg ttaccaaatt tttgaagata tatttttata aaacattaca   1800
atgttcattg gacttccgtt atttccggat ataaaatatc ttctttctgc gtagcaagca   1860
attacattac attaggtata ttaagaattt aaacatgtgt tttgaatcat taaattaatt   1920
```

```
agataagtaa gagaagaggt tgggagccta ataaaaacaa aacaaaaagg aaaaagtgga   1980
taggttgcca attgtgccga aaagaaagag aaagcatgaa cctcgtgcgt taaataagta   2040
tcagagacat actacatttt aaaaactctc acatgagaaa tcagaatcc               2089

<210>  7
<211>  1346
<212>  DNA
<213>  Arabidopsis thaliana

<220>
<221>  CDS
<222>  (85)..(642)
<223>  encoding expressed protein

<400>  7
gttattattc ctccatttat tcatctcaaa acccatatct ctctgtcttg atctctctct     60
cactttctaa taagatcaaa gaag atg tcg aaa gct acc ata gaa ctc gat       111
                           Met Ser Lys Ala Thr Ile Glu Leu Asp
                           1               5
ttc ctc gga ctt gag aag aaa caa acc aac aac gct cct aag cct aag      159
Phe Leu Gly Leu Glu Lys Lys Gln Thr Asn Asn Ala Pro Lys Pro Lys
10                  15                  20                  25
ttc cag aaa ttt ctc gat cgc cgt cgt agt ttc cga gat att caa ggt      207
Phe Gln Lys Phe Leu Asp Arg Arg Arg Ser Phe Arg Asp Ile Gln Gly
                30                  35                  40
gcg att tcg aaa atc gat ccg gag att atc aaa tcg ctg tta gct tcc      255
Ala Ile Ser Lys Ile Asp Pro Glu Ile Ile Lys Ser Leu Leu Ala Ser
            45                  50                  55
act gga aac aat tcc gat tca tcg gct aaa tct cgt tcg gtt ccg tct      303
Thr Gly Asn Asn Ser Asp Ser Ser Ala Lys Ser Arg Ser Val Pro Ser
        60                  65                  70
act ccg agg gaa gat cag cct cag atc ccg att tct ccg gtc cac gcg      351
Thr Pro Arg Glu Asp Gln Pro Gln Ile Pro Ile Ser Pro Val His Ala
    75                  80                  85
tct ctc gcc agg tct agt acc gaa ctc gtt tcg gga act gtt cct atg      399
Ser Leu Ala Arg Ser Ser Thr Glu Leu Val Ser Gly Thr Val Pro Met
90                  95                  100                 105
acg att ttc tac aat gga agt gtt tca gtt ttc caa gtg tct cgt aac      447
Thr Ile Phe Tyr Asn Gly Ser Val Ser Val Phe Gln Val Ser Arg Asn
                110                 115                 120
aaa gct ggt gaa att atg aag gtc gct aat gaa gca gca tct aag aaa      495
Lys Ala Gly Glu Ile Met Lys Val Ala Asn Glu Ala Ala Ser Lys Lys
            125                 130                 135
gac gag tcg tcg atg gag aca gat ctt tcg gta att ctt ccg acc act      543
Asp Glu Ser Ser Met Glu Thr Asp Leu Ser Val Ile Leu Pro Thr Thr
```

```
        140                  145                  150
cta aga cca aag ctc ttt ggc cag aat cta gaa gga gat ctt ccc atc        591
Leu Arg Pro Lys Leu Phe Gly Gln Asn Leu Glu Gly Asp Leu Pro Ile
    155                  160                  165
gca agg aga aag tca ctg caa cgt ttt ctc gag aag cgc aag gag agg        639
Ala Arg Arg Lys Ser Leu Gln Arg Phe Leu Glu Lys Arg Lys Glu Arg
170                 175                  180                  185
taa tgattcttca acaatccaag gatttttacc cccaaataat taaagaaagg             692
tttttatttt tctctctctc gaccttttt ttactataag ttatttaaga tagtaattat       752
gggtcctgcc tcttttactc tcacatacaa cttaagattc aactagtttt gttcaacaac      812
gcacatgctt atacgtagat agataatgga gatcagtagt aatatcggta tacgtaggtt      872
actattgtaa tggaactttt aaaaagcgcg ttgactttga gtctttgact ctagttctgt      932
ttgctacacc gacaagttat atttttcaaa atgatgagaa aacgaggaga aacaccggaa      992
aaaaatttga acttttactt ttatcagacc atacggccaa agaaagatct gtatattata     1052
taagttatca caaaacgcgg tttcacattt tctttttcgt cttgttgtgt ttgcagatta     1112
gtatcaacat ctccttacta tccgacatcg gcctaaacga tctcttttta gattgggaca     1172
tggaccaaat ttgtcttttt caatcggaag acatccatgt tcgtttttgg atttggctta     1232
tttccaatct tcttttgaag ccttcttcgt cgttgctaaa tcgtatacta ttcacgacaa     1292
acgtttttag gagattacgt tacctactaa gattatatat attggtttgt tttt           1346
```

<210> 8
<211> 185
<212> PRT
<213> Arabidopsis thaliana

<400> 8

```
Met Ser Lys Ala Thr Ile Glu Leu Asp Phe Leu Gly Leu Glu Lys Lys
1               5                   10                  15

Gln Thr Asn Asn Ala Pro Lys Pro Lys Phe Gln Lys Phe Leu Asp Arg
            20                  25                  30

Arg Arg Ser Phe Arg Asp Ile Gln Gly Ala Ile Ser Lys Ile Asp Pro
        35                  40                  45

Glu Ile Ile Lys Ser Leu Leu Ala Ser Thr Gly Asn Asn Ser Asp Ser
    50                  55                  60

Ser Ala Lys Ser Arg Ser Val Pro Ser Thr Pro Arg Glu Asp Gln Pro
65                  70                  75                  80

Gln Ile Pro Ile Ser Pro Val His Ala Ser Leu Ala Arg Ser Ser Thr
                85                  90                  95

Glu Leu Val Ser Gly Thr Val Pro Met Thr Ile Phe Tyr Asn Gly Ser
```

```
            100                 105                 110

Val Ser Val Phe Gln Val Ser Arg Asn Lys Ala Gly Glu Ile Met Lys
        115                 120                 125

Val Ala Asn Glu Ala Ala Ser Lys Lys Asp Glu Ser Ser Met Glu Thr
    130                 135                 140

Asp Leu Ser Val Ile Leu Pro Thr Thr Leu Arg Pro Lys Leu Phe Gly
145                 150                 155                 160

Gln Asn Leu Glu Gly Asp Leu Pro Ile Ala Arg Arg Lys Ser Leu Gln
                165                 170                 175

Arg Phe Leu Glu Lys Arg Lys Glu Arg
            180                 185

<210>  9
<211>  1289
<212>  DNA
<213>  Arabidopsis thaliana

<220>
<221>  promoter
<222>  (1)..(1289)
<223>  transcription regulating sequence from Arabidopsis thaliana gene
       At1g68850

<400>  9
ctcgaggctt ggagatgtta ttagtttaaa aaactctatt aaggtgcata agtcaggttc      60
aaatctaaga ggtcaacaca atcctctacc attataccaa aaccacctaa cactagtttt     120
tttcaactaa tgcatgtagc cctgccaata tataaccttg tttttaaccg tttttaagac     180
tgaaaatatt cttaaaataa taacaagttg acgacaaata aataattcaa attttagcga     240
agatcgcgtt gttactattt taataaatat tggactttca tttttttatg taatcgtata     300
attcaagatg ccgccaaaga gtggatctta ttttccctcc tactttttca tctagatttt     360
tcgtcatagg aagaaagaac agagaaagag aagaacactt agcaaaatta tttccaagga     420
aatgatataa ttaagctccc gctacaccta cacaaagttt acgaggttga gagtatttaa     480
taaaatttat aaagctcgac ttaaataact caaaaagagt tgggaaccta tttgcgacct     540
atatatctca ctaattactc atgatattat tcttaacaat agacagattt agttccaaac     600
taactacgga aacattgaaa aagaagttta gttgatttat ctaacatgca tgaaataata     660
tggacaagga tttgtacttt gtagttgcca tggtcgtgtc aacaacatag ttcaaatcct     720
attgagacta tgcaaattca tttctggaac tacattatat gtattctttt ttatccgaaa     780
aaaacactaa atatttctta ggtgaacaaa aaaaagagaa gtagatagtt ccggagcaga     840
acaaaacagt tgaacgaatt acaaaagaac gagaattcta gatttgtttc aagaatcagt     900
tttgatgtgc ttgcaacttt tgcaaacata caaacaaata tatctgcctt aataacaaaa     960
```

gtggaatatg aatcggaaaa aatagtacac ttttatggta atataataat ttgggttggt 1020
aataatctgc actagaattc aaataaattt tcatctagtt acctttgggt cataggagaa 1080
cccaacaacc atctcacaaa attagttgca tctcacttga cctacttctc aagacccttg 1140
taacacaaga aaactaaaaa ctctacaatg ccctcttctc aaaaaattat atatctctgg 1200
gaagtctcaa ccatcttctc taactcctat ctcaacacac atattttccc aaaatttgga 1260
tcaaaaagtt ttccgataga agaaattaa 1289

<210> 10
<211> 1297
<212> DNA
<213> Arabidopsis thaliana

<220>
<221> promoter
<222> (1)..(1297)
<223> transcription regulating sequence from Arabidopsis thaliana gene At1g68850

<400> 10
cacattctcg aggcttggag atgttattag tttaaaaaac tctattaagg tgcataagtc 60
aggttcaaat ctaagaggtc aacacaatcc tctaccatta taccaaaacc acctaacact 120
agttttttc aactaatgca tgtagccctg ccaatatata accttgtttt taaccgtttt 180
taagactgaa aatattctta aaataataac aagttgacga caaataaata attcaaattt 240
tagcgaagat cgcgttgtta ctattttaat aaatattgga ctttcatttt tttatgtaat 300
cgtataattc aagatgccgc caaagagtgg atcttatttt ccctcctact ttttcatcta 360
gatttttcgt cataggaaga aagaacagag aaagagaaga acacttagca aaattatttc 420
caaggaaatg atataattaa gctcccgcta cacctacaca aagtttacga ggttgagagt 480
atttaataaa atttataaag ctcgacttaa ataactcaaa aagagttggg aacctatttg 540
cgacctatat atctcactaa ttactcatga tattattctt aacaatagac agatttagtt 600
ccaaactaac tacggaaaca ttgaaaaaga agtttagttg atttatctaa catgcatgaa 660
ataatatgga caaggatttg tactttgtag ttgccatggt cgtgtcaaca acatagttca 720
aatcctattg agactatgca aattcatttc tggaactaca ttatatgtat tctttttat 780
ccgaaaaaaa cactaaatat ttcttaggtg aacaaaaaaa agagaagtag atagttccgg 840
agcagaacaa aacagttgaa cgaattacaa aagaacgaga attctagatt tgtttcaaga 900
atcagttttg atgtgcttgc aacttttgca aacatacaaa caaatatatc tgccttaata 960
acaaaagtgg aatatgaatc ggaaaaaata gtacactttt atggtaatat aataatttgg 1020
gttggtaata atctgcacta gaattcaaat aaattttcat ctagttacct ttgggtcata 1080
ggagaaccca acaaccatct cacaaaatta gttgcatctc acttgaccta cttctcaaga 1140
cccttgtaac acaagaaaac taaaaactct acaatgccct cttctcaaaa aattatatat 1200
ctctgggaag tctcaaccat cttctctaac tcctatctca acacacatat tttcccaaaa 1260
tttggatcaa aaagttttcc gatagaagaa attaaat 1297

<210> 11
<211> 1235
<212> DNA

<213> Arabidopsis thaliana

<220>
<221> promoter
<222> (1)..(1235)
<223> transcription regulating sequence from Arabidopsis thaliana gene At1g68850

<400> 11
ctcgaggctt ggagatgtta ttagtttaaa aaactctatt aaggtgcata agtcaggttc 60
aaatctaaga ggtcaacaca atcctctacc attataccaa aaccacctaa cactagtttt 120
tttcaactaa tgcatgtagc cctgccaata tataaccttg tttttaaccg tttttaagac 180
tgaaaatatt cttaaaataa taacaagttg acgacaaata aataattcaa attttagcga 240
agatcgcgtt gttactattt taataaatat tggactttca ttttttttatg taatcgtata 300
attcaagatg ccgccaaaga gtggatctta ttttccctcc tactttttca tctagatttt 360
tcgtcatagg aagaaagaac agagaaagag aagaacactt agcaaaatta tttccaagga 420
aatgatataa ttaagctccc gctacaccta cacaaagttt acgaggttga gagtatttaa 480
taaaatttat aaagctcgac ttaaataact caaaaagagt tgggaaccta tttgcgacct 540
atatatctca ctaattactc atgatattat tcttaacaat agacagattt agttccaaac 600
taactacgga aacattgaaa aagaagttta gttgatttat ctaacatgca tgaaataata 660
tggacaagga tttgtacttt gtagttgcca tggtcgtgtc aacaacatag ttcaaatcct 720
attgagacta tgcaaattca tttctggaac tacattatat gtattctttt ttatccgaaa 780
aaaacactaa atatttctta ggtgaacaaa aaaaagagaa gtagatagtt ccggagcaga 840
acaaaacagt tgaacgaatt acaaaagaac gagaattcta gatttgtttc aagaatcagt 900
tttgatgtgc ttgcaacttt tgcaaacata caaacaaata tatctgcctt aataacaaaa 960
gtggaatatg aatcggaaaa aatagtacac ttttatggta atataataat ttgggttggt 1020
aataatctgc actagaattc aaataaattt tcatctagtt acctttgggt cataggagaa 1080
cccaacaacc atctcacaaa attagttgca tctcacttga cctacttctc aagacccttg 1140
taacacaaga aaactaaaaa ctctacaatg ccctcttctc aaaaaattat atatctctgg 1200
gaagtctcaa ccatcttctc taactcctat ctcaa 1235

<210> 12
<211> 1241
<212> DNA
<213> Arabidopsis thaliana

<220>
<221> promoter
<222> (1)..(1241)
<223> transcription regulating sequence from Arabidopsis thaliana gene At1g68850

<400> 12
cacattctcg aggcttggag atgttattag tttaaaaaac tctattaagg tgcataagtc 60
aggttcaaat ctaagaggtc aacacaatcc tctaccatta taccaaaacc acctaacact 120

agttttttc aactaatgca tgtagccctg ccaatatata accttgtttt taaccgtttt 180
taagactgaa aatattctta aaataataac aagttgacga caaataaata attcaaattt 240
tagcgaagat cgcgttgtta ctattttaat aaatattgga ctttcatttt tttatgtaat 300
cgtataattc aagatgccgc caaagagtgg atcttatttt ccctcctact ttttcatcta 360
gatttttcgt cataggaaga aagaacagag aaagagaaga acacttagca aaattatttc 420
caaggaaatg atataattaa gctcccgcta cacctacaca aagtttacga ggttgagagt 480
atttaataaa atttataaag ctcgacttaa ataactcaaa aagagttggg aacctatttg 540
cgacctatat atctcactaa ttactcatga tattattctt aacaatagac agatttagtt 600
ccaaactaac tacggaaaca ttgaaaaaga agtttagttg atttatctaa catgcatgaa 660
ataatatgga caaggatttg tactttgtag ttgccatggt cgtgtcaaca acatagttca 720
aatcctattg agactatgca aattcatttc tggaactaca ttatatgtat tcttttttat 780
ccgaaaaaaa cactaaatat ttcttaggtg aacaaaaaaa agagaagtag atagttccgg 840
agcagaacaa aacagttgaa cgaattacaa aagaacgaga attctagatt tgtttcaaga 900
atcagttttg atgtgcttgc aacttttgca aacatacaaa caaatatatc tgccttaata 960
acaaaagtgg aatatgaatc ggaaaaaata gtacactttt atggtaatat aataatttgg 1020
gttggtaata atctgcacta gaattcaaat aaattttcat ctagttacct ttgggtcata 1080
ggagaaccca acaaccatct cacaaaatta gttgcatctc acttgaccta cttctcaaga 1140
cccttgtaac acaagaaaac taaaaactct acaatgccct cttctcaaaa aattatatat 1200
ctctgggaag tctcaaccat cttctctaac tcctatctca a 1241

<210> 13
<211> 2056
<212> DNA
<213> Arabidopsis thaliana

<220>
<221> promoter
<222> (1)..(2056)
<223> transcription regulating sequence from Arabidopsis thaliana gene At1g68850

<400> 13
tttttttgg tttccaaaaa gtttcaataa ttgaatacat tttattggtt gcagaattct 60
ctgctgttac tgttttgtat tttctagcat ttattttaca attttgttcc taaccttcta 120
acatagcaaa ttagcaatca taaactttta tgcatatata taagtgtaag acagtaaatc 180
ttgtatgtgg aaagacattt tccttcaaaa tataagttca taaacgtcaa gtcaattatg 240
gtcgactctc tttagagtac atactacata gattagattc agtcggattt aatgtgtact 300
taacttttta acttgcaggt cttgcatgac aagagtcgtg tcacaagacc attaaagtca 360
ctgaagtttg tgtttatggt gcacattgac atcatgtaga tggtattttg atgtaaacga 420
atgatatatt tgtaatatta caacttcaaa ggttacatct aaattttgag acttcgatga 480
tttgtttttt cttcgaatca ctcctgcgac tatgcatcag ccaaatccca aatgtgtgtc 540
aacatctcga aagcacaatc ctcattcgtt ttttactgag tgtgtgtctt tcccaagcaa 600
tatattcaaa aaaagtagaa agtaaatggc ccatgcaggt ttcgaacctg cgaccttcgc 660
gttattagca cgacgctcta accaactgag ctaatgggcc attttgcaat gacttgtttt 720
cttctaataa tactattttc aaacttgtga ttgcacagtt gcacattctc gaggcttgga 780

```
gatgttatta gtttaaaaaa ctctattaag gtgcataagt caggttcaaa tctaagaggt     840
caacacaatc ctctaccatt ataccaaaac cacctaacac tagtttttt caactaatgc      900
atgtagccct gccaatatat aaccttgttt ttaaccgttt ttaagactga aaatattctt     960
aaaataataa caagttgacg acaaataaat aattcaaatt ttagcgaaga tcgcgttgtt    1020
actattttaa taaatattgg actttcattt ttttatgtaa tcgtataatt caagatgccg    1080
ccaaagagtg gatcttattt tccctcctac tttttcatct agatttttcg tcataggaag    1140
aaagaacaga gaaagagaag aacacttagc aaaattattt ccaaggaaat gatataatta    1200
agctcccgct acacctacac aaagtttacg aggttgagag tatttaataa aatttataaa    1260
gctcgactta aataactcaa aaagagttgg gaacctattt gcgacctata tatctcacta    1320
attactcatg atattattct taacaataga cagatttagt tccaaactaa ctacggaaac    1380
attgaaaaag aagtttagtt gatttatcta acatgcatga aataatatgg acaaggattt    1440
gtactttgta gttgccatgg tcgtgtcaac aacatagttc aaatcctatt gagactatgc    1500
aaattcattt ctggaactac attatatgta ttcttttta tccgaaaaaa acactaaata    1560
tttcttaggt gaacaaaaaa aagagaagta gatagttccg gagcagaaca aaacagttga    1620
acgaattaca aaagaacgag aattctagat ttgtttcaag aatcagtttt gatgtgcttg    1680
caacttttgc aaacatacaa acaaatatat ctgccttaat aacaaaagtg gaatatgaat    1740
cggaaaaaat agtacacttt tatggtaata taataatttg ggttggtaat aatctgcact    1800
agaattcaaa taaattttca tctagttacc tttgggtcat aggagaaccc aacaaccatc    1860
tcacaaaatt agttgcatct cacttgacct acttctcaag acccttgtaa cacaagaaaa    1920
ctaaaaactc tacaatgccc tcttctcaaa aaattatata tctctgggaa gtctcaacca    1980
tcttctctaa ctcctatctc aacacacata ttttcccaaa atttggatca aaaagttttc    2040
cgatagaaga aattaa                                                    2056
```

<210> 14
<211> 2080
<212> DNA
<213> Arabidopsis thaliana

<220>
<221> promoter
<222> (1)..(2080)
<223> transcription regulating sequence from Arabidopsis thaliana gene At1g68850

<400> 14

```
tctcgtccca cttttttttt ggtttccaaa aagtttcaat aattgaatac attttattgg      60
ttgcagaatt ctctgctgtt actgttttgt attttctagc atttatttta caattttgtt     120
cctaaccttc taacatagca aattagcaat cataaacttt tatgcatata tataagtgta     180
agacagtaaa tcttgtatgt ggaaagacat tttccttcaa aatataagtt cataaacgtc     240
aagtcaatta tggtcgactc tctttagagt acatactaca tagattagat tcagtcggat     300
ttaatgtgta cttaactttt taacttgcag gtcttgcatg acaagagtcg tgtcacaaga     360
ccattaaagt cactgaagtt tgtgtttatg gtgcacattg acatcatgta gatggtattt     420
tgatgtaaac gaatgatata tttgtaatat tacaacttca aaggttacat ctaaattttg     480
agacttcgat gatttgtttt ttcttcgaat cactcctgcg actatgcatc agccaaatcc     540
caaatgtgtg tcaacatctc gaaagcacaa tcctcattcg ttttttactg agtgtgtgtc     600
```

tttcccaagc aatatattca aaaaaagtag aaagtaaatg gcccatgcag gtttcgaacc 660
tgcgaccttc gcgttattag cacgacgctc taaccaactg agctaatggg ccattttgca 720
atgacttgtt ttcttctaat aatactattt tcaaacttgt gattgcacag ttgcacattc 780
tcgaggcttg gagatgttat tagtttaaaa aactctatta aggtgcataa gtcaggttca 840
aatctaagag gtcaacacaa tcctctacca ttataccaaa accacctaac actagttttt 900
ttcaactaat gcatgtagcc ctgccaatat ataaccttgt ttttaaccgt ttttaagact 960
gaaaatattc ttaaaataat aacaagttga cgacaaataa ataattcaaa ttttagcgaa 1020
gatcgcgttg ttactatttt aataaatatt ggactttcat ttttttatgt aatcgtataa 1080
ttcaagatgc cgccaaagag tggatcttat tttccctcct actttttcat ctagattttt 1140
cgtcatagga agaaagaaca gagaaagaga agaacactta gcaaaattat ttccaaggaa 1200
atgatataat taagctcccg ctacacctac acaaagttta cgaggttgag agtatttaat 1260
aaaatttata aagctcgact taaataactc aaaaagagtt gggaacctat ttgcgaccta 1320
tatatctcac taattactca tgatattatt cttaacaata gacagattta gttccaaact 1380
aactacggaa acattgaaaa agaagtttag ttgatttatc taacatgcat gaaataatat 1440
ggacaaggat ttgtactttg tagttgccat ggtcgtgtca acaacatagt tcaaatccta 1500
ttgagactat gcaaattcat ttctggaact acattatatg tattcttttt tatccgaaaa 1560
aaacactaaa tatttcttag gtgaacaaaa aaaagagaag tagatagttc cggagcagaa 1620
caaaacagtt gaacgaatta caaaagaacg agaattctag atttgtttca agaatcagtt 1680
ttgatgtgct tgcaactttt gcaaacatac aaacaaatat atctgcctta ataacaaaag 1740
tggaatatga atcggaaaaa atagtacact tttatggtaa tataataatt tgggttggta 1800
ataatctgca ctagaattca aataaatttt catctagtta cctttgggtc ataggagaac 1860
ccaacaacca tctcacaaaa ttagttgcat ctcacttgac ctacttctca agacccttgt 1920
aacacaagaa aactaaaaac tctacaatgc cctcttctca aaaaattata tatctctggg 1980
aagtctcaac catcttctct aactcctatc tcaacacaca tattttccca aaatttggat 2040
caaaaagttt tccgatagaa gaaattaaat atgatgagac 2080

<210> 15
<211> 2002
<212> DNA
<213> Arabidopsis thaliana

<220>
<221> promoter
<222> (1)..(2002)
<223> transcription regulating sequence from Arabidopsis thaliana gene At1g68850

<400> 15
ttttttttgg tttccaaaaa gtttcaataa ttgaatacat tttattggtt gcagaattct 60
ctgctgttac tgttttgtat tttctagcat ttattttaca attttgttcc taaccttcta 120
acatagcaaa ttagcaatca taaactttta tgcatatata taagtgtaag acagtaaatc 180
ttgtatgtgg aaagacattt tccttcaaaa tataagttca taaacgtcaa gtcaattatg 240
gtcgactctc tttagagtac atactacata gattagattc agtcggattt aatgtgtact 300
taacttttta acttgcaggt cttgcatgac aagagtcgtg tcacaagacc attaaagtca 360
ctgaagtttg tgtttatggt gcacattgac atcatgtaga tggtattttg atgtaaacga 420

```
atgatatatt tgtaatatta caacttcaaa ggttacatct aaattttgag acttcgatga    480
tttgtttttt cttcgaatca ctcctgcgac tatgcatcag ccaaatccca aatgtgtgtc    540
aacatctcga aagcacaatc ctcattcgtt ttttactgag tgtgtgtctt tcccaagcaa    600
tatattcaaa aaaagtagaa agtaaatggc ccatgcaggt ttcgaacctg cgaccttcgc    660
gttattagca cgacgctcta accaactgag ctaatgggcc attttgcaat gacttgtttt    720
cttctaataa tactattttc aaacttgtga ttgcacagtt gcacattctc gaggcttgga    780
gatgttatta gtttaaaaaa ctctattaag gtgcataagt caggttcaaa tctaagaggt    840
caacacaatc ctctaccatt ataccaaaac cacctaacac tagttttttt caactaatgc    900
atgtagccct gccaatatat aaccttgttt ttaaccgttt ttaagactga aaatattctt    960
aaaataataa caagttgacg acaaataaat aattcaaatt ttagcgaaga tcgcgttgtt   1020
actattttaa taaatattgg actttcattt ttttatgtaa tcgtataatt caagatgccg   1080
ccaaagagtg gatcttattt tccctcctac tttttcatct agatttttcg tcataggaag   1140
aaagaacaga gaaagagaag aacacttagc aaaattattt ccaaggaaat gatataatta   1200
agctcccgct acacctacac aaagtttacg aggttgagag tatttaataa aatttataaa   1260
gctcgactta aataactcaa aaagagttgg gaacctattt gcgacctata tatctcacta   1320
attactcatg atattattct taacaataga cagatttagt tccaaactaa ctacggaaac   1380
attgaaaaag aagtttagtt gatttatcta acatgcatga aataatatgg acaaggattt   1440
gtactttgta gttgccatgg tcgtgtcaac aacatagttc aaatcctatt gagactatgc   1500
aaattcattt ctggaactac attatatgta ttcttttta tccgaaaaaa acactaaata   1560
tttcttaggt gaacaaaaaa aagagaagta gatagttccg gagcagaaca aaacagttga   1620
acgaattaca aaagaacgag aattctagat ttgtttcaag aatcagtttt gatgtgcttg   1680
caacttttgc aaacatacaa acaaatatat ctgccttaat aacaaaagtg gaatatgaat   1740
cggaaaaaat agtacacttt tatggtaata taataatttg ggttggtaat aatctgcact   1800
agaattcaaa taaattttca tctagttacc tttgggtcat aggagaaccc aacaaccatc   1860
tcacaaaatt agttgcatct cacttgacct acttctcaag acccttgtaa cacaagaaaa   1920
ctaaaaactc tacaatgccc tcttctcaaa aaattatata tctctgggaa gtctcaacca   1980
tcttctctaa ctcctatctc aa                                            2002
```

<210> 16
<211> 2014
<212> DNA
<213> Arabidopsis thaliana

<220>
<221> promoter
<222> (1)..(2014)
<223> transcription regulating sequence from Arabidopsis thaliana gene At1g68850

<400> 16

```
tctcgtccca cttttttttt ggtttccaaa aagtttcaat aattgaatac attttattgg     60
ttgcagaatt ctctgctgtt actgttttgt atttctagc atttatttta caattttgtt    120
cctaaccttc taacatagca aattagcaat cataaacttt tatgcatata tataagtgta    180
agacagtaaa tcttgtatgt ggaaagacat tttccttcaa aatataagtt cataaacgtc    240
aagtcaatta tggtcgactc tctttagagt acatactaca tagattagat tcagtcggat    300
```

```
ttaatgtgta cttaactttt taacttgcag gtcttgcatg acaagagtcg tgtcacaaga    360
ccattaaagt cactgaagtt tgtgtttatg gtgcacattg acatcatgta gatggtattt    420
tgatgtaaac gaatgatata tttgtaatat tacaacttca aaggttacat ctaaattttg    480
agacttcgat gatttgtttt ttcttcgaat cactcctgcg actatgcatc agccaaatcc    540
caaatgtgtg tcaacatctc gaaagcacaa tcctcattcg ttttttactg agtgtgtgtc    600
tttcccaagc aatatattca aaaaaagtag aaagtaaatg gcccatgcag gtttcgaacc    660
tgcgaccttc gcgttattag cacgacgctc taaccaactg agctaatggg ccattttgca    720
atgacttgtt ttcttctaat aatactattt tcaaacttgt gattgcacag ttgcacattc    780
tcgaggcttg gagatgttat tagtttaaaa aactctatta aggtgcataa gtcaggttca    840
aatctaagag gtcaacacaa tcctctacca ttataccaaa accacctaac actagttttt    900
ttcaactaat gcatgtagcc ctgccaatat ataaccttgt ttttaaccgt ttttaagact    960
gaaaatattc ttaaaataat aacaagttga cgacaaataa ataattcaaa ttttagcgaa   1020
gatcgcgttg ttactatttt aataaatatt ggactttcat ttttttatgt aatcgtataa   1080
ttcaagatgc cgccaaagag tggatcttat tttccctcct actttttcat ctagattttt   1140
cgtcatagga agaaagaaca gagaaagaga agaacactta gcaaaattat ttccaaggaa   1200
atgatataat taagctcccg ctacacctac acaaagttta cgaggttgag agtatttaat   1260
aaaatttata aagctcgact taaataactc aaaaagagtt gggaacctat ttgcgaccta   1320
tatatctcac taattactca tgatattatt cttaacaata gacagattta gttccaaact   1380
aactacggaa acattgaaaa agaagtttag ttgatttatc taacatgcat gaaataatat   1440
ggacaaggat ttgtactttg tagttgccat ggtcgtgtca acaacatagt tcaaatccta   1500
ttgagactat gcaaattcat ttctggaact acattatatg tattcttttt tatccgaaaa   1560
aaacactaaa tatttcttag gtgaacaaaa aaaagagaag tagatagttc cggagcagaa   1620
caaaacagtt gaacgaatta caaaagaacg agaattctag atttgtttca agaatcagtt   1680
ttgatgtgct tgcaactttt gcaaacatac aaacaaatat atctgcctta ataacaaaag   1740
tggaatatga atcggaaaaa atagtacact tttatggtaa tataataatt tgggttggta   1800
ataatctgca ctagaattca aataaatttt catctagtta cctttgggtc ataggagaac   1860
ccaacaacca tctcacaaaa ttagttgcat ctcacttgac ctacttctca agacccttgt   1920
aacacaagaa aactaaaaac tctacaatgc cctcttctca aaaaattata tatctctggg   1980
aagtctcaac catcttctct aactcctatc tcaa                                2014
```

<210> 17
<211> 1257
<212> DNA
<213> Arabidopsis thaliana

<220>
<221> CDS
<222> (57)..(1067)
<223> encoding putative peroxidase

<400> 17

```
cacacatatt ttcccaaaat ttggatcaaa aagttttccg atagaagaaa ttaaat atg     59
                                                              Met
                                                              1

atg aga ctc ctc ttt gta ttc ttc atg gtt cac acc atc ttt atc cca      107
```

```
Met Arg Leu Leu Phe Val Phe Phe Met Val His Thr Ile Phe Ile Pro
                5                   10                  15
tgc ttt tcc ttt gat aca ccg ggg aag gat ctt cct tta acc cta gat      155
Cys Phe Ser Phe Asp Thr Pro Gly Lys Asp Leu Pro Leu Thr Leu Asp
        20                  25                  30
tat tac aag tct act tgt cca acc gta ttt gac gtc atc aag aaa gaa      203
Tyr Tyr Lys Ser Thr Cys Pro Thr Val Phe Asp Val Ile Lys Lys Glu
    35                  40                  45
atg gaa tgc ata gtg aag gaa gat cct aga aat gca gcc ata att att      251
Met Glu Cys Ile Val Lys Glu Asp Pro Arg Asn Ala Ala Ile Ile Ile
50                  55                  60                  65
cgt ctt cac ttc cac gac tgc ttt gtc caa gga tgt gat gga tcg gtg      299
Arg Leu His Phe His Asp Cys Phe Val Gln Gly Cys Asp Gly Ser Val
                70                  75                  80
ttg cta gac gag aca gaa act cta cag gga gag aag aaa gct tct ccc      347
Leu Leu Asp Glu Thr Glu Thr Leu Gln Gly Glu Lys Lys Ala Ser Pro
            85                  90                  95
aac ata aat tca ttg aaa gga tac aaa att gtc gac aga atc aag aac      395
Asn Ile Asn Ser Leu Lys Gly Tyr Lys Ile Val Asp Arg Ile Lys Asn
        100                 105                 110
ata atc gaa tcc gaa tgt cct gga gtt gtt tca tgc gct gat ctt ctc      443
Ile Ile Glu Ser Glu Cys Pro Gly Val Val Ser Cys Ala Asp Leu Leu
    115                 120                 125
aca att ggt gct aga gat gct aca atc ctg gtg ggt ggg cct tac tgg      491
Thr Ile Gly Ala Arg Asp Ala Thr Ile Leu Val Gly Gly Pro Tyr Trp
130                 135                 140                 145
gat gtt cct gtg gga aga aaa gat tca aaa acc gca agc tac gag ctt      539
Asp Val Pro Val Gly Arg Lys Asp Ser Lys Thr Ala Ser Tyr Glu Leu
                150                 155                 160
gcc aca aca aac ctt cca act cca gaa gag ggt tta atc agc atc att      587
Ala Thr Thr Asn Leu Pro Thr Pro Glu Glu Gly Leu Ile Ser Ile Ile
            165                 170                 175
gct aag ttc tat tct caa ggt ctc tcg gtt gaa gac atg gtc gct ctt      635
Ala Lys Phe Tyr Ser Gln Gly Leu Ser Val Glu Asp Met Val Ala Leu
        180                 185                 190
ata gga gcg cac acg atc gga aaa gca caa tgt cgc aac ttc cga tcc      683
Ile Gly Ala His Thr Ile Gly Lys Ala Gln Cys Arg Asn Phe Arg Ser
    195                 200                 205
cga att tat gga gat ttt caa gtg acg tca gcc cta aat cca gtt tcg      731
Arg Ile Tyr Gly Asp Phe Gln Val Thr Ser Ala Leu Asn Pro Val Ser
210                 215                 220                 225
gag acg tac ttg gca agt ctt cga gag att tgt ccg gcg agt agc gga      779
Glu Thr Tyr Leu Ala Ser Leu Arg Glu Ile Cys Pro Ala Ser Ser Gly
                230                 235                 240
gaa ggt gat agt aac gtg acg gcg ata gac aat gtg acg ccg aat ctc      827
```

```
Glu Gly Asp Ser Asn Val Thr Ala Ile Asp Asn Val Thr Pro Asn Leu
            245                 250                 255

ttc gat aac tcg atc tac cac aca ctg cta aga gga gaa ggg tta ctg      875
Phe Asp Asn Ser Ile Tyr His Thr Leu Leu Arg Gly Glu Gly Leu Leu
        260                 265                 270

aat tcg gac cag gag atg tac acg agc ttg ttc ggg ata caa acg cgg      923
Asn Ser Asp Gln Glu Met Tyr Thr Ser Leu Phe Gly Ile Gln Thr Arg
    275                 280                 285

cga atc gtg agc aag tat gcg gag gat cca gtg gct ttc ttc gag caa      971
Arg Ile Val Ser Lys Tyr Ala Glu Asp Pro Val Ala Phe Phe Glu Gln
290                 295                 300                 305

ttc tcg aag tcg atg gta aag atg ggg aac att ttg aac tct gaa agc     1019
Phe Ser Lys Ser Met Val Lys Met Gly Asn Ile Leu Asn Ser Glu Ser
                310                 315                 320

ttg gct gat gga gaa gtt aga aga aat tgc aga ttt gtg aat aca tga     1067
Leu Ala Asp Gly Glu Val Arg Arg Asn Cys Arg Phe Val Asn Thr
            325                 330                 335

ttcatcaaca acgaaactaa aaaatggaga aaataatcta tttctgatct ttatttatat   1127
aagaaagaaa aaatttaatt tgatgttatt ctttccctta tttatttcta catttttaaa   1187
actatagggt tcttgaatgt ttaaaacctt attctaatga ttttcaattg atctgagtct   1247
gtagttgttt                                                          1257


<210>  18
<211>  336
<212>  PRT
<213>  Arabidopsis thaliana

<400>  18
Met Met Arg Leu Leu Phe Val Phe Phe Met Val His Thr Ile Phe Ile
1               5                   10                  15

Pro Cys Phe Ser Phe Asp Thr Pro Gly Lys Asp Leu Pro Leu Thr Leu
            20                  25                  30

Asp Tyr Tyr Lys Ser Thr Cys Pro Thr Val Phe Asp Val Ile Lys Lys
        35                  40                  45

Glu Met Glu Cys Ile Val Lys Glu Asp Pro Arg Asn Ala Ala Ile Ile
    50                  55                  60

Ile Arg Leu His Phe His Asp Cys Phe Val Gln Gly Cys Asp Gly Ser
65                  70                  75                  80

Val Leu Leu Asp Glu Thr Glu Thr Leu Gln Gly Glu Lys Lys Ala Ser
                85                  90                  95
```

Pro Asn Ile Asn Ser Leu Lys Gly Tyr Lys Ile Val Asp Arg Ile Lys
100 105 110

Asn Ile Ile Glu Ser Glu Cys Pro Gly Val Val Ser Cys Ala Asp Leu
115 120 125

Leu Thr Ile Gly Ala Arg Asp Ala Thr Ile Leu Val Gly Gly Pro Tyr
130 135 140

Trp Asp Val Pro Val Gly Arg Lys Asp Ser Lys Thr Ala Ser Tyr Glu
145 150 155 160

Leu Ala Thr Thr Asn Leu Pro Thr Pro Glu Glu Gly Leu Ile Ser Ile
165 170 175

Ile Ala Lys Phe Tyr Ser Gln Gly Leu Ser Val Glu Asp Met Val Ala
180 185 190

Leu Ile Gly Ala His Thr Ile Gly Lys Ala Gln Cys Arg Asn Phe Arg
195 200 205

Ser Arg Ile Tyr Gly Asp Phe Gln Val Thr Ser Ala Leu Asn Pro Val
210 215 220

Ser Glu Thr Tyr Leu Ala Ser Leu Arg Glu Ile Cys Pro Ala Ser Ser
225 230 235 240

Gly Glu Gly Asp Ser Asn Val Thr Ala Ile Asp Asn Val Thr Pro Asn
245 250 255

Leu Phe Asp Asn Ser Ile Tyr His Thr Leu Leu Arg Gly Glu Gly Leu
260 265 270

Leu Asn Ser Asp Gln Glu Met Tyr Thr Ser Leu Phe Gly Ile Gln Thr
275 280 285

Arg Arg Ile Val Ser Lys Tyr Ala Glu Asp Pro Val Ala Phe Phe Glu
290 295 300

Gln Phe Ser Lys Ser Met Val Lys Met Gly Asn Ile Leu Asn Ser Glu
305 310 315 320

Ser Leu Ala Asp Gly Glu Val Arg Arg Asn Cys Arg Phe Val Asn Thr
325 330 335

<210> 19
<211> 1023
<212> DNA
<213> Arabidopsis thaliana

<220>
<221> promoter
<222> (1)..(1023)
<223> transcription regulating sequence from Arabidopsis thaliana gene At4g36670

<400> 19
aatctcatcc actgatccac aaaccgtaat caccatcaaa atattcatgc aagtcaactt 60
gaggatattt acatactata attaagaaaa taccacaaat ttccaaatca agtttttttt 120
tttttttttt ggctattggc ttaattaaca aacaagaatt tggctattca cgtgattcaa 180
agtctaaact aatgaagccc acttgacttt tcgggcaact catctaaaac ttcaaacttt 240
gaaatctaat ttaaaaatgg acccatgacg ttttacactc tgactctcca tagttgactc 300
catgagtcca tccaaatcga gtgtttggtt acacagtgac atgaagtttt aaaattctcg 360
cgatctaaaa caagatattt ttaaaatatt cacgtgtata aaaacaagaa aatttaaaaa 420
tattcacgag gataaaaaca aaaagcacct attagaaata gctcttatcc aataaggcaa 480
tgataacgga ctcgtgtcgg tgtctaccca tatcttatta atcgaaaaaa aaatcttagt 540
tagtccaaaa ctcacccccca aagagtcaac tttgttacac atacgttctt atccaaaatc 600
caaagttaac ttattaacga ctaatgccaa ctcaggttta cacatgacat cagagcttcc 660
aagtttatag tctggtcaag ctgtaactgt taacttttaa ttacagtcaa ggtctgtaat 720
tagaatttga tcacaaaaca tgtttcattc tataaacata tattaaaacg atgtggatga 780
ctcttgtcca ttggattcaa ataactaaat catctcttaa cagtagcttg tggctttcac 840
gactactgtt ttgtcaacta tttttttttct atataatcac cgaatctttt ctttggtact 900
gagagagcaa gagagattag taagacctaa ctcttaacga acttttagat aaaagcttat 960
aactgagaga gacatagatc gacaaaagtc tctatacctt cttgtaacgt ggtggttaat 1020
taa 1023

<210> 20
<211> 1036
<212> DNA
<213> Arabidopsis thaliana

<220>
<221> promoter
<222> (1)..(1036)
<223> transcription regulating sequence from Arabidopsis thaliana gene At4g36670

<400> 20
gttataggtt caaatctcat ccactgatcc acaaaccgta atcaccatca aaatattcat 60

```
gcaagtcaac ttgaggatat ttacatacta taattaagaa aataccacaa atttccaaat    120
caagtttttt tttttttttt tggctattgg cttaattaac aaacaagaat ttggctattc    180
acgtgattca aagtctaaac taatgaagcc cacttgactt ttcgggcaac tcatctaaaa    240
cttcaaactt tgaaatctaa tttaaaaatg gacccatgac gttttacact ctgactctcc    300
atagttgact ccatgagtcc atccaaatcg agtgtttggt tacacagtga catgaagttt    360
taaaattctc gcgatctaaa acaagatatt tttaaaatat tcacgtgtat aaaaacaaga    420
aaatttaaaa atattcacga ggataaaaac aaaaagcacc tattagaaat agctcttatc    480
caataaggca atgataacgg actcgtgtcg gtgtctaccc atatcttatt aatcgaaaaa    540
aaaatcttag ttagtccaaa actcaccccc aaagagtcaa ctttgttaca catacgttct    600
tatccaaaat ccaaagttaa cttattaacg actaatgcca actcaggttt acacatgaca    660
tcagagcttc caagtttata gtctggtcaa gctgtaactg ttaactttta attacagtca    720
aggtctgtaa ttagaatttg atcacaaaac atgtttcatt ctataaacat atattaaaac    780
gatgtggatg actcttgtcc attggattca aataactaaa tcatctctta acagtagctt    840
gtggctttca cgactactgt tttgtcaact atttttttttc tatataatca ccgaatcttt    900
tctttggtac tgagagagca agagagatta gtaagaccta actcttaacg aacttttaga    960
taaaagctta taactgagag agacatagat cgacaaaagt ctctatacct tcttgtaacg   1020
tggtggttaa ttaatc                                                   1036
```

<210> 21
<211> 918
<212> DNA
<213> Arabidopsis thaliana

<220>
<221> promoter
<222> (1)..(918)
<223> transcription regulating sequence from Arabidopsis thaliana gene At4g36670

<400> 21

```
aatctcatcc actgatccac aaaccgtaat caccatcaaa atattcatgc aagtcaactt     60
gaggatattt acatactata attaagaaaa taccacaaat ttccaaatca agtttttttt    120
tttttttttt ggctattggc ttaattaaca aacaagaatt tggctattca cgtgattcaa    180
agtctaaact aatgaagccc acttgacttt tcgggcaact catctaaaac ttcaaacttt    240
gaaatctaat ttaaaaatgg acccatgacg ttttacactc tgactctcca tagttgactc    300
catgagtcca tccaaatcga gtgtttggtt acacagtgac atgaagtttt aaaattctcg    360
cgatctaaaa caagatattt ttaaaatatt cacgtgtata aaaacaagaa aatttaaaaa    420
tattcacgag gataaaaaca aaaagcacct attagaaata gctcttatcc aataaggcaa    480
tgataacgga ctcgtgtcgg tgtctaccca tatcttatta atcgaaaaaa aaatcttagt    540
tagtccaaaa ctcaccccca aagagtcaac tttgttacac atacgttctt atccaaaatc    600
caaagttaac ttattaacga ctaatgccaa ctcaggttta cacatgacat cagagcttcc    660
aagtttatag tctggtcaag ctgtaactgt taacttttaa ttacagtcaa ggtctgtaat    720
tagaatttga tcacaaaaca tgtttcattc tataaacata tattaaaacg atgtggatga    780
ctcttgtcca ttggattcaa ataactaaat catctcttaa cagtagcttg tggctttcac    840
gactactgtt ttgtcaacta ttttttttct atataatcac cgaatctttt ctttggtact    900
```

gagagagcaa gagagatt 918

<210> 22
<211> 929
<212> DNA
<213> Arabidopsis thaliana

<220>
<221> promoter
<222> (1)..(929)
<223> transcription regulating sequence from Arabidopsis thaliana gene At4g36670

<400> 22
gttataggtt caaatctcat ccactgatcc acaaaccgta atcaccatca aaatattcat 60
gcaagtcaac ttgaggatat ttacatacta taattaagaa aataccacaa atttccaaat 120
caagtttttt tttttttttt tggctattgg cttaattaac aaacaagaat ttggctattc 180
acgtgattca aagtctaaac taatgaagcc cacttgactt ttcgggcaac tcatctaaaa 240
cttcaaactt tgaaatctaa tttaaaaatg gacccatgac gttttacact ctgactctcc 300
atagttgact ccatgagtcc atccaaatcg agtgtttggt tacacagtga catgaagttt 360
taaaattctc gcgatctaaa acaagatatt tttaaaatat tcacgtgtat aaaaacaaga 420
aaatttaaaa atattcacga ggataaaaac aaaaagcacc tattagaaat agctcttatc 480
caataaggca atgataacgg actcgtgtcg gtgtctaccc atatcttatt aatcgaaaaa 540
aaaatcttag ttagtccaaa actcaccccc aaagagtcaa ctttgttaca catacgttct 600
tatccaaaat ccaaagttaa cttattaacg actaatgcca actcaggttt acacatgaca 660
tcagagcttc caagtttata gtctggtcaa gctgtaactg ttaactttta attacagtca 720
aggtctgtaa ttagaatttg atcacaaaac atgtttcatt ctataaacat atattaaaac 780
gatgtggatg actcttgtcc attggattca aataactaaa tcatctctta acagtagctt 840
gtggctttca cgactactgt tttgtcaact atttttttc tatataatca ccgaatcttt 900
tctttggtac tgagagagca agagagatt 929

<210> 23
<211> 2254
<212> DNA
<213> Arabidopsis thaliana

<220>
<221> promoter
<222> (1)..(2254)
<223> transcription regulating sequence from Arabidopsis thaliana gene At4g36670

<400> 23
gcccttcttc atactagtaa gtcaaatata aacttttgac gaaataatcc aataacatca 60
cctactcgtc cggaaacaga acagtacaaa gttggtgaat aaaatctatg gatattttat 120

```
atagtgagct ttttgagaaa cgtgatctac tttgatgtac taaagttaac ttttaacaat     180
gaaaacaaaa attgaaaaat taacaacgtt aaatcagaaa cgtaaataaa ctgaatcatc     240
atttcaacag ttgattccac aagctattaa aattttaaat attagaaagt attttaaaga     300
tgcttgaaac ttagcagaca gtttggttta aaagagtaaa agtggtaatg agctgatgtt     360
ggcggctaaa tctctaaaat cagtaaagca tatcgcgaac caaaagccaa agcaccaaat     420
gaaactgagt taggttcgtt gtacacacga tgttttccaa taaataatcc tccacaaaat     480
tcaaacaccg gatgtatctc gaaaacatta gatgttctat atacaaacat atagaatcat     540
ggtattgagt tcgtgatttt tttttgtaa catgatgtaa ttagtaattc atcctaatat     600
atgtaatcat ctttatattt tgataataca ttcgtgattc gttcgatgac attaccacac     660
gaggttacga ctgcgcacat atggtgtatt atgagtccac catacgataa attattaaaa     720
gtgtggtaga gagacttttc ttatggttga taaattatta ttttcaatat cgtcaaaatt     780
acttattcat tttctcttta tttggaccaa tttagattta gactcgatta gtttttctat     840
agatgtaggt tcttcctctc aagatgtagc gataggtttt agggttaaac tttgtcgtgg     900
atataggaag aaactgcatg tagtttttga tgggttacac ttttagttac aacacgaaag     960
tttatcggaa atactagttg gctcatggct gccttacgtt agacaattaa ttacaaacat    1020
ctcttttttt ctttgtcaat aattgtttct tattgtaggc atactattgg agaaacatga    1080
ggtacgaact tcccaaatca gttttttttt tttgggaaaa agttgaaatt atgcaaaaca    1140
ctgaaaaagg aaagaacaag taattaaaat gcaaaaaaaa atttttaatt aaaatgcatg    1200
aacatataaa caaataacat gttataggtt caaatctcat ccactgatcc acaaaccgta    1260
atcaccatca aaatattcat gcaagtcaac ttgaggatat ttacatacta taattaagaa    1320
aataccacaa atttccaaat caagtttttt tttttttttt tggctattgg cttaattaac    1380
aaacaagaat ttggctattc acgtgattca aagtctaaac taatgaagcc cacttgactt    1440
ttcgggcaac tcatctaaaa cttcaaactt tgaaatctaa tttaaaaatg gacccatgac    1500
gttttacact ctgactctcc atagttgact ccatgagtcc atccaaatcg agtgtttggt    1560
tacacagtga catgaagttt taaaattctc gcgatctaaa acaagatatt tttaaaatat    1620
tcacgtgtat aaaaacaaga aaatttaaaa atattcacga ggataaaaac aaaaagcacc    1680
tattagaaat agctcttatc caataaggca atgataacgg actcgtgtcg gtgtctaccc    1740
atatcttatt aatcgaaaaa aaaatcttag ttagtccaaa actcaccccc aaagagtcaa    1800
ctttgttaca catacgttct tatccaaaat ccaaagttaa cttattaacg actaatgcca    1860
actcaggttt acacatgaca tcagagcttc caagtttata gtctggtcaa gctgtaactg    1920
ttaactttta attacagtca aggtctgtaa ttagaatttg atcacaaaac atgtttcatt    1980
ctataaacat atattaaaac gatgtggatg actcttgtcc attggattca aataactaaa    2040
tcatctctta acagtagctt gtggctttca cgactactgt tttgtcaact attttttttc    2100
tatataatca ccgaatcttt tctttggtac tgagagagca agagagatta gtaagaccta    2160
actcttaacg aacttttaga taaaagctta taactgagag agacatagat cgacaaaagt    2220
ctctatacct tcttgtaacg tggtggttaa ttaa                                2254
```

<210> 24
<211> 2250
<212> DNA
<213> Arabidopsis thaliana

<220>
<221> promoter
<222> (1)..(2250)

<223> transcription regulating sequence from Arabidopsis thaliana gene At4g36670

<400> 24

```
cttcatacta gtaagtcaaa tataaacttt tgacgaaata atccaataac atcacctact      60
cgtccggaaa cagaacagta caaagttggt gaataaaatc tatggatatt ttatatagtg     120
agctttttga gaaacgtgat ctactttgat gtactaaagt taacttttaa caatgaaaac     180
aaaaattgaa aaattaacaa cgttaaatca gaaacgtaaa taaactgaat catcatttca     240
acagttgatt ccacaagcta ttaaaatttt aaatattaga aagtatttta aagatgcttg     300
aaacttagca gacagtttgg tttaaaagag taaaagtggt aatgagctga tgttggcggc     360
taaatctcta aaatcagtaa agcatatcgc gaaccaaaag ccaaagcacc aaatgaaact     420
gagttaggtt cgttgtacac acgatgtttt ccaataaata atcctccaca aaattcaaac     480
accggatgta tctcgaaaac attagatgtt ctatatacaa acatatagaa tcatggtatt     540
gagttcgtga tttttttttt gtaacatgat gtaattagta attcatccta atatatgtaa     600
tcatctttat attttgataa tacattcgtg attcgttcga tgacattacc acacgaggtt     660
acgactgcgc acatatggtg tattatgagt ccaccatacg ataaattatt aaaagtgtgg     720
tagagagact tttcttatgg ttgataaatt attattttca atatcgtcaa aattacttat     780
tcattttctc tttatttgga ccaatttaga tttagactcg attagttttt ctatagatgt     840
aggttcttcc tctcaagatg tagcgatagg ttttagggtt aaactttgtc gtggatatag     900
gaagaaactg catgtagttt ttgatgggtt acacttttag ttacaacacg aaagtttatc     960
ggaaatacta gttggctcat ggctgcctta cgttagacaa ttaattacaa acatctcttt    1020
ttttctttgt caataattgt ttcttattgt aggcatacta ttggagaaac atgaggtacg    1080
aacttcccaa atcagttttt ttttttgggg aaaaagttga aattatgcaa aacactgaaa    1140
aaggaaagaa caagtaatta aaatgcaaaa aaaaattttt aattaaaatg catgaacata    1200
taaacaaata acatgttata ggttcaaatc tcatccactg atccacaaac cgtaatcacc    1260
atcaaaatat tcatgcaagt caacttgagg atatttacat actataatta agaaaatacc    1320
acaaatttcc aaatcaagtt tttttttttt tttttggcta ttggcttaat taacaaacaa    1380
gaatttggct attcacgtga ttcaaagtct aaactaatga agcccacttg acttttcggg    1440
caactcatct aaaacttcaa actttgaaat ctaatttaaa aatggaccca tgacgtttta    1500
cactctgact ctccatagtt gactccatga gtccatccaa atcgagtgtt tggttacaca    1560
gtgacatgaa gttttaaaat tctcgcgatc taaaacaaga tatttttaaa atattcacgt    1620
gtataaaaac aagaaaattt aaaaatattc acgaggataa aaacaaaaag cacctattag    1680
aaatagctct tatccaataa ggcaatgata acggactcgt gtcggtgtct acccatatct    1740
tattaatcga aaaaaaaatc ttagttagtc caaaactcac ccccaaagag tcaactttgt    1800
tacacatacg ttcttatcca aaatccaaag ttaacttatt aacgactaat gccaactcag    1860
gtttacacat gacatcagag cttccaagtt tatagtctgg tcaagctgta actgttaact    1920
tttaattaca gtcaaggtct gtaattagaa tttgatcaca aaacatgttt cattctataa    1980
acatatatta aaacgatgtg gatgactctt gtccattgga ttcaaataac taaatcatct    2040
cttaacagta gcttgtggct ttcacgacta ctgttttgtc aactattttt tttctatata    2100
atcaccgaat cttttctttg gtactgagag agcaagagag attagtaaga cctaactctt    2160
aacgaacttt tagataaaag cttataactg agagagacat agatcgacaa aagtctctat    2220
accttcttgt aacgtggtgg ttaattaatc                                     2250
```

<210> 25
<211> 2149

<212> DNA
<213> Arabidopsis thaliana

<220>
<221> promoter
<222> (1)..(2149)
<223> transcription regulating sequence from Arabidopsis thaliana gene At4g36670

<400> 25
gcccttcttc atactagtaa gtcaaatata aacttttgac gaaataatcc aataacatca 60
cctactcgtc cggaaacaga acagtacaaa gttggtgaat aaaatctatg gatattttat 120
atagtgagct ttttgagaaa cgtgatctac tttgatgtac taaagttaac ttttaacaat 180
gaaaacaaaa attgaaaaat taacaacgtt aaatcagaaa cgtaaataaa ctgaatcatc 240
atttcaacag ttgattccac aagctattaa aattttaaat attagaaagt attttaaaga 300
tgcttgaaac ttagcagaca gtttggttta aaagagtaaa agtggtaatg agctgatgtt 360
ggcggctaaa tctctaaaat cagtaaagca tatcgcgaac caaaagccaa agcaccaaat 420
gaaactgagt taggttcgtt gtacacacga tgttttccaa taaataatcc tccacaaaat 480
tcaaacaccg gatgtatctc gaaaacatta gatgttctat atacaaacat atagaatcat 540
ggtattgagt tcgtgatttt ttttttgtaa catgatgtaa ttagtaattc atcctaatat 600
atgtaatcat ctttatattt tgataataca ttcgtgattc gttcgatgac attaccacac 660
gaggttacga ctgcgcacat atggtgtatt atgagtccac catacgataa attattaaaa 720
gtgtggtaga gagacttttc ttatggttga taaattatta ttttcaatat cgtcaaaatt 780
acttattcat tttctcttta tttggaccaa tttagattta gactcgatta gtttttctat 840
agatgtaggt tcttcctctc aagatgtagc gataggtttt agggttaaac tttgtcgtgg 900
atataggaag aaactgcatg tagtttttga tgggttacac ttttagttac aacacgaaag 960
tttatcggaa atactagttg gctcatggct gccttacgtt agacaattaa ttacaaacat 1020
ctcttttttt ctttgtcaat aattgtttct tattgtaggc atactattgg agaaacatga 1080
ggtacgaact tcccaaatca gttttttttt tttgggaaaa agttgaaatt atgcaaaaca 1140
ctgaaaaagg aaagaacaag taattaaaat gcaaaaaaaa atttttaatt aaaatgcatg 1200
aacatataaa caaataacat gttataggtt caaatctcat ccactgatcc acaaaccgta 1260
atcaccatca aaatattcat gcaagtcaac ttgaggatat ttacatacta taattaagaa 1320
aataccacaa atttccaaat caagtttttt tttttttttt tggctattgg cttaattaac 1380
aaacaagaat ttggctattc acgtgattca aagtctaaac taatgaagcc cacttgactt 1440
ttcgggcaac tcatctaaaa cttcaaactt tgaaatctaa tttaaaaatg gacccatgac 1500
gttttacact ctgactctcc atagttgact ccatgagtcc atccaaatcg agtgtttggt 1560
tacacagtga catgaagttt taaaattctc gcgatctaaa acaagatatt tttaaaatat 1620
tcacgtgtat aaaaacaaga aaatttaaaa atattcacga ggataaaaac aaaaagcacc 1680
tattagaaat agctcttatc caataaggca atgataacgg actcgtgtcg gtgtctaccc 1740
atatcttatt aatcgaaaaa aaaatcttag ttagtccaaa actcaccccc aaagagtcaa 1800
ctttgttaca catacgttct tatccaaaat ccaaagttaa cttattaacg actaatgcca 1860
actcaggttt acacatgaca tcagagcttc caagtttata gtctggtcaa gctgtaactg 1920
ttaactttta attacagtca aggtctgtaa ttagaatttg atcacaaaac atgtttcatt 1980
ctataaacat atattaaaac gatgtggatg actcttgtcc attggattca aataactaaa 2040
tcatctctta acagtagctt gtggctttca cgactactgt tttgtcaact attttttttc 2100

tatataatca ccgaatcttt tctttggtac tgagagagca agagagatt 2149

<210> 26
<211> 2143
<212> DNA
<213> Arabidopsis thaliana

<220>
<221> promoter
<222> (1)..(2143)
<223> transcription regulating sequence from Arabidopsis thaliana gene At4g36670

<400> 26
cttcatacta gtaagtcaaa tataaacttt tgacgaaata atccaataac atcacctact 60
cgtccggaaa cagaacagta caaagttggt gaataaaatc tatggatatt ttatatagtg 120
agctttttga gaaacgtgat ctactttgat gtactaaagt taacttttaa caatgaaaac 180
aaaaattgaa aaattaacaa cgttaaatca gaaacgtaaa taaactgaat catcatttca 240
acagttgatt ccacaagcta ttaaaatttt aaatattaga aagtatttta aagatgcttg 300
aaacttagca gacagtttgg tttaaaagag taaaagtggt aatgagctga tgttggcggc 360
taaatctcta aaatcagtaa agcatatcgc gaaccaaaag ccaaagcacc aaatgaaact 420
gagttaggtt cgttgtacac acgatgtttt ccaataaata atcctccaca aaattcaaac 480
accggatgta tctcgaaaac attagatgtt ctatatacaa acatatagaa tcatggtatt 540
gagttcgtga ttttttttt gtaacatgat gtaattagta attcatccta atatatgtaa 600
tcatctttat attttgataa tacattcgtg attcgttcga tgacattacc acacgaggtt 660
acgactgcgc acatatggtg tattatgagt ccaccatacg ataaattatt aaaagtgtgg 720
tagagagact tttcttatgg ttgataaatt attattttca atatcgtcaa aattacttat 780
tcattttctc tttatttgga ccaatttaga tttagactcg attagttttt ctatagatgt 840
aggttcttcc tctcaagatg tagcgatagg ttttagggtt aaactttgtc gtggatatag 900
gaagaaactg catgtagttt ttgatgggtt acacttttag ttacaacacg aaagtttatc 960
ggaaatacta gttggctcat ggctgcctta cgttagacaa ttaattacaa acatctcttt 1020
ttttctttgt caataattgt ttcttattgt aggcatacta ttggagaaac atgaggtacg 1080
aacttcccaa atcagttttt tttttttggg aaaaagttga aattatgcaa aacactgaaa 1140
aaggaaagaa caagtaatta aaatgcaaaa aaaaattttt aattaaaatg catgaacata 1200
taaacaaata acatgttata ggttcaaatc tcatccactg atccacaaac cgtaatcacc 1260
atcaaaatat tcatgcaagt caacttgagg atatttacat actataatta agaaaatacc 1320
acaaatttcc aaatcaagtt tttttttttt tttttggcta ttggcttaat taacaaacaa 1380
gaatttggct attcacgtga ttcaaagtct aaactaatga agcccacttg acttttcggg 1440
caactcatct aaaacttcaa actttgaaat ctaatttaaa aatggaccca tgacgtttta 1500
cactctgact ctccatagtt gactccatga gtccatccaa atcgagtgtt tggttacaca 1560
gtgacatgaa gttttaaaat tctcgcgatc taaaacaaga tatttttaaa atattcacgt 1620
gtataaaaac aagaaaattt aaaaatattc acgaggataa aaacaaaaag cacctattag 1680
aaatagctct tatccaataa ggcaatgata acggactcgt gtcggtgtct acccatatct 1740
tattaatcga aaaaaaaatc ttagttagtc caaaactcac ccccaaagag tcaactttgt 1800
tacacatacg ttcttatcca aaatccaaag ttaacttatt aacgactaat gccaactcag 1860

gtttacacat gacatcagag cttccaagtt tatagtctgg tcaagctgta actgttaact 1920
tttaattaca gtcaaggtct gtaattagaa tttgatcaca aaacatgttt cattctataa 1980
acatatatta aaacgatgtg gatgactctt gtccattgga ttcaaataac taaatcatct 2040
cttaacagta gcttgtggct ttcacgacta ctgttttgtc aactattttt tttctatata 2100
atcaccgaat cttttctttg gtactgagag agcaagagag att 2143

<210> 27
<211> 1280
<212> DNA
<213> Arabidopsis thaliana

<220>
<221> promoter
<222> (1)..(1280)
<223> transcription regulating sequence from Arabidopsis thaliana gene At4g36670

<400> 27
actagttggc tcatggctgc cttacgttag acaattaatt acaaacatct cttttttttct 60
ttgtcaataa ttgtttctta ttgtaggcat actattggag aaacatgagg tacgaacttc 120
ccaaatcagt tttttttttt gggaaaaagt tgaaattatg caaaacactg aaaaaggaaa 180
gaacaagtaa ttaaaatgca aaaaaaaatt tttaattaaa atgcatgaac atataaacaa 240
ataacatgtt ataggttcaa atctcatcca ctgatccaca aaccgtaatc accatcaaaa 300
tattcatgca agtcaacttg aggatattta catactataa ttaagaaaat accacaaatt 360
tccaaatcaa gttttttttt tttttttggc tattggctta attaacaaac aagaatttgg 420
ctattcacgt gattcaaagt ctaaactaat gaagcccact tgacttttcg ggcaactcat 480
ctaaaacttc aaactttgaa atctaattta aaaatggacc catgacgttt tacactctga 540
ctctccatag ttgactccat gagtccatcc aaatcgagtg tttggttaca cagtgacatg 600
aagttttaaa attctcgcga tctaaaacaa gatattttta aaatattcac gtgtataaaa 660
acaagaaaat ttaaaaatat tcacgaggat aaaaacaaaa agcacctatt agaaatagct 720
cttatccaat aaggcaatga taacggactc gtgtcggtgt ctacccatat cttattaatc 780
gaaaaaaaaa tcttagttag tccaaaactc acccccaaag agtcaacttt gttacacata 840
cgttcttatc caaaatccaa agttaactta ttaacgacta atgccaactc aggtttacac 900
atgacatcag agcttccaag tttatagtct ggtcaagctg taactgttaa cttttaatta 960
cagtcaaggt ctgtaattag aatttgatca caaaacatgt ttcattctat aaacatatat 1020
taaaacgatg tggatgactc ttgtccattg gattcaaata actaaatcat ctcttaacag 1080
tagcttgtgg ctttcacgac tactgttttg tcaactattt tttttctata taatcaccga 1140
atcttttctt tggtactgag agagcaagag agattagtaa gacctaactc ttaacgaact 1200
tttagataaa agcttataac tgagagagac atagatcgac aaaagtctct ataccttctt 1260
gtaacgtggt ggttaattaa 1280

<210> 28
<211> 1283
<212> DNA
<213> Arabidopsis thaliana

<220>
<221> promoter
<222> (1)..(1283)
<223> transcription regulating sequence from Arabidopsis thaliana gene At4g36670

<400> 28
```
ctagttggct catggctgcc ttacgttaga caattaatta caaacatctc tttttttctt      60
tgtcaataat tgtttcttat tgtaggcata ctattggaga aacatgaggt acgaacttcc     120
caaatcagtt tttttttttt gggaaaaagt tgaaattatg caaaacactg aaaaaggaaa     180
gaacaagtaa ttaaaatgca aaaaaaaatt tttaattaaa atgcatgaac atataaacaa     240
ataacatgtt ataggttcaa atctcatcca ctgatccaca aaccgtaatc accatcaaaa     300
tattcatgca agtcaacttg aggatattta catactataa ttaagaaaat accacaaatt     360
tccaaatcaa gttttttttt tttttttgg ctattggctt aattaacaaa caagaatttg      420
gctattcacg tgattcaaag tctaaactaa tgaagcccac ttgacttttc gggcaactca     480
tctaaaactt caaactttga aatctaattt aaaaatggac ccatgacgtt ttacactctg     540
actctccata gttgactcca tgagtccatc caaatcgagt gtttggttac acagtgacat     600
gaagttttaa aattctcgcg atctaaaaca agatattttt aaaatattca cgtgtataaa     660
aacaagaaaa tttaaaaata ttcacgagga taaaaacaaa aagcacctat tagaaatagc     720
tcttatccaa taaggcaatg ataacggact cgtgtcggtg tctacccata tcttattaat     780
cgaaaaaaaa atcttagtta gtccaaaact cacccccaaa gagtcaactt tgttacacat     840
acgttcttat ccaaaatcca aagttaactt attaacgact aatgccaact caggtttaca     900
catgacatca gagcttccaa gtttatagtc tggtcaagct gtaactgtta acttttaatt     960
acagtcaagg tctgtaatta gaatttgatc acaaaacatg tttcattcta taaacatata    1020
ttaaaacgat gtggatgact cttgtccatt ggattcaaat aactaaatca tctcttaaca    1080
gtagcttgtg gctttcacga ctactgtttt gtcaactatt ttttttctat ataatcaccg    1140
aatcttttct ttggtactga gagagcaaga gagattagta agacctaact cttaacgaac    1200
ttttagataa aagcttataa ctgagagaga catagatcga caaaagtctc tataccttct    1260
tgtaacgtgg tggttaatta atc                                            1283
```

<210> 29
<211> 1175
<212> DNA
<213> Arabidopsis thaliana

<220>
<221> promoter
<222> (1)..(1175)
<223> transcription regulating sequence from Arabidopsis thaliana gene At4g36670

<400> 29
```
actagttggc tcatggctgc cttacgttag acaattaatt acaaacatct cttttttttct     60
ttgtcaataa ttgtttctta ttgtaggcat actattggag aaacatgagg tacgaacttc     120
```

```
ccaaatcagt ttttttttt gggaaaaagt tgaaattatg caaaacactg aaaaaggaaa     180
gaacaagtaa ttaaaatgca aaaaaaaatt tttaattaaa atgcatgaac atataaacaa     240
ataacatgtt ataggttcaa atctcatcca ctgatccaca aaccgtaatc accatcaaaa     300
tattcatgca agtcaacttg aggatattta catactataa ttaagaaaat accacaaatt     360
tccaaatcaa gttttttttt tttttttggc tattggctta attaacaaac aagaatttgg     420
ctattcacgt gattcaaagt ctaaactaat gaagcccact tgacttttcg ggcaactcat     480
ctaaaacttc aaactttgaa atctaattta aaaatggacc catgacgttt tacactctga     540
ctctccatag ttgactccat gagtccatcc aaatcgagtg tttggttaca cagtgacatg     600
aagttttaaa attctcgcga tctaaaacaa gatattttta aaatattcac gtgtataaaa     660
acaagaaaat ttaaaaatat tcacgaggat aaaaacaaaa agcacctatt agaaatagct     720
cttatccaat aaggcaatga taacggactc gtgtcggtgt ctacccatat cttattaatc     780
gaaaaaaaaa tcttagttag tccaaaactc acccccaaag agtcaacttt gttacacata     840
cgttcttatc caaaatccaa agttaactta ttaacgacta atgccaactc aggtttacac     900
atgacatcag agcttccaag tttatagtct ggtcaagctg taactgttaa cttttaatta     960
cagtcaaggt ctgtaattag aatttgatca caaaacatgt ttcattctat aaacatatat    1020
taaaacgatg tggatgactc ttgtccattg gattcaaata actaaatcat ctcttaacag    1080
tagcttgtgg ctttcacgac tactgttttg tcaactattt tttttctata taatcaccga    1140
atcttttctt tggtactgag agagcaagag agatt                               1175
```

<210> 30
<211> 1176
<212> DNA
<213> Arabidopsis thaliana

<220>
<221> promoter
<222> (1)..(1176)
<223> transcription regulating sequence from Arabidopsis thaliana gene At4g36670

<400> 30

```
ctagttggct catggctgcc ttacgttaga caattaatta caaacatctc ttttttttctt     60
tgtcaataat tgtttcttat tgtaggcata ctattggaga aacatgaggt acgaacttcc    120
caaatcagtt ttttttttt gggaaaaagt tgaaattatg caaaacactg aaaaaggaaa    180
gaacaagtaa ttaaaatgca aaaaaaaatt tttaattaaa atgcatgaac atataaacaa    240
ataacatgtt ataggttcaa atctcatcca ctgatccaca aaccgtaatc accatcaaaa    300
tattcatgca agtcaacttg aggatattta catactataa ttaagaaaat accacaaatt    360
tccaaatcaa gttttttttt ttttttttgg ctattggctt aattaacaaa caagaatttg    420
gctattcacg tgattcaaag tctaaactaa tgaagcccac ttgacttttc gggcaactca    480
tctaaaactt caaactttga aatctaattt aaaaatggac ccatgacgtt ttacactctg    540
actctccata gttgactcca tgagtccatc caaatcgagt gtttggttac acagtgacat    600
gaagttttaa aattctcgcg atctaaaaca agatattttt aaaatattca cgtgtataaa    660
aacaagaaaa tttaaaaata ttcacgagga taaaaacaaa aagcacctat tagaaatagc    720
tcttatccaa taaggcaatg ataacggact cgtgtcggtg tctacccata tcttattaat    780
cgaaaaaaaa atcttagtta gtccaaaact cacccccaaa gagtcaactt tgttacacat    840
```

```
acgttcttat ccaaaatcca aagttaactt attaacgact aatgccaact caggtttaca      900
catgacatca gagcttccaa gtttatagtc tggtcaagct gtaactgtta acttttaatt      960
acagtcaagg tctgtaatta gaatttgatc acaaaacatg tttcattcta taaacatata     1020
ttaaaacgat gtggatgact cttgtccatt ggattcaaat aactaaatca tctcttaaca     1080
gtagcttgtg gctttcacga ctactgtttt gtcaactatt ttttttctat ataatcaccg     1140
aatcttttct ttggtactga gagagcaaga gagatt                               1176

<210>  31
<211>  1770
<212>  DNA
<213>  Arabidopsis thaliana

<220>
<221>  CDS
<222>  (108)..(1589)
<223>  encoding putative Arabidopsis thaliana mannitol transporter

<400>  31
agtaagacct aactcttaac gaacttttag ataaaagctt ataactgaga gagacataga       60
tcgacaaaag tctctatacc ttcttgtaac gtggtggtta attaatc atg gcc gat        116
                                                    Met Ala Asp
                                                    1

caa atc tcc ggc gag aag ccg gcc gga gtt aat aga ttc gct ctt caa        164
Gln Ile Ser Gly Glu Lys Pro Ala Gly Val Asn Arg Phe Ala Leu Gln
    5                   10                  15

tgt gct atc gtc gcc tcc atc gtc tcc atc atc ttt ggt tac gat act        212
Cys Ala Ile Val Ala Ser Ile Val Ser Ile Ile Phe Gly Tyr Asp Thr
20                  25                  30                  35

ggt gtt atg agt gga gcg atg gtg ttt ata gaa gaa gat ttg aag aca        260
Gly Val Met Ser Gly Ala Met Val Phe Ile Glu Glu Asp Leu Lys Thr
                40                  45                  50

aac gac gtt caa ata gaa gtt ctc act gga att ctc aac ctt tgt gcc        308
Asn Asp Val Gln Ile Glu Val Leu Thr Gly Ile Leu Asn Leu Cys Ala
            55                  60                  65

ctt gtc gga tca ttg ctc gcc gga aga acg tcg gac ata atc gga cgg        356
Leu Val Gly Ser Leu Leu Ala Gly Arg Thr Ser Asp Ile Ile Gly Arg
        70                  75                  80

cgt tac aca atc gtc ttg gcc tca ata cta ttc atg tta ggc tca ata        404
Arg Tyr Thr Ile Val Leu Ala Ser Ile Leu Phe Met Leu Gly Ser Ile
    85                  90                  95

ttg atg ggt tgg ggt ccg aat tat ccg gtt ctc cta tcc ggt aga tgc        452
Leu Met Gly Trp Gly Pro Asn Tyr Pro Val Leu Leu Ser Gly Arg Cys
100                 105                 110                 115

acc gct gga ctc gga gtc ggt ttt gct ctg atg gtt gct ccg gtt tac        500
Thr Ala Gly Leu Gly Val Gly Phe Ala Leu Met Val Ala Pro Val Tyr
```

```
                120                 125                 130
tct gcc gag atc gca act gct tca cat aga gga ctc tta gct tct ctt      548
Ser Ala Glu Ile Ala Thr Ala Ser His Arg Gly Leu Leu Ala Ser Leu
            135                 140                 145
cct cac ctt tgt atc agt ata ggg att tta cta ggt tat atc gtg aat      596
Pro His Leu Cys Ile Ser Ile Gly Ile Leu Leu Gly Tyr Ile Val Asn
        150                 155                 160
tac ttc ttc tcc aag tta cct atg cat atc ggt tgg aga ctc atg ctc      644
Tyr Phe Phe Ser Lys Leu Pro Met His Ile Gly Trp Arg Leu Met Leu
    165                 170                 175
ggt ata gcc gcg gtt ccg tcc cta gtg cta gcg ttc ggg atc ttg aaa      692
Gly Ile Ala Ala Val Pro Ser Leu Val Leu Ala Phe Gly Ile Leu Lys
180                 185                 190                 195
atg ccg gaa tct cca cgg tgg ttg att atg caa ggc cgt ctt aag gaa      740
Met Pro Glu Ser Pro Arg Trp Leu Ile Met Gln Gly Arg Leu Lys Glu
                200                 205                 210
ggc aag gag ata ttg gaa ttg gta tct aat tcc cct gaa gaa gca gaa      788
Gly Lys Glu Ile Leu Glu Leu Val Ser Asn Ser Pro Glu Glu Ala Glu
            215                 220                 225
ctc cgc ttt caa gac atc aaa gct gct gcg gga atc gac ccg aaa tgc      836
Leu Arg Phe Gln Asp Ile Lys Ala Ala Ala Gly Ile Asp Pro Lys Cys
        230                 235                 240
gta gac gat gtt gtg aaa atg gag ggt aag aag act cat ggt gaa gga      884
Val Asp Asp Val Val Lys Met Glu Gly Lys Lys Thr His Gly Glu Gly
    245                 250                 255
gtg tgg aaa gag ctc att cta aga cca act cct gca gtg aga cgt gtt      932
Val Trp Lys Glu Leu Ile Leu Arg Pro Thr Pro Ala Val Arg Arg Val
260                 265                 270                 275
ctt tta act gct ctt ggg att cat ttc ttc caa cac gcc tcc gga atc      980
Leu Leu Thr Ala Leu Gly Ile His Phe Phe Gln His Ala Ser Gly Ile
                280                 285                 290
gaa gca gtg ctt tta tac ggt ccg agg atc ttt aag aaa gca gga atc     1028
Glu Ala Val Leu Leu Tyr Gly Pro Arg Ile Phe Lys Lys Ala Gly Ile
            295                 300                 305
acg act aaa gac aag ctt ttc ttg gtt act atc ggt gtc gga atc atg     1076
Thr Thr Lys Asp Lys Leu Phe Leu Val Thr Ile Gly Val Gly Ile Met
        310                 315                 320
aaa acg acg ttt att ttc act gcg act tta ttg tta gac aag gta ggt     1124
Lys Thr Thr Phe Ile Phe Thr Ala Thr Leu Leu Leu Asp Lys Val Gly
    325                 330                 335
cga agg aag ctt ttg ttg acc agc gtt gga gga atg gtc att gcg ttg     1172
Arg Arg Lys Leu Leu Leu Thr Ser Val Gly Gly Met Val Ile Ala Leu
340                 345                 350                 355
aca atg ttg gga ttt ggg ctt aca atg gcc caa aat gct ggc ggg aaa     1220
Thr Met Leu Gly Phe Gly Leu Thr Met Ala Gln Asn Ala Gly Gly Lys
```

```
                  360                     365                     370
tta gcg tgg gct tta gta ctg agc ata gtt gcg gct tat agt ttc gtg      1268
Leu Ala Trp Ala Leu Val Leu Ser Ile Val Ala Ala Tyr Ser Phe Val
              375                     380                     385
gcg ttt ttc tct att ggg ctc ggc cca ata act tgg gtc tac agt tct      1316
Ala Phe Phe Ser Ile Gly Leu Gly Pro Ile Thr Trp Val Tyr Ser Ser
          390                     395                     400
gag gtt ttc ccg ttg aag ctt agg gca caa gga gcg agt ctc ggc gtt      1364
Glu Val Phe Pro Leu Lys Leu Arg Ala Gln Gly Ala Ser Leu Gly Val
    405                     410                     415
gcg gtg aac aga gta atg aac gcc acc gtg tcg atg tcg ttt ttg tcg      1412
Ala Val Asn Arg Val Met Asn Ala Thr Val Ser Met Ser Phe Leu Ser
420                     425                     430                     435
ttg act agt gcg ata acc acc ggt gga gct ttc ttt atg ttc gcc gga      1460
Leu Thr Ser Ala Ile Thr Thr Gly Gly Ala Phe Phe Met Phe Ala Gly
                  440                     445                     450
gtt gcg gca gtg gcg tgg aat ttc ttc ttc ttc ctc ttg ccg gag acg      1508
Val Ala Ala Val Ala Trp Asn Phe Phe Phe Phe Leu Leu Pro Glu Thr
              455                     460                     465
aaa gga aaa tca ctt gaa gaa atc gaa gcg ctt ttt caa aga gac ggt      1556
Lys Gly Lys Ser Leu Glu Glu Ile Glu Ala Leu Phe Gln Arg Asp Gly
          470                     475                     480
gat aaa gta cgc ggc gaa aac ggt gca gct tag catgatgaat ataatgttat   1609
Asp Lys Val Arg Gly Glu Asn Gly Ala Ala
    485                     490
cattaaatga aacgacagcg tttggggttt ctcactcatt acacgacgtc gtttagttgt   1669
acttttgtgt ggcttttatc aaattataaa tcaacggtca agattgacgc acgctttagt   1729
atttttttca ttaacaattt aagatcaacg gctaatacat c                        1770
```

<210> 32
<211> 493
<212> PRT
<213> Arabidopsis thaliana

<400> 32

```
Met Ala Asp Gln Ile Ser Gly Glu Lys Pro Ala Gly Val Asn Arg Phe
1               5                   10                  15

Ala Leu Gln Cys Ala Ile Val Ala Ser Ile Val Ser Ile Ile Phe Gly
            20                  25                  30

Tyr Asp Thr Gly Val Met Ser Gly Ala Met Val Phe Ile Glu Glu Asp
        35                  40                  45

Leu Lys Thr Asn Asp Val Gln Ile Glu Val Leu Thr Gly Ile Leu Asn
```

50 55 60

Leu Cys Ala Leu Val Gly Ser Leu Leu Ala Gly Arg Thr Ser Asp Ile
65 70 75 80

Ile Gly Arg Arg Tyr Thr Ile Val Leu Ala Ser Ile Leu Phe Met Leu
85 90 95

Gly Ser Ile Leu Met Gly Trp Gly Pro Asn Tyr Pro Val Leu Leu Ser
100 105 110

Gly Arg Cys Thr Ala Gly Leu Gly Val Gly Phe Ala Leu Met Val Ala
115 120 125

Pro Val Tyr Ser Ala Glu Ile Ala Thr Ala Ser His Arg Gly Leu Leu
130 135 140

Ala Ser Leu Pro His Leu Cys Ile Ser Ile Gly Ile Leu Leu Gly Tyr
145 150 155 160

Ile Val Asn Tyr Phe Phe Ser Lys Leu Pro Met His Ile Gly Trp Arg
165 170 175

Leu Met Leu Gly Ile Ala Ala Val Pro Ser Leu Val Leu Ala Phe Gly
180 185 190

Ile Leu Lys Met Pro Glu Ser Pro Arg Trp Leu Ile Met Gln Gly Arg
195 200 205

Leu Lys Glu Gly Lys Glu Ile Leu Glu Leu Val Ser Asn Ser Pro Glu
210 215 220

Glu Ala Glu Leu Arg Phe Gln Asp Ile Lys Ala Ala Ala Gly Ile Asp
225 230 235 240

Pro Lys Cys Val Asp Asp Val Val Lys Met Glu Gly Lys Lys Thr His
245 250 255

Gly Glu Gly Val Trp Lys Glu Leu Ile Leu Arg Pro Thr Pro Ala Val
260 265 270

Arg Arg Val Leu Leu Thr Ala Leu Gly Ile His Phe Phe Gln His Ala
275 280 285

Ser Gly Ile Glu Ala Val Leu Leu Tyr Gly Pro Arg Ile Phe Lys Lys

290 295 300

Ala Gly Ile Thr Thr Lys Asp Lys Leu Phe Leu Val Thr Ile Gly Val
305 310 315 320

Gly Ile Met Lys Thr Thr Phe Ile Phe Thr Ala Thr Leu Leu Leu Asp
325 330 335

Lys Val Gly Arg Arg Lys Leu Leu Leu Thr Ser Val Gly Gly Met Val
340 345 350

Ile Ala Leu Thr Met Leu Gly Phe Gly Leu Thr Met Ala Gln Asn Ala
355 360 365

Gly Gly Lys Leu Ala Trp Ala Leu Val Leu Ser Ile Val Ala Ala Tyr
370 375 380

Ser Phe Val Ala Phe Phe Ser Ile Gly Leu Gly Pro Ile Thr Trp Val
385 390 395 400

Tyr Ser Ser Glu Val Phe Pro Leu Lys Leu Arg Ala Gln Gly Ala Ser
405 410 415

Leu Gly Val Ala Val Asn Arg Val Met Asn Ala Thr Val Ser Met Ser
420 425 430

Phe Leu Ser Leu Thr Ser Ala Ile Thr Thr Gly Gly Ala Phe Phe Met
435 440 445

Phe Ala Gly Val Ala Ala Val Ala Trp Asn Phe Phe Phe Phe Leu Leu
450 455 460

Pro Glu Thr Lys Gly Lys Ser Leu Glu Glu Ile Glu Ala Leu Phe Gln
465 470 475 480

Arg Asp Gly Asp Lys Val Arg Gly Glu Asn Gly Ala Ala
485 490

<210> 33
<211> 1179
<212> DNA
<213> Arabidopsis thaliana

<220>
<221> promoter

<222> (1)..(1179)
<223> transcription regulating sequence from Arabidopsis thaliana gene At3g10920

<400> 33
actagtgaaa agtagagcgc acctattcct aaaagcagaa acccgggcat gaacgaatga 60
tatcatgatc acgatgagta agacatccga agaaacagat aagaaaggca acccggaaaa 120
ctcgaatata agccaggcta cagttgatat aatgataacc cccaaagaaa gatgtcgcct 180
cctccataag agcaagtcag cagctacatc aaagaacaaa gccatacaaa aatcacaaac 240
agtagctttt tgctggacct atgaactcat catgcgtatc cagagatttg aatcaatcaa 300
tttctcattc aaaatcaaac ctaaacctaa ctttcacctg ccaagttttc cattttctac 360
tatacccaaa atgctaaatg tacttacaca catgacgatt ttcacaacca accaaaatca 420
gaaaccctaa ctattccaac tatcatcccc aatttgaatt ttctgttctg taaatccgag 480
aaaattctca tcacaaacta ccaaaatgat gaaaaaacac aaattctgga aaaaagaact 540
cgcctttgcc accacccatg aactgatgaa ccgtaatctg ccgacccaac aatcgataat 600
ccgatgacga tcctccttca tttcttccat cgaaatcacc atcgatatca cttaaactat 660
ccatcggaga attaaaaaaa aaaaaccgaa tttacacaaa ttctccgact tccaggttcc 720
tatttcgatc tcgccgactc cgatttgaaa tttaccttaa agatacgatc tcagtacaat 780
ggtttgtgaa agcagctaga ttgatgggag aatgacagcg attgggtgga ttatcaagga 840
ggaaccaaat agaattggct ttcacatgca tccaacaaat tcaacatttg gaaattctcc 900
gtttctagtc aactttcttc atttctttat ttttttcctg tgaacaaaca aaaaagtaac 960
atttagccgt cacaaaatat taaaaattac gctttattat attaaggcct tgggctcact 1020
aatccaatat tacggcccat aaataaaaat cacatgtcta cgtgtcaaaa cagtcacttt 1080
tctctataca tgacaggcag tgatattatc ataaccctcc acttatacac atgttcattt 1140
ttggcgacca ctagaaggag aaacaaatct tcattccaa 1179

<210> 34
<211> 1187
<212> DNA
<213> Arabidopsis thaliana

<220>
<221> promoter
<222> (1)..(1187)
<223> transcription regulating sequence from Arabidopsis thaliana gene At3g10920

<400> 34
aagaagacta gtgaaaagta gagcgcacct attcctaaaa gcagaaaccc gggcatgaac 60
gaatgatatc atgatcacga tgagtaagac atccgaagaa acagataaga aaggcaaccc 120
ggaaaactcg aatataagcc aggctacagt tgatataatg ataacccccca aagaaagatg 180
tcgcctcctc cataagagca agtcagcagc tacatcaaag aacaaagcca tacaaaaatc 240
acaaacagta gctttttgct ggacctatga actcatcatg cgtatccaga gatttgaatc 300
aatcaatttc tcattcaaaa tcaaacctaa acctaacttt cacctgccaa gttttccatt 360
ttctactata cccaaaatgc taaatgtact tacacacatg acgattttca caaccaacca 420

```
aaatcagaaa ccctaactat tccaactatc atccccaatt tgaattttct gttctgtaaa    480
tccgagaaaa ttctcatcac aaactaccaa aatgatgaaa aaacacaaat tctggaaaaa    540
agaactcgcc tttgccacca cccatgaact gatgaaccgt aatctgccga cccaacaatc    600
gataatccga tgacgatcct ccttcatttc ttccatcgaa atcaccatcg atatcactta    660
aactatccat cggagaatta aaaaaaaaaa accgaattta cacaaattct ccgacttcca    720
ggttcctatt tcgatctcgc cgactccgat ttgaaattta ccttaaagat acgatctcag    780
tacaatggtt tgtgaaagca gctagattga tgggagaatg acagcgattg ggtggattat    840
caaggaggaa ccaaatagaa ttggctttca catgcatcca acaaattcaa catttggaaa    900
ttctccgttt ctagtcaact ttcttcattt ctttattttt ttcctgtgaa caaacaaaaa    960
agtaacattt agccgtcaca aaatattaaa aattacgctt tattatatta aggccttggg   1020
ctcactaatc caatattacg gcccataaat aaaaatcaca tgtctacgtg tcaaaacagt   1080
cacttttctc tatacatgac aggcagtgat attatcataa ccctccactt atacacatgt   1140
tcatttttgg cgaccactag aaggagaaac aaatcttcat tccaaca                  1187
```

<210> 35
<211> 1143
<212> DNA
<213> Arabidopsis thaliana

<220>
<221> promoter
<222> (1)..(1143)
<223> transcription regulating sequence from Arabidopsis thaliana gene At3g10920

<400> 35

```
actagtgaaa agtagagcgc acctattcct aaaagcagaa acccgggcat gaacgaatga     60
tatcatgatc acgatgagta agacatccga agaaacagat aagaaaggca acccggaaaa    120
ctcgaatata agccaggcta cagttgatat aatgataacc cccaaagaaa gatgtcgcct    180
cctccataag agcaagtcag cagctacatc aaagaacaaa gccatacaaa aatcacaaac    240
agtagctttt tgctggacct atgaactcat catgcgtatc cagagatttg aatcaatcaa    300
tttctcattc aaaatcaaac ctaaacctaa ctttcacctg ccaagttttc cattttctac    360
tatacccaaa atgctaaatg tacttacaca catgacgatt ttcacaacca accaaaatca    420
gaaaccctaa ctattccaac tatcatcccc aatttgaatt ttctgttctg taaatccgag    480
aaaattctca tcacaaacta ccaaaatgat gaaaaaacac aaattctgga aaaaagaact    540
cgcctttgcc accacccatg aactgatgaa ccgtaatctg ccgacccaac aatcgataat    600
ccgatgacga tcctccttca tttcttccat cgaaatcacc atcgatatca cttaaactat    660
ccatcggaga attaaaaaaa aaaaaccgaa tttacacaaa ttctccgact tccaggttcc    720
tatttcgatc tcgccgactc cgatttgaaa tttaccttaa agatacgatc tcagtacaat    780
ggtttgtgaa agcagctaga ttgatgggag aatgacagcg attgggtgga ttatcaagga    840
ggaaccaaat agaattggct ttcacatgca tccaacaaat tcaacatttg gaaattctcc    900
gtttctagtc aactttcttc atttctttat ttttttcctg tgaacaaaca aaaaagtaac    960
atttagccgt cacaaaatat taaaaattac gctttattat attaaggcct tgggctcact   1020
aatccaatat tacggcccat aaataaaaat cacatgtcta cgtgtcaaaa cagtcacttt   1080
tctctataca tgacaggcag tgatattatc ataaccctcc acttatacac atgttcattt   1140
```

ttg 1143

<210> 36
<211> 1149
<212> DNA
<213> Arabidopsis thaliana

<220>
<221> promoter
<222> (1)..(1149)
<223> transcription regulating sequence from Arabidopsis thaliana gene At3g10920

<400> 36
aagaagacta gtgaaaagta gagcgcacct attcctaaaa gcagaaaccc gggcatgaac 60
gaatgatatc atgatcacga tgagtaagac atccgaagaa acagataaga aaggcaaccc 120
ggaaaactcg aatataagcc aggctacagt tgatataatg ataacccca aagaaagatg 180
tcgcctcctc cataagagca agtcagcagc tacatcaaag aacaaagcca tacaaaaatc 240
acaaacagta gctttttgct ggacctatga actcatcatg cgtatccaga gatttgaatc 300
aatcaatttc tcattcaaaa tcaaacctaa acctaacttt cacctgccaa gttttccatt 360
ttctactata cccaaaatgc taaatgtact tacacacatg acgattttca caaccaacca 420
aaatcagaaa ccctaactat tccaactatc atccccaatt tgaattttct gttctgtaaa 480
tccgagaaaa ttctcatcac aaactaccaa aatgatgaaa aaacacaaat tctggaaaaa 540
agaactcgcc tttgccacca cccatgaact gatgaaccgt aatctgccga cccaacaatc 600
gataatccga tgacgatcct ccttcatttc ttccatcgaa atcaccatcg atatcactta 660
aactatccat cggagaatta aaaaaaaaaa accgaattta cacaaattct ccgacttcca 720
ggttcctatt tcgatctcgc cgactccgat ttgaaattta ccttaaagat acgatctcag 780
tacaatggtt tgtgaaagca gctagattga tgggagaatg acagcgattg ggtggattat 840
caaggaggaa ccaaatagaa ttggctttca catgcatcca acaaattcaa catttggaaa 900
ttctccgttt ctagtcaact ttcttcattt ctttattttt ttcctgtgaa caaacaaaaa 960
agtaacattt agccgtcaca aaatattaaa aattacgctt tattatatta aggccttggg 1020
ctcactaatc caatattacg gcccataaat aaaaatcaca tgtctacgtg tcaaaacagt 1080
cacttttctc tatacatgac aggcagtgat attatcataa ccctccactt atacacatgt 1140
tcattttg 1149

<210> 37
<211> 2419
<212> DNA
<213> Arabidopsis thaliana

<220>
<221> promoter
<222> (1)..(2419)
<223> transcription regulating sequence from Arabidopsis thaliana gene At3g10920

<400> 37

```
gaattcgtag gaatacgtaa tagagtttga tcatcttcat tgatatgacc atgtataata     60
ctataacaac acaagtgact acacctactc aatcatgtag tatagtcatt acttgtagac    120
ttgttgtagc actgtttttc ttttcctgac gtcatatttg tagcaagtcc gattaaattg    180
tgtaagtgag gttcttctgt gacgtgaaca cgtgacctaa cattctcagc atatgggcat    240
taaagttggg ctagtcaaat ccatctgaaa tgtgagtatt gttctgcgcc acgacgtcgt    300
ttaacgaaca gcggtgttaa actttttttg tatttttttcc tccagtggtg ttaagcttca    360
gacgaaggca agagttaatt tagttgaaca aagaaaccat cttatattca ttctggatat    420
ggcgtttagt tatactacaa atggtgaata acaagaaaag ctaaacaaaa acaagtaaaa    480
gcattctcag aatcaccaaa gttaagctca tgaatcttta tccttggata agctccatga    540
gagtctgctt ataacatttt cgtctactat tttgtagtgg tgagacaacc gcctgtgaat    600
cgtcccgcaa cacttgtcta ctttgctttg atatttactg tacaaagccg gtattgttac    660
agataggatg gtccctgttc acaccataaa aagtataaac ttcatataag aatttggttc    720
actgagttta aaacaacaat gtcgcaggaa ctaaaacgag atcaacaagc caacgtgggg    780
gatgaaactg aaggatcaaa aaacatagaa gctttttcct ttcatttgca tttaaggatc    840
aagttactaa ccgatgtaaa gaagagtgca gagagatata tagctaccaa tggcagagag    900
aagccacaga caaatcacca cctgctaaag aaaaatcgtc agaccaacag ttcagatgct    960
taacggttcc catgtcattt cactcaacat catcgtgagt ttctgaatgg gttagcttca   1020
taaccttgaa gaagagtcga aaatcgttgc caactgtgac atcatgagcc attacaagca   1080
agtgattgag ttttatacgg aaagaagctg cagcactatt caccatttcc tctgatagaa   1140
caagctctgg caacgaatgc aactgtctgc ttataaagag ataacaggta gaaatcatca   1200
ctaatcagtc acataacagc aaagaattaa gaaaaagaag actagtgaaa agtagagcgc   1260
acctattcct aaaagcagaa acccgggcat gaacgaatga tatcatgatc acgatgagta   1320
agacatccga agaaacagat aagaaaggca acccggaaaa ctcgaatata agccaggcta   1380
cagttgatat aatgataacc cccaaagaaa gatgtcgcct cctccataag agcaagtcag   1440
cagctacatc aaagaacaaa gccatacaaa aatcacaaac agtagctttt tgctggacct   1500
atgaactcat catgcgtatc cagagatttg aatcaatcaa tttctcattc aaaatcaaac   1560
ctaaacctaa ctttcacctg ccaagttttc cattttctac tatacccaaa atgctaaatg   1620
tacttacaca catgacgatt ttcacaacca accaaaatca gaaaccctaa ctattccaac   1680
tatcatcccc aatttgaatt ttctgttctg taaatccgag aaaattctca tcacaaacta   1740
ccaaaatgat gaaaaaacac aaattctgga aaaaagaact cgcctttgcc accacccatg   1800
aactgatgaa ccgtaatctg ccgacccaac aatcgataat ccgatgacga tcctccttca   1860
tttcttccat cgaaatcacc atcgatatca cttaaactat ccatcggaga attaaaaaaa   1920
aaaaaccgaa tttacacaaa ttctccgact tccaggttcc tatttcgatc tcgccgactc   1980
cgatttgaaa tttaccttaa agatacgatc tcagtacaat ggtttgtgaa agcagctaga   2040
ttgatgggag aatgacagcg attgggtgga ttatcaagga ggaaccaaat agaattggct   2100
ttcacatgca tccaacaaat tcaacatttg gaaattctcc gtttctagtc aactttcttc   2160
atttctttat ttttttcctg tgaacaaaca aaaaagtaac atttagccgt cacaaaatat   2220
taaaaattac gctttattat attaaggcct tgggctcact aatccaatat tacggcccat   2280
aaataaaaat cacatgtcta cgtgtcaaaa cagtcacttt tctctataca tgacaggcag   2340
tgatattatc ataaccctcc acttatacac atgttcattt ttggcgacca ctagaaggag   2400
aaacaaatct tcattccaa                                                 2419
```

<210> 38

<211> 2427
<212> DNA
<213> Arabidopsis thaliana

<220>
<221> promoter
<222> (1)..(2427)
<223> transcription regulating sequence from Arabidopsis thaliana gene At3g10920

<400> 38

```
aaaatggaat tcgtaggaat acgtaataga gtttgatcat cttcattgat atgaccatgt     60
ataatactat aacaacacaa gtgactacac ctactcaatc atgtagtata gtcattactt    120
gtagacttgt tgtagcactg tttttctttt cctgacgtca tatttgtagc aagtccgatt    180
aaattgtgta agtgaggttc ttctgtgacg tgaacacgtg acctaacatt ctcagcatat    240
gggcattaaa gttgggctag tcaaatccat ctgaaatgtg agtattgttc tgcgccacga    300
cgtcgtttaa cgaacagcgg tgttaaactt tttttgtatt ttttcctcca gtggtgttaa    360
gcttcagacg aaggcaagag ttaatttagt tgaacaaaga aaccatctta tattcattct    420
ggatatggcg tttagttata ctacaaatgg tgaataacaa gaaaagctaa acaaaaacaa    480
gtaaaagcat tctcagaatc accaaagtta agctcatgaa tctttatcct tggataagct    540
ccatgagagt ctgcttataa cattttcgtc tactattttg tagtggtgag acaaccgcct    600
gtgaatcgtc ccgcaacact tgtctacttt gctttgatat ttactgtaca aagccggtat    660
tgttacagat aggatggtcc ctgttcacac cataaaaagt ataaacttca tataagaatt    720
tggttcactg agtttaaaac aacaatgtcg caggaactaa aacgagatca acaagccaac    780
gtgggggatg aaactgaagg atcaaaaaac atagaagctt tttcctttca tttgcattta    840
aggatcaagt tactaaccga tgtaaagaag agtgcagaga gatatatagc taccaatggc    900
agagagaagc cacagacaaa tcaccacctg ctaaagaaaa atcgtcagac caacagttca    960
gatgcttaac ggttcccatg tcatttcact caacatcatc gtgagtttct gaatgggtta   1020
gcttcataac cttgaagaag agtcgaaaat cgttgccaac tgtgacatca tgagccatta   1080
caagcaagtg attgagtttt atacggaaag aagctgcagc actattcacc atttcctctg   1140
atagaacaag ctctggcaac gaatgcaact gtctgcttat aaagagataa caggtagaaa   1200
tcatcactaa tcagtcacat aacagcaaag aattaagaaa aagaagacta gtgaaaagta   1260
gagcgcacct attcctaaaa gcagaaaccc gggcatgaac gaatgatatc atgatcacga   1320
tgagtaagac atccgaagaa acagataaga aaggcaaccc ggaaaactcg aatataagcc   1380
aggctacagt tgatataatg ataacccccca aagaaagatg tcgcctcctc cataagagca   1440
agtcagcagc tacatcaaag aacaaagcca tacaaaaatc acaaacagta gctttttgct   1500
ggacctatga actcatcatg cgtatccaga gatttgaatc aatcaatttc tcattcaaaa   1560
tcaaacctaa acctaacttt cacctgccaa gttttccatt ttctactata cccaaaatgc   1620
taaatgtact tacacacatg acgattttca caaccaacca aaatcagaaa ccctaactat   1680
tccaactatc atccccaatt tgaattttct gttctgtaaa tccgagaaaa ttctcatcac   1740
aaactaccaa aatgatgaaa aaacacaaat tctggaaaaa agaactcgcc tttgccacca   1800
cccatgaact gatgaaccgt aatctgccga cccaacaatc gataatccga tgacgatcct   1860
ccttcatttc ttccatcgaa atcaccatcg atatcactta aactatccat cggagaatta   1920
aaaaaaaaaa accgaattta cacaaattct ccgacttcca ggttcctatt tcgatctcgc   1980
cgactccgat ttgaaattta ccttaaagat acgatctcag tacaatggtt tgtgaaagca   2040
```

gctagattga tgggagaatg acagcgattg ggtggattat caaggaggaa ccaaatagaa 2100
ttggctttca catgcatcca acaaattcaa catttggaaa ttctccgttt ctagtcaact 2160
ttcttcattt ctttattttt ttcctgtgaa caaacaaaaa agtaacattt agccgtcaca 2220
aaatattaaa aattacgctt tattatatta aggccttggg ctcactaatc caatattacg 2280
gcccataaat aaaaatcaca tgtctacgtg tcaaaacagt cacttttctc tatacatgac 2340
aggcagtgat attatcataa ccctccactt atacacatgt tcatttttgg cgaccactag 2400
aaggagaaac aaatcttcat tccaaca 2427

<210> 39
<211> 2383
<212> DNA
<213> Arabidopsis thaliana

<220>
<221> promoter
<222> (1)..(2383)
<223> transcription regulating sequence from Arabidopsis thaliana gene At3g10920

<400> 39
gaattcgtag gaatacgtaa tagagtttga tcatcttcat tgatatgacc atgtataata 60
ctataacaac acaagtgact acacctactc aatcatgtag tatagtcatt acttgtagac 120
ttgttgtagc actgtttttc ttttcctgac gtcatatttg tagcaagtcc gattaaattg 180
tgtaagtgag gttcttctgt gacgtgaaca cgtgacctaa cattctcagc atatgggcat 240
taaagttggg ctagtcaaat ccatctgaaa tgtgagtatt gttctgcgcc acgacgtcgt 300
ttaacgaaca gcggtgttaa actttttttg tatttttttcc tccagtggtg ttaagcttca 360
gacgaaggca agagttaatt tagttgaaca aagaaaccat cttatattca ttctggatat 420
ggcgtttagt tatactacaa atggtgaata acaagaaaag ctaaacaaaa acaagtaaaa 480
gcattctcag aatcaccaaa gttaagctca tgaatcttta tccttggata agctccatga 540
gagtctgctt ataacatttt cgtctactat tttgtagtgg tgagacaacc gcctgtgaat 600
cgtcccgcaa cacttgtcta ctttgctttg atatttactg tacaaagccg gtattgttac 660
agataggatg gtccctgttc acaccataaa aagtataaac ttcatataag aatttggttc 720
actgagttta aaacaacaat gtcgcaggaa ctaaaacgag atcaacaagc caacgtgggg 780
gatgaaactg aaggatcaaa aaacatagaa gctttttcct ttcatttgca tttaaggatc 840
aagttactaa ccgatgtaaa gaagagtgca gagagatata tagctaccaa tggcagagag 900
aagccacaga caaatcacca cctgctaaag aaaaatcgtc agaccaacag ttcagatgct 960
taacggttcc catgtcattt cactcaacat catcgtgagt ttctgaatgg gttagcttca 1020
taaccttgaa gaagagtcga aaatcgttgc caactgtgac atcatgagcc attacaagca 1080
agtgattgag ttttatacgg aaagaagctg cagcactatt caccatttcc tctgatagaa 1140
caagctctgg caacgaatgc aactgtctgc ttataaagag ataacaggta gaaatcatca 1200
ctaatcagtc acataacagc aaagaattaa gaaaaagaag actagtgaaa agtagagcgc 1260
acctattcct aaaagcagaa acccgggcat gaacgaatga tatcatgatc acgatgagta 1320
agacatccga agaaacagat aagaaaggca acccggaaaa ctcgaatata agccaggcta 1380
cagttgatat aatgataacc cccaaagaaa gatgtcgcct cctccataag agcaagtcag 1440
cagctacatc aaagaacaaa gccatacaaa aatcacaaac agtagctttt tgctggacct 1500

```
atgaactcat catgcgtatc cagagatttg aatcaatcaa tttctcattc aaaatcaaac   1560
ctaaacctaa ctttcacctg ccaagttttc cattttctac tatacccaaa atgctaaatg   1620
tacttacaca catgacgatt ttcacaacca accaaaatca gaaaccctaa ctattccaac   1680
tatcatcccc aatttgaatt ttctgttctg taaatccgag aaaattctca tcacaaacta   1740
ccaaaatgat gaaaaaacac aaattctgga aaaaagaact cgcctttgcc accacccatg   1800
aactgatgaa ccgtaatctg ccgacccaac aatcgataat ccgatgacga tcctccttca   1860
tttcttccat cgaaatcacc atcgatatca cttaaactat ccatcggaga attaaaaaaa   1920
aaaaaccgaa tttacacaaa ttctccgact tccaggttcc tatttcgatc tcgccgactc   1980
cgatttgaaa tttaccttaa agatacgatc tcagtacaat ggtttgtgaa agcagctaga   2040
ttgatgggag aatgacagcg attgggtgga ttatcaagga ggaaccaaat agaattggct   2100
ttcacatgca tccaacaaat tcaacatttg gaaattctcc gtttctagtc aactttcttc   2160
atttctttat tttttcctg tgaacaaaca aaaaagtaac atttagccgt cacaaaatat   2220
taaaaattac gctttattat attaaggcct tgggctcact aatccaatat tacggcccat   2280
aaataaaaat cacatgtcta cgtgtcaaaa cagtcacttt tctctataca tgacaggcag   2340
tgatattatc ataaccctcc acttatacac atgttcattt ttg                     2383
```

<210> 40
<211> 2389
<212> DNA
<213> Arabidopsis thaliana

<220>
<221> promoter
<222> (1)..(2389)
<223> transcription regulating sequence from Arabidopsis thaliana gene At3g10920

<400> 40

```
aaaatggaat tcgtaggaat acgtaataga gtttgatcat cttcattgat atgaccatgt     60
ataatactat aacaacacaa gtgactacac ctactcaatc atgtagtata gtcattactt    120
gtagacttgt tgtagcactg tttttctttt cctgacgtca tatttgtagc aagtccgatt    180
aaattgtgta agtgaggttc ttctgtgacg tgaacacgtg acctaacatt ctcagcatat    240
gggcattaaa gttgggctag tcaaatccat ctgaaatgtg agtattgttc tgcgccacga    300
cgtcgtttaa cgaacagcgg tgttaaactt tttttgtatt ttttcctcca gtggtgttaa    360
gcttcagacg aaggcaagag ttaatttagt tgaacaaaga aaccatctta tattcattct    420
ggatatggcg tttagttata ctacaaatgg tgaataacaa gaaaagctaa acaaaaacaa    480
gtaaaagcat tctcagaatc accaaagtta agctcatgaa tctttatcct tggataagct    540
ccatgagagt ctgcttataa cattttcgtc tactattttg tagtggtgag acaaccgcct    600
gtgaatcgtc ccgcaacact tgtctacttt gctttgatat ttactgtaca aagccggtat    660
tgttacagat aggatggtcc ctgttcacac cataaaaagt ataaacttca tataagaatt    720
tggttcactg agtttaaaac aacaatgtcg caggaactaa aacgagatca acaagccaac    780
gtgggggatg aaactgaagg atcaaaaaac atagaagctt tttcctttca tttgcattta    840
aggatcaagt tactaaccga tgtaaagaag agtgcagaga gatatatagc taccaatggc    900
agagagaagc cacagacaaa tcaccacctg ctaaagaaaa atcgtcagac caacagttca    960
gatgcttaac ggttcccatg tcatttcact caacatcatc gtgagtttct gaatgggtta   1020
```

```
gcttcataac cttgaagaag agtcgaaaat cgttgccaac tgtgacatca tgagccatta   1080
caagcaagtg attgagtttt atacggaaag aagctgcagc actattcacc atttcctctg   1140
atagaacaag ctctggcaac gaatgcaact gtctgcttat aaagagataa caggtagaaa   1200
tcatcactaa tcagtcacat aacagcaaag aattaagaaa aagaagacta gtgaaaagta   1260
gagcgcacct attcctaaaa gcagaaaccc gggcatgaac gaatgatatc atgatcacga   1320
tgagtaagac atccgaagaa acagataaga aaggcaaccc ggaaaactcg aatataagcc   1380
aggctacagt tgatataatg ataacccca aagaaagatg tcgcctcctc cataagagca   1440
agtcagcagc tacatcaaag aacaaagcca tacaaaaatc acaaacagta gctttttgct   1500
ggacctatga actcatcatg cgtatccaga gatttgaatc aatcaatttc tcattcaaaa   1560
tcaaacctaa acctaacttt cacctgccaa gttttccatt ttctactata cccaaaatgc   1620
taaatgtact tacacacatg acgattttca caaccaacca aaatcagaaa ccctaactat   1680
tccaactatc atccccaatt tgaattttct gttctgtaaa tccgagaaaa ttctcatcac   1740
aaactaccaa aatgatgaaa aaacacaaat tctggaaaaa agaactcgcc tttgccacca   1800
cccatgaact gatgaaccgt aatctgccga cccaacaatc gataatccga tgacgatcct   1860
ccttcatttc ttccatcgaa atcaccatcg atatcactta aactatccat cggagaatta   1920
aaaaaaaaaa accgaattta cacaaattct ccgacttcca ggttcctatt tcgatctcgc   1980
cgactccgat ttgaaattta ccttaaagat acgatctcag tacaatggtt tgtgaaagca   2040
gctagattga tgggagaatg acagcgattg ggtggattat caaggaggaa ccaaatagaa   2100
ttggctttca catgcatcca acaaattcaa catttggaaa ttctccgttt ctagtcaact   2160
ttcttcattt ctttattttt ttcctgtgaa caaacaaaaa agtaacattt agccgtcaca   2220
aaatattaaa aattacgctt tattatatta aggccttggg ctcactaatc caatattacg   2280
gcccataaat aaaaatcaca tgtctacgtg tcaaaacagt cacttttctc tatacatgac   2340
aggcagtgat attatcataa ccctccactt atacacatgt tcatttttg               2389
```

```
<210>  41
<211>  1006
<212>  DNA
<213>  Arabidopsis thaliana

<220>
<221>  CDS
<222>  (39)..(734)
<223>  encoding superoxide dismutase [Mn], mitochondrial SODA /
       manganese superoxide dismutase (MSD1)

<400>  41
gcgaccacta gaaggagaaa caaatcttca ttccaaca atg gcg att cgt tgt gta      56
                                          Met Ala Ile Arg Cys Val
                                          1               5

gcg agt aga aaa acc cta gcc ggc ttg aag gag aca tca tcg agg cta       104
Ala Ser Arg Lys Thr Leu Ala Gly Leu Lys Glu Thr Ser Ser Arg Leu
            10                  15                  20

ttg agg atc aga ggg att cag act ttt acg ctt cct gat ctt cct tac       152
Leu Arg Ile Arg Gly Ile Gln Thr Phe Thr Leu Pro Asp Leu Pro Tyr
        25                  30                  35
```

```
gat tat ggc gca ttg gaa ccg gcc att agt gga gag atc atg cag att      200
Asp Tyr Gly Ala Leu Glu Pro Ala Ile Ser Gly Glu Ile Met Gln Ile
    40                  45                  50
cat cac cag aag cat cac cag gct tat gtt act aat tac aat aat gct      248
His His Gln Lys His His Gln Ala Tyr Val Thr Asn Tyr Asn Asn Ala
55                  60                  65                  70
ctt gag cag ctt gat caa gct gtg aac aag gga gat gct tcc act gtt      296
Leu Glu Gln Leu Asp Gln Ala Val Asn Lys Gly Asp Ala Ser Thr Val
                75                  80                  85
gtt aag ttg cag agc gcc atc aaa ttc aac ggc gga ggt cat gtc aac      344
Val Lys Leu Gln Ser Ala Ile Lys Phe Asn Gly Gly Gly His Val Asn
            90                  95                  100
cat tcg att ttc tgg aag aac ctt gct cct tcc agt gaa ggt ggt gga      392
His Ser Ile Phe Trp Lys Asn Leu Ala Pro Ser Ser Glu Gly Gly Gly
        105                 110                 115
gag cca cca aaa gga tct ctt ggt agt gcc att gac gct cac ttt ggc      440
Glu Pro Pro Lys Gly Ser Leu Gly Ser Ala Ile Asp Ala His Phe Gly
    120                 125                 130
tcc ctt gaa ggt ctg gtg aaa aag atg agt gct gag ggt gct gca gtg      488
Ser Leu Glu Gly Leu Val Lys Lys Met Ser Ala Glu Gly Ala Ala Val
135                 140                 145                 150
caa ggc tca gga tgg gtg tgg ctc gga cta gac aaa gaa ctg aag aag      536
Gln Gly Ser Gly Trp Val Trp Leu Gly Leu Asp Lys Glu Leu Lys Lys
                155                 160                 165
cta gtt gtt gac aca act gcc aat cag gat cca tta gtg aca aaa gga      584
Leu Val Val Asp Thr Thr Ala Asn Gln Asp Pro Leu Val Thr Lys Gly
            170                 175                 180
gga agc ttg gta cct ctg gtg ggt ata gat gtt tgg gag cac gcc tac      632
Gly Ser Leu Val Pro Leu Val Gly Ile Asp Val Trp Glu His Ala Tyr
        185                 190                 195
tac ttg cag tac aaa aat gtg agg cct gag tat ctg aag aat gta tgg      680
Tyr Leu Gln Tyr Lys Asn Val Arg Pro Glu Tyr Leu Lys Asn Val Trp
    200                 205                 210
aaa gtg atc aac tgg aaa tat gca agc gag gtt tat gag aag gaa aac      728
Lys Val Ile Asn Trp Lys Tyr Ala Ser Glu Val Tyr Glu Lys Glu Asn
215                 220                 225                 230
aac tga atcgtttaca cgatgacata aggagatgaa ccagttccag ctcagctttt       784
Asn

gttttaaggt tgtctgaaac aaacttacag tgtctctttg gtttttaaga tttgctcaac    844
tcagctgtgt ggtacgttgt tttacaatga aagttttcaa gaataaaaat ttgctattat    904
tgtcagaaag cgctattgtt tattctacga agaaaacaaa acggaatctt attggtataa    964
taattacctc atttcaataa aacaataact aattttctcg tt                      1006
```

<210> 42

<211> 231
<212> PRT
<213> Arabidopsis thaliana

<400> 42

```
Met Ala Ile Arg Cys Val Ala Ser Arg Lys Thr Leu Ala Gly Leu Lys
1               5                   10                  15

Glu Thr Ser Ser Arg Leu Leu Arg Ile Arg Gly Ile Gln Thr Phe Thr
            20                  25                  30

Leu Pro Asp Leu Pro Tyr Asp Tyr Gly Ala Leu Glu Pro Ala Ile Ser
        35                  40                  45

Gly Glu Ile Met Gln Ile His His Gln Lys His His Gln Ala Tyr Val
    50                  55                  60

Thr Asn Tyr Asn Asn Ala Leu Glu Gln Leu Asp Gln Ala Val Asn Lys
65                  70                  75                  80

Gly Asp Ala Ser Thr Val Val Lys Leu Gln Ser Ala Ile Lys Phe Asn
                85                  90                  95

Gly Gly Gly His Val Asn His Ser Ile Phe Trp Lys Asn Leu Ala Pro
            100                 105                 110

Ser Ser Glu Gly Gly Gly Glu Pro Pro Lys Gly Ser Leu Gly Ser Ala
        115                 120                 125

Ile Asp Ala His Phe Gly Ser Leu Glu Gly Leu Val Lys Lys Met Ser
    130                 135                 140

Ala Glu Gly Ala Ala Val Gln Gly Ser Gly Trp Val Trp Leu Gly Leu
145                 150                 155                 160

Asp Lys Glu Leu Lys Lys Leu Val Val Asp Thr Thr Ala Asn Gln Asp
                165                 170                 175

Pro Leu Val Thr Lys Gly Gly Ser Leu Val Pro Leu Val Gly Ile Asp
            180                 185                 190

Val Trp Glu His Ala Tyr Tyr Leu Gln Tyr Lys Asn Val Arg Pro Glu
        195                 200                 205

Tyr Leu Lys Asn Val Trp Lys Val Ile Asn Trp Lys Tyr Ala Ser Glu
```

210 215 220

Val Tyr Glu Lys Glu Asn Asn
225 230

<210> 43
<211> 1009
<212> DNA
<213> Arabidopsis thaliana

<220>
<221> promoter
<222> (1)..(1009)
<223> transcription regulating sequence from Arabidopsis thaliana gene At1g33240

<400> 43
gttggtagtg agtgggtttg gctttggctt cgcataaact aatgcatggg tcaggttatc 60
gatccgacct gccaagatag ttccgagttt aaaattacat ggttatgtta attaattatg 120
cactatgtat gattcataaa ataatttcac tggctttgga cttactattt ttttcttttc 180
ttgacatcac taaaacacta ttgtttgata tgtttctata aatgtattat ttacacaata 240
acaaaaaaaa agagagaaag aaactgttga attggagagg aaagagagtg gggggaaaga 300
tgtcaaggct ggtccagctc aaatcgggta aaaactatta tcaaattcca attctgtccc 360
tccctttccc caaaaatctg attgtcacat accctaccaa tcagacaacg ccacgtggac 420
accttagaca tgatggtggg agaggacaac aacatgggag gcacatgatt ttgtgtttat 480
tatatttctc caaaacacaa tttttaccca aaaacaattt aatcggatca ctgagcaatg 540
agatgtttgc ggatccaaat ttaactttta aagccttcct tactctttgc tttataacaa 600
taataataat acacataaaa ttggatttaa ttaaatagat gtggaccttt ttttctaaat 660
aatataaaag aaggaagaaa ataaaataaa taaagaaaaa gagagagtga agaaaagtgg 720
gtttaacaat aatttgctgc aaagttagaa gaggcttcag ctttattaac ttcttgtttg 780
ctttcattct ctttctctct atctatcatc atcatcctcg taatcctctc ctttctcctt 840
cagacccctc ctagttgctg tttaaacaaa caaaaaaaga ctgcttctct ttcttatttc 900
tctcatcatc atcatcaatg gttttatgag atttatatca aaaaacattg aggagctaga 960
gagaaagaga gagtgtgtgt gtagaaaaag attgaaacat catcaagat 1009

<210> 44
<211> 1023
<212> DNA
<213> Arabidopsis thaliana

<220>
<221> promoter
<222> (1)..(1023)
<223> transcription regulating sequence from Arabidopsis thaliana gene At1g33240

<400> 44

```
atctgttata gtgttggtag tgagtgggtt tggctttggc ttcgcataaa ctaatgcatg     60
ggtcaggtta tcgatccgac ctgccaagat agttccgagt ttaaaattac atggttatgt    120
taattaatta tgcactatgt atgattcata aaataatttc actggctttg gacttactat    180
ttttttcttt tcttgacatc actaaaacac tattgtttga tatgtttcta taaatgtatt    240
atttacacaa taacaaaaaa aaagagagaa agaaactgtt gaattggaga ggaaagagag    300
tgggggggaaa gatgtcaagg ctggtccagc tcaaatcggg taaaaactat tatcaaattc    360
caattctgtc cctccctttc cccaaaaatc tgattgtcac ataccctacc aatcagacaa    420
cgccacgtgg acaccttaga catgatggtg ggagaggaca acaacatggg aggcacatga    480
ttttgtgttt attatatttc tccaaaacac aatttttacc caaaaacaat ttaatcggat    540
cactgagcaa tgagatgttt gcggatccaa atttaacttt taaagccttc cttactcttt    600
gctttataac aataataata atacacataa aattggattt aattaaatag atgtggacct    660
tttttctaa ataatataaa agaaggaaga aaataaaata aataaagaaa aagagagagt    720
gaagaaaagt gggtttaaca ataatttgct gcaaagttag aagaggcttc agctttatta    780
acttcttgtt tgctttcatt ctctttctct ctatctatca tcatcatcct cgtaatcctc    840
tcctttctcc ttcagacccc tcctagttgc tgtttaaaca aacaaaaaaa gactgcttct    900
ctttcttatt tctctcatca tcatcatcaa tggttttatg agatttatat caaaaaacat    960
tgaggagcta gagagaaaga gagagtgtgt gtgtagaaaa agattgaaac atcatcaaga   1020
ttg                                                                1023
```

<210> 45
<211> 785
<212> DNA
<213> Arabidopsis thaliana

<220>
<221> promoter
<222> (1)..(785)
<223> transcription regulating sequence from Arabidopsis thaliana gene At1g33240

<400> 45

```
gttggtagtg agtgggtttg gctttggctt cgcataaact aatgcatggg tcaggttatc     60
gatccgacct gccaagatag ttccgagttt aaaattacat ggttatgtta attaattatg    120
cactatgtat gattcataaa ataatttcac tggctttgga cttactattt ttttctttc    180
ttgacatcac taaaacacta ttgtttgata tgtttctata aatgtattat ttacacaata    240
acaaaaaaaa agagagaaag aaactgttga attggagagg aaagagagtg gggggaaaga    300
tgtcaaggct ggtccagctc aaatcgggta aaaactatta tcaaattcca attctgtccc    360
tccctttccc caaaaatctg attgtcacat accctaccaa tcagacaacg ccacgtggac    420
accttagaca tgatggtggg agaggacaac aacatgggag gcacatgatt ttgtgtttat    480
tatatttctc caaaacacaa tttttaccca aaaacaattt aatcggatca ctgagcaatg    540
agatgtttgc ggatccaaat ttaactttta aagccttcct tactctttgc tttataacaa    600
taataataat acacataaaa ttggatttaa ttaaatagat gtggaccttt ttttctaaat    660
aatataaaag aaggaagaaa ataaaataaa taaagaaaaa gagagagtga agaaaagtgg    720
```

gtttaacaat aatttgctgc aaagttagaa gaggcttcag ctttattaac ttcttgtttg 780
ctttc 785

<210> 46
<211> 797
<212> DNA
<213> Arabidopsis thaliana

<220>
<221> promoter
<222> (1)..(797)
<223> transcription regulating sequence from Arabidopsis thaliana gene At1g33240

<400> 46
atctgttata gtgttggtag tgagtgggtt tggctttggc ttcgcataaa ctaatgcatg 60
ggtcaggtta tcgatccgac ctgccaagat agttccgagt ttaaaattac atggttatgt 120
taattaatta tgcactatgt atgattcata aaataatttc actggctttg gacttactat 180
ttttttcttt tcttgacatc actaaaacac tattgtttga tatgtttcta taaatgtatt 240
atttacacaa taacaaaaaa aaagagagaa agaaactgtt gaattggaga ggaaagagag 300
tgggggggaaa gatgtcaagg ctggtccagc tcaaatcggg taaaaactat tatcaaattc 360
caattctgtc cctccctttc cccaaaaatc tgattgtcac ataccctacc aatcagacaa 420
cgccacgtgg acaccttaga catgatggtg ggagaggaca acaacatggg aggcacatga 480
ttttgtgttt attatatttc tccaaaacac aatttttacc caaaaacaat ttaatcggat 540
cactgagcaa tgagatgttt gcggatccaa atttaacttt taaagccttc cttactcttt 600
gctttataac aataataata atacacataa aattggattt aattaaatag atgtggacct 660
ttttttctaa ataatataaa agaaggaaga aaataaaata aataaagaaa aagagagagt 720
gaagaaaagt gggtttaaca ataatttgct gcaaagttag aagaggcttc agctttatta 780
acttcttgtt tgctttc 797

<210> 47
<211> 2819
<212> DNA
<213> Arabidopsis thaliana

<220>
<221> promoter
<222> (1)..(2819)
<223> transcription regulating sequence from Arabidopsis thaliana gene At1g33240

<400> 47
agagggtgag gtaagtggtc cataggattg tggatttagt gcggatttaa ggaaaggtct 60
ttggttggat agatttttaa gatgtgtgtg tgtgttctaa aactaagggt ttaaaatcaa 120
gagtgttgtg gtgtgagttg acttgtcatg aactctctga cacactggca aagtgagccc 180

```
aggttttgct agttgttttg tcgtaagcct tgaaattgac attatgatgc atcatggctt     240
ctatttaaag gggtcatat agttaatgca atgatcaaaa tagccccgag ttagattttt     300
ggtgagttag tagtgtaagt aaattctttg gattttgact ttgtgaggta tttatttatt     360
tggctgccac tgaaattgta gatatatttg tgagaggagg aatatggatt ttacgtgggt     420
gtgaaaattc atggaattac aaaaaaaaat atcgctggac cattttaact ataaatttgt     480
tttgtgaccc cgtcaaaatg atgttgactc gttatctttg gtaagtcctc gtatgacaca     540
agttgggaaa gtaatgagtt tgacaacttg ttgtgtccta ataagattag atttattggt     600
agagagagac gttgcttttg tgtgtttaag attagtgtgt taatcaaagt cttaatcaaa     660
actccctcat gagatctaga tctcttatgt aaaaagagag attcattcag tcagaaatca     720
aggaaggaaa ctccgtgagc atgttcttac cacctcgtat aattatattc ttattatact     780
tgtactaata ttaaagttgt ataattagga aaaaaagaa catatcacat gtaataagta     840
ataaatatag tgaaaaagga gaggcatgtg aaagtggggg gaagaagaag agaagacagc     900
tgtgtatccg ccgctctctc tctttctttc cccttcaaga aagttctcca cttccaccgc     960
ccaccttctt cttcttcttc accttcttct ctaaccatta ttattaatta gcatctttca    1020
ttttgttgtt agaaggaaat agtttgtttt cctttttttgt gtataactag aaatgagcgg   1080
tgtgtttttg tgtagagtgt gatttggtaa tgaaaatgag ttttttttt attttgtgta     1140
tgtttgtgta ggtgagaggt attaaagagt tgtataatgt gatagtatga gtgtctgcat    1200
cgatgtcttg agtttgcaac taattttaga tatgaggcta ttcaattttt gattgatcac    1260
ttatgtctat cttcaccaca gctttactat ctcaaaaaat gtacttattt tcattatttt    1320
tcacctaatt gttataaggt ttctttatta gtttgttatc ttgtactttt gtcgaaaatt    1380
ctggttacca ccaatacata aaatcatcat cttgactttt gttttgattc acatatatat    1440
gtgcaaagga aattaacagt ttcattttta attaaaaacc aaacgagtta ctaaaaaaaa    1500
aagcgatgtc aagctatcaa catttaacta aacgaatgtc ttattttttg gagaaaaaaa    1560
taaattcaaa gaacatatca aagatcactt gacgtcacgt ttgtttattt atgggatttt    1620
gcagaccatg catgttcttg attccaaatt atgccaacat acaagaatca cttgtaaaat    1680
tgtagcgtcc aaatgagtct agcaactttg ttatcgtgac caagccatca tatcaattca    1740
tgtatgctaa attcgctgca aattcagtaa aaagtatgtt taaaatgaca taaacgaaat    1800
ctgttatagt gttggtagtg agtgggtttg gctttggctt cgcataaact aatgcatggg    1860
tcaggttatc gatccgacct gccaagatag ttccgagttt aaaattacat ggttatgtta    1920
attaattatg cactatgtat gattcataaa ataatttcac tggctttgga cttactattt    1980
ttttcttttc ttgacatcac taaaacacta ttgtttgata tgtttctata aatgtattat    2040
ttacacaata acaaaaaaaa agagagaaag aaactgttga attggagagg aaagagagtg    2100
gggggaaaga tgtcaaggct ggtccagctc aaatcgggta aaaactatta tcaaattcca    2160
attctgtccc tccctttccc caaaaatctg attgtcacat accctaccaa tcagacaacg    2220
ccacgtggac accttagaca tgatggtggg agaggacaac aacatgggag gcacatgatt    2280
ttgtgtttat tatatttctc caaaacacaa tttttaccca aaaacaattt aatcggatca    2340
ctgagcaatg agatgtttgc ggatccaaat ttaactttta aagccttcct tactctttgc    2400
tttataacaa taataataat acacataaaa ttggatttaa ttaaatagat gtggaccttt    2460
ttttctaaat aatataaaag aaggaagaaa ataaaataaa taaagaaaaa gagagagtga    2520
agaaaagtgg gtttaacaat aatttgctgc aaagttagaa gaggcttcag ctttattaac    2580
ttcttgtttg ctttcattct ctttctctct atctatcatc atcatcctcg taatcctctc    2640
ctttctcctt cagacccctc ctagttgctg tttaaacaaa caaaaaaaga ctgcttctct    2700
ttcttatttc tctcatcatc atcatcaatg gttttatgag atttatatca aaaaacattg    2760
aggagctaga gagaaagaga gagtgtgtgt gtagaaaaag attgaaacat catcaagat     2819
```

<210> 48
<211> 2833
<212> DNA
<213> Arabidopsis thaliana

<220>
<221> promoter
<222> (1)..(2833)
<223> transcription regulating sequence from Arabidopsis thaliana gene At1g33240

<400> 48
atatccacta gtagagggtg aggtaagtgg tccataggat tgtggattta gtgcggattt 60
aaggaaaggt ctttggttgg atagattttt aagatgtgtg tgtgtgttct aaaactaagg 120
gtttaaaatc aagagtgttg tggtgtgagt tgacttgtca tgaactctct gacacactgg 180
caaagtgagc ccaggttttg ctagttgttt tgtcgtaagc cttgaaattg acattatgat 240
gcatcatggc ttctatttaa aggggggtcat atagttaatg caatgatcaa aatagccccg 300
agttagattt ttggtgagtt agtagtgtaa gtaaattctt tggattttga ctttgtgagg 360
tatttattta tttggctgcc actgaaattg tagatatatt tgtgagagga ggaatatgga 420
ttttacgtgg gtgtgaaaat tcatggaatt acaaaaaaaa atatcgctgg accattttaa 480
ctataaattt gttttgtgac cccgtcaaaa tgatgttgac tcgttatctt tggtaagtcc 540
tcgtatgaca caagttggga aagtaatgag tttgacaact tgttgtgtcc taataagatt 600
agatttattg gtagagagag acgttgcttt tgtgtgttta agattagtgt gttaatcaaa 660
gtcttaatca aaactccctc atgagatcta gatctcttat gtaaaaagag agattcattc 720
agtcagaaat caaggaagga aactccgtga gcatgttctt accacctcgt ataattatat 780
tcttattata cttgtactaa tattaaagtt gtataattag gaaaaaaaag aacatatcac 840
atgtaataag taataaatat agtgaaaaag gagaggcatg tgaaagtggg gggaagaaga 900
agagaagaca gctgtgtatc cgccgctctc tctctttctt tccccttcaa gaaagttctc 960
cacttccacc gcccaccttc ttcttcttct tcaccttctt ctctaaccat tattattaat 1020
tagcatcttt cattttgttg ttagaaggaa atagtttgtt ttcctttttt gtgtataact 1080
agaaatgagc ggtgtgtttt tgtgtagagt gtgatttggt aatgaaaatg agtttttttt 1140
ttattttgtg tatgtttgtg taggtgagag gtattaaaga gttgtataat gtgatagtat 1200
gagtgtctgc atcgatgtct tgagtttgca actaatttta gatatgaggc tattcaattt 1260
ttgattgatc acttatgtct atcttcacca cagctttact atctcaaaaa atgtacttat 1320
tttcattatt tttcacctaa ttgttataag gtttctttat tagtttgtta tcttgtactt 1380
ttgtcgaaaa ttctggttac caccaataca taaaatcatc atcttgactt ttgttttgat 1440
tcacatatat atgtgcaaag gaaattaaca gtttcatttt taattaaaaa ccaaacgagt 1500
tactaaaaaa aaaagcgatg tcaagctatc aacatttaac taaacgaatg tcttattttt 1560
tggagaaaaa aataaattca aagaacatat caaagatcac ttgacgtcac gtttgtttat 1620
ttatgggatt ttgcagacca tgcatgttct tgattccaaa ttatgccaac atacaagaat 1680
cacttgtaaa attgtagcgt ccaaatgagt ctagcaactt tgttatcgtg accaagccat 1740
catatcaatt catgtatgct aaattcgctg caaattcagt aaaaagtatg tttaaaatga 1800
cataaacgaa atctgttata gtgttggtag tgagtgggtt tggctttggc ttcgcataaa 1860
ctaatgcatg ggtcaggtta tcgatccgac ctgccaagat agttccgagt ttaaaattac 1920
atggttatgt taattaatta tgcactatgt atgattcata aaataatttc actggctttg 1980

gacttactat ttttttcttt tcttgacatc actaaaacac tattgtttga tatgtttcta 2040
taaatgtatt atttacacaa taacaaaaaa aaagagagaa agaaactgtt gaattggaga 2100
ggaaagagag tgggggggaaa gatgtcaagg ctggtccagc tcaaatcggg taaaaactat 2160
tatcaaattc caattctgtc cctccctttc cccaaaaatc tgattgtcac ataccctacc 2220
aatcagacaa cgccacgtgg acaccttaga catgatggtg ggagaggaca acaacatggg 2280
aggcacatga ttttgtgttt attatatttc tccaaaacac aatttttacc caaaaacaat 2340
ttaatcggat cactgagcaa tgagatgttt gcggatccaa atttaacttt taaagccttc 2400
cttactcttt gctttataac aataataata atacacataa aattggattt aattaaatag 2460
atgtggacct ttttttctaa ataatataaa agaaggaaga aaataaaata aataaagaaa 2520
aagagagagt gaagaaaagt gggtttaaca ataatttgct gcaaagttag aagaggcttc 2580
agctttatta acttcttgtt tgctttcatt ctctttctct ctatctatca tcatcatcct 2640
cgtaatcctc tcctttctcc ttcagacccc tcctagttgc tgtttaaaca aacaaaaaaa 2700
gactgcttct ctttcttatt tctctcatca tcatcatcaa tggttttatg agatttatat 2760
caaaaaacat tgaggagcta gagagaaaga gagagtgtgt gtgtagaaaa agattgaaac 2820
atcatcaaga ttg 2833

<210> 49
<211> 2595
<212> DNA
<213> Arabidopsis thaliana

<220>
<221> promoter
<222> (1)..(2595)
<223> transcription regulating sequence from Arabidopsis thaliana gene At1g33240

<400> 49
agagggtgag gtaagtggtc cataggattg tggatttagt gcggatttaa ggaaaggtct 60
ttggttggat agatttttaa gatgtgtgtg tgtgttctaa aactaagggt ttaaaatcaa 120
gagtgttgtg gtgtgagttg acttgtcatg aactctctga cacactggca aagtgagccc 180
aggttttgct agttgttttg tcgtaagcct tgaaattgac attatgatgc atcatggctt 240
ctatttaaag gggtcatat agttaatgca atgatcaaaa tagccccgag ttagattttt 300
ggtgagttag tagtgtaagt aaattctttg gattttgact ttgtgaggta tttatttatt 360
tggctgccac tgaaattgta gatatatttg tgagaggagg aatatggatt ttacgtgggt 420
gtgaaaattc atggaattac aaaaaaaaat atcgctggac cattttaact ataaatttgt 480
tttgtgaccc cgtcaaaatg atgttgactc gttatctttg gtaagtcctc gtatgacaca 540
agttgggaaa gtaatgagtt tgacaacttg ttgtgtccta ataagattag atttattggt 600
agagagagac gttgcttttg tgtgtttaag attagtgtgt taatcaaagt cttaatcaaa 660
actccctcat gagatctaga tctcttatgt aaaaagagag attcattcag tcagaaatca 720
aggaaggaaa ctccgtgagc atgttcttac cacctcgtat aattatattc ttattatact 780
tgtactaata ttaaagttgt ataattagga aaaaaaagaa catatcacat gtaataagta 840
ataaatatag tgaaaaagga gaggcatgtg aaagtggggg gaagaagaag agaagacagc 900
tgtgtatccg ccgctctctc tctttctttc cccttcaaga aagttctcca cttccaccgc 960
ccaccttctt cttcttcttc accttcttct ctaaccatta ttattaatta gcatctttca 1020

ttttgttgtt agaaggaaat agtttgtttt cctttttttgt gtataactag aaatgagcgg 1080
tgtgtttttg tgtagagtgt gatttggtaa tgaaaatgag tttttttttt attttgtgta 1140
tgtttgtgta ggtgagaggt attaaagagt tgtataatgt gatagtatga gtgtctgcat 1200
cgatgtcttg agtttgcaac taattttaga tatgaggcta ttcaattttt gattgatcac 1260
ttatgtctat cttcaccaca gctttactat ctcaaaaaat gtacttattt tcattatttt 1320
tcacctaatt gttataaggt ttctttatta gtttgttatc ttgtactttt gtcgaaaatt 1380
ctggttacca ccaatacata aaatcatcat cttgactttt gttttgattc acatatatat 1440
gtgcaaagga aattaacagt ttcattttta attaaaaacc aaacgagtta ctaaaaaaaa 1500
aagcgatgtc aagctatcaa catttaacta aacgaatgtc ttattttttg gagaaaaaaa 1560
taaattcaaa gaacatatca aagatcactt gacgtcacgt ttgtttattt atgggatttt 1620
gcagaccatg catgttcttg attccaaatt atgccaacat acaagaatca cttgtaaaat 1680
tgtagcgtcc aaatgagtct agcaactttg ttatcgtgac caagccatca tatcaattca 1740
tgtatgctaa attcgctgca aattcagtaa aaagtatgtt taaaatgaca taaacgaaat 1800
ctgttatagt gttggtagtg agtgggtttg gctttggctt cgcataaact aatgcatggg 1860
tcaggttatc gatccgacct gccaagatag ttccgagttt aaaattacat ggttatgtta 1920
attaattatg cactatgtat gattcataaa ataatttcac tggctttgga cttactattt 1980
ttttcttttc ttgacatcac taaaacacta ttgtttgata tgtttctata aatgtattat 2040
ttacacaata acaaaaaaaa agagagaaag aaactgttga attggagagg aaagagagtg 2100
gggggaaaga tgtcaaggct ggtccagctc aaatcgggta aaaactatta tcaaattcca 2160
attctgtccc tccctttccc caaaaatctg attgtcacat accctaccaa tcagacaacg 2220
ccacgtggac accttagaca tgatggtggg agaggacaac aacatgggag gcacatgatt 2280
ttgtgtttat tatatttctc caaaacacaa tttttaccca aaaacaattt aatcggatca 2340
ctgagcaatg agatgtttgc ggatccaaat ttaacttta aagccttcct tactctttgc 2400
tttataacaa taataataat acacataaaa ttggatttaa ttaaatagat gtggaccttt 2460
ttttctaaat aatataaaag aaggaagaaa ataaaataaa taaagaaaaa gagagagtga 2520
agaaaagtgg gtttaacaat aatttgctgc aaagttagaa gaggcttcag ctttattaac 2580
ttcttgtttg ctttc 2595

<210> 50
<211> 2607
<212> DNA
<213> Arabidopsis thaliana

<220>
<221> promoter
<222> (1)..(2607)
<223> transcription regulating sequence from Arabidopsis thaliana gene At1g33240

<400> 50
atatccacta gtagagggtg aggtaagtgg tccataggat tgtggattta gtgcggattt 60
aaggaaaggt ctttggttgg atagattttt aagatgtgtg tgtgtgttct aaaactaagg 120
gtttaaaatc aagagtgttg tggtgtgagt tgacttgtca tgaactctct gacacactgg 180
caaagtgagc ccaggttttg ctagttgttt tgtcgtaagc cttgaaattg acattatgat 240
gcatcatggc ttctatttaa agggggtcat atagttaatg caatgatcaa aatagccccg 300

```
agttagattt ttggtgagtt agtagtgtaa gtaaattctt tggattttga ctttgtgagg    360
tatttattta tttggctgcc actgaaattg tagatatatt tgtgagagga ggaatatgga    420
ttttacgtgg gtgtgaaaat tcatggaatt acaaaaaaaa atatcgctgg accattttaa    480
ctataaattt gttttgtgac cccgtcaaaa tgatgttgac tcgttatctt tggtaagtcc    540
tcgtatgaca caagttggga aagtaatgag tttgacaact tgttgtgtcc taataagatt    600
agatttattg gtagagagag acgttgcttt tgtgtgttta agattagtgt gttaatcaaa    660
gtcttaatca aaactccctc atgagatcta gatctcttat gtaaaaagag agattcattc    720
agtcagaaat caaggaagga aactccgtga gcatgttctt accacctcgt ataattatat    780
tcttattata cttgtactaa tattaaagtt gtataattag gaaaaaaaag aacatatcac    840
atgtaataag taataaatat agtgaaaaag gagaggcatg tgaaagtggg gggaagaaga    900
agagaagaca gctgtgtatc cgccgctctc tctctttctt tccccttcaa gaaagttctc    960
cacttccacc gcccaccttc ttcttcttct tcaccttctt ctctaaccat tattattaat   1020
tagcatcttt cattttgttg ttagaaggaa atagtttgtt ttcctttttt gtgtataact   1080
agaaatgagc ggtgtgtttt tgtgtagagt gtgatttggt aatgaaaatg agtttttttt   1140
ttattttgtg tatgtttgtg taggtgagag gtattaaaga gttgtataat gtgatagtat   1200
gagtgtctgc atcgatgtct tgagtttgca actaatttta gatatgaggc tattcaattt   1260
ttgattgatc acttatgtct atcttcacca cagctttact atctcaaaaa atgtacttat   1320
tttcattatt tttcacctaa ttgttataag gtttctttat tagtttgtta tcttgtactt   1380
ttgtcgaaaa ttctggttac caccaataca taaaatcatc atcttgactt ttgttttgat   1440
tcacatatat atgtgcaaag gaaattaaca gtttcatttt taattaaaaa ccaaacgagt   1500
tactaaaaaa aaaagcgatg tcaagctatc aacatttaac taaacgaatg tcttattttt   1560
tggagaaaaa aataaattca aagaacatat caaagatcac ttgacgtcac gtttgtttat   1620
ttatgggatt ttgcagacca tgcatgttct tgattccaaa ttatgccaac atacaagaat   1680
cacttgtaaa attgtagcgt ccaaatgagt ctagcaactt tgttatcgtg accaagccat   1740
catatcaatt catgtatgct aaattcgctg caaattcagt aaaaagtatg tttaaaatga   1800
cataaacgaa atctgttata gtgttggtag tgagtgggtt tggctttggc ttcgcataaa   1860
ctaatgcatg ggtcaggtta tcgatccgac ctgccaagat agttccgagt ttaaaattac   1920
atggttatgt taattaatta tgcactatgt atgattcata aaataatttc actggctttg   1980
gacttactat ttttttcttt tcttgacatc actaaaacac tattgtttga tatgtttcta   2040
taaatgtatt atttacacaa taacaaaaaa aaagagagaa agaaactgtt gaattggaga   2100
ggaaagagag tgggggaaa gatgtcaagg ctggtccagc tcaaatcggg taaaaactat   2160
tatcaaattc caattctgtc cctccctttc cccaaaaatc tgattgtcac ataccctacc   2220
aatcagacaa cgccacgtgg acaccttaga catgatggtg ggagaggaca acaacatggg   2280
aggcacatga ttttgtgttt attatatttc tccaaaacac aatttttacc caaaaacaat   2340
ttaatcggat cactgagcaa tgagatgttt gcggatccaa atttaacttt taaagccttc   2400
cttactcttt gctttataac aataataata atacacataa aattggattt aattaaatag   2460
atgtggacct tttttctaa ataatataaa agaaggaaga aaataaaata aataaagaaa   2520
aagagagagt gaagaaaagt gggtttaaca ataatttgct gcaaagttag aagaggcttc   2580
agctttatta acttcttgtt tgctttc                                       2607
```

<210> 51
<211> 2624
<212> DNA
<213> Arabidopsis thaliana

<220>
<221> CDS
<222> (227)..(2236)
<223> encoding putative Arabidopsis thaliana trihelix DNA-binding protein

<400> 51
```
attctctttc tctctatcta tcatcatcat cctcgtaatc ctctcctttc tccttcagac      60
ccctcctagt tgctgtttaa acaaacaaaa aaagactgct tctctttctt atttctctca     120
tcatcatcat caatggtttt atgagattta tatcaaaaaa cattgaggag ctagagagaa     180
agagagagtg tgtgtgtaga aaaagattga aacatcatca agattg atg gag caa        235
                                                   Met Glu Gln
                                                   1
gga gga ggt ggt ggt ggt aat gaa gtt gtg gag gaa gct tca cct att      283
Gly Gly Gly Gly Gly Gly Asn Glu Val Val Glu Glu Ala Ser Pro Ile
    5                   10                  15
agt tca aga cct cct gct aac aac tta gaa gag ctt atg aga ttc tca      331
Ser Ser Arg Pro Pro Ala Asn Asn Leu Glu Glu Leu Met Arg Phe Ser
20                  25                  30                  35
gcc gcc gcg gat gac ggt gga tta gga ggt gga ggt gga gga gga gga      379
Ala Ala Ala Asp Asp Gly Gly Leu Gly Gly Gly Gly Gly Gly Gly Gly
                40                  45                  50
gga gga agt gct tct tct tca tcg gga aat cga tgg ccg aga gaa gaa      427
Gly Gly Ser Ala Ser Ser Ser Ser Gly Asn Arg Trp Pro Arg Glu Glu
            55                  60                  65
act tta gct ctt ctt cgg atc cga tcc gat atg gat tct act ttt cgt      475
Thr Leu Ala Leu Leu Arg Ile Arg Ser Asp Met Asp Ser Thr Phe Arg
        70                  75                  80
gat gct act ctc aaa gct cct ctt tgg gaa cat gtt tcc agg aag cta      523
Asp Ala Thr Leu Lys Ala Pro Leu Trp Glu His Val Ser Arg Lys Leu
    85                  90                  95
ttg gag tta ggt tac aaa cga agt tca aag aaa tgc aaa gag aaa ttc      571
Leu Glu Leu Gly Tyr Lys Arg Ser Ser Lys Lys Cys Lys Glu Lys Phe
100                 105                 110                 115
gaa aac gtt cag aaa tat tac aaa cgt act aaa gaa act cgc ggt ggt      619
Glu Asn Val Gln Lys Tyr Tyr Lys Arg Thr Lys Glu Thr Arg Gly Gly
                120                 125                 130
cgt cat gat ggt aaa gct tac aag ttc ttc tct cag ctt gaa gct ctc      667
Arg His Asp Gly Lys Ala Tyr Lys Phe Phe Ser Gln Leu Glu Ala Leu
            135                 140                 145
aac act act cct cct tca tct tcc ctc gac gtt act cct ctc tcc gtc      715
Asn Thr Thr Pro Pro Ser Ser Ser Leu Asp Val Thr Pro Leu Ser Val
        150                 155                 160
gct aat ccc att ctc atg cct tct tct tct tct tct cca ttt ccc gta      763
Ala Asn Pro Ile Leu Met Pro Ser Ser Ser Ser Ser Pro Phe Pro Val
```

```
    165                 170                 175
ttc tct caa ccg caa ccg caa acg caa acg caa ccg cct caa acg cat      811
Phe Ser Gln Pro Gln Pro Gln Thr Gln Thr Gln Pro Pro Gln Thr His
180                 185                 190                 195
aat gtc tct ttt act cct act cca cca cct ctt cca ctt cct tca atg      859
Asn Val Ser Phe Thr Pro Thr Pro Pro Pro Leu Pro Leu Pro Ser Met
                200                 205                 210
ggt ccg ata ttt acc ggt gtt act ttc tcg tct cat agc tca tcg acg      907
Gly Pro Ile Phe Thr Gly Val Thr Phe Ser Ser His Ser Ser Ser Thr
            215                 220                 225
gct tca gga atg ggg tct gat gat gat gac gac gat atg gac gtt gat      955
Ala Ser Gly Met Gly Ser Asp Asp Asp Asp Asp Asp Met Asp Val Asp
        230                 235                 240
cag gct aac att gcg ggt tct agt agc cga aaa cgc aaa cgt gga aac     1003
Gln Ala Asn Ile Ala Gly Ser Ser Ser Arg Lys Arg Lys Arg Gly Asn
    245                 250                 255
cgc ggt gga ggc ggt aaa atg atg gaa ttg ttt gaa ggt ttg gtg aga     1051
Arg Gly Gly Gly Gly Lys Met Met Glu Leu Phe Glu Gly Leu Val Arg
260                 265                 270                 275
caa gta atg caa aag caa gcg gct atg caa agg agt ttc ttg gaa gct     1099
Gln Val Met Gln Lys Gln Ala Ala Met Gln Arg Ser Phe Leu Glu Ala
                280                 285                 290
ctt gag aag aga gag caa gaa cgt ctt gat cgt gaa gaa gct tgg aaa     1147
Leu Glu Lys Arg Glu Gln Glu Arg Leu Asp Arg Glu Glu Ala Trp Lys
            295                 300                 305
cgt caa gaa atg gct cgg tta gct cga gaa cac gag gtc atg tct caa     1195
Arg Gln Glu Met Ala Arg Leu Ala Arg Glu His Glu Val Met Ser Gln
        310                 315                 320
gaa cga gcc gcc tct gct tct cgt gac gcc gca atc att tca ttg att     1243
Glu Arg Ala Ala Ser Ala Ser Arg Asp Ala Ala Ile Ile Ser Leu Ile
    325                 330                 335
cag aaa att act ggc cat acc att cag tta cct cct tct ttg tca tct     1291
Gln Lys Ile Thr Gly His Thr Ile Gln Leu Pro Pro Ser Leu Ser Ser
340                 345                 350                 355
caa ccg cct cca ccg tat caa ccg cca ccc gcg gtc act aaa cgt gtg     1339
Gln Pro Pro Pro Pro Tyr Gln Pro Pro Pro Ala Val Thr Lys Arg Val
                360                 365                 370
gcg gaa cca cca tta tca aca gct caa tct caa tca caa caa cca ata     1387
Ala Glu Pro Pro Leu Ser Thr Ala Gln Ser Gln Ser Gln Gln Pro Ile
            375                 380                 385
atg gcg att cca caa caa caa att ctt cct cct cct cct cct tct cat     1435
Met Ala Ile Pro Gln Gln Gln Ile Leu Pro Pro Pro Pro Pro Ser His
        390                 395                 400
cct cac gct cat caa cca gaa cag aaa caa caa caa caa cca caa caa     1483
Pro His Ala His Gln Pro Glu Gln Lys Gln Gln Gln Gln Pro Gln Gln
```

```
    405                 410                 415
gag atg gtc atg agc tcg gaa caa tca tca tta cca tca tca tca aga      1531
Glu Met Val Met Ser Ser Glu Gln Ser Ser Leu Pro Ser Ser Ser Arg
420                 425                 430                 435
tgg cca aag gca gag att cta gcg ctt ata aac ctg aga agt gga atg      1579
Trp Pro Lys Ala Glu Ile Leu Ala Leu Ile Asn Leu Arg Ser Gly Met
                440                 445                 450
gaa cca agg tac caa gat aat gta cct aaa gga ctt cta tgg gaa gag      1627
Glu Pro Arg Tyr Gln Asp Asn Val Pro Lys Gly Leu Leu Trp Glu Glu
            455                 460                 465
atc tca act tca atg aag aga atg gga tac aac aga aac gct aag aga      1675
Ile Ser Thr Ser Met Lys Arg Met Gly Tyr Asn Arg Asn Ala Lys Arg
        470                 475                 480
tgt aaa gag aaa tgg gaa aac ata aac aaa tac tac aag aaa gtt aaa      1723
Cys Lys Glu Lys Trp Glu Asn Ile Asn Lys Tyr Tyr Lys Lys Val Lys
    485                 490                 495
gaa agc aac aag aaa cgt cct caa gat gct aag act tgt cct tac ttt      1771
Glu Ser Asn Lys Lys Arg Pro Gln Asp Ala Lys Thr Cys Pro Tyr Phe
500                 505                 510                 515
cac cgc ctc gat ctt ctt tac cgc aac aaa gta ctc ggt agt ggc ggt      1819
His Arg Leu Asp Leu Leu Tyr Arg Asn Lys Val Leu Gly Ser Gly Gly
                520                 525                 530
ggt tct agc act tct ggt cta cct caa gac caa aaa cag agt ccg gtc      1867
Gly Ser Ser Thr Ser Gly Leu Pro Gln Asp Gln Lys Gln Ser Pro Val
            535                 540                 545
act gcg atg aaa ccg cca caa gaa gga ctt gtt aat gtt caa caa act      1915
Thr Ala Met Lys Pro Pro Gln Glu Gly Leu Val Asn Val Gln Gln Thr
        550                 555                 560
cat ggg tca gct tca act gag gaa gaa gag cct ata gag gaa agt cca      1963
His Gly Ser Ala Ser Thr Glu Glu Glu Glu Pro Ile Glu Glu Ser Pro
    565                 570                 575
caa gga aca gaa aag cca gaa gac ctt gtg atg aga gag ctg att caa      2011
Gln Gly Thr Glu Lys Pro Glu Asp Leu Val Met Arg Glu Leu Ile Gln
580                 585                 590                 595
caa caa cag caa cta caa caa caa gaa tca atg ata ggt gag tat gaa      2059
Gln Gln Gln Gln Leu Gln Gln Gln Glu Ser Met Ile Gly Glu Tyr Glu
                600                 605                 610
aag att gaa gag tct cac aat tat aat aac atg gag gaa gag gaa gat      2107
Lys Ile Glu Glu Ser His Asn Tyr Asn Asn Met Glu Glu Glu Glu Asp
            615                 620                 625
cag gaa atg gat gag gaa gaa cta gac gag gat gag aag tcc gcg gct      2155
Gln Glu Met Asp Glu Glu Glu Leu Asp Glu Asp Glu Lys Ser Ala Ala
        630                 635                 640
ttc gag att gcg ttt caa agc cct gca aac aga gga ggc aat ggc cat      2203
Phe Glu Ile Ala Phe Gln Ser Pro Ala Asn Arg Gly Gly Asn Gly His
```

```
    645                 650                 655
acg gaa cca cct ttc ttg aca atg gtt cag taa aatcagaatc attgtttcaa   2256
Thr Glu Pro Pro Phe Leu Thr Met Val Gln
660                 665
gaaaatgtac ttatgtgtgc atagttttct aaacaaaaca cccaaaaaca caaaacacaa   2316
acacacacac acacacatct gacaccacaa actgaaatat caaggtctca tcgatcttcg   2376
atttttcagt gaatacaatt ctttttcaca ttctttccct tttgttcttt tctttctcac   2436
tgtctcttgt ttgtttcttc ttgttcattg ttttcttctt ccaaccccca catgaacaaa   2496
aaactctagc taaaaggtct tttagattat tttggagttg taatttcatc agcaagtctc   2556
acactttttt tttttctctt aaaacatttg ttgattggtt aattggggaa taaagagtct   2616
ttatttta                                                            2624
```

<210> 52
<211> 669
<212> PRT
<213> Arabidopsis thaliana

<400> 52

```
Met Glu Gln Gly Gly Gly Gly Gly Gly Asn Glu Val Val Glu Glu Ala
1               5                   10                  15

Ser Pro Ile Ser Ser Arg Pro Pro Ala Asn Asn Leu Glu Glu Leu Met
            20                  25                  30

Arg Phe Ser Ala Ala Ala Asp Asp Gly Gly Leu Gly Gly Gly Gly Gly
        35                  40                  45

Gly Gly Gly Gly Gly Ser Ala Ser Ser Ser Ser Gly Asn Arg Trp Pro
    50                  55                  60

Arg Glu Glu Thr Leu Ala Leu Leu Arg Ile Arg Ser Asp Met Asp Ser
65                  70                  75                  80

Thr Phe Arg Asp Ala Thr Leu Lys Ala Pro Leu Trp Glu His Val Ser
                85                  90                  95

Arg Lys Leu Leu Glu Leu Gly Tyr Lys Arg Ser Ser Lys Lys Cys Lys
            100                 105                 110

Glu Lys Phe Glu Asn Val Gln Lys Tyr Tyr Lys Arg Thr Lys Glu Thr
        115                 120                 125

Arg Gly Gly Arg His Asp Gly Lys Ala Tyr Lys Phe Phe Ser Gln Leu
    130                 135                 140
```

```
Glu Ala Leu Asn Thr Thr Pro Pro Ser Ser Ser Leu Asp Val Thr Pro
145                 150                 155                 160

Leu Ser Val Ala Asn Pro Ile Leu Met Pro Ser Ser Ser Ser Ser Pro
                165                 170                 175

Phe Pro Val Phe Ser Gln Pro Gln Pro Gln Thr Gln Thr Gln Pro Pro
            180                 185                 190

Gln Thr His Asn Val Ser Phe Thr Pro Thr Pro Pro Pro Leu Pro Leu
        195                 200                 205

Pro Ser Met Gly Pro Ile Phe Thr Gly Val Thr Phe Ser Ser His Ser
    210                 215                 220

Ser Ser Thr Ala Ser Gly Met Gly Ser Asp Asp Asp Asp Asp Asp Met
225                 230                 235                 240

Asp Val Asp Gln Ala Asn Ile Ala Gly Ser Ser Ser Arg Lys Arg Lys
                245                 250                 255

Arg Gly Asn Arg Gly Gly Gly Gly Lys Met Met Glu Leu Phe Glu Gly
            260                 265                 270

Leu Val Arg Gln Val Met Gln Lys Gln Ala Ala Met Gln Arg Ser Phe
        275                 280                 285

Leu Glu Ala Leu Glu Lys Arg Glu Gln Glu Arg Leu Asp Arg Glu Glu
    290                 295                 300

Ala Trp Lys Arg Gln Glu Met Ala Arg Leu Ala Arg Glu His Glu Val
305                 310                 315                 320

Met Ser Gln Glu Arg Ala Ala Ser Ala Ser Arg Asp Ala Ala Ile Ile
                325                 330                 335

Ser Leu Ile Gln Lys Ile Thr Gly His Thr Ile Gln Leu Pro Pro Ser
            340                 345                 350

Leu Ser Ser Gln Pro Pro Pro Pro Tyr Gln Pro Pro Pro Ala Val Thr
        355                 360                 365

Lys Arg Val Ala Glu Pro Pro Leu Ser Thr Ala Gln Ser Gln Ser Gln
    370                 375                 380
```

```
Gln Pro Ile Met Ala Ile Pro Gln Gln Gln Ile Leu Pro Pro Pro Pro
385                 390                 395                 400

Pro Ser His Pro His Ala His Gln Pro Glu Gln Lys Gln Gln Gln Gln
                405                 410                 415

Pro Gln Gln Glu Met Val Met Ser Ser Glu Gln Ser Ser Leu Pro Ser
            420                 425                 430

Ser Ser Arg Trp Pro Lys Ala Glu Ile Leu Ala Leu Ile Asn Leu Arg
        435                 440                 445

Ser Gly Met Glu Pro Arg Tyr Gln Asp Asn Val Pro Lys Gly Leu Leu
    450                 455                 460

Trp Glu Glu Ile Ser Thr Ser Met Lys Arg Met Gly Tyr Asn Arg Asn
465                 470                 475                 480

Ala Lys Arg Cys Lys Glu Lys Trp Glu Asn Ile Asn Lys Tyr Tyr Lys
                485                 490                 495

Lys Val Lys Glu Ser Asn Lys Lys Arg Pro Gln Asp Ala Lys Thr Cys
            500                 505                 510

Pro Tyr Phe His Arg Leu Asp Leu Leu Tyr Arg Asn Lys Val Leu Gly
        515                 520                 525

Ser Gly Gly Gly Ser Ser Thr Ser Gly Leu Pro Gln Asp Gln Lys Gln
    530                 535                 540

Ser Pro Val Thr Ala Met Lys Pro Pro Gln Glu Gly Leu Val Asn Val
545                 550                 555                 560

Gln Gln Thr His Gly Ser Ala Ser Thr Glu Glu Glu Glu Pro Ile Glu
                565                 570                 575

Glu Ser Pro Gln Gly Thr Glu Lys Pro Glu Asp Leu Val Met Arg Glu
            580                 585                 590

Leu Ile Gln Gln Gln Gln Gln Leu Gln Gln Gln Glu Ser Met Ile Gly
        595                 600                 605

Glu Tyr Glu Lys Ile Glu Glu Ser His Asn Tyr Asn Asn Met Glu Glu
    610                 615                 620
```

```
Glu Glu Asp Gln Glu Met Asp Glu Glu Glu Leu Asp Glu Asp Glu Lys
625                 630                 635                 640

Ser Ala Ala Phe Glu Ile Ala Phe Gln Ser Pro Ala Asn Arg Gly Gly
                645                 650                 655

Asn Gly His Thr Glu Pro Pro Phe Leu Thr Met Val Gln
            660                 665
```

<210> 53
<211> 993
<212> DNA
<213> Arabidopsis thaliana

<220>
<221> promoter
<222> (1)..(993)
<223> transcription regulating sequence from Arabidopsis thaliana gene At1g28440

<400> 53

```
tatcttccat attttctttg catttttgt aaataactac atattattat cttcaaaaat      60
gaatttcaca tttcaacaaa ataaaatatt ttcatgtttc tacatgcatg ctagtttatt    120
ctcttaatag ttgatacaat attcctaata ctctttaaga cgtgcataaa ctttttaaaa    180
ttgaatgata attaatttaa ttcacatcga ctttagacta cgttccaagt aaacttccaa    240
cacattctgt tttttttttt ttgatatgtt ctaacaatat ttgtacttta ctactttaca    300
aacgccgata ataaataaaa ctgtcggaat atatctcatt ccatctccaa aaaaaaaaaa    360
aaaaaaatca gatatctaca tttcaaaatt aatgtttacc gtaccttctt ttcaccttgc    420
aattttttta aaatgcacat ttactctact aactaacttt tcatttacat taaaaacata    480
tataatgaaa tcaaatttgt atgcaaattc catatatatt ataaagctaa aattatttaa    540
ttgaaaattt acaatcttat cgtctcctac ataactaatt taaaaacagt tgcattctct    600
taaacaacaa agtacaaaaa aagaatgttt ccttccatat ggctatttag ctagatcatc    660
attattagta acattctctt ttataatcag ataaacacag gataattata caatgttgga    720
aactcgacat agatactaat aattgcaaac aaataattag agagaatcgt tatagaaaac    780
aaaaacaaaa taaatgatag tagaaaaaca aatgattttt atagcattca cagtcagagt    840
cacgataatt cacctgtctc cggtcaacgc tttttataga ctctcttctt ctcttccttc    900
aaagcactct ctcacaaaca ctctcttctt catactctct tatctctctc tctttcttca    960
accaccgtca tagataccgg ggaagacgaa gaa                                 993
```

<210> 54
<211> 1010
<212> DNA
<213> Arabidopsis thaliana

<220>

<221> promoter
<222> (1)..(1010)
<223> transcription regulating sequence from Arabidopsis thaliana gene At1g28440

<400> 54
```
gatatcttat cctatcttcc atattttctt tgcatttttt gtaaataact acatattatt      60
atcttcaaaa atgaatttca catttcaaca aaataaaata ttttcatgtt tctacatgca     120
tgctagttta ttctcttaat agttgataca atattcctaa tactctttaa gacgtgcata     180
aactttttaa aattgaatga taattaattt aattcacatc gactttagac tacgttccaa     240
gtaaacttcc aacacattct gttttttttt tttttgatat gttctaacaa tatttgtact     300
ttactacttt acaaacgccg ataataaata aaactgtcgg aatatatctc attccatctc     360
caaaaaaaaa aaaaaaaaaa aatcagatat ctacatttca aaattaatgt ttaccgtacc     420
ttcttttcac cttgcaattt ttttaaaatg cacatttact ctactaacta acttttcatt     480
tacattaaaa acatatataa tgaaatcaaa tttgtatgca aattccatat atattataaa     540
gctaaaatta tttaattgaa aatttacaat cttatcgtct cctacataac taatttaaaa     600
acagttgcat tctcttaaac aacaaagtac aaaaaaagaa tgtttccttc catatggcta     660
tttagctaga tcatcattat tagtaacatt ctcttttata atcagataaa cacaggataa     720
ttatacaatg ttggaaactc gacatagata ctaataattg caaacaaata attagagaga     780
atcgttatag aaaacaaaaa caaaataaat gatagtagaa aaacaaatga tttttatagc     840
attcacagtc agagtcacga taattcacct gtctccggtc aacgcttttt atagactctc     900
ttcttctctt ccttcaaagc actctctcac aaacactctc ttcttcatac tctcttatct     960
ctctctcttt cttcaaccac cgtcatagat accggggaag acgaagaaac               1010
```

<210> 55
<211> 905
<212> DNA
<213> Arabidopsis thaliana

<220>
<221> promoter
<222> (1)..(905)
<223> transcription regulating sequence from Arabidopsis thaliana gene At1g28440

<400> 55
```
tatcttccat attttctttg catttttttgt aaataactac atattattat cttcaaaaat      60
gaatttcaca tttcaacaaa ataaaatatt ttcatgtttc tacatgcatg ctagtttatt     120
ctcttaatag ttgatacaat attcctaata ctctttaaga cgtgcataaa ctttttaaaa     180
ttgaatgata attaatttaa ttcacatcga ctttagacta cgttccaagt aaacttccaa     240
cacattctgt tttttttttt ttgatatgtt ctaacaatat ttgtacttta ctactttaca     300
aacgccgata ataaataaaa ctgtcggaat atatctcatt ccatctccaa aaaaaaaaaa     360
aaaaaaatca gatatctaca tttcaaaatt aatgtttacc gtaccttctt ttcaccttgc     420
aatttttttta aaatgcacat ttactctact aactaacttt tcatttacat taaaaacata     480
tataatgaaa tcaaatttgt atgcaaattc catatatatt ataaagctaa aattatttaa     540
```

ttgaaaattt acaatcttat cgtctcctac ataactaatt taaaaacagt tgcattctct 600
taaacaacaa agtacaaaaa aagaatgttt ccttccatat ggctatttag ctagatcatc 660
attattagta acattctctt ttataatcag ataaacacag gataattata caatgttgga 720
aactcgacat agatactaat aattgcaaac aaataattag agagaatcgt tatagaaaac 780
aaaaacaaaa taaatgatag tagaaaaaca aatgattttt atagcattca cagtcagagt 840
cacgataatt cacctgtctc cggtcaacgc tttttataga ctctcttctt ctcttccttc 900
aaagc 905

<210> 56
<211> 920
<212> DNA
<213> Arabidopsis thaliana

<220>
<221> promoter
<222> (1)..(920)
<223> transcription regulating sequence from Arabidopsis thaliana gene At1g28440

<400> 56
gatatcttat cctatcttcc atattttctt tgcatttttt gtaaataact acatattatt 60
atcttcaaaa atgaatttca catttcaaca aaataaaata ttttcatgtt tctacatgca 120
tgctagttta ttctcttaat agttgataca atattcctaa tactctttaa gacgtgcata 180
aactttttaa aattgaatga taattaattt aattcacatc gactttagac tacgttccaa 240
gtaaacttcc aacacattct gttttttttt tttttgatat gttctaacaa tatttgtact 300
ttactacttt acaaacgccg ataataaata aaactgtcgg aatatatctc attccatctc 360
caaaaaaaaa aaaaaaaaaa aatcagatat ctacatttca aaattaatgt ttaccgtacc 420
ttcttttcac cttgcaattt ttttaaaatg cacatttact ctactaacta acttttcatt 480
tacattaaaa acatatataa tgaaatcaaa tttgtatgca aattccatat atattataaa 540
gctaaaatta tttaattgaa aatttacaat cttatcgtct cctacataac taatttaaaa 600
acagttgcat tctcttaaac aacaaagtac aaaaaaagaa tgtttccttc catatggcta 660
tttagctaga tcatcattat tagtaacatt ctcttttata atcagataaa cacaggataa 720
ttatacaatg ttggaaactc gacatagata ctaataattg caaacaaata attagagaga 780
atcgttatag aaaacaaaaa caaaataaat gatagtagaa aaacaaatga tttttatagc 840
attcacagtc agagtcacga taattcacct gtctccggtc aacgcttttt atagactctc 900
ttcttctctt ccttcaaagc 920

<210> 57
<211> 2258
<212> DNA
<213> Arabidopsis thaliana

<220>
<221> promoter
<222> (1)..(2258)

<223> transcription regulating sequence from Arabidopsis thaliana gene At1g28440

<400> 57

```
cattgaggag cgtaatgcct tcaaaccatt tatataagaa cttgaactct caaaatatag     60
atcaaaggtt gcttgcttgt acacaatact cgtaacaaat taatagttct attaaataat    120
tccaaagtga caatgttatg aaactataag aggacatcaa tcacacgtaa tggaaacttg    180
attaactaag taatgaaatt aaaaatataa acaaaactaa tttgacggtt tctttcaatc    240
aactagctag caacaatttg taggatgaaa gagactttgc ttataacaat ctcataagac    300
agagatttgt agctattgta gtgtacgtaa tttcttaaat gtctatctta taggatacaa    360
gtgtcgaact ccgtttagtg taccctaatt tttttttgac aacttgtatt gtgtgtttca    420
cagaaactat tagaaacacg aagaagagaa gactgcttgg aatcaatcta tctccaaaat    480
ttcactcatc tatctcttta aatatcattc aaatggtatg ttgattaaag tgatgctttt    540
ctcaaccgat acatatgtta gaacatacgt tttctaacta acatatatgt aggcaaaatt    600
tgtgttgttc ttttatgagt ttattatttt gttaaattaa gattaactaa aataaaatca    660
aattccaact gataaactaa gattgagatt atgatagtaa acgtaacaat tctcataatg    720
taacgggcat gaaatccaaa tcgtaagaaa ataaaaacaa aaatagataa caattttttt    780
taactggatt caacaatgta ttcacgtcct caaaaacaaa attcttgaac acaagtacag    840
ttagtgcaac tctttttcac atcctacaaa ataatttacg atttcgttta gccgtaacaa    900
cttatgaaaa ccaagttgtg agaataagaa caaagtgtac acacagtttg tttttcacaa    960
tacattattt tacgtcttcc aattttgttg catatcaacc taaaatttaa aaaccataaa   1020
gaactcttat tcgactcgaa tactttttta agactatgac atctctaaga taagcaatac   1080
tcatgactct cgcttgagaa gtgcattctc atattgacta caccattagg caatatgttt   1140
cctaacgttt aaccatacaa gtttgtcccc ctctttccct ttgcaatact aattagtata   1200
acaaatttca ttagagaatg tttaatttca taaatatctc aatattttga tatcttatcc   1260
tatcttccat attttctttg cattttttgt aaataactac atattattat cttcaaaaat   1320
gaatttcaca tttcaacaaa ataaaatatt ttcatgtttc tacatgcatg ctagtttatt   1380
ctcttaatag ttgatacaat attcctaata ctctttaaga cgtgcataaa ctttttaaaa   1440
ttgaatgata attaatttaa ttcacatcga ctttagacta cgttccaagt aaacttccaa   1500
cacattctgt tttttttttt tttgatatgt tctaacaata tttgtacttt actactttac   1560
aaacgccgat aataaataaa actgtcggaa tatatctcat tccatctcca aaaaaaaaaa   1620
aaaaaaaaaa tcagatatct acatttcaaa attaatgttt accgtacctt cttttcacct   1680
tgcaattttt ttaaaatgca catttactct actaactaac ttttcattta cattaaaaac   1740
atatataatg aaatcaaatt tgtatgcaaa ttccatatat attataaagc taaaattatt   1800
taattgaaaa tttacaatct tatcgtctcc tacataacta atttaaaaac agttgcattc   1860
tcttaaacaa caaagtacaa aaaaagaatg tttccttcca tatggctatt tagctagatc   1920
atcattatta gtaacattct cttttataat cagataaaca caggataatt atacaatgtt   1980
ggaaactcga catagatact aataattgca aacaaataat tagagagaat cgttatagaa   2040
aacaaaaaca aaataaatga tagtagaaaa acaaatgatt tttatagcat tcacagtcag   2100
agtcacgata attcacctgt ctccggtcaa cgctttttat agactctctt cttctcttcc   2160
ttcaaagcac tctctcacaa acactctctt cttcatactc tcttatctct ctctctttct   2220
tcaaccaccg tcatagatac cggggaagac gaagaaac                           2258
```

<210> 58
<211> 2168

<212> DNA
<213> Arabidopsis thaliana

<220>
<221> promoter
<222> (1)..(2168)
<223> transcription regulating sequence from Arabidopsis thaliana gene At1g28440

<400> 58

```
cattgaggag cgtaatgcct tcaaaccatt tatataagaa cttgaactct caaaatatag     60
atcaaaggtt gcttgcttgt acacaatact cgtaacaaat taatagttct attaaataat    120
tccaaagtga caatgttatg aaactataag aggacatcaa tcacacgtaa tggaaacttg    180
attaactaag taatgaaatt aaaaatataa acaaaactaa tttgacggtt tctttcaatc    240
aactagctag caacaatttg taggatgaaa gagactttgc ttataacaat ctcataagac    300
agagatttgt agctattgta gtgtacgtaa tttcttaaat gtctatctta taggatacaa    360
gtgtcgaact ccgtttagtg taccctaatt tttttttgac aacttgtatt gtgtgtttca    420
cagaaactat tagaaacacg aagaagagaa gactgcttgg aatcaatcta tctccaaaat    480
ttcactcatc tatctcttta aatatcattc aaatggtatg ttgattaaag tgatgctttt    540
ctcaaccgat acatatgtta gaacatacgt tttctaacta acatatatgt aggcaaaatt    600
tgtgttgttc ttttatgagt ttattatttt gttaaattaa gattaactaa aataaaatca    660
aattccaact gataaactaa gattgagatt atgatagtaa acgtaacaat tctcataatg    720
taacgggcat gaaatccaaa tcgtaagaaa ataaaaacaa aaatagataa caattttttt    780
taactggatt caacaatgta ttcacgtcct caaaaacaaa attcttgaac acaagtacag    840
ttagtgcaac tctttttcac atcctacaaa ataatttacg atttcgttta gccgtaacaa    900
cttatgaaaa ccaagttgtg agaataagaa caaagtgtac acacagtttg tttttcacaa    960
tacattattt tacgtcttcc aattttgttg catatcaacc taaaatttaa aaaccataaa   1020
gaactcttat tcgactcgaa tacttttta agactatgac atctctaaga taagcaatac   1080
tcatgactct cgcttgagaa gtgcattctc atattgacta caccattagg caatatgttt   1140
cctaacgttt aaccatacaa gtttgtcccc ctctttccct ttgcaatact aattagtata   1200
acaaatttca ttagagaatg tttaatttca taaatatctc aatattttga tatcttatcc   1260
tatcttccat atttctttg catttttgt aaataactac atattattat cttcaaaaat   1320
gaatttcaca tttcaacaaa ataaaatatt ttcatgtttc tacatgcatg ctagtttatt   1380
ctcttaatag ttgatacaat attcctaata ctctttaaga cgtgcataaa ctttttaaaa   1440
ttgaatgata attaatttaa ttcacatcga ctttagacta cgttccaagt aaacttccaa   1500
cacattctgt ttttttttt tttgatatgt tctaacaata tttgtacttt actactttac   1560
aaacgccgat aataaataaa actgtcggaa tatatctcat tccatctcca aaaaaaaaaa   1620
aaaaaaaaaa tcagatatct acatttcaaa attaatgttt accgtacctt cttttcacct   1680
tgcaattttt ttaaaatgca catttactct actaactaac ttttcattta cattaaaaac   1740
atatataatg aaatcaaatt tgtatgcaaa ttccatatat attataaagc taaaattatt   1800
taattgaaaa tttacaatct tatcgtctcc tacataacta atttaaaaac agttgcattc   1860
tcttaaacaa caaagtacaa aaaaagaatg tttccttcca tatggctatt tagctagatc   1920
atcattatta gtaacattct cttttataat cagataaaca caggataatt atacaatgtt   1980
ggaaactcga catagatact aataattgca aacaaataat tagagagaat cgttatagaa   2040
aacaaaaaca aaataaatga tagtagaaaa acaaatgatt tttatagcat tcacagtcag   2100
```

```
agtcacgata attcacctgt ctccggtcaa cgctttttat agactctctt cttctcttcc   2160
ttcaaagc                                                            2168


<210>  59
<211>  3357
<212>  DNA
<213>  Arabidopsis thaliana


<220>
<221>  CDS
<222>  (91)..(3081)
<223>  encoding putative leucine-rich repeat transmembrane protein
       kinase

<400>  59
actctctcac aaacactctc ttcttcatac tctcttatct ctctctcttt cttcaaccac    60
cgtcatagat accggggaag acgaagaaac atg tat ctt ctc ttt ctc ttc tta    114
                                 Met Tyr Leu Leu Phe Leu Phe Leu
                                 1               5
ctt ttc ccc acc gtc ttc tct ctt aac caa gac ggt ttc att ctt caa     162
Leu Phe Pro Thr Val Phe Ser Leu Asn Gln Asp Gly Phe Ile Leu Gln
    10                  15                  20
caa gtc aag ctc tcg tta gac gac cca gac tca tat ctc tct tcc tgg     210
Gln Val Lys Leu Ser Leu Asp Asp Pro Asp Ser Tyr Leu Ser Ser Trp
25                  30                  35                  40
aac tcc aac gat gct tct cct tgt cgg tgg tcc ggc gtt tcc tgc gcc     258
Asn Ser Asn Asp Ala Ser Pro Cys Arg Trp Ser Gly Val Ser Cys Ala
                45                  50                  55
ggt gat ttc tcc tcc gtc act tcc gta gac ctc tcc agc gct aat ctc     306
Gly Asp Phe Ser Ser Val Thr Ser Val Asp Leu Ser Ser Ala Asn Leu
            60                  65                  70
gcc gga cct ttt cct tca gtc att tgt cgt ctc tct aat ctg gct cat     354
Ala Gly Pro Phe Pro Ser Val Ile Cys Arg Leu Ser Asn Leu Ala His
        75                  80                  85
ctc tct ttg tac aat aac tcc atc aac tct act ctt cct ctc aac atc     402
Leu Ser Leu Tyr Asn Asn Ser Ile Asn Ser Thr Leu Pro Leu Asn Ile
    90                  95                  100
gct gct tgt aag agt ctt caa act ctc gat ctc tct cag aat cta ctc     450
Ala Ala Cys Lys Ser Leu Gln Thr Leu Asp Leu Ser Gln Asn Leu Leu
105                 110                 115                 120
acc ggt gag ctt cca caa act ctt gcc gat att ccg act ttg gtt cac     498
Thr Gly Glu Leu Pro Gln Thr Leu Ala Asp Ile Pro Thr Leu Val His
                125                 130                 135
tta gat ttg acc ggt aac aac ttt tcc ggt gac att ccg gcg agt ttc     546
Leu Asp Leu Thr Gly Asn Asn Phe Ser Gly Asp Ile Pro Ala Ser Phe
```

```
            140                 145                 150
ggc aaa ttc gaa aac cta gag gtt ctt tct ctt gtt tac aat ctc tta       594
Gly Lys Phe Glu Asn Leu Glu Val Leu Ser Leu Val Tyr Asn Leu Leu
        155                 160                 165
gac ggt acg att cct ccg ttt ctc ggc aac atc agc acg ttg aag atg       642
Asp Gly Thr Ile Pro Pro Phe Leu Gly Asn Ile Ser Thr Leu Lys Met
    170                 175                 180
ctg aat ctt tcg tat aac ccc ttt agt ccg agt cgg atc ccg ccg gag       690
Leu Asn Leu Ser Tyr Asn Pro Phe Ser Pro Ser Arg Ile Pro Pro Glu
185                 190                 195                 200
ttc ggg aac ttg acg aat ctc gag gtt atg tgg ctc act gag tgt cat       738
Phe Gly Asn Leu Thr Asn Leu Glu Val Met Trp Leu Thr Glu Cys His
                205                 210                 215
tta gtc gga cag atc cct gac tcg ctg ggt caa ctc agt aaa ctc gtt       786
Leu Val Gly Gln Ile Pro Asp Ser Leu Gly Gln Leu Ser Lys Leu Val
            220                 225                 230
gat tta gac ctt gcg ctc aac gac ctt gta ggt cat att cct cct tcg       834
Asp Leu Asp Leu Ala Leu Asn Asp Leu Val Gly His Ile Pro Pro Ser
        235                 240                 245
ctc ggt ggt ttg act aac gtc gtt cag att gag ctg tac aac aac tcg       882
Leu Gly Gly Leu Thr Asn Val Val Gln Ile Glu Leu Tyr Asn Asn Ser
    250                 255                 260
ttg acc gga gag att cca ccg gag ctc ggg aat ttg aaa tcg ttg aga       930
Leu Thr Gly Glu Ile Pro Pro Glu Leu Gly Asn Leu Lys Ser Leu Arg
265                 270                 275                 280
ctt ctc gac gcg tcg atg aat cag tta acc ggg aaa ata ccg gac gag       978
Leu Leu Asp Ala Ser Met Asn Gln Leu Thr Gly Lys Ile Pro Asp Glu
                285                 290                 295
ctt tgc cgt gtg ccg ttg gag agt ttg aat ctc tac gag aac aat cta      1026
Leu Cys Arg Val Pro Leu Glu Ser Leu Asn Leu Tyr Glu Asn Asn Leu
            300                 305                 310
gaa ggt gag ctt ccg gcg agt ata gcg tta tct ccg aac ttg tac gag      1074
Glu Gly Glu Leu Pro Ala Ser Ile Ala Leu Ser Pro Asn Leu Tyr Glu
        315                 320                 325
att aga ata ttc gga aac cgg tta acc ggt gga tta cca aaa gac ctc      1122
Ile Arg Ile Phe Gly Asn Arg Leu Thr Gly Gly Leu Pro Lys Asp Leu
    330                 335                 340
ggt cta aac tcg ccg ttg aga tgg ttg gat gtt tcg gaa aac gaa ttt      1170
Gly Leu Asn Ser Pro Leu Arg Trp Leu Asp Val Ser Glu Asn Glu Phe
345                 350                 355                 360
tcc ggc gac tta ccg gcg gat ctg tgt gcg aaa gga gag cta gag gag      1218
Ser Gly Asp Leu Pro Ala Asp Leu Cys Ala Lys Gly Glu Leu Glu Glu
                365                 370                 375
ttg ttg att ata cac aat tcc ttc tcc ggc gtt ata ccg gag agt ctc      1266
Leu Leu Ile Ile His Asn Ser Phe Ser Gly Val Ile Pro Glu Ser Leu
```

```
            380                 385                 390
gcc gat tgc agg agc ttg aca cgt atc cgg tta gct tat aac cgg ttt      1314
Ala Asp Cys Arg Ser Leu Thr Arg Ile Arg Leu Ala Tyr Asn Arg Phe
        395                 400                 405
tcc ggt tca gtt cct aca ggt ttc tgg gga ttg cct cat gtt aac ttg      1362
Ser Gly Ser Val Pro Thr Gly Phe Trp Gly Leu Pro His Val Asn Leu
    410                 415                 420
ctt gag ctc gtc aac aac tcg ttc tcc ggt gaa att tcg aag tcc att      1410
Leu Glu Leu Val Asn Asn Ser Phe Ser Gly Glu Ile Ser Lys Ser Ile
425                 430                 435                 440
gga ggt gct tca aat ctc tcg ctg ttg att ctc tcc aac aat gaa ttc      1458
Gly Gly Ala Ser Asn Leu Ser Leu Leu Ile Leu Ser Asn Asn Glu Phe
                445                 450                 455
acc gga tct ttg ccg gag gaa att ggt tct ttg gat aat ctt aat cag      1506
Thr Gly Ser Leu Pro Glu Glu Ile Gly Ser Leu Asp Asn Leu Asn Gln
            460                 465                 470
ttg tcg gca agt ggg aac aag ttc agt ggc tcg ttg cct gat agc ttg      1554
Leu Ser Ala Ser Gly Asn Lys Phe Ser Gly Ser Leu Pro Asp Ser Leu
        475                 480                 485
atg agt ctt gga gaa tta ggt act ctt gat ctt cat ggt aat cag ttt      1602
Met Ser Leu Gly Glu Leu Gly Thr Leu Asp Leu His Gly Asn Gln Phe
    490                 495                 500
tca gga gag tta act tct gga atc aaa tct tgg aag aag ctc aac gag      1650
Ser Gly Glu Leu Thr Ser Gly Ile Lys Ser Trp Lys Lys Leu Asn Glu
505                 510                 515                 520
tta aac tta gcc gat aac gaa ttc acc ggt aaa att cca gat gaa atc      1698
Leu Asn Leu Ala Asp Asn Glu Phe Thr Gly Lys Ile Pro Asp Glu Ile
                525                 530                 535
ggg agt ttg tca gta ttg aac tat ctt gat ctt tct ggt aac atg ttc      1746
Gly Ser Leu Ser Val Leu Asn Tyr Leu Asp Leu Ser Gly Asn Met Phe
            540                 545                 550
tct ggc aag atc ccg gtt tca ttg cag agt ttg aag cta aac cag ctg      1794
Ser Gly Lys Ile Pro Val Ser Leu Gln Ser Leu Lys Leu Asn Gln Leu
        555                 560                 565
aat ctg tct tat aac cgg tta tcg ggt gac tta ccg cct tct tta gcg      1842
Asn Leu Ser Tyr Asn Arg Leu Ser Gly Asp Leu Pro Pro Ser Leu Ala
    570                 575                 580
aaa gat atg tat aag aat agc ttc att ggg aac ccg gga ttg tgt ggg      1890
Lys Asp Met Tyr Lys Asn Ser Phe Ile Gly Asn Pro Gly Leu Cys Gly
585                 590                 595                 600
gat atc aag gga ttg tgt ggc tct gag aat gaa gct aag aag aga ggc      1938
Asp Ile Lys Gly Leu Cys Gly Ser Glu Asn Glu Ala Lys Lys Arg Gly
                605                 610                 615
tat gta tgg ctt ctt aga tcg att ttc gta ctt gct gcg atg gtg ctt      1986
Tyr Val Trp Leu Leu Arg Ser Ile Phe Val Leu Ala Ala Met Val Leu
```

```
              620                 625                 630
ctt gcg ggt gtt gct tgg ttc tac ttc aag tac agg act ttc aag aaa     2034
Leu Ala Gly Val Ala Trp Phe Tyr Phe Lys Tyr Arg Thr Phe Lys Lys
        635                 640                 645
gca aga gcc atg gag aga tct aag tgg act cta atg tcg ttc cac aaa     2082
Ala Arg Ala Met Glu Arg Ser Lys Trp Thr Leu Met Ser Phe His Lys
    650                 655                 660
ctc ggg ttc agt gag cat gag att ctt gaa agc ttg gat gaa gat aat     2130
Leu Gly Phe Ser Glu His Glu Ile Leu Glu Ser Leu Asp Glu Asp Asn
665                 670                 675                 680
gtg att gga gct gga gct tca ggt aaa gtt tac aag gtt gta ctc acc     2178
Val Ile Gly Ala Gly Ala Ser Gly Lys Val Tyr Lys Val Val Leu Thr
                685                 690                 695
aat ggg gaa act gtt gcg gtt aag cgt tta tgg aca ggt tct gtt aag     2226
Asn Gly Glu Thr Val Ala Val Lys Arg Leu Trp Thr Gly Ser Val Lys
            700                 705                 710
gaa act gga gat tgt gat cca gag aaa ggt tac aaa cct gga gtt caa     2274
Glu Thr Gly Asp Cys Asp Pro Glu Lys Gly Tyr Lys Pro Gly Val Gln
        715                 720                 725
gat gag gct ttt gaa gct gaa gtt gag aca ttg ggt aag att agg cat     2322
Asp Glu Ala Phe Glu Ala Glu Val Glu Thr Leu Gly Lys Ile Arg His
    730                 735                 740
aag aac att gtg aag cta tgg tgt tgc tgt tct aca aga gac tgc aag     2370
Lys Asn Ile Val Lys Leu Trp Cys Cys Cys Ser Thr Arg Asp Cys Lys
745                 750                 755                 760
ctc ttg gtt tat gag tac atg cct aat ggt agt ttg gga gat ttg ctt     2418
Leu Leu Val Tyr Glu Tyr Met Pro Asn Gly Ser Leu Gly Asp Leu Leu
                765                 770                 775
cat agc agt aaa gga gga atg ttg gga tgg caa acg agg ttt aag att     2466
His Ser Ser Lys Gly Gly Met Leu Gly Trp Gln Thr Arg Phe Lys Ile
            780                 785                 790
atc tta gat gcg gct gag ggg ctt tcg tat ctt cac cat gat tct gtt     2514
Ile Leu Asp Ala Ala Glu Gly Leu Ser Tyr Leu His His Asp Ser Val
        795                 800                 805
cct ccg att gtg cat aga gat att aag tca aac aat att ttg atc gat     2562
Pro Pro Ile Val His Arg Asp Ile Lys Ser Asn Asn Ile Leu Ile Asp
    810                 815                 820
gga gat tat ggt gca aga gtt gct gat ttt ggt gtg gct aaa gct gtc     2610
Gly Asp Tyr Gly Ala Arg Val Ala Asp Phe Gly Val Ala Lys Ala Val
825                 830                 835                 840
gac ttg acc gga aaa gct cct aaa tcg atg tca gtg atc gct ggt tca     2658
Asp Leu Thr Gly Lys Ala Pro Lys Ser Met Ser Val Ile Ala Gly Ser
                845                 850                 855
tgc ggt tat atc gca cca gaa tac gca tat acg ctt cgt gtg aac gag     2706
Cys Gly Tyr Ile Ala Pro Glu Tyr Ala Tyr Thr Leu Arg Val Asn Glu
```

```
            860                 865                 870
aaa agc gac atc tac agt ttc ggg gta gtg atc ctt gag ata gta act      2754
Lys Ser Asp Ile Tyr Ser Phe Gly Val Val Ile Leu Glu Ile Val Thr
        875                 880                 885
agg aaa cgc ccg gtt gat cca gaa ctt ggg gag aag gat ttg gtg aag      2802
Arg Lys Arg Pro Val Asp Pro Glu Leu Gly Glu Lys Asp Leu Val Lys
    890                 895                 900
tgg gtt tgc tct aca ttg gac caa aaa ggc ata gag cat gtg ata gac      2850
Trp Val Cys Ser Thr Leu Asp Gln Lys Gly Ile Glu His Val Ile Asp
905                 910                 915                 920
ccg aaa ctc gac tct tgt ttc aaa gaa gag ata agc aaa atc ctc aac      2898
Pro Lys Leu Asp Ser Cys Phe Lys Glu Glu Ile Ser Lys Ile Leu Asn
                925                 930                 935
gtt gga ctc ctc tgc acg agt ccg ttg ccc att aac cga cct tcc atg      2946
Val Gly Leu Leu Cys Thr Ser Pro Leu Pro Ile Asn Arg Pro Ser Met
            940                 945                 950
agg cgt gtg gtt aag atg ttg caa gaa att ggt ggt gga gac gaa gat      2994
Arg Arg Val Val Lys Met Leu Gln Glu Ile Gly Gly Gly Asp Glu Asp
        955                 960                 965
agc cta cac aag ata aga gat gac aag gat ggc aag tta aca cct tat      3042
Ser Leu His Lys Ile Arg Asp Asp Lys Asp Gly Lys Leu Thr Pro Tyr
    970                 975                 980
tac aac gaa gac acc tca gac caa gga agt ata gct tga gacacaaatg       3091
Tyr Asn Glu Asp Thr Ser Asp Gln Gly Ser Ile Ala
985                 990                 995
gagaaagaaa agtgttttgc ccaaaaaagc tctctcaatt tttggtgctt gaagctgtca   3151
atttagggca ttgaatcaag attctctcac cccttttag ggttttgatc taaaagtgag    3211
aatcagagat tgtcgaaatt tttataatat atataaagaa gtttctaatt cagcgtttca   3271
gtttctccac tgtataaagc tattagtaaa ctctggtttt gtaagagttt ttatctttgg   3331
taaatgcaaa tgaaaaagtt gcgatc                                        3357
```

<210> 60
<211> 996
<212> PRT
<213> Arabidopsis thaliana

<400> 60

```
Met Tyr Leu Leu Phe Leu Phe Leu Leu Phe Pro Thr Val Phe Ser Leu
1               5                   10                  15

Asn Gln Asp Gly Phe Ile Leu Gln Gln Val Lys Leu Ser Leu Asp Asp
            20                  25                  30

Pro Asp Ser Tyr Leu Ser Ser Trp Asn Ser Asn Asp Ala Ser Pro Cys
        35                  40                  45
```

Arg Trp Ser Gly Val Ser Cys Ala Gly Asp Phe Ser Ser Val Thr Ser
50 55 60

Val Asp Leu Ser Ser Ala Asn Leu Ala Gly Pro Phe Pro Ser Val Ile
65 70 75 80

Cys Arg Leu Ser Asn Leu Ala His Leu Ser Leu Tyr Asn Asn Ser Ile
85 90 95

Asn Ser Thr Leu Pro Leu Asn Ile Ala Ala Cys Lys Ser Leu Gln Thr
100 105 110

Leu Asp Leu Ser Gln Asn Leu Leu Thr Gly Glu Leu Pro Gln Thr Leu
115 120 125

Ala Asp Ile Pro Thr Leu Val His Leu Asp Leu Thr Gly Asn Asn Phe
130 135 140

Ser Gly Asp Ile Pro Ala Ser Phe Gly Lys Phe Glu Asn Leu Glu Val
145 150 155 160

Leu Ser Leu Val Tyr Asn Leu Leu Asp Gly Thr Ile Pro Pro Phe Leu
165 170 175

Gly Asn Ile Ser Thr Leu Lys Met Leu Asn Leu Ser Tyr Asn Pro Phe
180 185 190

Ser Pro Ser Arg Ile Pro Pro Glu Phe Gly Asn Leu Thr Asn Leu Glu
195 200 205

Val Met Trp Leu Thr Glu Cys His Leu Val Gly Gln Ile Pro Asp Ser
210 215 220

Leu Gly Gln Leu Ser Lys Leu Val Asp Leu Asp Leu Ala Leu Asn Asp
225 230 235 240

Leu Val Gly His Ile Pro Pro Ser Leu Gly Gly Leu Thr Asn Val Val
245 250 255

Gln Ile Glu Leu Tyr Asn Asn Ser Leu Thr Gly Glu Ile Pro Pro Glu
260 265 270

Leu Gly Asn Leu Lys Ser Leu Arg Leu Leu Asp Ala Ser Met Asn Gln
275 280 285

Leu Thr Gly Lys Ile Pro Asp Glu Leu Cys Arg Val Pro Leu Glu Ser
290 295 300

Leu Asn Leu Tyr Glu Asn Asn Leu Glu Gly Glu Leu Pro Ala Ser Ile
305 310 315 320

Ala Leu Ser Pro Asn Leu Tyr Glu Ile Arg Ile Phe Gly Asn Arg Leu
325 330 335

Thr Gly Gly Leu Pro Lys Asp Leu Gly Leu Asn Ser Pro Leu Arg Trp
340 345 350

Leu Asp Val Ser Glu Asn Glu Phe Ser Gly Asp Leu Pro Ala Asp Leu
355 360 365

Cys Ala Lys Gly Glu Leu Glu Glu Leu Leu Ile Ile His Asn Ser Phe
370 375 380

Ser Gly Val Ile Pro Glu Ser Leu Ala Asp Cys Arg Ser Leu Thr Arg
385 390 395 400

Ile Arg Leu Ala Tyr Asn Arg Phe Ser Gly Ser Val Pro Thr Gly Phe
405 410 415

Trp Gly Leu Pro His Val Asn Leu Leu Glu Leu Val Asn Asn Ser Phe
420 425 430

Ser Gly Glu Ile Ser Lys Ser Ile Gly Gly Ala Ser Asn Leu Ser Leu
435 440 445

Leu Ile Leu Ser Asn Asn Glu Phe Thr Gly Ser Leu Pro Glu Glu Ile
450 455 460

Gly Ser Leu Asp Asn Leu Asn Gln Leu Ser Ala Ser Gly Asn Lys Phe
465 470 475 480

Ser Gly Ser Leu Pro Asp Ser Leu Met Ser Leu Gly Glu Leu Gly Thr
485 490 495

Leu Asp Leu His Gly Asn Gln Phe Ser Gly Glu Leu Thr Ser Gly Ile
500 505 510

Lys Ser Trp Lys Lys Leu Asn Glu Leu Asn Leu Ala Asp Asn Glu Phe
515 520 525

```
Thr Gly Lys Ile Pro Asp Glu Ile Gly Ser Leu Ser Val Leu Asn Tyr
    530                 535                 540

Leu Asp Leu Ser Gly Asn Met Phe Ser Gly Lys Ile Pro Val Ser Leu
545                 550                 555                 560

Gln Ser Leu Lys Leu Asn Gln Leu Asn Leu Ser Tyr Asn Arg Leu Ser
                565                 570                 575

Gly Asp Leu Pro Pro Ser Leu Ala Lys Asp Met Tyr Lys Asn Ser Phe
            580                 585                 590

Ile Gly Asn Pro Gly Leu Cys Gly Asp Ile Lys Gly Leu Cys Gly Ser
        595                 600                 605

Glu Asn Glu Ala Lys Lys Arg Gly Tyr Val Trp Leu Leu Arg Ser Ile
    610                 615                 620

Phe Val Leu Ala Ala Met Val Leu Leu Ala Gly Val Ala Trp Phe Tyr
625                 630                 635                 640

Phe Lys Tyr Arg Thr Phe Lys Lys Ala Arg Ala Met Glu Arg Ser Lys
                645                 650                 655

Trp Thr Leu Met Ser Phe His Lys Leu Gly Phe Ser Glu His Glu Ile
            660                 665                 670

Leu Glu Ser Leu Asp Glu Asp Asn Val Ile Gly Ala Gly Ala Ser Gly
        675                 680                 685

Lys Val Tyr Lys Val Val Leu Thr Asn Gly Glu Thr Val Ala Val Lys
    690                 695                 700

Arg Leu Trp Thr Gly Ser Val Lys Glu Thr Gly Asp Cys Asp Pro Glu
705                 710                 715                 720

Lys Gly Tyr Lys Pro Gly Val Gln Asp Glu Ala Phe Glu Ala Glu Val
                725                 730                 735

Glu Thr Leu Gly Lys Ile Arg His Lys Asn Ile Val Lys Leu Trp Cys
            740                 745                 750

Cys Cys Ser Thr Arg Asp Cys Lys Leu Leu Val Tyr Glu Tyr Met Pro
        755                 760                 765
```

Asn Gly Ser Leu Gly Asp Leu Leu His Ser Ser Lys Gly Gly Met Leu
770 775 780

Gly Trp Gln Thr Arg Phe Lys Ile Ile Leu Asp Ala Ala Glu Gly Leu
785 790 795 800

Ser Tyr Leu His His Asp Ser Val Pro Pro Ile Val His Arg Asp Ile
805 810 815

Lys Ser Asn Asn Ile Leu Ile Asp Gly Asp Tyr Gly Ala Arg Val Ala
820 825 830

Asp Phe Gly Val Ala Lys Ala Val Asp Leu Thr Gly Lys Ala Pro Lys
835 840 845

Ser Met Ser Val Ile Ala Gly Ser Cys Gly Tyr Ile Ala Pro Glu Tyr
850 855 860

Ala Tyr Thr Leu Arg Val Asn Glu Lys Ser Asp Ile Tyr Ser Phe Gly
865 870 875 880

Val Val Ile Leu Glu Ile Val Thr Arg Lys Arg Pro Val Asp Pro Glu
885 890 895

Leu Gly Glu Lys Asp Leu Val Lys Trp Val Cys Ser Thr Leu Asp Gln
900 905 910

Lys Gly Ile Glu His Val Ile Asp Pro Lys Leu Asp Ser Cys Phe Lys
915 920 925

Glu Glu Ile Ser Lys Ile Leu Asn Val Gly Leu Leu Cys Thr Ser Pro
930 935 940

Leu Pro Ile Asn Arg Pro Ser Met Arg Arg Val Val Lys Met Leu Gln
945 950 955 960

Glu Ile Gly Gly Gly Asp Glu Asp Ser Leu His Lys Ile Arg Asp Asp
965 970 975

Lys Asp Gly Lys Leu Thr Pro Tyr Tyr Asn Glu Asp Thr Ser Asp Gln
980 985 990

Gly Ser Ile Ala
995

<210> 61
<211> 27
<212> DNA
<213> Artificial

<220>
<223> oligonucleotide primer

<400> 61
tgttaggaat tcagcagcca catatgc 27

<210> 62
<211> 28
<212> DNA
<213> Artificial

<220>
<223> oligonucleotide primer

<400> 62
ctttcgccat ggtctttgat cttattag 28

<210> 63
<211> 29
<212> DNA
<213> Artificial

<220>
<223> oligonucleotide primer

<400> 63
ggattcccat ggctcatgtg agagttttt 29

<210> 64
<211> 22
<212> DNA
<213> Artificial

<220>
<223> oligonucleotide primer

<400> 64
cgtttagaat tcagaatccg ac 22

<210> 65
<211> 28
<212> DNA
<213> Artificial

<220>
<223> oligonucleotide primer

<400> 65
ctttcgccat ggtctttgat cttattag 28

<210> 66
<211> 29
<212> DNA
<213> Artificial

<220>
<223> oligonucleotide primer

<400> 66
ggattcccat ggctcatgtg agagttttt 29

<210> 67
<211> 23
<212> DNA
<213> Artificial

<220>
<223> oligonucleotide primer

<400> 67
cacattctcg aggcttggag atg 23

<210> 68
<211> 29
<212> DNA
<213> Artificial

<220>
<223> oligonucleotide primer

<400> 68
gtctcaggat ccttaatttc ttctatcgg 29

<210> 69

<211> 30
<212> DNA
<213> Artificial

<220>
<223> oligonucleotide primer

<400> 69
ttgagaggat ccttagagaa gatggttgag 30

<210> 70
<211> 27
<212> DNA
<213> Artificial

<220>
<223> oligonucleotide primer

<400> 70
tctcgtcccg ggtttttttt ggtttcc 27

<210> 71
<211> 29
<212> DNA
<213> Artificial

<220>
<223> oligonucleotide primer

<400> 71
gtctcaggat ccttaatttc ttctatcgg 29

<210> 72
<211> 30
<212> DNA
<213> Artificial

<220>
<223> oligonucleotide primer

<400> 72
ttgagaggat ccttagagaa gatggttgag 30

<210> 73
<211> 26

<212> DNA
<213> Artificial

<220>
<223> oligonucleotide primer

<400> 73
gttataggat ccaatctcat ccactg 26

<210> 74
<211> 25
<212> DNA
<213> Artificial

<220>
<223> oligonucleotide primer

<400> 74
gatcggccat ggttaattaa ccacc 25

<210> 75
<211> 28
<212> DNA
<213> Artificial

<220>
<223> oligonucleotide primer

<400> 75
aatctcccat ggtctctcag taccaaag 28

<210> 76
<211> 29
<212> DNA
<213> Artificial

<220>
<223> oligonucleotide primer

<400> 76
tgttgagaat tctgctttct tcatactag 29

<210> 77
<211> 25
<212> DNA

<213> Artificial

<220>
<223> oligonucleotide primer

<400> 77
gatcggccat ggttaattaa ccacc 25

<210> 78
<211> 28
<212> DNA
<213> Artificial

<220>
<223> oligonucleotide primer

<400> 78
aatctcccat ggtctctcag taccaaag 28

<210> 79
<211> 26
<212> DNA
<213> Artificial

<220>
<223> oligonucleotide primer

<400> 79
ggaaatacta gttggctcat ggctgc 26

<210> 80
<211> 25
<212> DNA
<213> Artificial

<220>
<223> oligonucleotide primer

<400> 80
gatcggccat ggttaattaa ccacc 25

<210> 81
<211> 28
<212> DNA
<213> Artificial

<220>
<223> oligonucleotide primer

<400> 81
aatctcccat ggtctctcag taccaaag 28

<210> 82
<211> 23
<212> DNA
<213> Artificial

<220>
<223> oligonucleotide primer

<400> 82
aagaagacta gtgaaaagta gag 23

<210> 83
<211> 23
<212> DNA
<213> Artificial

<220>
<223> oligonucleotide primer

<400> 83
gaatcgccat ggttggaatg aag 23

<210> 84
<211> 29
<212> DNA
<213> Artificial

<220>
<223> oligonucleotide primer

<400> 84
caaaaaccat ggtgtgtata agtggaggg 29

<210> 85
<211> 23
<212> DNA
<213> Artificial

<220>
<223> oligonucleotide primer

<400> 85
aaaatggaat tcgtaggaat acg 23

<210> 86
<211> 23
<212> DNA
<213> Artificial

<220>
<223> oligonucleotide primer

<400> 86
gaatcgccat ggttggaatg aag 23

<210> 87
<211> 29
<212> DNA
<213> Artificial

<220>
<223> oligonucleotide primer

<400> 87
caaaaaccat ggtgtgtata agtggaggg 29

<210> 88
<211> 26
<212> DNA
<213> Artificial

<220>
<223> oligonucleotide primer

<400> 88
atctgtacta gtgttggtag tgagtg 26

<210> 89
<211> 24
<212> DNA
<213> Artificial

<220>

<223> oligonucleotide primer

<400> 89
cttgctccat ggatcttgat gatg 24

<210> 90
<211> 28
<212> DNA
<213> Artificial

<220>
<223> oligonucleotide primer

<400> 90
gaaagcccat gggaagttaa taaagctg 28

<210> 91
<211> 23
<212> DNA
<213> Artificial

<220>
<223> oligonucleotide primer

<400> 91
atatccacta gtagagggtg agg 23

<210> 92
<211> 24
<212> DNA
<213> Artificial

<220>
<223> oligonucleotide primer

<400> 92
cttgctccat ggatcttgat gatg 24

<210> 93
<211> 28
<212> DNA
<213> Artificial

<220>
<223> oligonucleotide primer

<400> 93
gaaagcccat gggaagttaa taaagctg 28

<210> 94
<211> 28
<212> DNA
<213> Artificial

<220>
<223> oligonucleotide primer

<400> 94
gatatcggat cctatcttcc atattttc 28

<210> 95
<211> 25
<212> DNA
<213> Artificial

<220>
<223> oligonucleotide primer

<400> 95
gaagatccat ggttcttcgt cttcc 25

<210> 96
<211> 27
<212> DNA
<213> Artificial

<220>
<223> oligonucleotide primer

<400> 96
gctttgccat gggagaagaa gagagtc 27

<210> 97
<211> 26
<212> DNA
<213> Artificial

<220>
<223> oligonucleotide primer

<400> 97
cattgaggat cctaatgcct tcaaac 26

<210> 98
<211> 25
<212> DNA
<213> Artificial

<220>
<223> oligonucleotide primer

<400> 98
gaagatccat ggttcttcgt cttcc 25

<210> 99
<211> 27
<212> DNA
<213> Artificial

<220>
<223> oligonucleotide primer

<400> 99
gctttgccat gggagaagaa gagagtc 27

<210> 100
<211> 8986
<212> DNA
<213> Artificial

<220>
<223> binary vector pSUN0301

<400> 100
cgttgtaaaa cgacggccag tgaattcgag ctcggtacct cgagcccggg cgatatcgga 60
tccactagtc tagagtcgat cgaccatggt acgtcctgta gaaaccccaa cccgtgaaat 120
caaaaaactc gacggcctgt gggcattcag tctggatcgc gaaaactgtg gaattggtca 180
gcgttggtgg gaaagcgcgt tacaagaaag ccgggcaatt gctgtgccag gcagttttaa 240
cgatcagttc gccgatgcag atattcgtaa ttatgcgggc aacgtctggt atcagcgcga 300
agtctttata ccgaaaggtt gggcaggcca gcgtatcgtg ctgcgtttcg atgcggtcac 360
tcattacggc aaagtgtggg tcaataatca ggaagtgatg gagcatcagg gcggctatac 420
gccatttgaa gccgatgtca cgccgtatgt tattgccggg aaaagtgtac gtaagtttct 480
gcttctacct ttgatatata tataataatt atcattaatt agtagtaata taatatttca 540
aatatttttt tcaaaataaa agaatgtagt atatagcaat tgcttttctg tagtttataa 600
gtgtgtatat tttaatttat aacttttcta atatatgacc aaaatttgtt gatgtgcagg 660

```
tatcaccgtt tgtgtgaaca acgaactgaa ctggcagact atcccgccgg gaatggtgat     720
taccgacgaa aacggcaaga aaaagcagtc ttacttccat gatttcttta actatgccgg     780
aatccatcgc agcgtaatgc tctacaccac gccgaacacc tgggtggacg atatcaccgt     840
ggtgacgcat gtcgcgcaag actgtaacca cgcgtctgtt gactggcagg tggtggccaa     900
tggtgatgtc agcgttgaac tgcgtgatgc ggatcaacag gtggttgcaa ctggacaagg     960
cactagcggg actttgcaag tggtgaatcc gcacctctgg caaccgggtg aaggttatct    1020
ctatgaactg tgcgtcacag ccaaaagcca gacagagtgt gatatctacc cgcttcgcgt    1080
cggcatccgg tcagtggcag tgaagggcga acagttcctg attaaccaca aaccgttcta    1140
ctttactggc tttggtcgtc atgaagatgc ggacttacgt ggcaaaggat tcgataacgt    1200
gctgatggtg cacgaccacg cattaatgga ctggattggg gccaactcct accgtacctc    1260
gcattaccct tacgctgaag agatgctcga ctgggcagat gaacatggca tcgtggtgat    1320
tgatgaaact gctgctgtcg gctttaacct ctctttaggc attggtttcg aagcgggcaa    1380
caagccgaaa gaactgtaca gcgaagaggc agtcaacggg gaaactcagc aagcgcactt    1440
acaggcgatt aaagagctga tagcgcgtga caaaaaccac ccaagcgtgg tgatgtggag    1500
tattgccaac gaaccggata cccgtccgca agtgcacggg aatatttcgc cactggcgga    1560
agcaacgcgt aaactcgacc cgacgcgtcc gatcacctgc gtcaatgtaa tgttctgcga    1620
cgctcacacc gataccatca gcgatctctt tgatgtgctg tgcctgaacc gttattacgg    1680
atggtatgtc caaagcggcg atttggaaac ggcagagaag gtactggaaa aagaacttct    1740
ggcctggcag gagaaactgc atcagccgat tatcatcacc gaatacggcg tggatacgtt    1800
agccgggctg cactcaatgt acaccgacat gtggagtgaa gagtatcagt gtgcatggct    1860
ggatatgtat caccgcgtct ttgatcgcgt cagcgccgtc gtcggtgaac aggtatggaa    1920
tttcgccgat tttgcgacct cgcaaggcat attgcgcgtt ggcggtaaca agaaagggat    1980
cttcactcgc gaccgcaaac cgaagtcggc ggcttttctg ctgcaaaaac gctggactgg    2040
catgaacttc ggtgaaaaac cgcagcaggg aggcaaacaa tgaatcaaca actctcctgg    2100
cgcaccatcg tcggctacag cctcgggaat tgctaccgag ctcggtaccc ggcgcaaaaa    2160
tcaccagtct ctctctacaa atctatctct ctctattttt ctccagaata atgtgtgagt    2220
agttcccaga taagggaatt agggttctta tagggtttcg ctcatgtgtt gagcatataa    2280
gaaaccctta gtatgtattt gtatttgtaa aatacttcta tcaataaaat ttctaattcc    2340
taaaaccaaa atccagtgac cgggtaccga gctcgaattt cgacctgcag gcatgcaagc    2400
ttggcgtaat catggtcata gctgtttcct actagatctg attgtcgttt cccgccttca    2460
gtttaaacta tcagtgtttg acaggatata ttggcgggta aacctaagag aaaagagcgt    2520
ttattagaat aatcggatat ttaaaagggc gtgaaaaggt ttatccgttc gtccatttgt    2580
atgtccatga taagtcgcgc tgtatgtgtt tgtttgaata ttcatggaac gcagtggcgg    2640
ttttcatggc ttgttatgac tgtttttttg gggtacagtc tatgcctcgg gcatccaagc    2700
agcaagcgcg ttacgccgtg ggtcgatgtt tgatgttatg gagcagcaac gatgttacgc    2760
agcagggcag tcgccctaaa acaaagttaa acatcatggg ggaagcggtg atcgccgaag    2820
tatcgactca actatcagag gtagttggcg tcatcgagcg ccatctcgaa ccgacgttgc    2880
tggccgtaca tttgtacggc tccgcagtgg atggcggcct gaagccacac agtgatattg    2940
atttgctggt tacggtgacc gtaaggcttg atgaaacaac gcggcgagct ttgatcaacg    3000
accttttgga aacttcggct tcccctggag agagcgagat tctccgcgct gtagaagtca    3060
ccattgttgt gcacgacgac atcattccgt ggcgttatcc agctaagcgc gaactgcaat    3120
ttggagaatg gcagcgcaat gacattcttg caggtatctt cgagccagcc acgatcgaca    3180
ttgatctggc tatcttgctg acaaaagcaa gagaacatag cgttgccttg gtaggtccag    3240
cggcggagga actctttgat ccggttcctg aacaggatct atttgaggcg ctaaatgaaa    3300
ccttaacgct atggaactcg ccgcccgact gggctggcga tgagcgaaat gtagtgctta    3360
```

cgttgtcccg catttggtac agcgcagtaa ccggcaaaat cgcgccgaag gatgtcgctg 3420
ccgactgggc aatggagcgc ctgccggccc agtatcagcc cgtcatactt gaagctagac 3480
aggcttatct tggacaagaa gaagatcgct tggcctcgcg cgcagatcag ttggaagaat 3540
ttgtccacta cgtgaaaggc gagatcacca aggtagtcgg caaataatgt ctagctagaa 3600
attcgttcaa gccgacgccg cttcgcggcg cggcttaact caagcgttag atgcactaag 3660
cacataattg ctcacagcca aactatcagg tcaagtctgc ttttattatt tttaagcgtg 3720
cataataagc cctacacaaa ttgggagata tatcatgcat gaccaaaatc ccttaacgtg 3780
agttttcgtt ccactgagcg tcagaccccg tagaaaagat caaaggatct tcttgagatc 3840
cttttttct gcgcgtaatc tgctgcttgc aaacaaaaaa accaccgcta ccagcggtgg 3900
tttgtttgcc ggatcaagag ctaccaactc tttttccgaa ggtaactggc ttcagcagag 3960
cgcagatacc aaatactgtc cttctagtgt agccgtagtt aggccaccac ttcaagaact 4020
ctgtagcacc gcctacatac ctcgctctgc taatcctgtt accagtggct gctgccagtg 4080
gcgataagtc gtgtcttacc gggttggact caagacgata gttaccggat aaggcgcagc 4140
ggtcgggctg aacggggggt tcgtgcacac agcccagctt ggagcgaacg acctacaccg 4200
aactgagata cctacagcgt gagctatgag aaagcgccac gcttcccgaa gggagaaagg 4260
cggacaggta tccggtaagc ggcagggtcg gaacaggaga gcgcacgagg gagcttccag 4320
ggggaaacgc ctggtatctt tatagtcctg tcgggtttcg ccacctctga cttgagcgtc 4380
gatttttgtg atgctcgtca ggggggcgga gcctatggaa aaacgccagc aacgcggcct 4440
ttttacggtt cctggccttt tgctggcctt ttgctcacat gttctttcct gcgttatccc 4500
ctgattctgt ggataaccgt attaccgcct ttgagtgagc tgataccgct cgccgcagcc 4560
gaacgaccga gcgcagcgag tcagtgagcg aggaagcgga agagcgcctg atgcggtatt 4620
ttctccttac gcatctgtgc ggtatttcac accgcatagg ccgcgatagg ccgacgcgaa 4680
gcggcggggc gtagggagcg cagcgaccga agggtaggcg ctttttgcag ctcttcggct 4740
gtgcgctggc cagacagtta tgcacaggcc aggcgggttt taagagtttt aataagtttt 4800
aaagagtttt aggcggaaaa atcgcctttt ttctctttta tatcagtcac ttacatgtgt 4860
gaccggttcc caatgtacgg ctttgggttc ccaatgtacg ggttccggtt cccaatgtac 4920
ggctttgggt tcccaatgta cgtgctatcc acaggaaaga gaccttttcg accttttcc 4980
cctgctaggg caatttgccc tagcatctgc tccgtacatt aggaaccggc ggatgcttcg 5040
ccctcgatca ggttgcggta gcgcatgact aggatcgggc cagcctgccc cgcctcctcc 5100
ttcaaatcgt actccggcag gtcatttgac ccgatcagct tgcgcacggt gaaacagaac 5160
ttcttgaact ctccggcgct gccactgcgt tcgtagatcg tcttgaacaa ccatctggct 5220
tctgccttgc ctgcggcgcg gcgtgccagg cggtagagaa aacggccgat gccgggatcg 5280
atcaaaaagt aatcggggtg aaccgtcagc acgtccgggt tcttgccttc tgtgatctcg 5340
cggtacatcc aatcagctag ctcgatctcg atgtactccg gccgcccggt ttcgctcttt 5400
acgatcttgt agcggctaat caaggcttca ccctcggata ccgtcaccag gcggccgttc 5460
ttggccttct tcgtacgctg catggcaacg tgcgtggtgt ttaaccgaat gcaggtttct 5520
accaggtcgt ctttctgctt tccgccatcg gctcgccggc agaacttgag tacgtccgca 5580
acgtgtggac ggaacacgcg gccgggcttg tctcccttcc cttcccggta tcggttcatg 5640
gattcggtta gatgggaaac cgccatcagt accaggtcgt aatcccacac actggccatg 5700
ccggccggcc ctgcggaaac ctctacgtgc ccgtctggaa gctcgtagcg gatcacctcg 5760
ccagctcgtc ggtcacgctt cgacagacgg aaaacggcca cgtccatgat gctgcgacta 5820
tcgcgggtgc ccacgtcata gagcatcgga acgaaaaaat ctggttgctc gtcgcccttg 5880
ggcggcttcc taatcgacgg cgcaccggct gccggcggtt gccgggattc tttgcggatt 5940
cgatcagcgg ccccttgcca cgattcaccg ggcgtgctt ctgcctcgat gcgttgccgc 6000
tgggcggcct gcgcggcctt caacttctcc accaggtcat cacccagcgc cgcgccgatt 6060

```
tgtaccgggc cggatggttt gcgaccgctc acgccgattc ctcgggcttg ggggttccag   6120
tgccattgca gggccggcag acaacccagc cgcttacgcc tggccaaccg cccgttcctc   6180
cacacatggg gcattccacg gcgtcggtgc ctggttgttc ttgattttcc atgccgcctc   6240
ctttagccgc taaaattcat ctactcattt attcatttgc tcatttactc tggtagctgc   6300
gcgatgtatt cagatagcag ctcggtaatg gtcttgcctt ggcgtaccgc gtacatcttc   6360
agcttggtgt gatcctccgc cggcaactga aagttgaccc gcttcatggc tggcgtgtct   6420
gccaggctgg ccaacgttgc agccttgctg ctgcgtgcgc tcggacggcc ggcacttagc   6480
gtgtttgtgc ttttgctcat ttctctttta cctcattaac tcaaatgagt tttgatttaa   6540
tttcagcggc cagcgcctgg acctcgcggg cagcgtcgcc ctcgggttct gattcaagaa   6600
cggttgtgcc ggcggcggca gtgcctgggt agctcacgcg ctgcgtgata cgggactcaa   6660
gaatgggcag ctcgtacccg gccagcgcct cggcaacctc accgccgatg cgcgtgcctt   6720
tgatcgcccg cgacacgaca aaggccgctt gtagccttcc atccgtgacc tcaatgcgct   6780
gcttaaccag ctccaccagg tcggcggtgg cccatatgtc gtaagggctt ggctgcaccg   6840
gaatcagcac gaagtcggct gccttgatcg cggacacagc caagtccgcc gcctggggcg   6900
ctccgtcgat cactacgaag tcgcgccggc cgatggcctt cacgtcgcgg tcaatcgtcg   6960
ggcggtcgat gccgacaacg gttagcggtt gatcttcccg cacggccgcc caatcgcggg   7020
cactgccctg gggatcggaa tcgactaaca gaacatcggc cccggcgagt tgcagggcgc   7080
gggctagatg ggttgcgatg gtcgtcttgc ctgacccgcc tttctggtta agtacagcga   7140
taaccttcat gcgttcccct tgcgtatttg tttatttact catcgcatca tatacgcagc   7200
gaccgcatga cgcaagctgt tttactcaaa tacacatcac ctttttagac gcgtggtgat   7260
tttgtgccga gctgccggtc ggggagctgt tggctggctg gtggcaggat atattgtggt   7320
gtaaacaaat tgacgcttag acaacttaat aacacattgc ggacgtcttt aatgtactga   7380
attaacatcc gtttgatact tgtctaaaat tggctgattt cgagtgcatc tatgcataaa   7440
aacaatctaa tgacaattat taccaagcag tgatcctgtc aaacactgat agtttaaact   7500
gaaggcggga aacgacaatc tgatcatgag cggagaatta agggagtcac gttatgaccc   7560
ccgccgatga cgcgggacaa gccgttttac gtttggaact gacagaaccg caacgttgaa   7620
ggagccactc agccgcgggt ttctggagtt taatgagcta agcacatacg tcagaaacca   7680
ttattgcgcg ttcaaaagtc gcctaaggtc actatcagct agcaaatatt tcttgtcaaa   7740
aatgctccac tgacgttcca taaattcccc tcggtatcca attagagtct catattcact   7800
ctcaatccaa ataatctgca ccggatctgg atcgtttcgc atgattgaac aagatggatt   7860
gcacgcaggt tctccggccg cttgggtgga gaggctattc ggctatgact gggcacaaca   7920
gacaatcggc tgctctgatg ccgccgtgtt ccggctgtca gcgcaggggc gcccggttct   7980
ttttgtcaag accgacctgt ccggtgccct gaatgaactg caggacgagg cagcgcggct   8040
atcgtggctg gccacgacgg gcgttccttg cgcagctgtg ctcgacgttg tcactgaagc   8100
gggaagggac tggctgctat tgggcgaagt gccggggcag gatctcctgt catctcacct   8160
tgctcctgcc gagaaagtat ccatcatggc tgatgcaatg cggcggctgc atacgcttga   8220
tccggctacc tgcccattcg accaccaagc gaaacatcgc atcgagcgag cacgtactcg   8280
gatggaagcc ggtcttgtcg atcaggatga tctggacgaa gagcatcagg ggctcgcgcc   8340
agccgaactg ttcgccaggc tcaaggcgcg catgcccgac ggcgaggatc tcgtcgtgac   8400
acatggcgat gcctgcttgc cgaatatcat ggtggaaaat ggccgctttt ctggattcat   8460
cgactgtggc cggctgggtg tggcggaccg ctatcaggac atagcgttgg ctacccgtga   8520
tattgctgaa gagcttggcg gcgaatgggc tgaccgcttc ctcgtgcttt acggtatcgc   8580
cgctcccgat tcgcagcgca tcgccttcta tcgccttctt gacgagttct tctgagcggg   8640
acccaagctc tagatcttgc tgcgttcgga tattttcgtg gagttcccgc cacagacccg   8700
gatgatcccc gatcgttcaa acatttggca ataaagtttc ttaagattga atcctgttgc   8760
```

```
cggtcttgcg atgattatca tataatttct gttgaattac gttaagcatg taataattaa   8820
catgtaatgc atgacgttat ttatgagatg ggtttttatg attagagtcc cgcaattata   8880
catttaatac gcgatagaaa acaaaatata gcgcgcaaac taggataaat tatcgcgcgc   8940
ggtgtcatct atgttactag atcgggcctc ctgtcaagct ctgagt                  8986
```